# EUROPEAN PATENT APPLICATION

(11) **EP 4 707 391 A1**
(43) Date of publication of application: **11.03.2026**
(21) Application number: 24199586.9
(22) Date of filing: 10.09.2024
(51) Int. Cl.: C12N 15/113, A61K 31/7105, A61K 31/713, A61P 7/04

(54) **NUCLEIC ACID COMPOUNDS FOR ZPI INHIBITION**

(71) Applicant: e-therapeutics PLC, London W2 6BD (GB)
(72) Inventor: The designation of the inventor has not yet been filed
(74) Representative: CMS Cameron McKenna Nabarro Olswang LLP

(57) **Abstract**

The present invention provides novel nucleic acid compounds suitable for therapeutic use. Additionally, the present invention provides methods of making these compounds, as well as methods of using such compounds for the treatment of various diseases and conditions.

## Description

### FIELD

The present invention provides novel nucleic acid compounds, suitable for therapeutic use. Additionally, the present invention provides methods of making these compounds, as well as methods of using such compounds for the treatment of various diseases and conditions.

### BACKGROUND OF THE INVENTION

Nucleic acid compounds have important therapeutic applications in medicine. Nucleic acids can be used to silence genes that are responsible for a particular disease. Gene-silencing prevents formation of a protein by inhibiting translation. Importantly, gene-silencing agents are a promising alternative to traditional small, organic compounds that inhibit the function of the protein linked to the disease. siRNA, antisense RNA, and micro-RNA are oligonucleotides / oligonucleosides that prevent the formation of proteins by gene-silencing.

A number of modified siRNA compounds in particular have been developed in the last two decades for diagnostic and therapeutic purposes, including siRNA / RNAi therapeutic agents for the treatment of various diseases including central-nervous-system diseases, inflammatory diseases, metabolic disorders, oncology, infectious diseases, and ocular diseases.

The present invention relates to nucleic acid compounds that inhibit the expression of the gene ZPI, for use in the treatment and / or prevention of disease.

### STATEMENTS OF INVENTION

According to a first aspect of the present invention, there is provided a nucleic acid for inhibiting expression of ZPI, comprising a duplex region that comprises a first strand and a second strand that is at least partially complementary to the first strand, wherein said first strand is: (i) at least partially complementary to a portion of RNA transcribed from the ZPI gene, and (ii) comprises at least 17 contiguous nucleosides differing by 0 or 1 nucleosides from any one of the first strand sequences as listed in Table 2.

According to a second aspect of the present invention, there is provided a nucleic acid for inhibiting expression of ZPI, comprising a duplex region that comprises a first strand and a second strand that is at least partially complementary to the first strand, wherein said first strand is: (i) at least partially complementary to a portion of RNA transcribed from the ZPI gene, and (ii) comprises at least 17 contiguous nucleosides differing by 0 or 1 nucleosides from any one of the first strand modified sequences as listed in Table 3.

A nucleic acid as described herein, wherein the first strand comprises nucleosides 2-18 of any one of the sequences according to the above first and second aspects of the present invention.

A nucleic acid according to the above first aspect of the present invention, wherein the second strand comprises a nucleoside sequence of at least 17 contiguous nucleosides differing by 0 or 1 nucleosides from any one of the second strand sequences as listed in Table 2, and wherein the second strand has a region of at least 85% complementarity over the 17 contiguous nucleosides to the first strand.

A nucleic acid according to the above first aspect of the present invention, wherein the second strand comprises a nucleoside sequence of at least 17 contiguous nucleosides differing by 0 or 1 nucleosides from any one of the second strand sequences as listed in Table 2, and wherein the duplex region comprises at least 14, 15, 16 or 17 complementary base pairs.

A nucleic acid according to the above second aspect of the present invention, wherein the second strand comprises a nucleoside sequence of at least 17 contiguous nucleosides differing by 0 or 1 nucleosides from any one of the second strand modified sequences as listed in Table 4, and wherein the second strand has a region of at least 85% complementarity over the 17 contiguous nucleosides to the first strand.

A nucleic acid according to the above second aspect of the present invention, wherein the second strand comprises a nucleoside sequence of at least 17 contiguous nucleosides differing by 0 or 1 nucleosides from any one of the second strand modified sequences as listed in Table 4, and wherein the duplex region comprises at least 14, 15, 16 or 17 complementary base pairs.

A nucleic acid according to the above first aspect of the present invention, wherein the first strand comprises any one of the first strand sequences as listed in Table 2.

A nucleic acid according to the above second aspect of the present invention, wherein the first strand comprises any one of the first strand modified sequences as listed in Table 3.

A nucleic acid according to the above first aspect of the present invention, wherein the second strand comprises any one of the second strand sequences as listed in Table 2.

A nucleic acid according to the above second aspect of the present invention, wherein the second strand comprises any one of the second strand modified sequences as listed in Table 4.

A nucleic acid according to the invention, wherein the first strand comprises SEQ ID NO: 54 or SEQ ID NO: 51.A nucleic acid according to the invention, wherein the first strand comprises SEQ ID NO: 148 or SEQ ID NO: 145.

A nucleic acid according to the invention, wherein the second strand comprises SEQ ID NO: 101 or SEQ ID NO: 98.

A nucleic acid according to the invention, wherein the second strand comprises SEQ ID NO: 195 or SEQ ID NO: 192.

A nucleic acid according to the invention, comprising first and second strands that comprise, consist of, or consist essentially of a nucleoside sequence differing by 0 or 1 nucleotides from any one of the following combinations of first and second sequences:

| Unmodified first strand | Unmodified second strand |
|---|---|
| SEQ ID NO: 54 | SEQ ID NO: 101 |
| SEQ ID NO: 51 | SEQ ID NO: 98 |

A nucleic acid according to the invention, comprising first and second strands that comprise, consist of, or consist essentially of a nucleoside sequence differing by 0 or 1 nucleotides from any one of the following combinations of first and second sequences:

| Unmodified first strand | Unmodified second strand |
|---|---|
| SEQ ID NO: 54 | SEQ ID NO: 101 |

A nucleic acid according to the invention, comprising first and second strands that comprise, consist of, or consist essentially of a nucleoside sequence differing by 0 or 1 nucleotides from any one of the following combinations of first and second sequences:

| Modified first strand | Modified second strand |
|---|---|
| SEQ ID NO: 148 | SEQ ID NO: 195 |
| SEQ ID NO: 145 | SEQ ID NO: 192 |

A nucleic acid according to the invention, comprising first and second strands that comprise, consist of, or consist essentially of a nucleoside sequence differing by 0 or 1 nucleotides from any one of the following combinations of first and second sequences:

| Modified first strand | Modified second strand |
|---|---|
| SEQ ID NO: 148 | SEQ ID NO: 195 |

A conjugate for inhibiting expression of ZPI target gene in a cell, said conjugate comprising a nucleic acid as disclosed herein and one or more ligand moieties.

A pharmaceutical composition comprising a nucleic acid as disclosed herein, in combination with a pharmaceutically acceptable excipient or carrier.

A nucleic acid or pharmaceutical composition, for use in therapy.

A nucleic acid or pharmaceutical composition, for use in prevention or treatment of a disease related to a disorder of haemostasis, such as haemophilia.

A nucleic acid or pharmaceutical composition, for use in prevention or treatment of Von Willebrand disease.A nucleic acid or pharmaceutical composition, for use in prevention or treatment of Factor X Deficiency.

### FIGURES

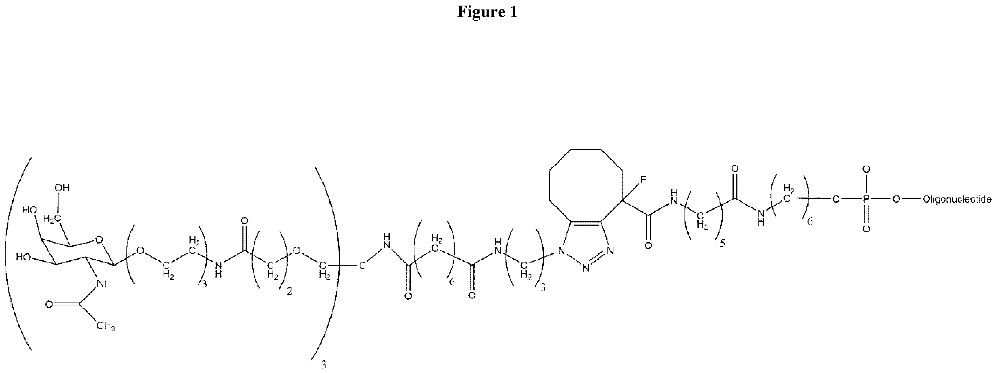

**Figure 1****:** Linker and ligand portions of constructs suitable for use according to the present invention including tether 1a. While Figure 1 depicts the linker to be conjugated to an oligonucleotide, it is to be understood that the present invention also encompasses conjugates of the same linker with an oligonucleoside as disclosed herein.
   It should also be understood that while Figure 1 depicts as a product molecules based on the linker and ligand portions as specifically depicted in Figure 1 attached to an oligonucleoside moiety as also depicted herein, this product may alternatively further comprise, or consist essentially of, molecules wherein the linker and ligand portions are essentially as depicted in Figure 1 attached to an oligonucleoside moiety but having the F substituent as shown in Figure 1 on the cyclo-octyl ring replaced by a substituent, which could occur as a result of hydrolytic displacement, such as an OH substituent, or the OH substituent could be synthesized as a linker in its own right. In this way, (a) tether 1a constructs can consist essentially of molecules having linker and ligand portions specifically as depicted in Figure 1, with a F substituent on the cyclo-octyl ring; or (b) tether 1a constructs can consist essentially of molecules having linker and ligand portions essentially as depicted in Figure 1 but having the F substituent as shown in Figure 1 on the cyclo-octyl ring replaced by an OH substituent, or (c) tether 1a constructs can comprise a mixture of molecules as defined in (a) and/or (b).

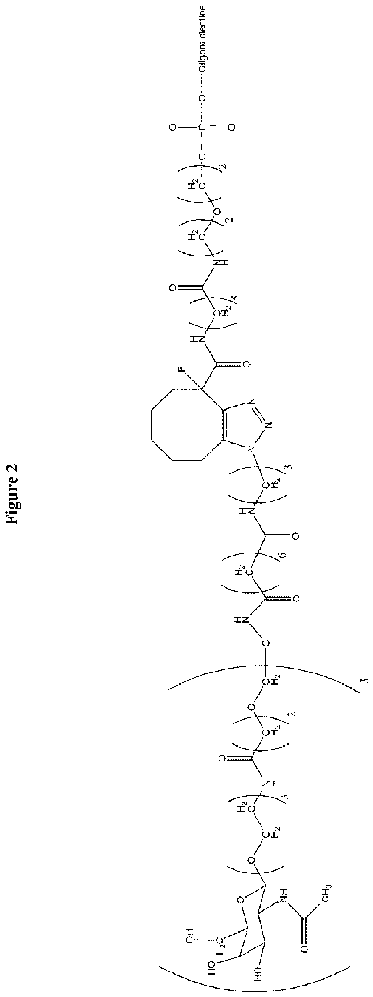

**Figure 2****:** Linker and ligand portions of constructs suitable for use according to the present invention including tether 1b. While Figure 2 depicts the linker to be conjugated to an oligonucleotide, it is to be understood that the present invention also encompasses conjugates of the same linker with an oligonucleoside as disclosed herein.
   The comments made in relation to Figure 1 and the possible replacement of the F substituent as shown in Figure 1 on the cyclo-octyl ring replaced by a substituent, which could occur as a result of hydrolytic displacement, such as an OH substituent, or the OH substituent could be synthesized as a linker in its own right, apply equally to tether 1b constructs. In this way, (a) tether 1b constructs can consist essentially of molecules having linker and ligand portions specifically as depicted in Figure 2, with a F substituent on the cyclo-octyl ring; or (b) tether 1b constructs can consist essentially of molecules having linker and ligand portions essentially as depicted in Figure 2 but having the F substituent as shown in Figure 2 on the cyclo-octyl ring replaced by an OH substituent, or (c) tether 1b constructs can comprise a mixture of molecules as defined in (a) and/or (b).

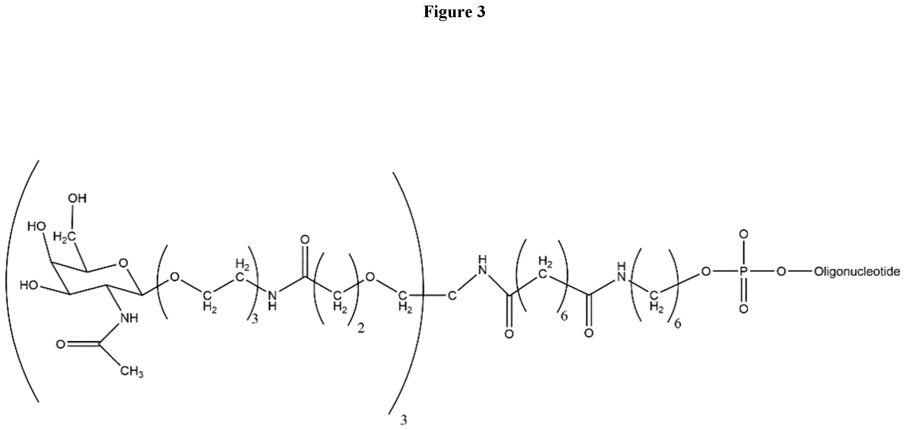

**Figure 3****:** Linker and ligand portions of constructs suitable for use according to the present invention including tether 2a. While Figure 3 depicts the linker to be conjugated to an oligonucleotide, it is to be understood that the present invention also encompasses conjugates of the same linker with an oligonucleoside as disclosed herein.

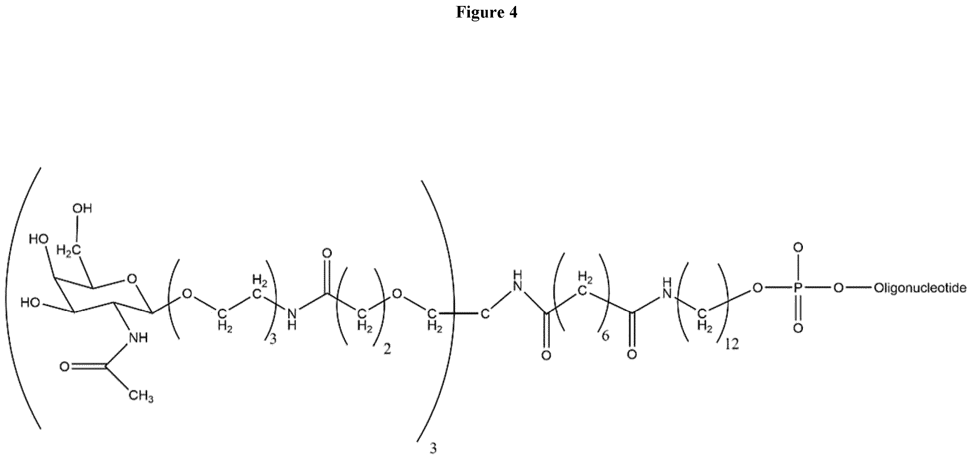

**Figure 4****:** Linker and ligand portions of constructs suitable for use according to the present invention including tether 2b. While Figure 4 depicts the linker to be conjugated to an oligonucleotide, it is to be understood that the present invention also encompasses conjugates of the same linker with an oligonucleoside as disclosed herein.

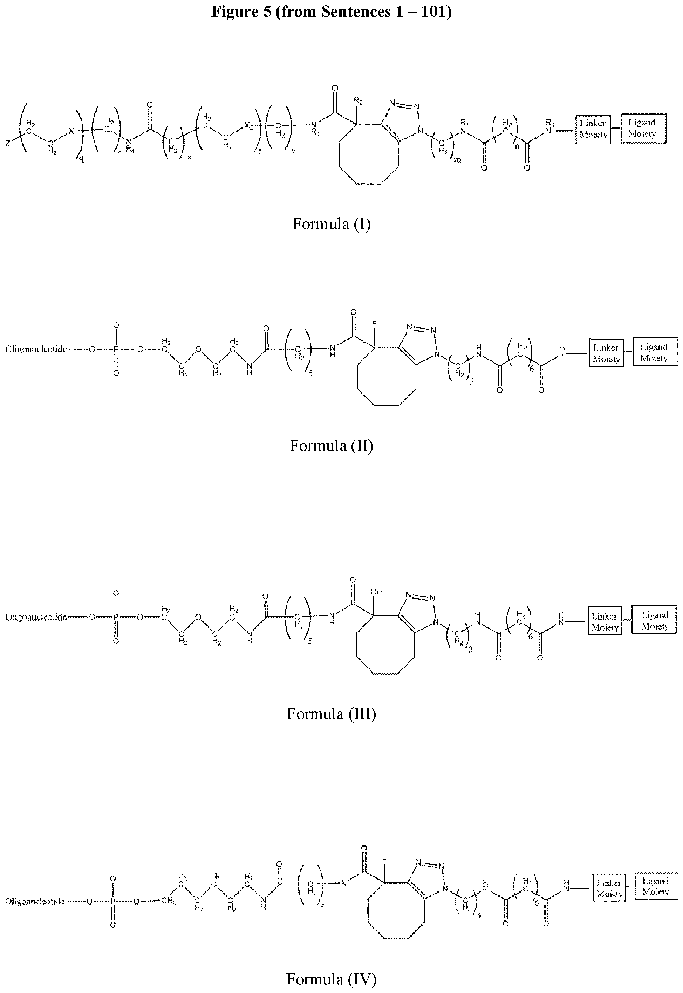

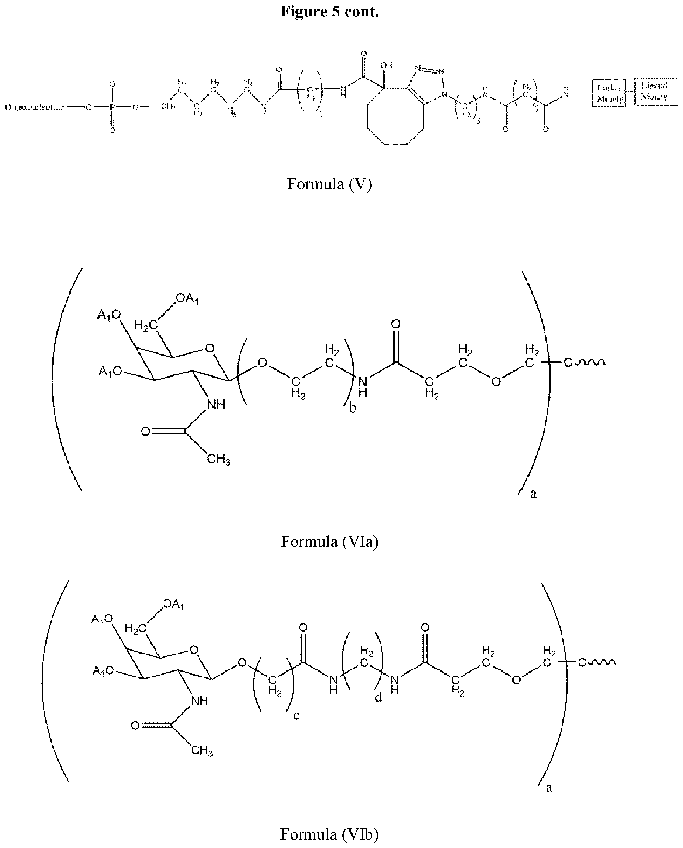

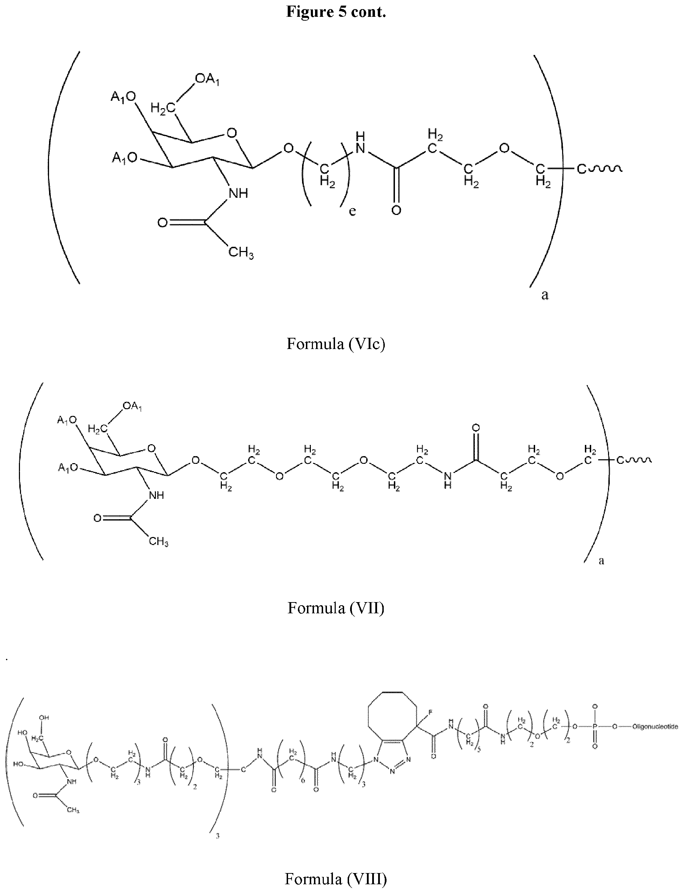

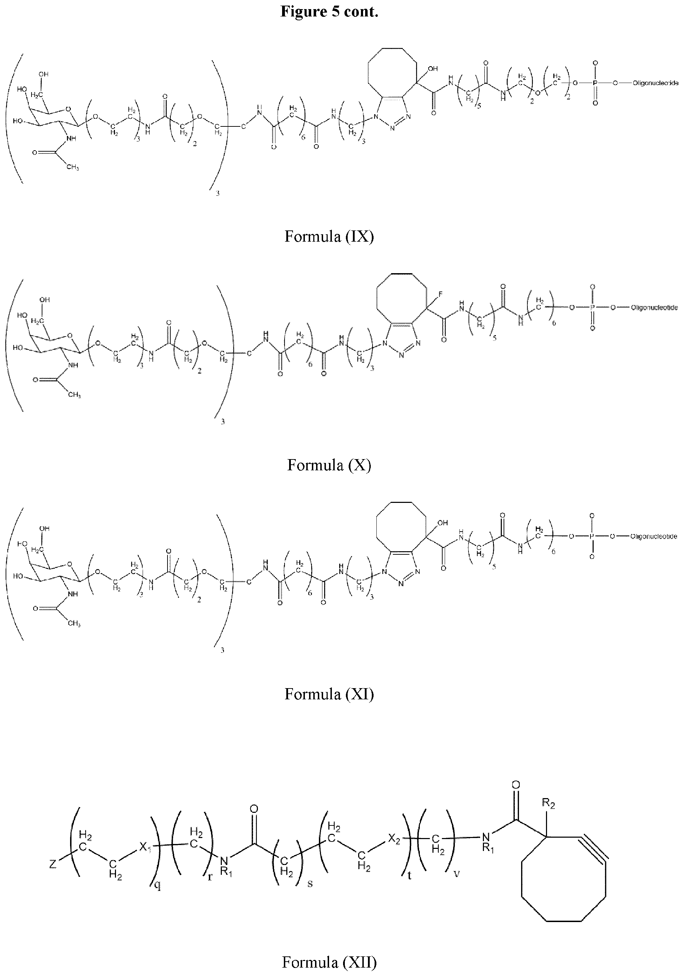

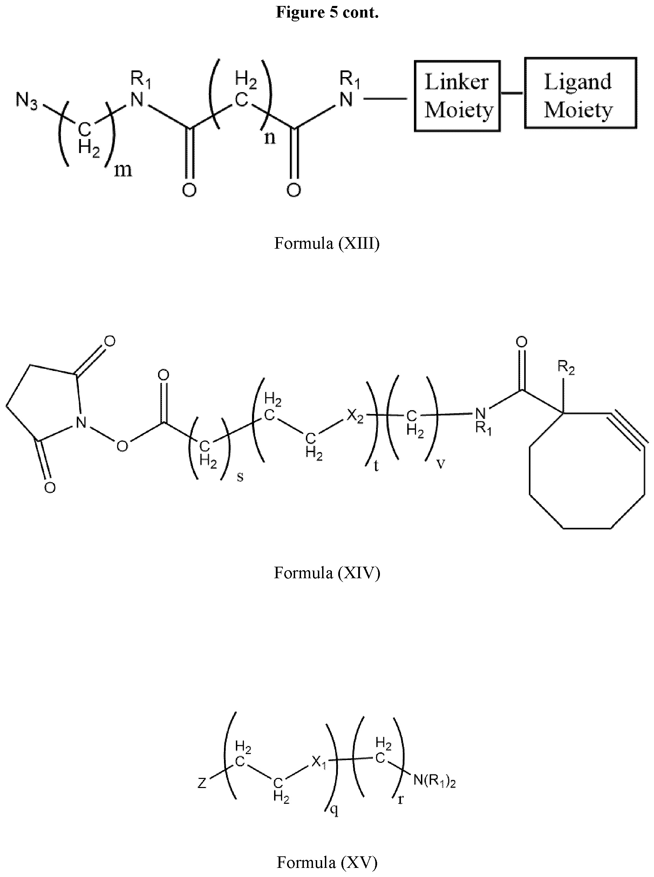

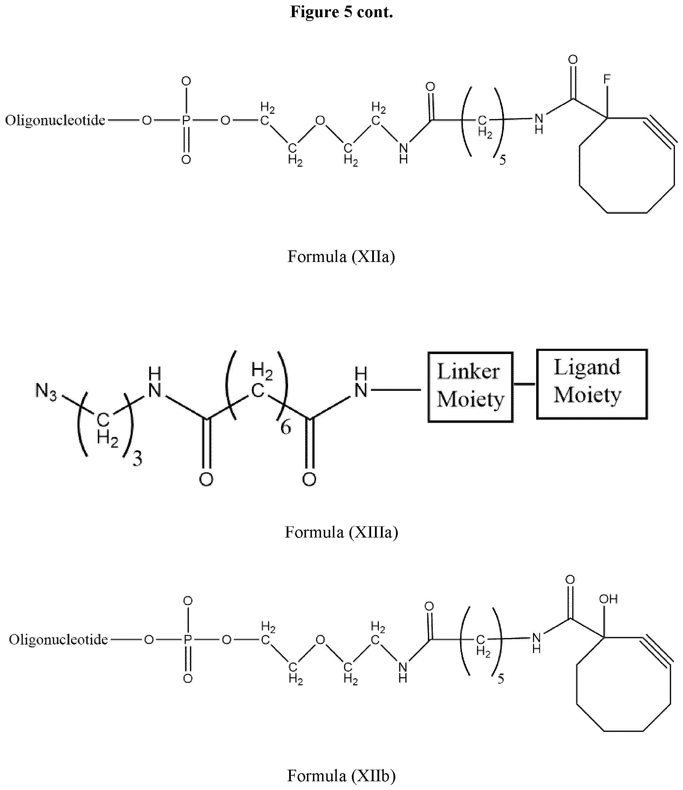

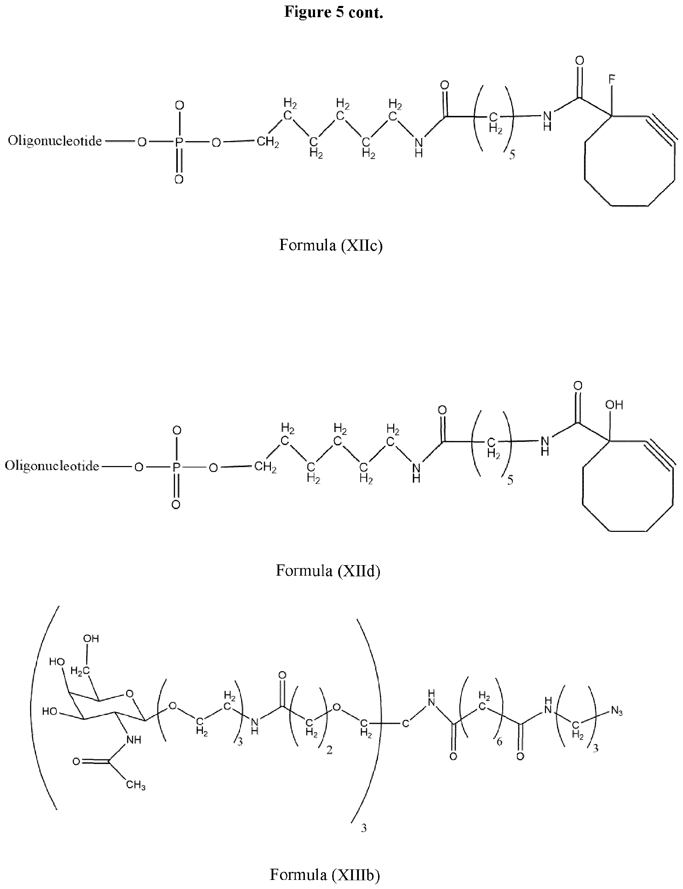

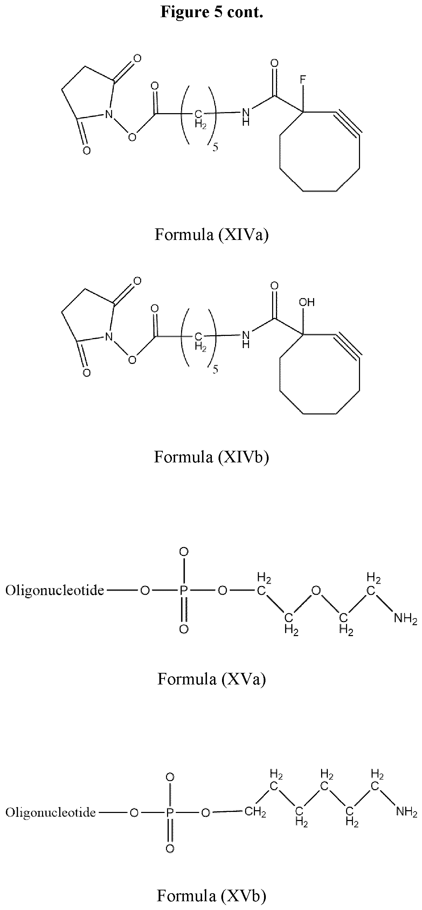

**Figure 5****:** Formulae described in Sentences 1-101 disclosed herein.

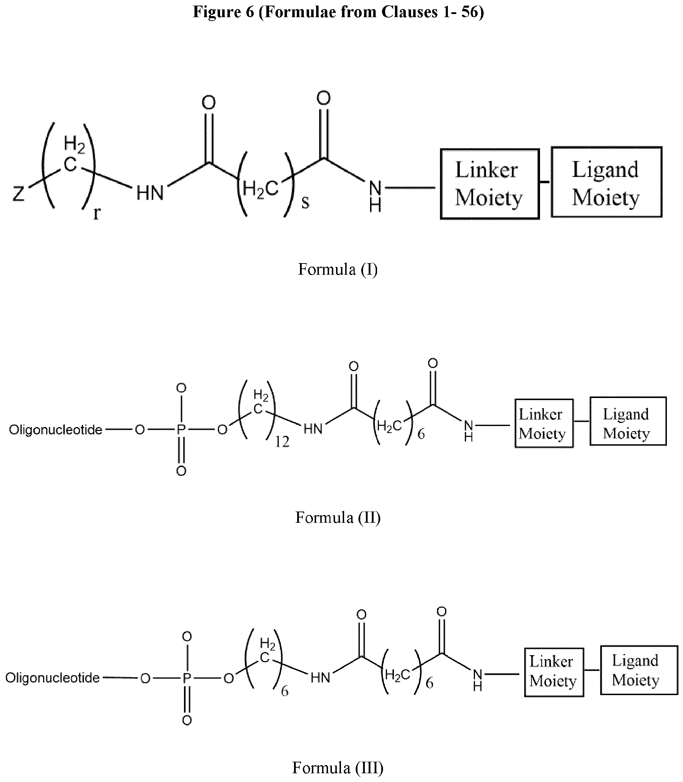

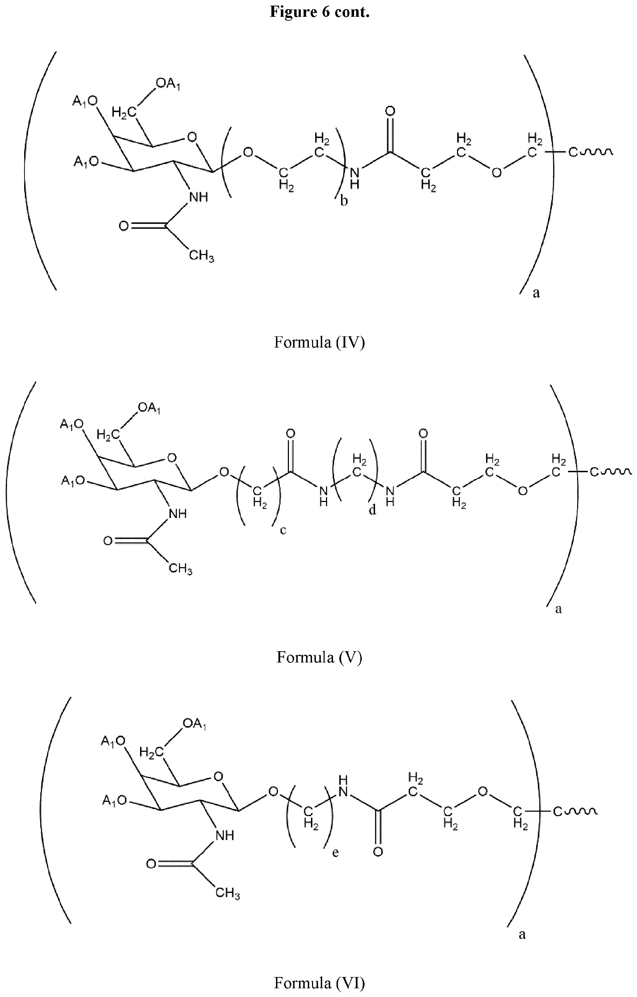

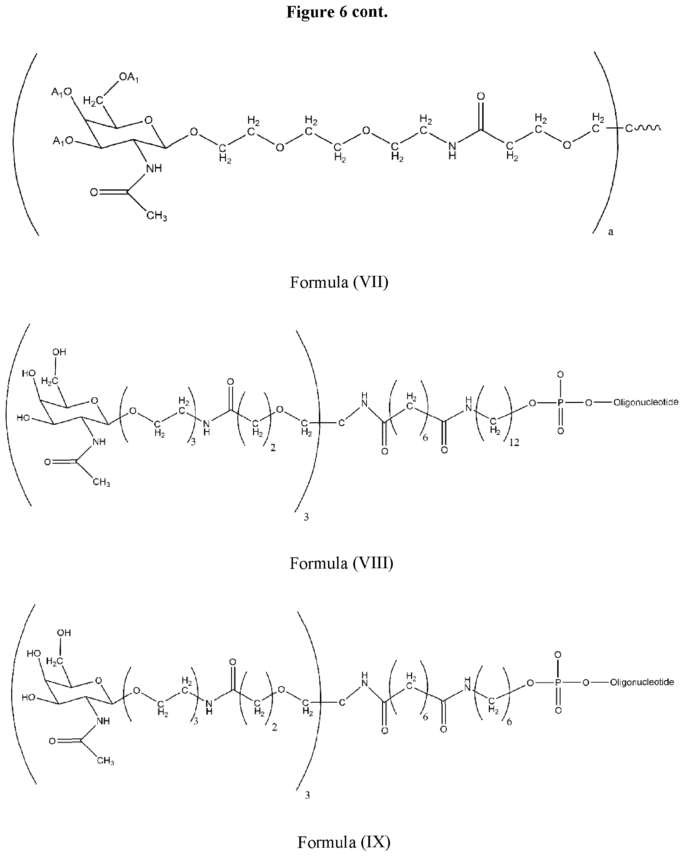

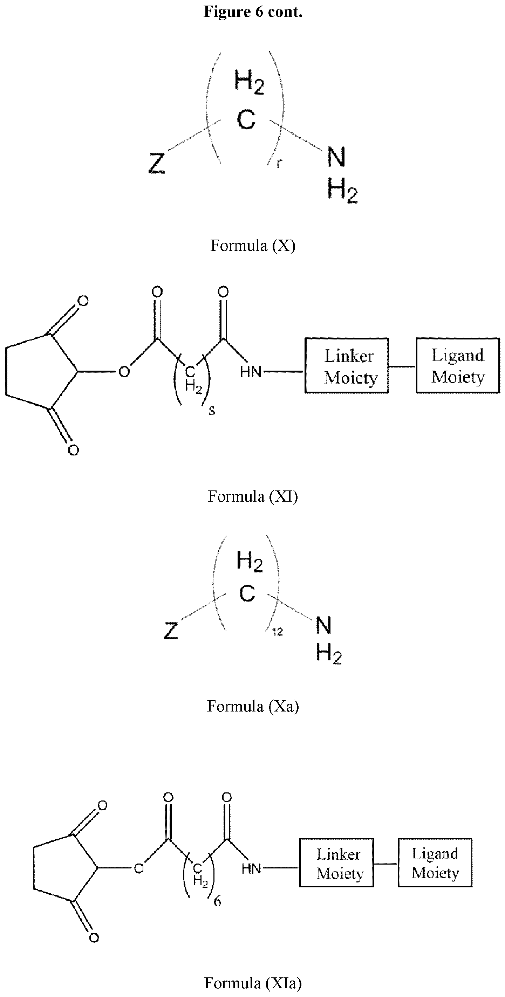

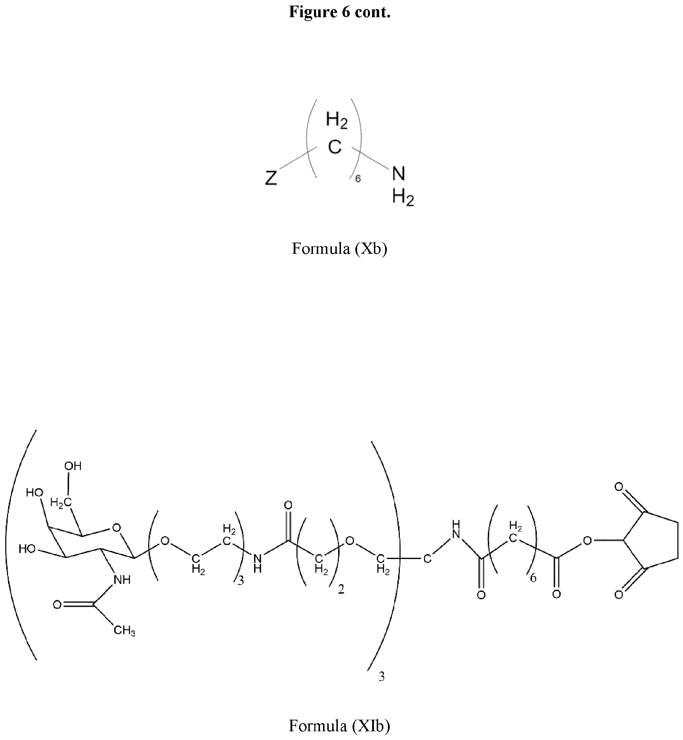

**Figure 6****:** Formulae described in Clauses 1-56 disclosed herein

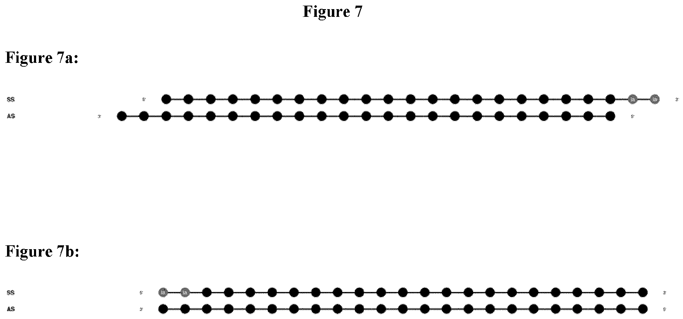

**Figures 7a and 7b****:** Inverted abasic constructs that can be used with nucleic acid sequences according to the present invention as described herein. For Figure 7a, a GalNAc linker is attached to the 5' end region of the sense strand in use (not depicted in Figure 7a). For Figure 7b, a GalNAc linker is attached to the 3' end region of the sense strand in use (not depicted in Figure 7b).
   iaia as shown at the 3' end region of the sense strand in Figure 7a represents (i) two abasic nucleosides provided as the penultimate and terminal nucleosides at the 3' end region of the sense strand, (ii) wherein a 3'-3' reversed linkage is provided between the antepenultimate nucleoside (namely at position 21 of the sense strand, wherein position 1 is the terminal 5' nucleoside of the sense strand) and the adjacent penultimate abasic residue of the sense strand, and (iii) the linkage between the terminal and penultimate abasic nucleosides is 5'-3' when reading towards the 3' end region comprising the terminal and penultimate abasic nucleosides.
   iaia as shown at the 5' end region of the sense strand in Figure 7b represents (i) two abasic nucleosides provided as the penultimate and terminal nucleosides at the 5' end region of the sense strand, (ii) wherein a 5'-5' reversed linkage is provided between the antepenultimate nucleoside (namely at position 1 of the sense strand, not including the iaia motif at the 5' end region of the sense strand in the nucleoside position numbering on the sense strand) and the adjacent penultimate abasic residue of the sense strand, and (iii) the linkage between the terminal and penultimate abasic nucleosides is 3'-5' when reading towards the 5' end region comprising the terminal and penultimate abasic nucleosides.

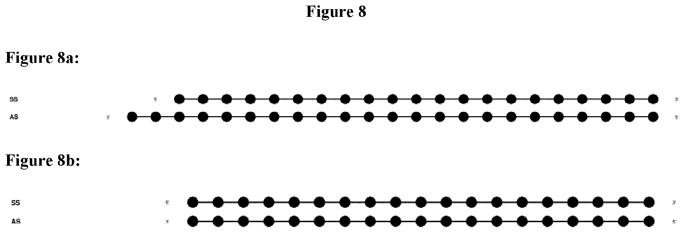

**Figures 8a and 8b****:** Duplex constructs according to Table 5.
**Figure 9****:** The correlation between predicted and experimentally determined siRNA efficacy values i.e. maximum RNA knockdown where 1 represents maximum knockdown and 0 is no reduction in mRNA levels. Data displayed are for the test dataset in the best performing siRNAdesignR model.
**Figure 10****:** Performance metrics for the best performing siRNAdesignR model in the test data set and also the validation dataset. In both cases the model scored above 0.5 in the Precision@20 metric, and the best performing siRNA (experimentally determined) was in the top 20 predictions of the model (nSiRNAsForBest).
**Figure 11****:** siRNAdesignR ranking for 276 siRNAs. These rankings were tested in an in vitro model (Huh7 cells) demonstrating strong correlation (Spearman correlation coefficient 0.743).
**Figure 12****:** A dose response curve of SLC25A5 mRNA knockdown following 24 hour exposure to siRNA. Cells were tested in triplicate repeats on two separate days (replicates 1 and 2). Data are mean +/- standard deviation with knockdown normalised to untreated wells.
**Figure 13****:** Change in SLC25A5 mRNA knockdown over 28 days following one subcutaneous dose of siRNA at day 0. As mRNA measurements are taken from liver tissue measurements are taken from different mice at each time point. Data are mean +/- standard deviation from 16 mice per timepoint per dose, normalised to saline control.
**Figure 14****:** Change in SLC25A5 protein expression over 28 days following one subcutaneous dose of siRNA at day 0. As mRNA measurements are taken from liver tissue measurements are taken from different mice at each time point. Data are mean +/- standard deviation from 16 mice per timepoint per dose, normalized to saline control.
**Figure 15****:** Results of dose-response experiments for inhibition of B4GALT1 mRNA expression in human Huh7 cells. Data are mean +/- standard deviation from triplicate repeats with knockdown normalized to untreated wells. Dotted curves represent 95% confidence intervals. Dotted lines and shaded areas represent the mean relative expression +/- standard deviation from untreated wells on the same plate.
**Figure 16****:** Results of time course experiments for inhibition of B4GALT1 mRNA expression in C57BL/6 mice. Data are mean +/- standard deviation from 12 mice per timepoint per dose, normalised mice prior to treatment with siRNA construct.
**Figure 17****:** Results of an RNAi molecule screen targeting the expression of B4GALT1 in human Huh7 cells. Data are mean +/- standard deviation from triplicate repeats with knockdown at 3 nM normalised to untreated cells. ETX-M00001217 was included as a non-targeting control and ETX-M00001850 was the lead sequence from the original screen included for comparison.
**Figure 18****:** Overview of the hydrodynamic injection (HDI) study in mice.
**Figure 19****:** Results of an siRNA molecule screen targeting the expression of human ZPI following hydrodynamic injection in BALB/c mice.

### DEFINITIONS

The "first strand", also called the antisense strand or guide strand herein and which can be used interchangeably herein, refers to the nucleic acid strand, e.g. the strand of an siRNA, e.g. a dsiRNA, which includes a region that is substantially complementary to a target sequence, e.g. to an mRNA. As used herein, the term "region of complementarity" refers to the region on the antisense strand that is substantially complementary to a sequence, for example a target sequence. Where the region of complementarity is not fully complementary to the target sequence, the mismatches can typically be in the internal or terminal regions of the molecule. In some embodiments, a double stranded nucleic acid e.g. an siRNA agent of the invention includes a nucleoside mismatch in the antisense strand.

The "second strand" (also called the sense strand or passenger strand herein, and which can be used interchangeably herein), refers to the strand of a nucleic acid e.g. siRNA that includes a region that is substantially complementary to a region of the antisense strand as that term is defined herein.

In the context of molecule comprising a nucleic acid provided with a ligand moiety, optionally also with a linker moiety, the nucleic acid of the invention may be referred to as an oligonucleoside or an oligonucleoside moiety.

Oligonucleotides are short nucleic acid polymers. Whilst oligonucleotides contain phosphodiester bonds between the nucleoside component thereof (base plus sugar), the present invention is not limited to oligonucleotides always joined by such a phosphodiester bond between adjacent nucleosides, and other oligomers of nucleosides joined by bonds which are bonds other than a phosphodiester bond are contemplated. For example, a bond between nucleosides may be a phosphorothioate bond. Therefore, the term "oligonucleoside" as used herein covers both oligonucleotides and other oligomers of nucleosides. An oligonucleoside which is a nucleic acid having at least a portion which is an oligonucleotide is preferred according to the present invention. An oligonucleoside having one or more, or a majority of, phosphodiester backbone bonds between nucleosides is also preferred according to the present invention. An oligonucleoside having one or more, or a majority of, phosphodiester backbone bonds between nucleosides, and also having one or more phosphorothioate backbone bonds between nucleosides (typically in a terminal region of the first and / or second strands) is also preferred according to the present invention.

It is preferred herein that the nucleic acid according to the invention is a double stranded oligonucleoside comprising one or more phosphorothioate backbone bonds between nucleosides. Accordingly, in all instances in which the present application refers to an oligonucleotide, particularly in the chemical structures disclosed herein, the oligonucleotide may equally be an oligonucleoside as defined herein. Similarly, in all instances in which the present application refers to an oligonucleoside, particularly in the chemical structures disclosed herein, the oligonucleoside may specifically be an oligonucleotide as defined herein.

In some embodiments, a double stranded nucleic acid e.g. siRNA agent of the invention includes a nucleoside mismatch in the sense strand. In some embodiments, the nucleoside mismatch is, for example, within 5, 4, 3, 2, or 1 nucleosides from the 3 '-end of the nucleic acid e.g. siRNA.

In another embodiment, the nucleoside mismatch is, for example, in the 3'- terminal nucleoside of the nucleic acid e.g. siRNA.

A "target sequence" (which may also be called a target RNA or a target mRNA) refers to a contiguous portion of the nucleoside sequence of an mRNA molecule formed during the transcription of a gene, including mRNA that is a product of RNA processing of a primary transcription product, or can be a contiguous portion of the nucleoside sequence of any RNA molecule such as a LNCRNA which it is desired to inhibit.

The target sequence may be from about 10-35 nucleosides in length, e.g., about 15-30 nucleosides in length. For example, the target sequence can be from about 15-30 nucleosides, 15-29, 15-28, 15-27, 15-26, 15-25, 15-24, 15-23, 15-22, 15-21, 15-20, 15-19, 15-18, 15-17, 18-30, 18-29, 18-28, 18-27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20-24, 20-23, 20-22, 20- 21, 21-30, 21-29, 21-28, 21-27, 21-26, 21-25, 21-24, 21-23, or 21-22 nucleosides in length. Ranges and lengths intermediate to the above recited ranges and lengths are also contemplated to be part of the invention.

The term "ribonucleoside" or "nucleoside" can also refer to a modified nucleoside, as further detailed below.

A nucleic acid can be a DNA or an RNA, and can comprise modified nucleosides. RNA is a preferred nucleic acid.

The terms "iRNA", "siRNA", "RNAi agent," and "iRNA agent," "RNA interference agent" as used interchangeably herein, refer to an agent that contains RNA, and which mediates the targeted cleavage of an RNA transcript via an RNA-induced silencing complex (RISC) pathway. siRNA directs the sequence-specific degradation of mRNA through RNA interference (RNAi).

A double stranded RNA is referred to herein as a "double stranded siRNA (dsiRNA) agent", "double stranded siRNA (dsiRNA) molecule", "double stranded RNA (dsRNA) agent", "double stranded RNA (dsRNA) molecule", "dsiRNA agent", "dsiRNA molecule", or "dsiRNA", which refers to a complex of ribonucleic acid molecules, having a duplex structure comprising two antiparallel and substantially complementary nucleic acid strands, referred to as having "sense" and "antisense" orientations with respect to a target RNA.

The majority of nucleosides of each strand of the nucleic acid, e.g. a dsiRNA molecule, are preferably ribonucleosides, but in that case each or both strands can also include one or more non-ribonucleosides, e.g., a deoxyribonucleoside or a modified nucleoside. In addition, as used in this specification, an "siRNA" may include ribonucleosides with chemical modifications.

The term "modified nucleoside" refers to a nucleoside having, independently, a modified sugar moiety, a modified internucleoside linkage, or modified nucleobase, or any combination thereof. Thus, the term modified nucleoside encompasses substitutions, additions or removal of, e.g., a functional group or atom, to internucleoside linkages, sugar moieties, or nucleobases. Any such modifications, as used in an siRNA type molecule, are encompassed by "iRNA" or "RNAi agent" or "siRNA" or "siRNA agent" for the purposes of this specification and claims.

The duplex region of a nucleic acid of the invention e.g. a dsRNA may range from about 9 to 40 base pairs in length such as 9 to 36 base pairs in length, e.g., about 15- 30 base pairs in length, for example, about 9, 10, 11, 12, 13, 14, 15, 16, 17, 18, 19, 20, 21, 22, 23, 24, 25, 26, 27, 28, 29, 30, 31, 32, 33, 34, 35, or 36 base pairs in length, such as about 15-30, 15-29, 15-28, 15-27, 15-26, 15-25, 15-24, 15-23, 15-22, 15-21, 15-20, 15-19, 15-18, 15-17, 18-30, 18-29, 18-28, 18- 27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20-24,20-23, 20-22, 20-21, 21-30, 21-29, 21-28, 21-27, 21-26, 21-25, 21-24, 21-23, or 21-22 base pairs in length.

The two strands forming the duplex structure may be different portions of one larger molecule, or they may be separate molecules e.g. RNA molecules.

The term "nucleoside overhang" refers to at least one unpaired nucleoside that extends from the duplex structure of a nucleic acid according to the present invention. A nucleic acid according to the present invention can comprise an overhang of at least one nucleoside; alternatively the overhang can comprise at least two nucleosides, at least three nucleosides, at least four nucleosides, at least five nucleosides or more. A nucleoside overhang can comprise or consist of a nucleoside/nucleoside analog, including a deoxynucleoside. The overhang(s) can be on the sense strand, the antisense strand, or any combination thereof. Furthermore, the nucleoside(s) of an overhang can be present on the 5'-end, 3'-end, or both ends of either an antisense or sense strand.

In certain embodiments, the antisense strand has a 1-10 nucleoside, e.g., 0-3, 1-3, 2-4, 2-5, 4-10, 5-10, 1, 2, 3, 4, 5, 6, 7, 8, 9, or 10 nucleoside overhang at the 3'-end or the 5'-end.

"Blunt" or "blunt end" means that there are no unpaired nucleosides at that end of the double stranded nucleic acid, i.e., no nucleoside overhang. The nucleic acids of the invention include those with no nucleoside overhang at one end or with no nucleoside overhangs at either end.

Unless otherwise indicated, the term "complementary," when used to describe a first nucleoside sequence in relation to a second nucleoside sequence, refers to the ability of an oligonucleoside comprising the first nucleoside sequence to hybridize and form a duplex structure under certain conditions with an oligonucleoside comprising the second nucleoside sequence, as will be understood by the skilled person. Such conditions can, for example, be stringent conditions, where stringent conditions can include: 400 mM NaCl, 40 mM PIPES pH 6.4, 1 mM EDTA, 50°C or 70°C for 12-16 hours followed by washing (see, e.g., "Molecular Cloning: A Laboratory Manual, Sambrook, et al. (1989) Cold Spring Harbor Laboratory Press).

Complementary sequences within nucleic acid e.g. a dsiRNA, as described herein, include base-pairing of the oligonucleoside comprising a first nucleoside sequence to an oligonucleoside comprising a second nucleoside sequence over the entire length of one or both nucleoside sequences. Such sequences can be referred to as "fully complementary" with respect to each other herein. However, where a first sequence is referred to as "substantially complementary" or "partially complementary" with respect to a second sequence herein, the two sequences can be fully complementary, or they can form one or more mismatched base pairs, such as 2, 4, or 5 mismatched base pairs, but preferably not more than 5 , while retaining the ability to hybridize under the conditions most relevant to their ultimate application, e.g., inhibition of gene expression via a RISC pathway. Overhangs shall not be regarded as mismatches with regard to the determination of complementarity. For example, a nucleic acid e.g. dsiRNA comprising one oligonucleoside 17 nucleosides in length and another oligonucleoside 19 nucleosides in length, wherein the longer oligonucleoside comprises a sequence of 17 nucleosides that is fully complementary to the shorter oligonucleoside, can yet be referred to as "fully complementary".

"Complementary" sequences, as used herein, can also include, or be formed entirely from, non-Watson-Crick base pairs or base pairs formed from non-natural and modified nucleosides, in so far as the above requirements with respect to their ability to hybridize are fulfilled. Such non-Watson- Crick base pairs include, but are not limited to, G:U Wobble or Hoogstein base pairing.

The terms "complementary," "fully complementary" and "substantially/partially complementary" herein can be used with respect to the base matching between the sense strand and the antisense strand of a nucleic acid e.g., dsiRNA, or between the antisense strand of a double stranded nucleic acid e.g. siRNA agent and a target sequence.

Within the present invention, the second strand of the nucleic acid according to the invention, in particular a dsiRNA for inhibiting expression of ZPI, is at least partially complementary to the first strand of said nucleic acid. In certain embodiments, a first and second strand of a nucleic acid according to the invention are partially complementary if they form a duplex region having a length of at least 17 base pairs and comprising not more than 1, 2, 3, 4, or 5 mismatched base pairs.

In certain embodiments, a first and second strand of the nucleic acid according to the invention are partially complementary if they form a duplex region having a length of 19 base pairs and comprising not more than 1, 2, 3, 4, or 5 mismatched base pairs. In certain embodiments, a first and second strand of the nucleic acid according to the invention are partially complementary if they form a duplex region having a length of 21 base pairs comprising not more than 1, 2, 3, 4, or 5 mismatched base pairs.

Alternatively, a first and second strand of the nucleic acid according to the invention are partially complementary if they form a duplex region having a length of at least 17 base pairs, wherein at least 14, 15, 16 or 17 of said base pairs are complementary base pairs, in particular Watson-Crick base pairs.

In certain embodiments, a first and second strand of the nucleic acid according to the invention are partially complementary if they form a duplex region having a length of 19 base pairs, wherein at least 14, 15, 16, 17, 18 or all 19 base pairs are complementary base pairs, in particular Watson-Crick base pairs. In certain embodiments, a first and second strand of the nucleic acid according to the invention are partially complementary if they form a duplex region having a length of 21 base pairs, wherein at least 16, 17, 18, 19, 20 or all 21 base pairs are complementary base pairs, in particular Watson-Crick base pairs.

As used herein, a nucleic acid that is "substantially complementary" or "partially complementary" to at least part of a messenger RNA (mRNA) refers to a nucleic acid that is substantially or partially complementary to a contiguous portion of the mRNA of interest (e.g., an mRNA encoding a gene). In certain embodiments, the contiguous portion of the mRNA is a sequence as listed in Table 1, i.e., any one of SEQ ID NOs: 1-47. For example, a nucleic acid is complementary to at least a part of an mRNA of a gene of interest if the sequence is substantially or partially complementary to a non-interrupted portion of an mRNA encoding that gene.

Accordingly, in some preferred embodiments, the antisense oligonucleosides as disclosed herein are fully complementary to the target gene sequence.

In other embodiments, the antisense oligonucleosides disclosed herein are substantially or partially complementary to a target RNA sequence and comprise a contiguous nucleoside sequence which is at least about 80% complementary over its entire length to the equivalent region of the target RNA sequence, such as at least about 85%, 86%, 87%, 88%, 89%, about 90%, 91 %, 92%, 93%, 94%, 95%, 96%, 97%, 98%, or 99% complementary or 100% complementary.

In certain embodiments, the first (antisense) strand of a nucleic acid according to the invention is partially or fully complementary to a contiguous portion of RNA transcribed from the ZPI gene. In certain embodiments, the first strand of the nucleic acid according to the invention is partially or fully complementary to a contiguous portion of at least 17 nucleosides of the ZPI mRNA. In certain embodiments, the first strand of the nucleic acid according to the invention is partially or fully complementary to a contiguous portion of 17, 18, 19, 20, 21, 22 or 23 nucleosides of the ZPI mRNA. In certain embodiments, the first strand of the nucleic acid according to the invention is partially or fully complementary to a contiguous portion of 17, 18, 19, 20, 21, 22 or 23 nucleosides of any one of the sequences as listed in Table 1, i.e., any one of SEQ ID NOs: 1-47.

In certain embodiments, the first (antisense) strand of the nucleic acid according to the invention is partially complementary to a contiguous portion of the ZPI mRNA if it comprises a contiguous nucleoside sequence of at least 17 nucleosides, wherein at least 14, 15, 16 or 17 nucleosides of said contiguous nucleoside sequence are complementary to a contiguous portion of the ZPI mRNA. In certain embodiments, the first strand of the nucleic acid according to the invention comprises a contiguous nucleoside sequence of at least 17 nucleosides, wherein at least 14, 15, 16 or 17 nucleosides of said contiguous nucleoside sequence are complementary to a contiguous portion of any one of the sequences listed in Table 1, i.e., any one of SEQ ID NOs: 1-47. In certain embodiments, the first strand of the nucleic acid according to the invention comprises a contiguous nucleoside sequence of 19 nucleosides, wherein at least 14, 15, 16, 17, 18 or all 19 nucleosides of said contiguous nucleoside sequence are complementary to a contiguous portion of any one of the sequences listed in Table 1, i.e., any one of SEQ ID NOs: 1-47. In certain embodiments, the first strand of the nucleic acid according to the invention comprises a contiguous nucleoside sequence of 23 nucleosides, wherein at least 18, 19, 20, 21, 22 or all 23 nucleosides of said contiguous nucleoside sequence are complementary to a contiguous portion of any one of the sequences listed in Table 1, i.e., any one of SEQ ID NOs: 1-47.

In some embodiments, a nucleic acid e.g. an siRNA of the invention includes a sense strand that is substantially or partially complementary to an antisense oligonucleoside which, in turn, is complementary to a target gene sequence and comprises a contiguous nucleoside sequence. The nucleoside sequence of the sense strand is typically at least about 80% complementary over its entire length to the equivalent region of the nucleoside sequence of the antisense strand, such as about 85%, 86%, 87%, 88%, 89%, 90%, 91 %, 92%, 93%, 94%, 95%, 96%, 97%, 98%, or 99% complementary, or 100% complementary.

In some embodiments, a nucleic acid e.g. an siRNA of the invention includes an antisense strand that is substantially or partially complementary to the target sequence and comprises a contiguous nucleoside sequence which is at least 80% complementary over its entire length to the target sequence such as about 85%, 86%, 87%, 88%, 89%, 90%, 91 %, 92%, 93%, 94%, 95%, 96%, 97%, 98%, or 99% complementary, or 100% complementary.

As used herein, a "subject" is an animal, such as a mammal, including a primate (such as a human, a non-human primate, e.g., a monkey, and a chimpanzee), or a non-primate or a bird that expresses the target gene, either endogenously or heterologously, when the target gene sequence has sufficient complementarity to the nucleic acid e.g. siRNA agent to promote target knockdown. In certain preferred embodiments, the subject is a human.

The terms "treating" or "treatment" refer to a beneficial or desired result including, but not limited to, alleviation or amelioration of one or more symptoms associated with gene expression. "Treatment" can also mean prolonging survival as compared to expected survival in the absence of treatment.

The terms "prevent" or "prevention" as used herein are defined as eliminating or reducing the likelihood of occurrence of one or more symptoms of a disease or disorder. For example, the inhibitor disclosed herein can be used to prevent the occurrence of a disease related to a disorder of haemostasis, such as haemophilia.

In an embodiment, the inhibitor disclosed herein can be used to prevent the occurrence of Von Willebrand disease.

In an embodiment, the inhibitor disclosed herein can be used to prevent the occurrence of Factor X Deficiency.

"Therapeutically effective amount," as used herein, is intended to include the amount of a nucleic acid e.g. an siRNA that, when administered to a patient for treating a subject having disease, is sufficient to effect treatment of the disease (e.g., by diminishing, ameliorating or maintaining the existing disease or one or more symptoms of disease or its related comorbidities).

The phrase "pharmaceutically acceptable" is employed herein to refer to compounds, materials, compositions, or dosage forms which are suitable for use in contact with the tissues of human subjects and animal subjects without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition, or vehicle, such as a liquid or solid filler, diluent, excipient, manufacturing aid or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the subject being treated.

Where a value or range of values of a parameter are recited, it is intended that values and ranges intermediate to the recited values are also intended to be part of this invention.

The articles "a" and "an" are used herein to refer to one or to more than one (i.e. to at least one) of the grammatical object of the article.

The term "including" is used herein to mean, and is used interchangeably with, the phrase "including but not limited to".

The term "or" is used herein to mean, and is used interchangeably with, the term "and/or," unless context clearly indicates otherwise. For example, "sense strand or antisense strand" is understood as "sense strand or antisense strand or sense strand and antisense strand."

The term "about" is used herein to mean within the typical ranges of tolerances in the art. For example, "about" can be understood as about 2 standard deviations from the mean. In certain embodiments, about means +10%. In certain embodiments, about means +5%. When about is present before a series of numbers or a range, it is understood that "about" can modify each of the numbers in the series or range.

The term "at least" prior to a number or series of numbers is understood to include the number adjacent to the term "at least", and all subsequent numbers or integers that could logically be included, as clear from context. For example, the number of nucleosides in a nucleic acid molecule must be an integer. For example, "at least 18 nucleosides of a 21 nucleoside nucleic acid molecule" means that 18, 19, 20, or 21 nucleosides have the indicated property. When at least is present before a series of numbers or a range, it is understood that "at least" can modify each of the numbers in the series or range.

As used herein, "no more than" or "less than" is understood as the value adjacent to the phrase and logical lower values or integers, as logical from context, to zero. For example, a duplex with an overhang of "no more than 2 nucleosides" has a 2, 1, or 0 nucleoside overhang. When "no more than" is present before a series of numbers or a range, it is understood that "no more than" can modify each of the numbers in the series or range.

The terminal region of a strand is the last 5 nucleosides from the 5' or the 3' end.

A nucleobase sequence is the sequence of the bases of the nucleic acid in an oligomer.

Positions of the first and second strands, such as 'position 1' are counted from the 5' end of the relevant strand, excluding any inverted abasic nucleosides.Various embodiments of the invention can be combined as determined appropriate by one of skill in the art.

### Abasic Nucleosides

In certain embodiments, there are 1, e.g. 2, e.g. 3, e.g. 4 or more abasic nucleosides present in nucleic acids according to the present invention. Abasic nucleosides are modified nucleosides because they lack the base normally seen at position 1 of the sugar moiety. Typically, there will be a hydrogen at position 1 of the sugar moiety of the abasic nucleosides present in a nucleic acid according to the present invention.

The abasic nucleosides are in the terminal region of the second strand, preferably located within the terminal 5 nucleosides of the end of the strand. The terminal region may be the terminal 5 nucleosides, which includes abasic nucleosides.

The second strand may comprise, as preferred features (which are all specifically contemplated in combination unless mutually exclusive):
2, or more than 2, abasic nucleosides in a terminal region of the second strand; and / or
2, or more than 2, abasic nucleosides in either the 5' or 3' terminal region of the second strand; and / or
2, or more than 2, abasic nucleosides in either the 5' or 3' terminal region of the second strand, wherein the abasic nucleosides are present in an overhang as herein described; and/or
2, or more than 2, consecutive abasic nucleosides in a terminal region of the second strand, wherein preferably one such abasic nucleoside is a terminal nucleoside; and / or
2, or more than 2, consecutive abasic nucleosides in either the 5' or 3' terminal region of the second strand, wherein preferably one such abasic nucleoside is a terminal nucleoside in either the 5' or 3' terminal region of the second strand; and / or
a reversed internucleoside linkage connects at least one abasic nucleoside to an adjacent basic nucleoside in a terminal region of the second strand; and / or
a reversed internucleoside linkage connects at least one abasic nucleoside to an adjacent basic nucleoside in either the 5' or 3' terminal region of the second strand; and /or
an abasic nucleoside as the penultimate nucleoside which is connected via the reversed linkage to the nucleoside which is not the terminal nucleoside (called the antepenultimate nucleoside herein); and/or
abasic nucleosides as the 2 terminal nucleosides connected via a 5'-3' linkage when reading the strand in the direction towards the terminus comprising the terminal nucleosides;
abasic nucleosides as the 2 terminal nucleosides connected via a 3'-5' linkage when reading the strand in the direction towards the terminus comprising the terminal nucleosides;
abasic nucleosides as the terminal 2 positions, wherein the penultimate nucleoside is connected via the reversed linkage to the antepenultimate nucleoside, and wherein the reversed linkage is a 5-5' reversed linkage or a 3'-3' reversed linkage;
abasic nucleosides as the terminal 2 positions, wherein the penultimate nucleoside is connected via the reversed linkage to the antepenultimate nucleoside, and wherein either
(1) the reversed linkage is a 5-5' reversed linkage and the linkage between the terminal and penultimate abasic nucleosides is 3'5' when reading towards the terminus comprising the terminal and penultimate abasic nucleosides; or
(2) the reversed linkage is a 3-3' reversed linkage and the linkage between the terminal and penultimate abasic nucleosides is 5'3' when reading towards the terminus comprising the terminal and penultimate abasic nucleosides.

Preferably there is an abasic nucleoside at the terminus of the second strand.

Preferably there are 2 or at least 2 abasic nucleosides in the terminal region of the second strand, preferably at the terminal and penultimate positions.

Preferably 2 or more abasic nucleosides are consecutive, for example all abasic nucleosides may be consecutive. For example, the terminal 1 or terminal 2 or terminal 3 or terminal 4 nucleosides may be abasic nucleosides.

An abasic nucleoside may also be linked to an adjacent nucleoside through a 5'-3' phosphodiester linkage or reversed linkage unless there is only 1 abasic nucleoside at the terminus, in which case it will have a reversed linkage to the adjacent nucleoside.

A reversed linkage (which may also be referred to as an inverted linkage, which is also seen in the art), comprises either a 5'-5', a 3'3', a 3'-2' or a 2'-3' phosphodiester linkage between the adjacent sugar moieties of the nucleosides.

Abasic nucleosides which are not terminal will have 2 phosphodiester bonds, one with each adjacent nucleoside, and these may be a reversed linkage or may be a 5'-3 phosphodiester bond or may be one of each.

A preferred embodiment comprises 2 abasic nucleosides at the terminal and penultimate positions of the second strand, and wherein the reversed internucleoside linkage is located between the penultimate (abasic) nucleoside and the antepenultimate nucleoside.

Preferably there are 2 abasic nucleosides at the terminal and penultimate positions of the second strand and the penultimate nucleoside is linked to the antepenultimate nucleoside through a reversed internucleoside linkage and is linked to the terminal nucleoside through a 5'-3' or 3'-5' phosphodiester linkage (reading in the direction of the terminus of the molecule).

Preferably a nucleic acid according to the present invention comprises one or more abasic nucleosides, optionally wherein the one or more abasic nucleosides are in a terminal region of the second strand, and/or wherein at least one abasic nucleoside is linked to an adjacent basic nucleoside through a reversed internucleoside linkage.

Typically the second strand comprises 2 consecutive abasic nucleosides in the 5' terminal region of the second strand, wherein one such abasic nucleoside is a terminal nucleoside at the 5' terminal region of the second strand and the other abasic nucleoside is a penultimate nucleoside at the 5' terminal region of the second strand, wherein: (a) said penultimate abasic nucleoside is connected to an adjacent first basic nucleoside in an adjacent 5' near terminal region through a reversed internucleoside linkage; and (b) the reversed linkage is a 5-5' reversed linkage; and (c) the linkage between the terminal and penultimate abasic nucleosides is 3'5' when reading towards the terminus comprising the terminal and penultimate abasic nucleosides. More typically, (i) the first strand and the second strand each has a length of 23 nucleosides; (ii) two phosphorothioate internucleoside linkages are respectively between three consecutive positions in said 5' near terminal region of the second strand, wherein a first phosphorothioate internucleoside linkage is present between said adjacent first basic nucleoside of (a) and an adjacent second basic nucleoside in said 5' near terminal region of the second strand, and a second phosphorothioate internucleoside linkage is present between said adjacent second basic nucleoside and an adjacent third basic nucleoside in said 5' near terminal region of the second strand; (iii) two phosphorothioate internucleoside linkages are respectively between three consecutive positions in both 5' and 3' terminal regions of the first strand, whereby a terminal nucleoside respectively at each of the 5' and 3' terminal regions of said first strand is each attached to a respective 5' and 3' adjacent penultimate nucleoside by a phosphorothioate internucleoside linkage, and each first 5' and 3' penultimate nucleoside is attached to a respective 5' and 3' adjacent antepenultimate nucleoside by a phosphorothioate internucleoside linkage; and (iv) the second strand of the nucleic acid is conjugated directly or indirectly to one or more ligand moieties at the 3' terminal region of the second strand.

Alternatively the second strand comprises 2 consecutive abasic nucleosides preferably in an overhang in the 3' terminal region of the second strand, wherein one such abasic nucleoside is a terminal nucleoside at the 3' terminal region of the second strand and the other abasic nucleoside is a penultimate nucleoside at the 3' terminal region of the second strand, wherein: (a) said penultimate abasic nucleoside is connected to an adjacent first basic nucleoside in an adjacent 3' near terminal region through a reversed internucleoside linkage; and (b) the reversed linkage is a 3-3' reversed linkage; and (c) the linkage between the terminal and penultimate abasic nucleosides is 5'-3' when reading towards the terminus comprising the terminal and penultimate abasic nucleosides. More typically, (i) the first strand and the second strand each has a length of 23 nucleosides; (ii) two phosphorothioate internucleoside linkages are respectively between three consecutive positions in said 3' near terminal region of the second strand, wherein a first phosphorothioate internucleoside linkage is present between said adjacent first basic nucleoside of (a) and an adjacent second basic nucleoside in said 3' near terminal region of the second strand, and a second phosphorothioate internucleoside linkage is present between said adjacent second basic nucleoside and an adjacent third basic nucleoside in said 3' near terminal region of the second strand; (iii) two phosphorothioate internucleoside linkages are respectively between three consecutive positions in both 5' and 3' terminal regions of the first strand, whereby a terminal nucleoside respectively at each of the 5' and 3' terminal regions of said first strand is each attached to a respective 5' and 3' adjacent penultimate nucleoside by a phosphorothioate internucleoside linkage, and each first 5' and 3' penultimate nucleoside is attached to a respective 5' and 3' adjacent antepenultimate nucleoside by a phosphorothioate internucleoside linkage; and (iv) the second strand of the nucleic acid is conjugated directly or indirectly to one or more ligand moieties at the 5' terminal region of the second strand.

Examples of the structures are as follows (where the specific RNA nucleosides shown are not limiting and could be any RNA nucleoside):
A A 3'-3' reversed bond (and also showing the 5'-3 direction of the last phosphodiester bond between the two abasic molecules reading towards the terminus of the molecule) 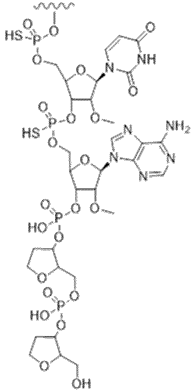
B Illustrating a 5'-5' reversed bond (and also showing the 3'-5' direction of the last phosphodiester bond between the two abasic molecules reading towards the terminus of the molecule)

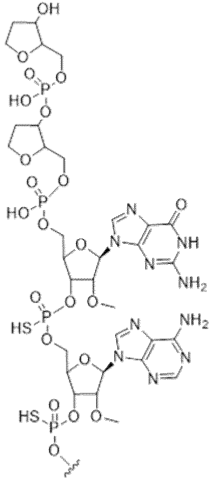

The abasic nucleoside or abasic nucleosides present in the nucleic acid are provided in the presence of a reversed internucleoside linkage or linkages, namely a 5'-5' or a 3 '-3' reversed internucleoside linkage. A reversed linkage occurs as a result of a change of orientation of an adjacent nucleoside sugar, such that the sugar will have a 3' - 5' orientation as opposed to the conventional 5' - 3' orientation (with reference to the numbering of ring atoms on the nucleoside sugars). The abasic nucleoside or nucleosides as present in the nucleic acids of the invention preferably include such inverted nucleoside sugars.

In the case of a terminal nucleoside having an inverted orientation, then this will result in an "inverted" end configuration for the overall nucleic acid. Whilst certain structures drawn and referenced herein are represented using conventional 5' - 3' direction (with reference to the numbering of ring atoms on the nucleoside sugars), it will be appreciated that the presence of a terminal nucleoside having a change of orientation and a proximal 3'-3' reversed linkage, will result in a nucleic acid having an overall 5'- 5' end structure (i.e. the conventional 3' end nucleoside becomes a 5' end nucleoside). Alternatively, it will be appreciated that the presence of a terminal nucleoside having a change of orientation and a proximal 5'-5' reversed linkage will result in a nucleic acid with an overall 3'- 3' end structure.

The proximal 3'-3' or 5'-5' reversed linkage as herein described, may comprise the reversed linkage being directly adjacent / attached to a terminal nucleoside having an inverted orientation, such as a single terminal nucleoside having an inverted orientation. Alternatively, the proximal 3'-3' or 5'-5' reversed linkage as herein described, may comprise the reversed linkage being adjacent 2, or more than 2, nucleosides having an inverted orientation, such as 2, or more than 2, terminal region nucleosides having an inverted orientation, such as the terminal and penultimate nucleosides. In this way, the reversed linkage may be attached to a penultimate nucleoside having an inverted orientation. While a skilled addressee will appreciate that inverted orientations as described above can result in nucleic acid molecules having overall 3' - 3' or 5'- 5' end structures as described herein, it will also be appreciated that with the presence of one or more additional reversed linkages and / or nucleosides having an inverted orientation, then the overall nucleic acid may have 3' - 5' end structures corresponding to the conventionally positioned 5' / 3' ends.

In one aspect the nucleic acid may have a 3'-3' reversed linkage, and the terminal sugar moiety may comprise a 5' OH rather than a 5' phosphate group at the 5' position of that terminal sugar.

A skilled person would therefore clearly understand that 5'- 5', 3'-3' and 3' - 5' (reading in the direction of that terminus) end variants of the more conventional 5'- 3' structures (with reference to the numbering of ring atoms on the end nucleoside sugars) drawn herein are included in the scope of the disclosure, where a reversed linkage or linkages is / are present.

In the situation of e.g., a reversed internucleoside linkage and / or one or more nucleosides having an inverted orientation creating an inverted end, and where the relative position of a linkage (e.g., to a linker) or the location of an internal feature (such as a modified nucleoside) is defined relative to the 5' or 3' end of the nucleic acid, then the 5' or 3' end is the conventional 5' or 3' end which would have existed had a reversed linkage not been in place, and wherein the conventional 5' or 3' end is determined by consideration of the directionality of the majority of the internal nucleoside linkages and / or nucleoside orientation within the nucleic acid. It is possible to tell from these internal bonds and / or nucleoside orientation which ends of the nucleic acid would constitute the conventional 5' and 3' ends (with reference to the numbering of ring atoms on the end nucleoside sugars) of the molecule absent the reversed linkage.

For example, in the structure shown below there are abasic residues in the first 2 positions located at the 5' end. Where the terminal nucleoside has an inverted orientation then the 5' end indicated in the diagram below, which is the conventional 5' end, can in fact comprise a 3' OH in view of the inverted nucleoside at the terminal position. Nevertheless the majority of the molecule will comprise conventional internucleoside linkages that run from the 3' OH of the sugar to the 5' phosphate of the next sugar, when reading in the standard 5' [PO₄] to 3' [OH] direction of a nucleic acid molecule (with reference to the numbering of ring atoms on the nucleoside sugars), which can be used to determine the conventional 5' and 3' ends that would be found absent the inverted end configuration.
5' A -A -Me- Me- Me-Me- Me- Me- F -Me- F -F - F -Me- Me- Me- Me-Me-Me- Me- Me- Me-Me 3'

In some embodiments, the second (sense) strand of the nucleic acid according to the invention comprises 2 consecutive abasic nucleosides in the 5' terminal region as shown in the following 5' terminal motif 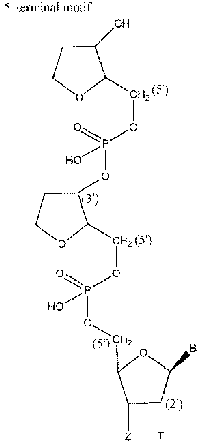 wherein:
B represents a nucleoside base,
T represent H, OH or a 2' ribose modification,
Z represents the remaining nucleosides of said second strand.

In some embodiments, the second (sense) strand of the nucleic acid according to the invention comprises 2 consecutive abasic nucleosides in the 5' terminal region as shown in the following 5' terminal motif 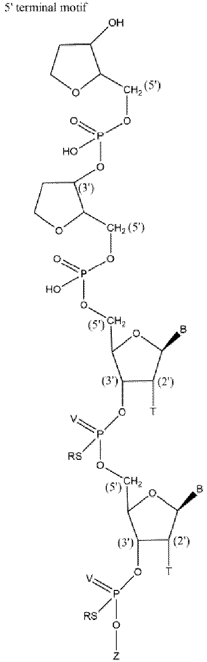 wherein:
B represents a nucleoside base,
T represents H, OH or a 2' ribose modification (preferably a 2' ribose modification, more preferably a 2'Me or 2'F ribose modification),
V represents O or S (preferably O),
R represents H or C₁₋₄ alkyl (preferably H),
Z represents the remaining nucleosides of said second strand,
more preferably the following 5' terminal motif 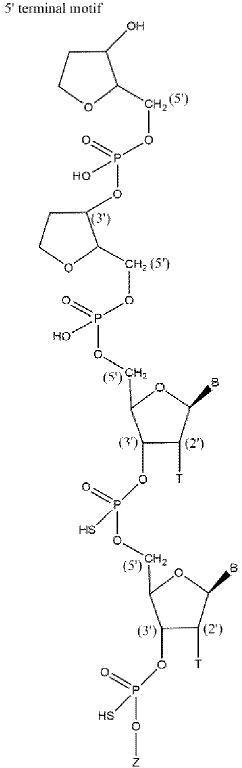 wherein:
   B represents a nucleoside base,
   T represents a 2' ribose modification (preferably a 2'Me or 2'F ribose modification),
   Z represents the remaining nucleosides of said second strand.

The reversed bond is preferably located at the end of the nucleic acid e.g., RNA which is distal to a ligand moiety, such as a GalNAc containing portion, of the molecule.

GalNAc-siRNA constructs with a 5'-GalNAc on the sense strand can have a reversed linkage on the opposite end of the sense strand.

GalNAc-siRNA constructs with a 3'-GalNAc on the sense strand can have a reversed linkage on the opposite end of the sense strand.

In a preferred embodiment, the second (sense) strand of the nucleic acid according to the invention comprises 2 consecutive abasic nucleosides in the 5' terminal region as shown in the following 5' terminal motif 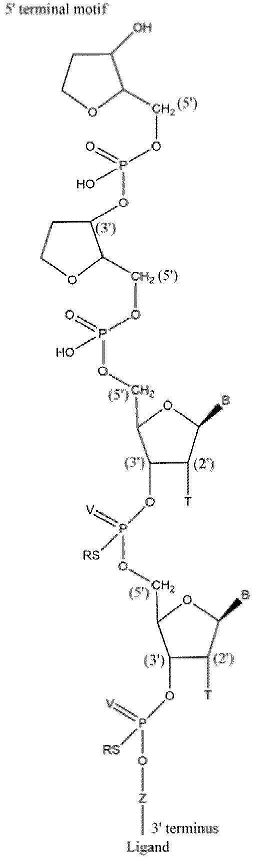 wherein:
B represents a nucleoside base,
T represent H, OH or a 2' ribose modification (preferably a 2' ribose modification, more preferably a 2'Me or 2'F ribose modification),
V represent O or S (preferably O),
R represent H or C₁₋₄ alkyl (preferably H),
Z comprises 11 to 26 contiguous nucleosides, preferably 15 to 21 contiguous nucleosides, and more preferably 19 contiguous nucleosides, more preferably the following 5' terminal motif 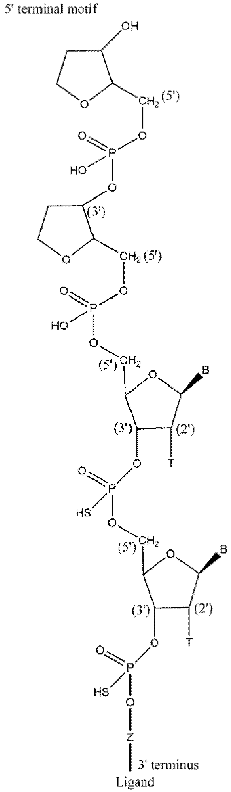 wherein:
   B represents a nucleoside base,
   T represents a 2' ribose modification (preferably a 2'Me or 2'F ribose modification),
   Z comprises 19 contiguous nucleosides.

### Nucleic Acid Lengths

In one aspect the i) the first strand of the nucleic acid has a length in the range of 17 to 30 nucleosides, preferably 19 to 25 nucleosides, more preferably 19 or 23 nucleosides; and / or ii) the second strand of the nucleic acid has a length in the range of 17 to 30 nucleosides, preferably 19 to 25 nucleosides, more preferably 19 or 21 nucleosides.

Typically the duplex region of the nucleic acid is between 17 and 30 nucleosides in length, more preferably is 19 or 21 nucleosides in length. Similarly, the region of complementarity between the first strand and the portion of RNA transcribed from the ZPI gene is between 17 and 30 nucleosides in length.

### Nucleic Acid Modifications

In certain embodiments, the nucleic acid e.g. an RNA of the invention e.g., a dsiRNA, does not comprise further modifications, e.g., chemical modifications or conjugations known in the art and described herein.

In other preferred embodiments, the nucleic acid e.g. RNA of the invention, e.g., a dsiRNA, is further chemically modified to enhance stability or other beneficial characteristics.

In certain embodiments of the invention, substantially all of the nucleosides are modified.

The nucleic acids featured in the invention can be synthesized or modified by methods well established in the art, such as those described in "Current protocols in nucleic acid chemistry," Beaucage, S.L. et al. (Edrs.), John Wiley & Sons, Inc., New York, NY, USA, which is hereby incorporated herein by reference.

Modifications include, for example, end modifications, e.g., 5'-end modifications (phosphorylation, conjugation, inverted linkages) or 3 '-end modifications (conjugation, DNA nucleosides within an RNA, or RNA nucleosides within a DNA, inverted linkages, etc.); base modifications, e.g., replacement with stabilizing bases, destabilizing bases, or bases that base pair with an expanded repertoire of partners, conjugated bases; sugar modifications (e.g., at the 2'-position or 4'- position) or replacement of the sugar; or backbone modifications, including modification or replacement of the phosphodiester linkages.

Specific examples of nucleic acids such as siRNA compounds useful in the embodiments described herein include, but are not limited to RNAs containing modified backbones or no natural internucleoside linkages. Nucleic acids such as RNAs having modified backbones include, among others, those that do not have a phosphorus atom in the backbone. For the purposes of this specification, and as sometimes referenced in the art, modified nucleic acids e.g., RNAs that do not have a phosphorus atom in their internucleoside backbone can also be considered to be oligonucleosides. In some embodiments, a modified nucleic acid e.g., an siRNA will have a phosphorus atom in its internucleoside backbone.

Modified nucleic acid e.g. RNA backbones include, for example, phosphorothioates, chiral phosphorothioates, phosphorodithioates, phosphotriesters, aminoalkyl phosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5'-linked analogs of these, and those having inverted polarity wherein the adjacent pairs of nucleoside units are linked 5'-3' or 5'-2'. Various salts, mixed salts and free acid forms are also included.

Modified nucleic acids e.g. RNAs can also contain one or more substituted sugar moieties. The nucleic acids e.g. siRNAs, e.g., dsiRNAs, featured herein can include one of the following at the 2'-position: OH; F; O-, S-, or N-alkyl; O-, S-, or N-alkenyl; O-, S- or N-alkynyl; or O-alkyl-O-alkyl, wherein the alkyl, alkenyl and alkynyl can be substituted or unsubstituted. 2' O- methyl and 2' -F are preferred modifications.

In certain preferred embodiments, the nucleic acid comprises at least one modified nucleoside.

The nucleic acid of the invention may comprise one or more modified nucleosides on the first strand and/or the second strand.

In some embodiments, substantially all of the nucleosides of the sense strand and all of the nucleosides of the antisense strand comprise a modification.

In some embodiments, all of the nucleosides of the sense strand and substantially all of the nucleosides of the antisense strand comprise a modification.

In some embodiments, all of the nucleosides of the sense strand and all of the nucleosides of the antisense strand comprise a modification.

In one embodiment, at least one of the modified nucleosides is selected from the group consisting of a deoxy- nucleoside, a 3 '-terminal deoxy-thymine (dT) nucleoside, a 2'-O-methyl modified nucleoside (also called herein 2'-Me, where Me is a methoxy), a 2'-fluoro modified nucleoside, a 2'-deoxy- modified nucleoside, a locked nucleoside, an unlocked nucleoside, a conformationally restricted nucleoside, a constrained ethyl nucleoside, an abasic nucleoside, a 2' -amino- modified nucleoside, a 2'- O-allyl- modified nucleoside, 2' -C-alkyl- modified nucleoside, 2'-hydroxly-modified nucleoside, a 2'- methoxyethyl modified nucleoside, a 2'-O-alkyl-modified nucleoside, a morpholino nucleoside, a phosphoramidate, a non-natural base comprising nucleoside, a tetrahydropyran modified nucleoside, a 1 ,5-anhydrohexitol modified nucleoside, a cyclohexenyl modified nucleoside, a nucleoside comprising a phosphorothioate group, a nucleoside comprising a methylphosphonate group, a nucleoside comprising a 5 '-phosphate, and a nucleoside comprising a 5 '-phosphate mimic. In another embodiment, the modified nucleosides comprise a short sequence of 3 '-terminal deoxy-thymine nucleosides (dT).

Modifications on the nucleosides may preferably be selected from the group including, but not limited to, LNA, HNA, CeNA, 2 -methoxyethyl, 2'-O-alkyl, 2 -O-allyl, 2'-C-allyl, 2'-fluoro, 2'-deoxy, 2'- hydroxyl, and combinations thereof. In another embodiment, the modifications on the nucleosides are 2-O-methyl ("2-Me") or 2'-fluoro modifications.

One preferred modification is a modification at the 2'-OH group of the ribose sugar, optionally selected from 2'-Me or 2'-F modifications.

In certain embodiments, the nucleic acid e.g., siRNA agent further comprises at least one phosphorothioate or methylphosphonate internucleoside linkage. For example the phosphorothioate or methylphosphonate internucleoside linkage can be at the 3 '-terminus or in the terminal region of one strand, i.e., the sense strand or the antisense strand; or at the ends of both strands, the sense strand and the antisense strand.

In certain embodiments, the phosphorothioate or methylphosphonate internucleoside linkage is at the 5 'terminus or in the terminal region of one strand, i.e., the sense strand or the antisense strand; or at the ends of both strands, the sense strand and the antisense strand.

In certain embodiments, a phosphorothioate or a methylphosphonate internucleoside linkage is at both the 5'- and 3 '-terminus or in the terminal region of one strand, i.e., the sense strand or the antisense strand; or at the ends of both strands, the sense strand and the antisense strand.

Any nucleic acid may comprise one or more phosphorothioate (PS) modifications within the nucleic acid, such as at least two PS internucleoside bonds at the ends of a strand.

At least one of the oligoribonucleoside strands preferably comprises at least two consecutive phosphorothioate modifications in the last 3 nucleosides of the oligonucleoside.

The invention therefore also relates to: A nucleic acid disclosed herein which comprises phosphorothioate internucleoside linkages respectively between at least two or three consecutive positions, such as in a 5' and/or 3' terminal region and/or near terminal region of the second strand, whereby said near terminal region is preferably adjacent said terminal region wherein said one or more abasic nucleosides of said second strand is / are located.

A nucleic acid disclosed herein which comprises phosphorothioate internucleoside linkages respectively between at least two or three consecutive positions in a 5' and / or 3' terminal region of the first strand, whereby preferably the terminal position at the 5' and / or 3' terminal region of said first strand is attached to its adjacent position by a phosphorothioate internucleoside linkage.

The nucleic acid strand may be an RNA comprising a phosphorothioate internucleoside linkage between the three nucleosides contiguous with 2 terminally located abasic nucleosides.

A preferred nucleic acid is a double stranded RNA comprising 2 adjacent abasic nucleosides at the 5' terminus of the second strand and a ligand moiety comprising one or more GalNAc ligand moieties at the opposite 3' end of the second strand. Further preferred, the same nucleic acid may also comprise a phosphorothioate bond between nucelotides at positions 3-4 and 4-5 of the second strand, reading from the position 1 of the second strand.

Position 1 of the first or the second strand is the nucleoside which is the closest to the end of the nucleic acid (ignoring any abasic nucleosides) and that is joined to an adjacent nucleoside (at Position 2) via a 3' to 5' internal bond, with reference to the bonds between the sugar moieties of the backbone, and reading in a direction away from that end of the molecule.

It can therefore be seen that "position 1 of the sense strand" is the 5' most nucleoside (not including abasic nucleosides) at the conventional 5' end of the sense strand. Typically, the nucleoside at this position 1 of the sense strand will be equivalent to the 5' nucleoside of the selected target nucleic acid sequence, and more generally the sense strand will have equivalent nucleosides to those of the target nucleic acid sequence starting from this position 1 of the sense strand, whilst also allowing for acceptable mismatches between the sequences.

As used herein, "position 1 of the antisense strand" is the 5' most nucleoside (not including abasic nucleosides) at the conventional 5' end of the antisense strand. As hereinbefore described, there will be a region of complementarity between the sense and antisense strands, and in this way the antisense strand will also have a region of complementarity to the target nucleic acid sequence as referred to above.

Preferred modifications that can be used with sequences according to the present invention can be as follows:
Modification 1:
   First strand modification:
      NmsNfsNmNfNmNfNmNfNfNmNmNmNmNfNmNfNmNmNmNmNmsNmsNm (5' to 3')
   Second strand modification:
      iaiaNmsNmsNmNmNmNmNfNfNfNfNfNmNmNmNmNmNmNmNfNmNm (5' to 3')
Modification 2:
   First strand modification:
      NmsNfsNmNfNmNfNmNfNfNmNmNmNmNfNmNfNmNmNmNmNmsNmsNm (5' to 3')
   Second strand modification:
      iaiaNmsNmsNmNmNmNfNfNmNfNfNfNfNmNmNmNmNmNmNmNmNm (5' to 3')
Modification 3:
   First strand modification:
      NmsNfsNmNfNmNfNmNfNfNmNmNmNmNfNmNfNmNmNmNmNmsNmsNm (5' to 3')
   Second strand modification:
      iaiaNmsNmsNmNmNmNmNfNmNfNfNfNfNmNmNmNmNmNmNmNmNm (5' to 3')
Modification 4:
   First strand modification:
      NmsNfsNmNfNmNfNmNmNmNmNmNmNmNfNmNfNmNmNmNmNmsNmsNm (5' to 3')
   Second strand modification:
      iaiaNmsNmsNmNmNmNmNfNmNfNfNfNfNmNmNmNmNmNmNmNmNm (5' to 3')
Modification 5:
   First strand modification:
      NmsNfsNmNmNmNfNmNmNfNmNmNmNmNfNmNfNmNmNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 6:
   First strand modification:
      NmsNfsNmNmNmNfNmNmNmNmNmNmNmNfNmNfNmNfNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 7:
   First strand modification:
      NmsNfsNmNmNmNyNmNmNmNmNmNmNmNfNmNfNmNmNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 8:
   First strand modification:
      NmsNfsNmNmNmNyNmNfNfNmNmNmNmNfNmNfNmNmNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 9:
   First strand modification:
      NmsNfsNmNmNmNfNmNmNmNmNmNmNmNfNmNfNmNmNmNfNmsNmsNm (5' to 3')
   Second strand modification:
      iaiaNmsNmsNmNmNmNmNmNmNfNfNfNmNmNmNmNmNmNmNmNmNm (5' to 3')
Modification 10:
   First strand modification:
      NmsNfsNmNfNmNfNmNmNmNmNmNmNmNfNmNfNmNmNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 11:
   First strand modification:
      NmsNfsNmNfNmNfNmNfNfNmNmNmNmNfNmNfNmNmNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 12:
   First strand modification:
      NmsNfsNmNmNmNfNmNfNfNmNmNmNmNfNmNfNmNfNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 13:
   First strand modification:
      NmsNfsNmNmNmNfNmNfNfNmNmNmNmNfNmNfNmNmNmNfNmsNmsNm (5' to 3')
   Second strand modification:
      iaiaNmsNmsNmNmNmNmNmNmNfNfNfNmNmNmNmNmNmNmNmNmNm (5' to 3')
   wherein in each of the above modifications:
   ia represents an inverted abasic nucleoside;
   Nm represents a 2'Me ribose modified nucleoside;
   Nf represents a 2'F ribose modified nucleoside;
   Ny represents a nucleoside with a thermally destabilizing modification, preferably wherein the destabilizing modification is selected from a modified unlocked nucleic acid (UNA) and a glycol nucleic acid (GNA), more preferably a glycol nucleic acid, most preferably an (S)-glycol nucleic acid;
   s represents a phosphorothioate internucleoside bond.

In a particularly preferred embodiment, the sequences according to the present invention have the following modification pattern:
Modification 13:
First strand modification:
   NmsNfsNmNmNmNfNmNfNfNmNmNmNmNfNmNfNmNmNmNfNmsNmsNm (5' to 3')
Second strand modification:
   iaiaNmsNmsNmNmNmNmNmNmNfNfNfNmNmNmNmNmNmNmNmNmNm (5' to 3')

In certain embodiments, the nucleic acid comprises a 5' vinylphosphonate (5'VP) modification. The 5'VP modification is preferably on the antisense strand, but can be on the sense strand as well, or instead. Preferably, the 5'VP modification is a 5'-(E)-vinylphosphonate (5'-(E)-VP) modification.

A 5'-VP modification is a stable phosphate mimic added at the 5' end of an oligonucleoside. It is a modification in which the 5' carbon forms a double bond to a 6' carbon linked to the phosphorus. Such modifications are described in Haraszti et al 2017 (Haraszti et al., 5'-Vinylphosphonate improves tissue accumulation and efficacy of conjugated siRNAs in vivo. Nucleic Acids Res. 2017 Jul 27;45(13):7581-7592. doi: 10.1093/nar/gkx507. PMID: 28591791; PMCID: PMC5570069).

### Conjugation

Another modification of the nucleic acid e.g., RNA e.g., an siRNA of the invention involves linking the nucleic acid e.g., the siRNA to one or more ligand moieties e.g. to enhance the activity, cellular distribution, or cellular uptake of the nucleic acid e.g. siRNA e.g. into a cell.

In certain embodiments, the inhibitor according to the invention is conjugated to a ligand moiety that enables and/or facilitates targeting of hepatocytes. In certain embodiments, targeting of hepatocytes is achieved using N-acetylgalactosamine (GalNAc) conjugates as described in more detail herein below. That is, in certain embodiments, the inhibitor according to the invention is an siRNA-GalNAc conjugate.

In some embodiments, the ligand moiety described can be attached to a nucleic acid e.g., an siRNA oligonucleoside, via a linker that can be cleavable or non-cleavable. The term "linker" or "linking group" means an organic moiety that connects two parts of a compound, e.g., covalently attaches two parts of a compound.

The ligand can be attached to the 3' or 5' end of the sense strand.

The ligand is preferably conjugated to 3' end of the sense strand of the nucleic acid e.g., an siRNA agent.

The invention therefore relates in a further aspect to a conjugate for inhibiting expression of a target gene in a cell, said conjugate comprising a nucleic acid portion and one or more ligand moieties, said nucleic acid portion comprising a nucleic acid as disclosed herein.

In one aspect the second strand of the nucleic acid is conjugated directly or indirectly (e.g., via a linker) to the one or more ligand moiety(s), wherein said ligand moiety is typically present at a terminal region of the second strand, preferably at the 3' terminal region thereof.

In certain embodiments, the ligand moiety comprises a GalNAc or GalNAc derivative attached to the nucleic acid e.g., dsiRNA through a linker.

Therefore, the invention relates to a conjugate wherein the ligand moiety comprises:
i) one or more GalNAc ligands; and/or
ii) one or more GalNAc ligand derivatives; and/or
iii) one or more GalNAc ligands conjugated to said nucleic acid through a linker.

Said GalNAc ligand may be conjugated directly or indirectly to the 5' or 3' terminal region of the second strand of the nucleic acid, preferably at the 3' terminal region thereof.

GalNAc ligands are well known in the art and described in, inter alia, EP3775207A1.

In some embodiments, the GalNAc ligand is comprised in any one of the linkers shown in Figures 1 to 4 or Figure 5 (Formula XI), wherein the "oligonucleotide" may be any nucleic acid disclosed herein. Accordingly, the "oligonucleotide" may comprise other bonds than a phosphodiester bond, such as one or more phosphorothioate bonds. Preferably, the nucleic acid according to the invention is a double stranded oligonucleoside as defined herein and the linker is conjugated to the second strand, more preferably to the 3' terminal region of the second strand, via a phosphodiester bond.

In some embodiments, the GalNAc ligand is comprised in the linker shown in Figure 3, wherein the "oligonucleotide" may be any nucleic acid disclosed herein. Accordingly, the "oligonucleotide" may comprise other bonds than a phosphodiester bond, such as one or more phosphorothioate bonds. Preferably, the nucleic acid according to the invention is a double stranded oligonucleoside as defined herein and the linker is conjugated to the second strand, more preferably to the 3' terminal region of the second strand, via a phosphodiester bond.

In some embodiments, the GalNAc ligand is comprised in the linker shown in Figure 5 (Formula XI), wherein the "oligonucleotide" may be any nucleic acid disclosed herein. Accordingly, the "oligonucleotide" may comprise other bonds than a phosphodiester bond, such as one or more phosphorothioate bonds. Preferably, the nucleic acid according to the invention is a double stranded oligonucleoside as defined herein and the linker is conjugated to the second strand, more preferably to the 3' terminal region of the second strand, via a phosphodiester bond.

In some embodiments, the GalNAc ligand is comprised in any one of the linkers shown in Figures 1 to 4 or Figure 5 (Formula XI), wherein the "oligonucleotide" represents a nucleic acid according to the invention, wherein the nucleic acid according to the invention comprises a modified or unmodified second strand comprising or consisting of any one of SEQ ID NO:95 to SEQ ID NO: 141, preferably wherein the linker is conjugated to the 3' terminal region of the second strand, i.e., to the 3' terminal region of any one of SEQ ID NO:95 to SEQ ID NO: 141, via a phosphodiester bond.

In some embodiments, the GalNAc ligand is comprised in the linker shown in Figure 3, wherein the "oligonucleotide" represents a nucleic acid according to the invention, wherein the nucleic acid according to the invention comprises a modified or unmodified second strand comprising or consisting of any one of SEQ ID NO:95 to SEQ ID NO: 141, preferably wherein the linker is conjugated to the 3' terminal region of the second strand, i.e., to the 3' terminal region of any one of SEQ ID NO:95 to SEQ ID NO: 141, via a phosphodiester bond.

In some embodiments, the GalNAc ligand is comprised in the linker shown in Figure 5 (Formula XI), wherein the "oligonucleotide" represents a nucleic acid according to the invention, wherein the nucleic acid according to the invention comprises a modified or unmodified second strand comprising or consisting of any one of SEQ ID NO:95 to SEQ ID NO: 141, preferably wherein the linker is conjugated to the 3' terminal region of the second strand, i.e., to the 3' terminal region of any one SEQ ID NO:95 to SEQ ID NO: 141, via a phosphodiester bond.

In some embodiments, the GalNAc ligand is comprised in any one of the linkers shown in Figures 1 to 4 or Figure 5 (Formula XI), wherein the "oligonucleotide" represents a nucleic acid according to the invention, wherein the nucleic acid according to the invention comprises a modified second strand comprising or consisting of any one of SEQ ID NO: 189 to SEQ ID NO:235, preferably wherein the linker is conjugated to the 3' terminal region of the second strand, i.e., to the 3' terminal region of any one of SEQ ID NO: 189 to SEQ ID NO:235, via a phosphodiester bond.

In some embodiments, the GalNAc ligand is comprised in the linker shown in Figure 3, wherein the "oligonucleotide" represents a nucleic acid according to the invention, wherein the nucleic acid according to the invention comprises a modified second strand comprising or consisting of any one of SEQ ID NO: 189 to SEQ ID NO:235, preferably wherein the linker is conjugated to the 3' terminal region of the second strand, i.e., to the 3' terminal region of any one of SEQ ID NO: 189 to SEQ ID NO:235, via a phosphodiester bond.

In some embodiments, the GalNAc ligand is comprised in the linker shown in Figure 5 (Formula XI), wherein the "oligonucleotide" represents a nucleic acid according to the invention, wherein the nucleic acid according to the invention comprises a modified second strand comprising or consisting of any one of SEQ ID NO: 189 to SEQ ID NO:235, preferably wherein the linker is conjugated to the 3' terminal region of the second strand, i.e., to the 3' terminal region of any one of SEQ ID NO: 189 to SEQ ID NO:235, via a phosphodiester bond.

In some embodiments, the GalNAc ligand is comprised in the linker shown in Figures 1 to 4 or Figure 5 (Formula XI), wherein the "oligonucleotide" represents a nucleic acid according to the invention, wherein the nucleic acid according to the invention comprises a modified second strand comprising or consisting of any one of SEQ ID NO: 189 to SEQ ID NO:235, preferably SEQ ID NO: 195 or SEQ ID NO: 192, wherein the second strand has the following structure 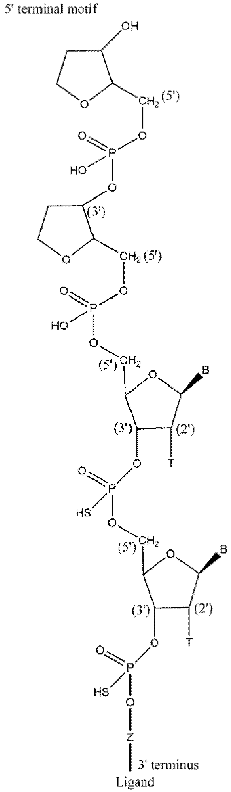 wherein:
T represents a 2'Me ribose modification,
B represents the nucleoside bases of the first two basic nucleosides in the 5' terminal region of any one of SEQ ID NO: 189 to SEQ ID NO:235, preferably SEQ ID NO: 195 or SEQ ID NO: 192, and
Z represents the remaining 19 contiguous basic nucleosides of any one of SEQ ID NO: 189 to SEQ ID NO:235, preferably SEQ ID NO: 195 or SEQ ID NO: 192.

In some embodiments, the GalNAc ligand is comprised in the linker shown in Figure 5 (Formula XI), wherein the "oligonucleotide" represents a nucleic acid according to the invention, wherein the nucleic acid according to the invention comprises a modified second strand comprising or consisting of any one of SEQ ID NO: 189 to SEQ ID NO:235, preferably SEQ ID NO: 195 or SEQ ID NO: 192, wherein the second strand has the following structure 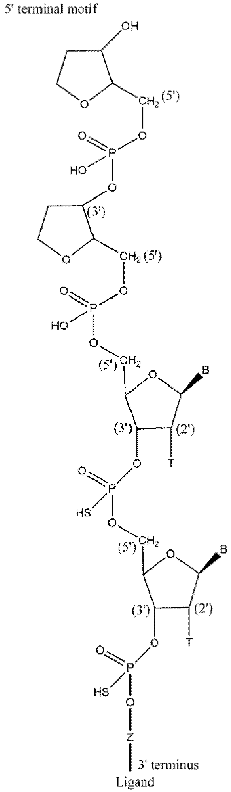 wherein:
T represents a 2'Me ribose modification,
B represents the nucleoside bases of the first two basic nucleosides in the 5' terminal region of any one of SEQ ID NO: 189 to SEQ ID NO:235, and
Z represents the remaining 19 contiguous basic nucleosides of any one of SEQ ID NO: 189 to SEQ ID NO:235.

In some embodiments, the GalNAc ligand is comprised in the linker shown in Figure 3, wherein the "oligonucleotide" represents a nucleic acid according to the invention, wherein the nucleic acid according to the invention comprises a modified second strand comprising or consisting of any one of SEQ ID NO: 189 to SEQ ID NO:235, preferably SEQ ID NO: 195 or SEQ ID NO: 192, wherein the second strand has the following structure 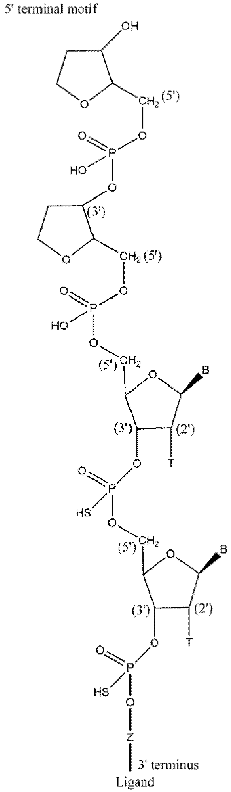 wherein:
T represents a 2'Me ribose modification,
B represents the nucleoside bases of the first two basic nucleosides in the 5' terminal region of any one of SEQ ID NO: 189 to SEQ ID NO:235, preferably SEQ ID NO: 195 or SEQ ID NO: 192, and
Z represents the remaining 19 contiguous basic nucleosides of any one of SEQ ID NO: 189 to SEQ ID NO:235, preferably SEQ ID NO: 195 or SEQ ID NO: 192.

### Vector And Cell

In one aspect, the invention provides a cell containing a nucleic acid, such as inhibitory RNA [RNAi] as described herein.

In one aspect, the invention provides a cell comprising a vector as described herein.

In one aspect the invention provides a vector comprising an oligonucleoside inhibitor, e.g.an iRNA e.g. siRNA.

### Pharmaceutically Acceptable Compositions

In one aspect, the invention provides a pharmaceutical composition for inhibiting expression of a target gene, the composition comprising a nucleic acid as disclosed herein.

The pharmaceutically acceptable composition may comprise an excipient and or carrier.

Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) lubricating agents, such as magnesium stearate, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen- free water; (17) isotonic saline; (18) Ringer's solution; (19) ethyl alcohol; (20) pH buffered solutions; (21) polyesters, polycarbonates and/or poly anhydrides; (22) bulking agents, such as polypeptides and amino acids (23) serum component, such as serum albumin, HDL and LDL; and (22) other non-toxic compatible substances employed in pharmaceutical formulations.

Typical pharmaceutical carriers include, but are not limited to, binding agents (e.g., pregelatinized maize starch, polyvinylpyrrolidone or hydroxypropyl methylcellulose, etc.); fillers (e.g., lactose and other sugars, microcrystalline cellulose, pectin, gelatin, calcium sulfate, ethyl cellulose, polyacrylates or calcium hydrogen phosphate, etc.); lubricants (e.g., magnesium stearate, talc, silica, colloidal silicon dioxide, stearic acid, metallic stearates, hydrogenated vegetable oils, corn starch, polyethylene glycols, sodium benzoate, sodium acetate, etc.); disintegrants (e.g., starch, sodium starch glycolate, etc.); and wetting agents (e.g., sodium lauryl sulphate, etc).

Pharmaceutically acceptable organic or inorganic excipients suitable for non-parenteral administration which do not deleteriously react with nucleic acids can also be used to formulate the compositions of the present invention. Suitable pharmaceutically acceptable excipients include, but are not limited to, water, salt solutions, alcohols, polyethylene glycols, gelatin, lactose, amylose, magnesium stearate, talc, silicic acid, viscous paraffin, hydroxymethylcellulose, polyvinylpyrrolidone, and the like.

Formulations for topical administration of nucleic acids can include sterile and non-sterile aqueous solutions, non-aqueous solutions in common solvents such as alcohols, or solutions of the nucleic acids in liquid or solid oil bases. The solutions can also contain buffers, diluents and other suitable additives. Pharmaceutically acceptable organic or inorganic excipients suitable for non- parenteral administration which do not deleteriously react with nucleic acids can be used.

In one embodiment, the nucleic acid or composition is administered in an unbuffered solution. In certain embodiments, the unbuffered solution is saline or water. In other embodiments, the nucleic acid e.g. siRNA agent is administered in a buffered solution. In such embodiments, the buffer solution can comprise acetate, citrate, prolamine, carbonate, or phosphate, or any combination thereof. For example, the buffer solution can be phosphate buffered saline (PBS).

### Dosages

The pharmaceutical compositions of the invention may be administered in dosages sufficient to inhibit expression of a gene. In general, a suitable dose of a nucleic acid e.g. an siRNA of the invention will be in the range of about 0.001 to about 200.0 milligrams per kilogram body weight of the recipient per day, generally in the range of about 1 to 50 mg per kilogram body weight per day. Typically, a suitable dose of a nucleic acid e.g. an siRNA of the invention will be in the range of about 0.1 mg/kg to about 5.0 mg/kg, e.g., about 0.3 mg/kg and about 3.0 mg/kg.

A repeat-dose regimen may include administration of a therapeutic amount of a nucleic acid e.g. siRNA on a regular basis, such as every other day or once a year. In certain embodiments, the nucleic acid e.g. siRNA is administered about once per month to about once per quarter (i.e., about once every three months).

In various embodiments, the nucleic acid e.g. siRNA agent is administered at a dose of about 0.01 mg/kg to about 10 mg/kg or about 0.5 mg/kg to about 50 mg/kg. In some embodiments, the nucleic acid e.g. siRNA agent is administered at a dose of about 10 mg/kg to about 30 mg/kg. In certain embodiments, the nucleic acid e.g. siRNA agent is administered at a dose selected from about 0.5 mg/kg 1 mg/kg, 1.5 mg/kg, 3 mg/kg, 5 mg/kg, 10 mg/kg, and 30 mg/kg. In certain embodiments, the nucleic acid e.g. agent is administered about once per week, once per month, once every other two months, or once a quarter (i.e., once every three months) at a dose of about 0.1 mg/kg to about 5.0 mg/kg. In certain embodiments, the nucleic acid e.g. siRNA agent is administered to the subject once a week. In certain embodiments, the nucleic acid e.g. siRNA agent is administered to the subject once a month. In certain embodiments, the nucleic acid e.g. siRNA agent is administered once per quarter (i.e., every three months).

After an initial treatment regimen, the treatments can be administered on a less frequent basis. For example, after administration weekly or biweekly for three months, administration can be repeated once per month, for six months, or a year; or longer.

The pharmaceutical composition can be administered once daily, or administered as two, three, or more sub-doses at appropriate intervals throughout the day or even using continuous infusion or delivery through a controlled release formulation. In that case, the nucleic acid e.g. siRNA contained in each sub-dose must be correspondingly smaller in order to achieve the total daily dosage. The dosage unit can also be compounded for delivery over several days, e.g., using a conventional sustained release formulation which provides sustained release of the nucleic acid e.g. siRNA over a several day period. Sustained release formulations are well known in the art and are particularly useful for delivery of agents at a particular site, such as could be used with the agents of the present invention. In this embodiment, the dosage unit contains a corresponding multiple of the daily dose.

In other embodiments, a single dose of the pharmaceutical compositions can be long lasting, such that subsequent doses are administered at not more than 3, 4, or 5 day intervals, or at not more than 1, 2, 3, or 4 week intervals. In some embodiments of the invention, a single dose of the pharmaceutical compositions of the invention is administered once per week. In other embodiments of the invention, a single dose of the pharmaceutical compositions of the invention is administered bimonthly. In certain embodiments, the siRNA is administered about once per month to about once per quarter (i.e., about once every three months), or even every 6 months or 12 months.

Estimates of effective dosages and in vivo half-lives for the individual nucleic acid e.g. siRNAs encompassed by the invention can be made using conventional methodologies or on the basis of in vivo testing using an appropriate animal model, as known in the art.

The pharmaceutical compositions of the present invention can be administered in a number of ways depending upon whether local or systemic treatment is desired and upon the area to be treated. Administration can be topical (e.g., by a transdermal patch), pulmonary, e.g., by inhalation or insufflation of powders or aerosols, including by nebulizer; intratracheal, intranasal, epidermal and transdermal, oral or parenteral. Parenteral administration includes intravenous, intraarterial, subcutaneous, intraperitoneal, or intramuscular injection or infusion; subdermal, e.g. via an implanted device; or intracranial, e.g., by intraparenchymal, intrathecal or intraventricular administration. In certain preferred embodiments, the compositions are administered by intravenous infusion or injection. In certain embodiments, the compositions are administered by subcutaneous injection.

In one embodiment, the nucleic acid e.g. agent is administered to the subject subcutaneously.

The nucleic acid e.g. siRNA can be delivered in a manner to target a particular tissue (e.g. in particular liver cells).

### Methods For Inhibiting ZPI Gene Expression

The present invention also provides methods of inhibiting expression of ZPI gene in a cell. The methods include contacting a cell with a nucleic acid of the invention e.g. siRNA agent, such as double stranded siRNA agent, in an amount effective to inhibit expression of the ZPI gene in the cell, thereby inhibiting expression of the ZPI gene in the cell. It is to be noted that a nucleic acid "for inhibiting the expression of ZPI" is a nucleic acid that is capable of inhibiting ZPI expression, preferably as described herein below.

Contacting of a cell with the nucleic acid e.g. an siRNA, such as a double stranded siRNA agent, may be done in vitro or in vivo. Contacting a cell in vivo with nucleic acid e.g. includes contacting a cell or group of cells within a subject, e.g., a human subject, with the nucleic acid e.g. siRNA. Combinations of in vitro and in vivo methods of contacting a cell are also possible. Contacting a cell may be direct or indirect, as discussed above. Furthermore, contacting a cell may be accomplished via a targeting ligand moiety, including any ligand moiety described herein or known in the art. In preferred embodiments, the targeting ligand moiety is a carbohydrate moiety, e.g. a GalNAc3 ligand, or any other ligand moiety that directs the siRNA agent to a site of interest.

The term "inhibiting," as used herein, is used interchangeably with "reducing," "silencing," "downregulating", "suppressing", and other similar terms, and includes any level of inhibition.

In some embodiments of the methods of the invention, expression of ZPI gene is inhibited by at least 30%, 35%, 40%, 45%, 50%, 55%, 60%, 65%, 70%, 75%, 80%, 85%, 90%, or 95%, or to below the level of detection of the assay, preferably when determined by qPCR as described herein and/or when the siRNA is introduced into the target cell by transfection. In certain embodiments, the methods include a clinically relevant inhibition of expression of ZPI target gene e.g. as demonstrated by a clinically relevant outcome after treatment of a subject with an agent to reduce the expression of the gene.

In some embodiments, when transfected into the cells, the nucleic acid of the invention inhibits expression of the ZPI gene with an IC50 value lower than 2500 pM, 2400 pM, 2300 pM, 2200 pM, 2100 pM, 2000 pM, 1900 pM, 1800 pM, 1700 pM, 1600 pM, 1500 pM, 1400 pM, 1300 pM, 1200 pM, 1100 pM, 1000 pM, 900 pM, 800 pM, 700 pM, 600 pM, 500 pM, 400 pM, 300 pM, 200 pM or 100 pM, preferably when determined by qPCR, more preferably by reverse transcriptase (RT)-qPCR, as described herein.

In a preferred embodiment, when transfected into the cells, the nucleic acid of the invention inhibits expression of the ZPI gene with an IC50 value lower than 2500 pM. In a more preferred embodiment, when transfected into the cells, the nucleic acid of the invention inhibits expression of the ZPI gene with an IC50 value lower than 1000 pM. In an even more preferred embodiment, when transfected into the cells, the nucleic acid of the invention inhibits expression of the ZPI gene with an IC50 value lower than 500 pM. In a most preferred embodiment, when transfected into the cells, the nucleic acid of the invention inhibits expression of the ZPI gene with an IC50 value lower than 100 pM.

Inhibition of expression of the ZPI gene may be quantified by the following method:
Huh7 cells (human hepatocyte-derived cell line, obtained from JCRB Cell Bank) may be maintained in Dulbecco's Modified Eagle Medium (DMEM) supplemented with 10% FBS at 37°C in an atmosphere of 5% CO₂. Cells may then be transfected with siRNA duplexes targeting ZPI mRNA or a negative control siRNA (siRNA-control; sense strand 5'-UUCUCCGAACGUGUCACGUTT-3' (SEQ ID NO:237), antisense strand 5'-ACGUGACACGUUCGGAGAATT-3' (SEQ ID NO:238)) using 10x3-fold serial dilutions over a final duplex concentration range of 20 nM to 1 pM. Transfection may be carried out by adding 9.7 µL Opti-MEM (ThermoFisher) plus 0.3 µL Lipofectamine RNAiMAX (ThermoFisher) to 10 µL of each siRNA duplex. The mixture may be incubated at room temperature for 15 minutes before being added to 100 µL of complete growth medium containing 20,000 Huh7 cells. Cells may be incubated for 24 hours at 37°C/5% CO₂ prior to total RNA purification using a RNeasy 96 Kit (Qiagen). Each duplex may be tested by transfection in duplicate wells in a single experiment.
cDNA synthesis may be performed using FastQuant RT (with gDNase) Kit (Tiangen). Real-time quantitative PCR (qPCR) may be performed on an ABI Prism 7900HT or ABI QuantStudio 7 with primers specific for human ZPI (Hs01547819_m1) and human GAPDH (Hs02786624_g1) using a TaqMan Gene Expression Assay Kit (ThermoFisher Scientific).
qPCR may be performed in duplicate on cDNA derived from each well and the mean cycle threshold (Ct) calculated. Relative ZPI expression may be calculated from mean Ct values using the comparative Ct (ΔΔCt) method, normalised to GAPDH and relative to untreated cells. Maximum percent inhibition of ZPI expression and IC50 values may be calculated using a four parameter (variable slope) model using GraphPad Prism 9.

Alternatively or in addition, inhibition of expression of the ZPI gene may be characterized by a reduction of mean relative expression of the ZPI gene.

In some embodiments, when cells are transfected with 0.1 nM of the nucleic acid of the invention, the mean relative expression of ZPI is below 1, 0.9, 0.8, 0.7, 0.6, 0.5, or 0.4, preferably when determined by qPCR, more preferably by reverse transcriptase (RT)-qPCR, as described herein.

In some embodiments, when cells are transfected with 3 nM of the nucleic acid of the invention, the mean relative expression of ZPI is below 1, 0.9, 0.8, 0.7, 0.6, 0.5, 0.4, 0.3, 0.2 or 0.1, preferably when determined by qPCR, more preferably by reverse transcriptase (RT)-qPCR, as described herein.

Mean relative expression of the ZPI gene may be quantified by the following method:
Huh7 cells (human hepatocyte-derived cell line, obtained from JCRB Cell Bank) may be maintained in Dulbecco's Modified Eagle Medium (DMEM) supplemented with 10% FBS at 37°C in at atmosphere of 5% CO₂. Cells may be transfected with siRNA duplexes targeting ZPI mRNA or a negative control siRNA (siRNA-control; sense strand 5'-UUCUCCGAACGUGUCACGUTT-3' (SEQ ID NO:237), antisense strand 5'-ACGUGACACGUUCGGAGAATT-3'(SEQ ID NO:238)) at a final duplex concentration of 3 nM. Transfection may be carried out by adding 9.7 µL Opti-MEM (ThermoFisher) plus 0.3 µL Lipofectamine RNAiMAX (ThermoFisher) to 10 µL of each siRNA duplex. The mixture may be incubated at room temperature for 15 minutes before being added to 100 µL of complete growth medium containing 20,000 Huh7 cells. Cells may be incubated for 24 hours at 37°C/5% CO₂ prior to total RNA purification using a RNeasy 96 Kit (Qiagen). Each duplex may be tested by transfection in duplicate wells in two independent experiments.
cDNA synthesis may be performed using FastQuant RT (with gDNase) Kit (Tiangen). Real-time quantitative PCR (qPCR) may be performed on an ABI Prism 7900HT or ABI QuantStudio 7 with primers specific for human ZPI (Hs01547819_m1) and human GAPDH (Hs02786624_g1) using FastStart Universal Probe Master Kit (Roche).
qPCR may be performed in duplicate on cDNA derived from each well and the mean Ct calculated. Relative ZPI expression may be calculated from mean Ct values using the comparative Ct (ΔΔCt) method, normalised to GAPDH and relative to untreated cells.

Inhibition of the expression of ZPI gene may be manifested by a reduction of the amount of mRNA of the target ZPI gene in comparison to a suitable control.

In other embodiments, inhibition of the expression of ZPI gene may be assessed in terms of a reduction of a parameter that is functionally linked to gene expression, e.g, protein expression or signaling pathways.

### Methods Of Treating Or Preventing Diseases Associated With ZPI Gene Expression

The present invention also provides methods of using nucleic acid e.g. an siRNA of the invention or a composition containing nucleic acid e.g. an siRNA of the invention to reduce or inhibit ZPI gene expression in a cell or reduce expression or function of a target. The methods include contacting the cell with a nucleic acid e.g. dsiRNA of the invention and maintaining the cell for a time sufficient to obtain degradation of the mRNA transcript of ZPI, thereby inhibiting expression of the ZPI gene in the cell. Reduction in gene expression or function of a target can be assessed by any methods known in the art.

In the methods of the invention the cell may be contacted in vitro or in vivo, i.e., the cell may be within a subject.

A cell suitable for treatment using the methods of the invention may be any cell that expresses a gene of interest associated with a disease related to a disorder of haemostasis, such as haemophilia.

A cell suitable for treatment using the methods of the invention may be any cell that expresses a gene of interest associated with Von Willebrand disease.

A cell suitable for treatment using the methods of the invention may be any cell that expresses a gene of interest associated with Factor X Deficiency.

The in vivo methods of the invention may include administering to a subject a composition containing a nucleic acid of the invention e.g. an siRNA, where the nucleic acid e.g. siRNA includes a nucleoside sequence that is complementary to at least a part of an RNA transcript of ZPI gene of the mammal to be treated.

The present invention further provides methods of treatment of a subject in need thereof. The treatment methods of the invention include administering a nucleic acid such as an siRNA of the invention to a subject, e.g., a subject that would benefit from a reduction or inhibition of the expression of ZPI gene, in a therapeutically effective amount e.g. a nucleic acid such as an siRNA targeting ZPI or a pharmaceutical composition comprising the nucleic acid targeting ZPI. The disease to be treated is a disease related to a disorder of haemostasis, such as haemophilia.

In another preferred embodiment, the disease related to a disorder of haemostasis is Von Willebrand disease.

In an embodiment the disease related to a disorder of haemostasis is Factor X Deficiency.

The nucleic acid according to the invention may be used in the prevention and/or treatment of a disease related to a disorder of haemostasis, such as haemophilia. As used herein, the term "haemophilia" refers to a group of disease states broadly characterized by reduced blood clotting or coagulation. Haemophilia may refer to Type A, Type B, or Type C haemophilia, or to the composite of all three diseases types.

The nucleic acid according to the invention may be used in the prevention and/or treatment of Von Willebrand disease.

The patient to be treated may be a patient that already has a disease related to a disorder of haemostasis or that is at risk of developing a disease related to a disorder of haemostasis. That is, in certain embodiments, the nucleic acid of the present invention may be used in the treatment and/or management of an existing disease related to a disorder of haemostasis. Treatment and/or management of an existing disease related to a disorder of haemostasis with the nucleic acid of the present invention may prevent worsening of the disease related to a disorder of haemostasis and/or reverse the disease related to a disorder of haemostasis. In some instances, treatment of an existing disease related to a disorder of haemostasis with the nucleic acid of the present invention may even cure the disease related to a disorder of haemostasis. In certain embodiments, the nucleic acid of the present invention may be used to prevent manifestation of a disease related to a disorder of haemostasis in a patient that is at risk of developing a disease related to a disorder of haemostasis.

The skilled person is capable of diagnosing whether a patient has a disease related to a disorder of haemostasis or is at risk of developing a disease related to a disorder of haemostasis. For example, diagnosing a disease related to a disorder of haemostasis may involve clotting factor tests as known in the art.

Haemophilia, or hemophilia is a mostly inherited genetic disorder that impairs the body's ability to make blood clots, a process needed to stop bleeding. This results in subjects bleeding for a longer time after an injury, easy bruising, and an increased risk of bleeding inside joints or the brain. Subjects with a mild case of the disease may have symptoms only after an accident or during surgery. Bleeding into a joint, also referred to as haemarthrosis, can result in permanent damage while bleeding in the brain can result in long term headaches, seizures, or a decreased level of consciousness.

There are two main types of haemophilia: haemophilia A, which occurs due to low amounts of clotting factor VIII, and haemophilia B, which occurs due to low levels of clotting factor IX. They are typically inherited from one's parents through an X chromosome carrying a nonfunctional gene. Rarely a new mutation may occur during early development or haemophilia may develop later in life due to antibodies forming against a clotting factor. Other types include haemophilia C, which occurs due to low levels of factor XI, Von Willebrand disease, which occurs due to low levels of a substance called von Willebrand factor, and parahaemophilia, which occurs due to low levels of factor V. Haemophilia A, B, and C prevent the intrinsic pathway from functioning properly; this clotting pathway is necessary when there is damage to the endothelium of a blood vessel. Acquired haemophilia is associated with cancers, autoimmune disorders, and pregnancy. Diagnosis is by testing the blood for its ability to clot and its levels of clotting factors.

In certain embodiments, the nucleic acid of the present invention is suitable for treatment, or for treatment of haemophilia A, B and/or C. In certain embodiments, the nucleic acid of the present invention is suitable for treatment, or for treatment of haemophilia A and/or B. In certain embodiments, the nucleic acid of the present invention is suitable for treatment, or for treatment of acquired haemophilia. In certain embodiments, the nucleic acid of the present invention is suitable for treatment, or for treatment of Willebrand disease. In certain embodiments, the nucleic acid of the present invention is suitable for treatment, or for treatment of parahaemophilia.

Without wishing to being bound by theory, treatment with the nucleic acid of the invention results in a boost of clotting factor levels such that bleeding can be reduced or prevented. Thus, in a preferred embodiment, treatment with the nucleic acid of the invention reduces or prevents bleeding episodes in a subject suffering from haemophilia. In another preferred embodiment, treatment with the nucleic acid of the invention reduces or prevents bleeding into a joint of a subject suffering from haemophilia. In certain embodiments, treatment with the nucleic acid of the invention reduces or prevents bleeding into a muscle or into the brain of a subject suffering from haemophilia.

Alternatively or in addition, treatment of a subject, preferably a subject having a disorder of haemostasis, such as haemophilia, with the nucleic acid of the invention may result in one or more of more of the following:
In certain embodiments, treatment of a subject, preferably a subject having a disorder of haemostasis, such as haemophilia, with the nucleic acid of the invention results in one or more of the following: reduced bone marrow hyperplasia, reduced osteoarthritis, reduced chondrocyte degeneration/necrosis, reduced haemorrhage, reduced haemosiderin deposition, reduced occurrence of haematoma, reduced osteoclastogenic bone resorption, reduced osteolysis, reduced periostitis, reduced sub-chondral bone sclerosis, reduced tendon degeneration, reduced tendonitis, and/or reduced tenosynovitis.

In a particular embodiment, the invention relates to a nucleic acid suitable for use, or for use, in treatment of haemophilia, wherein the treatment of haemophilia is characterized by reduced bleeding and one or more of: reduced bone marrow hyperplasia, reduced osteoarthritis, reduced chondrocyte degeneration/necrosis, reduced haemorrhage, reduced haemosiderin deposition, reduced haematoma, reduced osteoclastogenic bone resorption, reduced osteolysis, reduced periostitis, reduced sub-chondral bone sclerosis, reduced tendon degeneration, reduced tendonitis, and/or reduced tenosynovitis.

Throughout this disclosure, it is specifically contemplated that the disease or disorder (including the disease related to a disorder of haemostasis) can be Von Willebrand disease.

A nucleic acid e.g. siRNA of the invention may be administered as a "free" nucleic acid or "free" siRNA, administered in the absence of a pharmaceutical composition. The naked nucleic acid may be in a suitable buffer solution. The buffer solution may comprise acetate, citrate, prolamine, carbonate, or phosphate, or any combination thereof. In one embodiment, the buffer solution is phosphate buffered saline (PBS). The pH and osmolarity of the buffer solution can be adjusted such that it is suitable for administering to a subject.

Alternatively, a nucleic acid e.g. siRNA of the invention may be administered as a pharmaceutical composition, such as a dsiRNA liposomal formulation.

In one embodiment, the method includes administering a composition featured herein such that expression of ZPI gene is decreased, such as for about 1, 2, 3, 4, 5, 6, 7, 8, 12, 16, 18, 24 hours, 28, 32, or about 36 hours. In one embodiment, expression of ZPI target gene is decreased for an extended duration, e.g., at least about two, three, four days or more, e.g., about one week, two weeks, three weeks, or four weeks or longer, e.g., about 1 month, 2 months, or 3 months. Subjects can be administered a therapeutic amount of nucleic acid e.g. siRNA, such as about 0.01 mg/kg to about 200 mg/kg, so as to prevent and/or treat a disease related to a disorder of haemostasis, such as haemophilia.

The nucleic acid e.g. siRNA can be administered by intravenous infusion over a period of time, on a regular basis. In certain embodiments, after an initial treatment regimen, the treatments can be administered on a less frequent basis. Administration of the siRNA can reduce gene product levels of ZPI target gene , e.g., in a cell or tissue of the patient by at least about 20%, 25%, 30%, 35%, 40%, 45%, 50%, 55%, 60%, 65%, 70%, 75%, 80%, 85%, 90%, or 95%, or below the level of detection of the assay method used. In certain embodiments, administration results in clinical stabilization or preferably clinically relevant reduction of at least one sign or symptom of a ZPI gene-associated disorder.

Alternatively, the nucleic acid e.g. siRNA can be administered subcutaneously, i.e., by subcutaneous injection. One or more injections may be used to deliver the desired daily dose of nucleic acid e.g., siRNA to a subject. The injections may be repeated over a period of time. The administration may be repeated on a regular basis. In certain embodiments, after an initial treatment regimen, the treatments can be administered on a less frequent basis. A repeat-dose regimen may include administration of a therapeutic amount of nucleic acid on a regular basis, such as every other day or to once a year. In certain embodiments, the nucleic acid is administered about once per month to about once per quarter (i.e., about once every three months).

In one aspect the present invention may be applied in the compounds, processes, compositions or uses of the following Sentences numbered 1-101 wherein reference to any Formula in the Sentences 1-101 refers only to those Formulas that are defined within Sentences 1-101. These formulae are reproduced in Figure 5. Specifically, an oligonucleoside moiety as represented by Z in any of the following sentences can comprise a nucleic acid for inhibiting expression of ZPI as defined in any of the claims hereinafter.
1. A compound comprising the following structure: 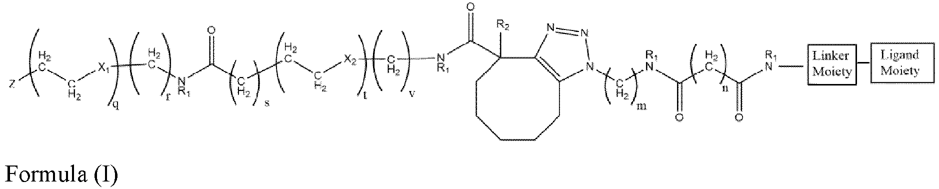 wherein:
   R₁ at each occurrence is independently selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₁ and X₂ at each occurrence are independently selected from the group consisting of methylene, oxygen and sulfur;
   m is an integer of from 1 to 6;
   n is an integer of from 1 to 10;
   q, r, s, t, v are independently integers from 0 to 4, with the proviso that:
      (i) q and r cannot both be 0 at the same time; and
      (ii) s, t and v cannot all be 0 at the same time;
   Z is an oligonucleoside moiety.
2. A compound according to Sentence 1, wherein R₁ is hydrogen at each occurrence.
3. A compound according to Sentence 1, wherein R₁ is methyl.
4. A compound according to Sentence 1, wherein R₁ is ethyl.
5. A compound according to any of Sentences 1 to 4, wherein R₂ is hydroxy.
6. A compound according to any of Sentences 1 to 4, wherein R₂ is halo.
7. A compound according to Sentence 6, wherein R₂ is fluoro.
8. A compound according to Sentence 6, wherein R₂ is chloro.
9. A compound according to Sentence 6, wherein R₂ is bromo.
10. A compound according to Sentence 6, wherein R₂ is iodo.
11. A compound according to Sentence 6, wherein R₂ is nitro.
12. A compound according to any of Sentences 1 to 11, wherein X₁ is methylene.
13. A compound according to any of Sentences 1 to 11, wherein X₁ is oxygen.
14. A compound according to any of Sentences 1 to 11, wherein X₁ is sulfur.
15. A compound according to any of Sentences 1 to 14, wherein X₂ is methylene.
16. A compound according to any of Sentences 1 to 15, wherein X₂ is oxygen.
17. A compound according to any of Sentences 1 to 16, wherein X₂ is sulfur.
18. A compound according to any of Sentences 1 to 17, wherein m = 3.
19. A compound according to any of Sentences 1 to 18, wherein n = 6.
20. A compound according to Sentences 13 and 15, wherein X₁ is oxygen and X₂ is methylene, and preferably wherein:
   q = 1,
   r = 2,
   s = 1,
   t = 1,
   v= 1.
21. A compound according to Sentences 12 and 15, wherein both X₁ and X₂ are methylene, and preferably wherein:
   q = 1,
   r = 3,
   s = 1,
   t = 1,
   v= 1.
22. A compound according to any of Sentences 1 to 21, wherein Z is: 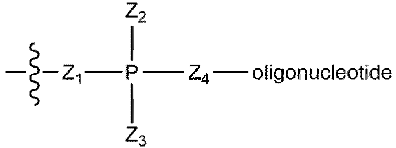 wherein:
   Z₁, Z₂, Z₃, Z₄ are independently at each occurrence oxygen or sulfur; and
   one the bonds between P and Z₂, and P and Z₃ is a single bond and the other bond is a double bond.
23. A compound according to Sentence 22, wherein said oligonucleoside is an RNA compound capable of modulating, preferably inhibiting, expression of a target gene.
24. A compound according to Sentence 23, wherein said RNA compound comprises an RNA duplex comprising first and second strands, wherein the first strand is at least partially complementary to an RNA sequence of a target gene, and the second strand is at least partially complementary to said first strand, and wherein each of the first and second strands have 5' and 3' ends.
25. A compound according to Sentence 24, wherein the RNA compound is attached at the 5' end of its second strand to the adjacent phosphate.
26. A compound according to Sentence 24, wherein the RNA compound is attached at the 3' end of its second strand to the adjacent phosphate.
27. A compound of Formula (II): 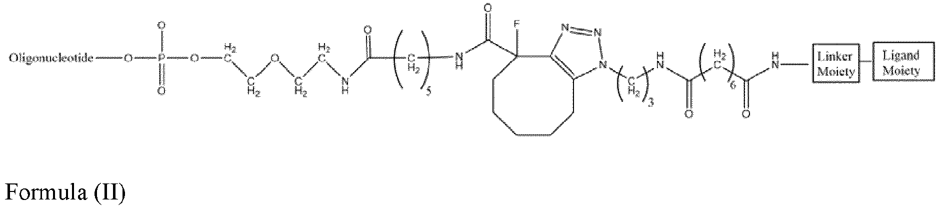
28. A compound of Formula (III): 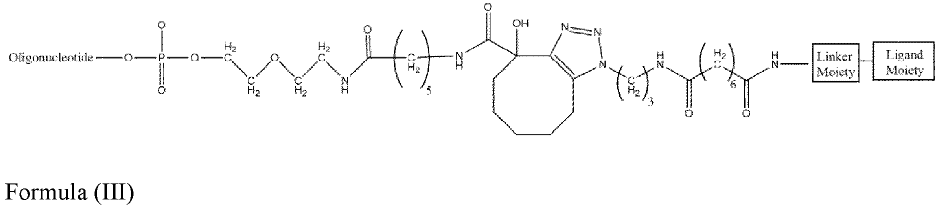
29. A compound according to Sentence 27 or 28, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
30. A composition comprising a compound of Formula (II) as defined in Sentence 27, and a compound of Formula (III) as defined in Sentence 28, optionally dependent on Sentence 29.
31. A composition according to Sentence 30, wherein said compound of Formula (III) as defined in Sentence 28 is present in an amount in the range of 10 to 15% by weight of said composition.
32. A compound of Formula (IV): 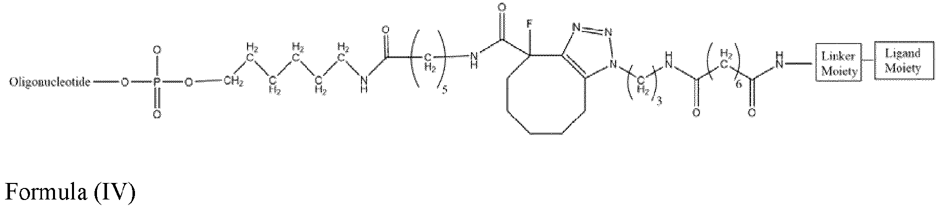
33. A compound of Formula (V): 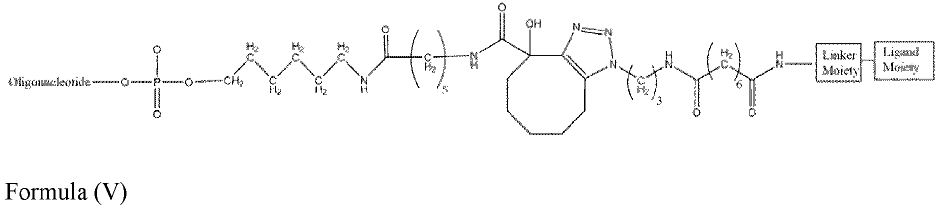
34. A compound according to Sentence 32 or 33, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
35. A composition comprising a compound of Formula (IV) as defined in Sentence 32, and a compound of Formula (V) as defined in Sentence 33, optionally dependent on Sentence 34.
36. A composition according to Sentence 35, wherein said compound of Formula (V) as defined in Sentence 33 is present in an amount in the range of 10 to 15% by weight of said composition.
37. A compound as defined in any of Sentences 1 to 29, or 32 to 34, wherein the oligonucleoside comprises an RNA duplex which further comprises one or more riboses modified at the 2' position, preferably a plurality of riboses modified at the 2' position.
38. A compound according to Sentence 37, wherein the modifications are chosen from 2'-O-methyl, 2'-deoxy-fluoro, and 2'-deoxy.
39. A compound according to any of Sentences 1 to 29, or 32 to 34, or 37 to 38, wherein the oligonucleoside further comprises one or more degradation protective moieties at one or more ends.
40. A compound according to Sentence 39, wherein said one or more degradation protective moieties are not present at the end of the oligonucleoside strand that carries the ligand moieties, and / or wherein said one or more degradation protective moieties is selected from phosphorothioate internucleoside linkages, phosphorodithioate internucleoside linkages and inverted abasic nucleosides, wherein said inverted abasic nucleosides are present at the distal end of the strand that carries the ligand moieties.
41. A compound according to any of Sentences 1 to 29, or 32 to 34, or 37 to 40, wherein said ligand moiety as depicted in Formula (I) in Sentence 1 comprises one or more ligands.
42. A compound according to Sentence 41, wherein said ligand moiety as depicted in Formula (I) in Sentence 1 comprises one or more carbohydrate ligands.
43. A compound according to Sentence 42, wherein said one or more carbohydrates can be a monosaccharide, disaccharide, trisaccharide, tetrasaccharide, oligosaccharide or polysaccharide.
44. A compound according to Sentence 43, wherein said one or more carbohydrates comprise one or more galactose moieties, one or more lactose moieties, one or more N-AcetylGalactosamine moieties, and / or one or more mannose moieties.
45. A compound according to Sentence 44, wherein said one or more carbohydrates comprise one or more N-Acetyl-Galactosamine moieties.
46. A compound according to Sentence 45, which comprises two or three N-AcetylGalactosamine moieties.
47. A compound according to any of Sentences 41 to 46, wherein said one or more ligands are attached in a linear configuration, or in a branched configuration.
48. A compound according to Sentence 47, wherein said one or more ligands are attached as a biantennary or triantennary branched configuration.
49. A compound according to Sentences 46 to 48, wherein said moiety: 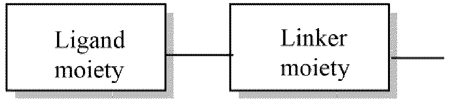 as depicted in Formula (I) in Sentence 1 is any of Formulae (VIa), (VIb) or (VIc), preferably Formula (VIa): 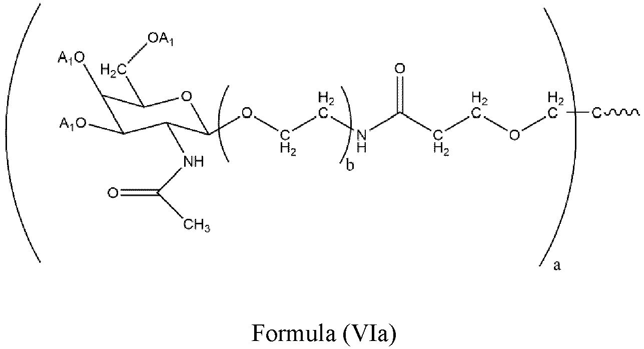 wherein:
   A_{I} is hydrogen, or a suitable hydroxy protecting group;
   a is an integer of 2 or 3; and
   b is an integer of 2 to 5; or 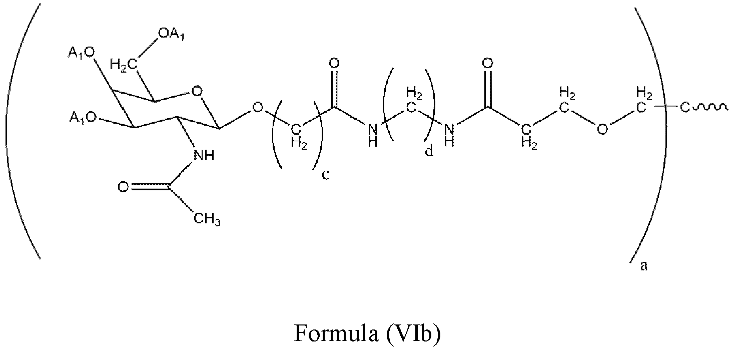 wherein:
      A_{I} is hydrogen, or a suitable hydroxy protecting group;
      a is an integer of 2 or 3; and
      c and d are independently integers of 1 to 6; or 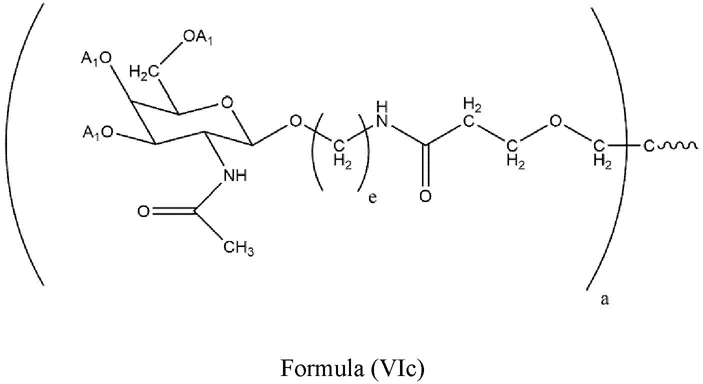 wherein:
         A_{I} is hydrogen, or a suitable hydroxy protecting group;
         a is an integer of 2 or 3; and
         e is an integer of 2 to 10.
50. A compound according to Sentences 46 to 48, wherein said moiety: 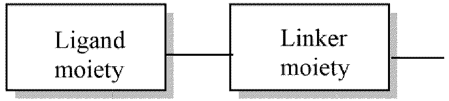 as depicted in Formula (I) in Sentence 1 is Formula (VII): 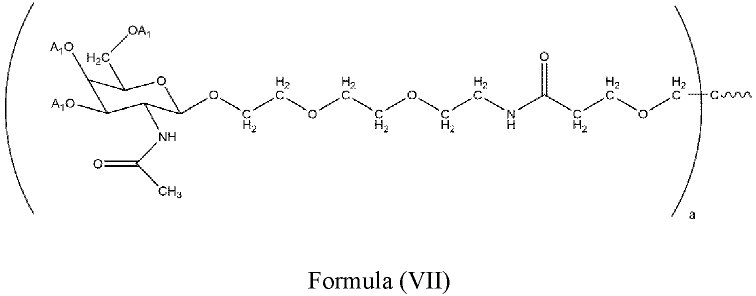 wherein:
   A_{I} is hydrogen;
   a is an integer of 2 or 3.
51. A compound according to Sentence 49 or 50, wherein a = 2.
52. A compound according to Sentence 49 or 50, wherein a = 3.
53. A compound according to Sentence 49, wherein b = 3.
54. A compound of Formula (VIII): 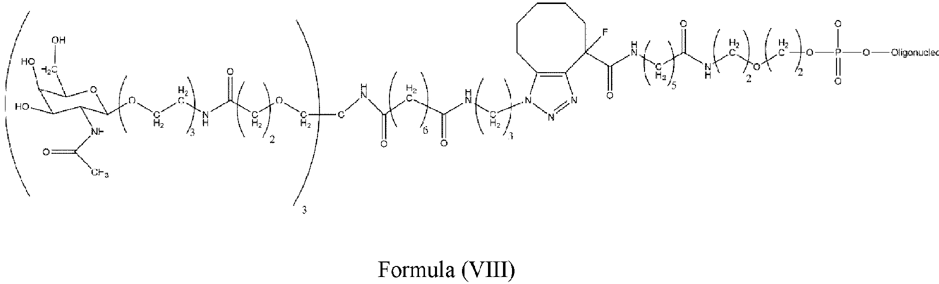
55. A compound of Formula (IX): 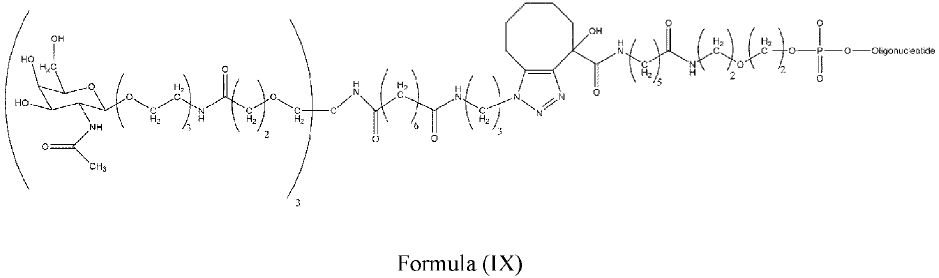
56. A compound according to Sentence 54 or 55, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
57. A composition comprising a compound of Formula (VIII) as defined in Sentence 54, and a compound of Formula (IX) as defined in Sentence 55, optionally dependent on Sentence 56.
58. A composition according to Sentence 57, wherein said compound of Formula (IX) as defined in Sentence 55 is present in an amount in the range of 10 to 15% by weight of said composition.
59. A compound of Formula (X): 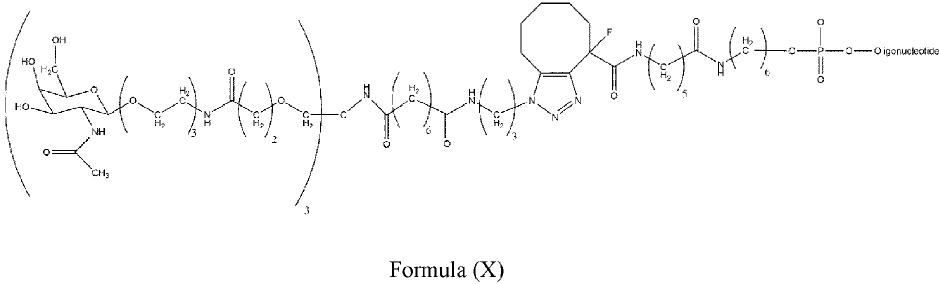
60. A compound of Formula (XI): 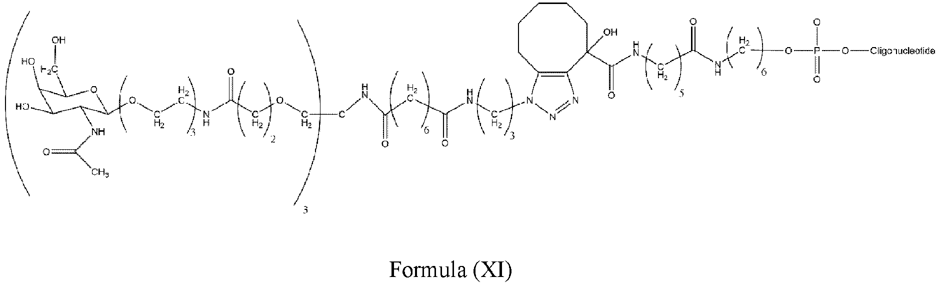
61. A compound according to Sentence 59 or 60, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
62. A composition comprising a compound of Formula (X) as defined in Sentence 59, and a compound of Formula (XI) as defined in Sentence 60, optionally dependent on Sentence 61.
63. A composition according to Sentence 62, wherein said compound of Formula (XI) as defined in Sentence 60 is present in an amount in the range of 10 to 15% by weight of said composition.
64. A compound as defined in any of Sentences 54 to 63, wherein the oligonucleoside comprises an RNA duplex which further comprises one or more riboses modified at the 2' position, preferably a plurality of riboses modified at the 2' position.
65. A compound according to Sentence 64, wherein the modifications are chosen from 2'-O-methyl, 2'-deoxy-fluoro, and 2'-deoxy.
66. A compound according to any of Sentences 54 to 65, wherein the oligonucleoside further comprises one or more degradation protective moieties at one or more ends.
67. A compound according to Sentence 66, wherein said one or more degradation protective moieties are not present at the end of the oligonucleoside strand that carries the ligand moieties, and / or wherein said one or more degradation protective moieties is selected from phosphorothioate internucleoside linkages, phosphorodithioate internucleoside linkages and inverted abasic nucleosides, wherein said inverted abasic nucleosides are present at the distal end of the strand that carries the ligand moieties, as shown in any of Formulae (VIII), (IX), (X) or (XI) in any of Sentences 54, 55, 59 or 60.
68. A process of preparing a compound according to any of Sentences 1 to 29, 32 to 34, 37 to 56, 59 to 61, and 64 to 67, and / or a composition according to any of Sentences 30, 31, 35, 36, 57, 58, 62, 63, which comprises reacting compounds of Formulae (XII) and (XIII): 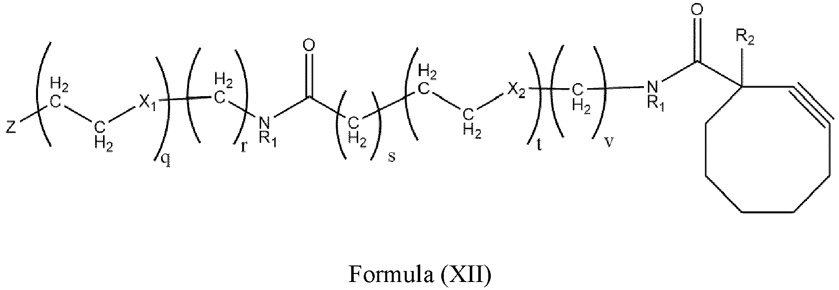 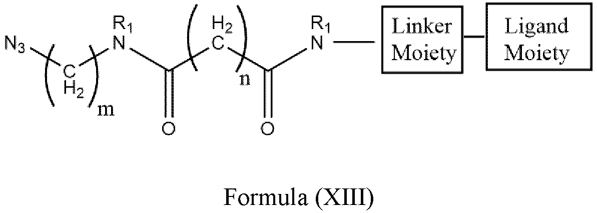 herein:
   R₁ at each occurrence is independently selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₁ and X₂ at each occurrence are independently selected from the group consisting of methylene, oxygen and sulfur;
   m is an integer of from 1 to 6;
   n is an integer of from 1 to 10;
   q, r, s, t, v are independently integers from 0 to 4, with the proviso that:
      (i) q and r cannot both be 0 at the same time; and
      (ii) s, t and v cannot all be 0 at the same time;
   Z is an oligonucleoside moiety;
   and where appropriate carrying out deprotection of the ligand and / or annealing of a second strand for the oligonucleoside moiety.
69. A process according to Sentence 68, wherein a compound of Formula (XII) is prepared by reacting compounds of Formulae (XIV) and (XV): 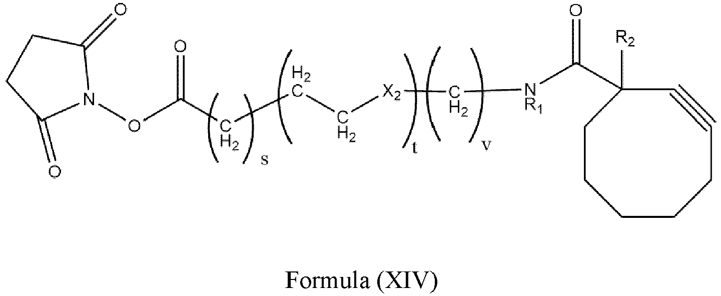 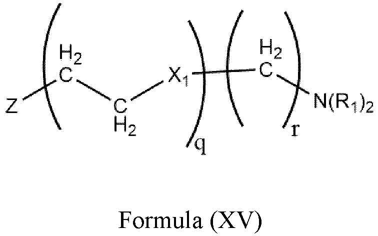
   R₁ at each occurrence is independently selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₁ and X₂ at each occurrence are independently selected from the group consisting of methylene, oxygen and sulfur;
   q, r, s, t, v are independently integers from 0 to 4, with the proviso that:
      (i) q and r cannot both be 0 at the same time; and
      (ii) s, t and v cannot all be 0 at the same time;
   Z is an oligonucleoside moiety.
70. A process according to Sentence 68, to prepare a compound according to any of Sentences 20, 25, 27, 29, 54, 56, and / or a composition according to any of Sentences 30, 31, 57, 58, wherein:
   compound of Formula (XII) is Formula (XIIa): 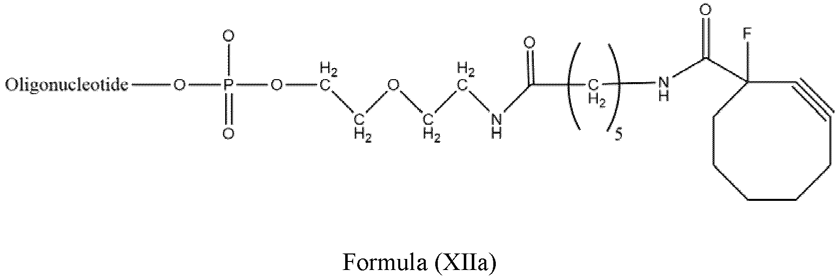 and compound of Formula (XIII) is Formula (XIIIa): 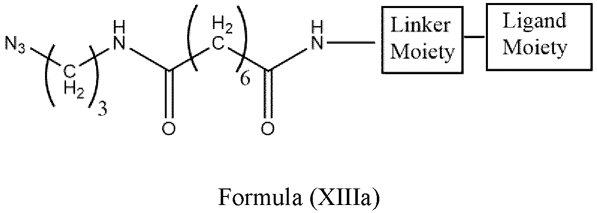 wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
71. A process according to Sentence 68, to prepare a compound according to any of Sentences 20, 25, 28, 29, 55, 56, and / or a composition according to any of Sentences 30, 31, 57, 58, wherein:
   compound of Formula (XII) is Formula (XIIb): 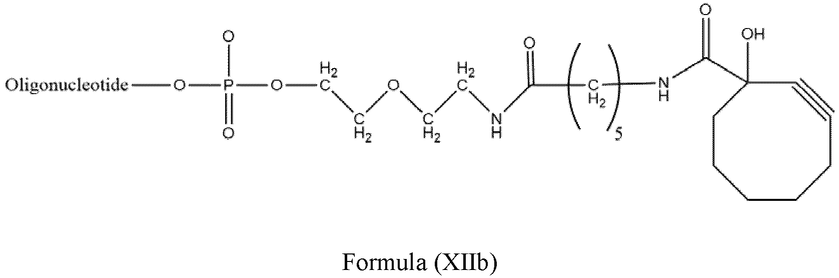 and compound of Formula (XIII) is Formula (XIIIa): 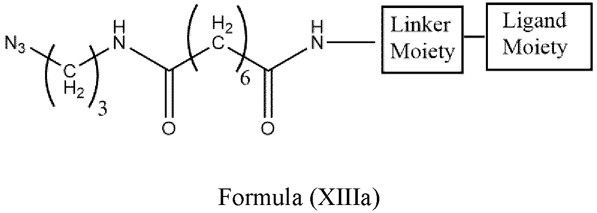 wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
72. A process according to Sentence 68, to prepare a compound according to any of Sentences 21, 26, 32, 34, 59, 61, and / or a composition according to any of Sentences 35, 36, 62, 63, wherein:
   compound of Formula (XII) is Formula (XIIc): 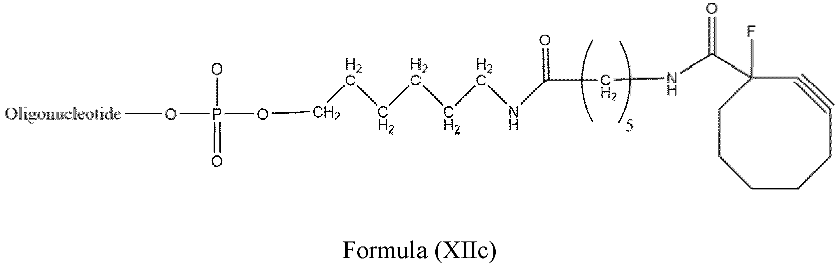 and compound of Formula (XIII) is Formula (XIIIa): 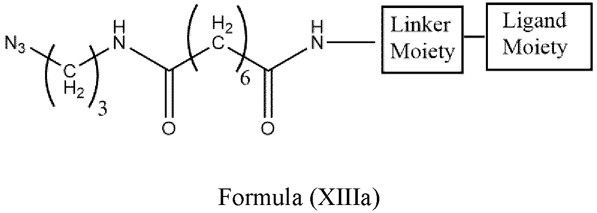 wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
73. A process according to Sentence 68, to prepare a compound according to any of Sentences 21, 26, 33, 34, 60, 61, and / or a composition according to any of Sentences 35, 36, 62, 63, wherein:
   compound of Formula (XII) is Formula (XIId): 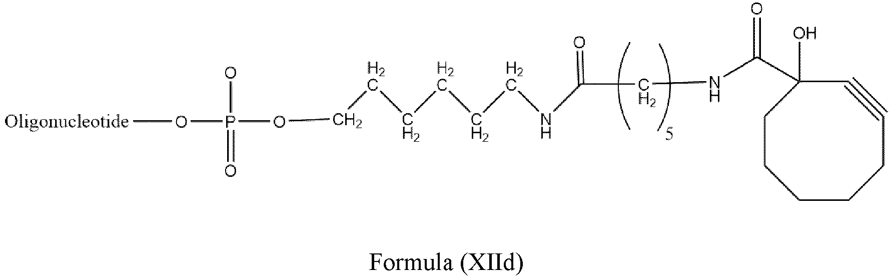 and compound of Formula (XIII) is Formula (XIIIa): 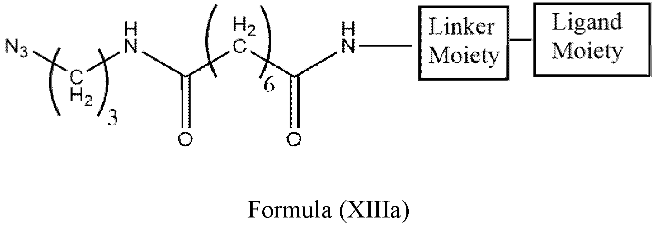 wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
74. A process according to any of Sentences 70 to 73, wherein:
   compound of Formula (XIIIa) is Formula (XIIIb): 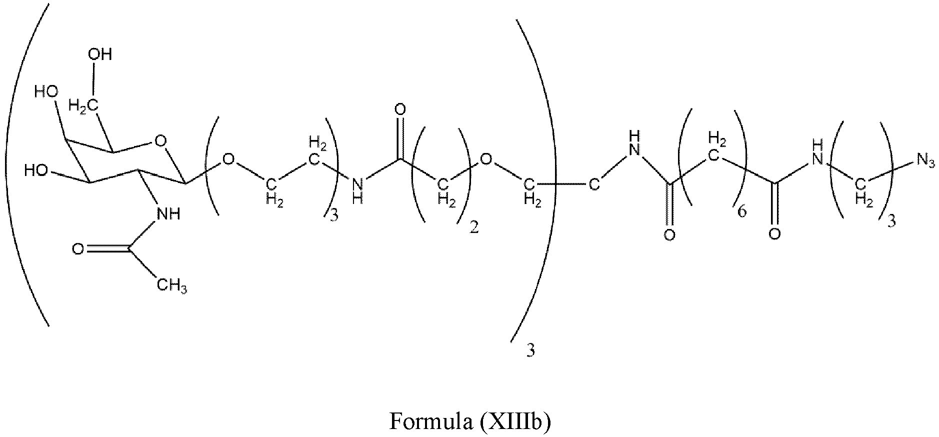
75. A process according to Sentences 69, as dependent on Sentences 70 to 73, wherein:
   compound of Formula (XIV) is either Formula (XIVa) or Formula (XIVb): 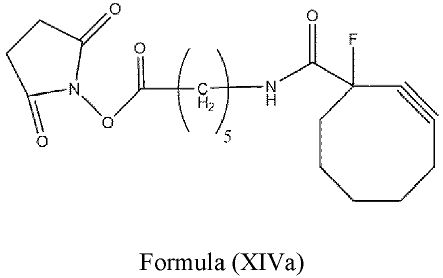 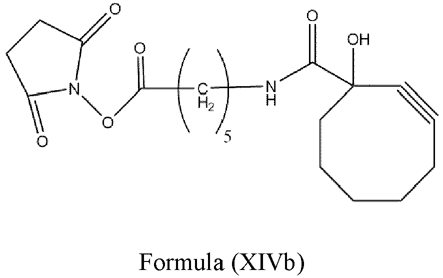 and compound of Formula (XV) is either Formula (XVa) or Formula (XIVb): 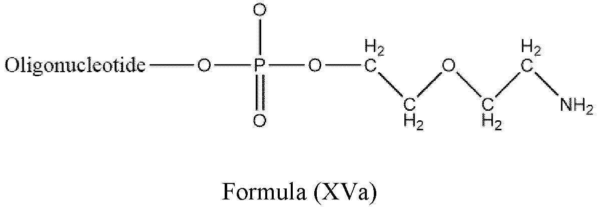 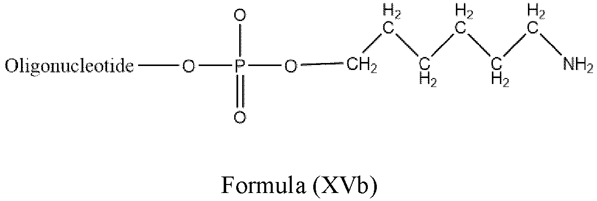 wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein (i) said RNA duplex is attached at the 5' end of its second strand to the adjacent phosphate in Formula (XVa), or (ii) said RNA duplex is attached at the 3' end of its second strand to the adjacent phosphate in Formula (XVb).
76. A compound of Formula (XII): 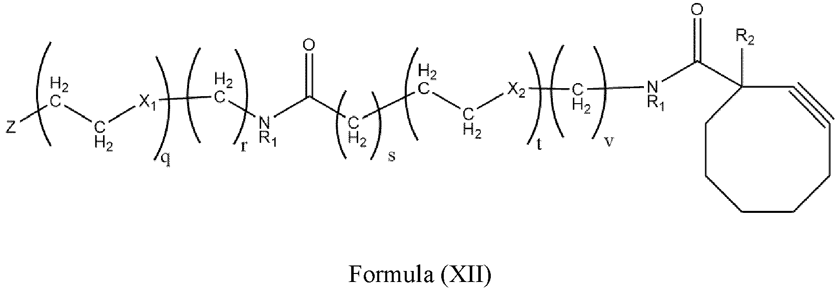 wherein:
   R₁ at each occurrence is independently selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₁ and X₂ at each occurrence are independently selected from the group consisting of methylene, oxygen and sulfur;
   q, r, s, t, v are independently integers from 0 to 4, with the proviso that:
      (i) q and r cannot both be 0 at the same time; and
      (ii) s, t and v cannot all be 0 at the same time;
   Z is an oligonucleoside moiety.
77. A compound of Formula (XIIa): 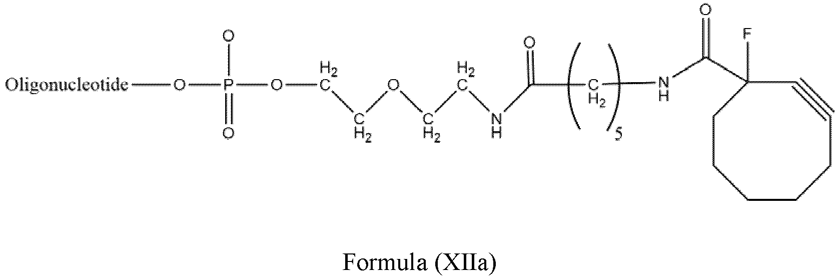
78. A compound of Formula (XIIb): 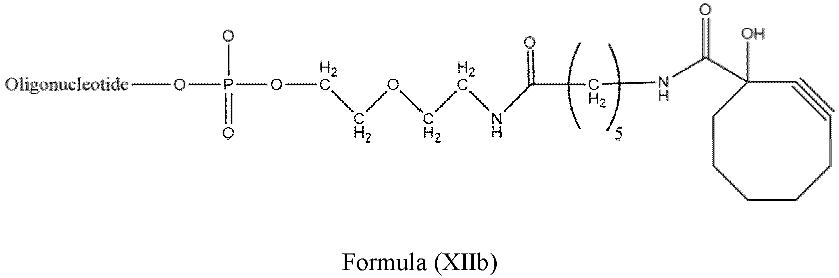
79. A compound of Formula (XIIc): 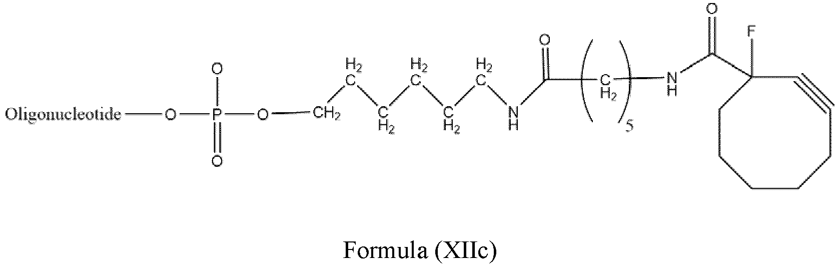
80. A compound of Formula (XIId): 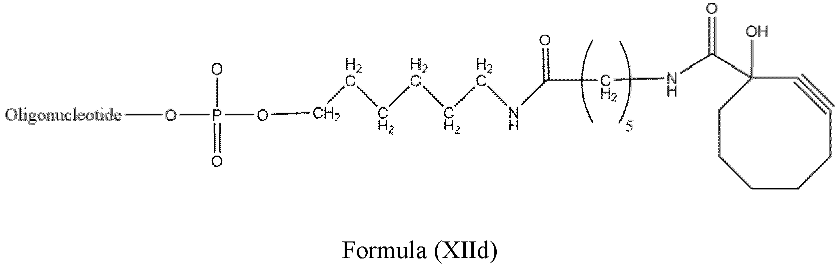
81. A compound of Formula (XIII): 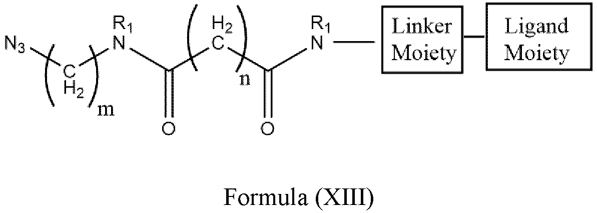 wherein:
   R₁ at each occurrence is independently selected from the group consisting of hydrogen, methyl and ethyl;
   m is an integer of from 1 to 6;
   n is an integer of from 1 to 10.
82. A compound of Formula (XIIIa): 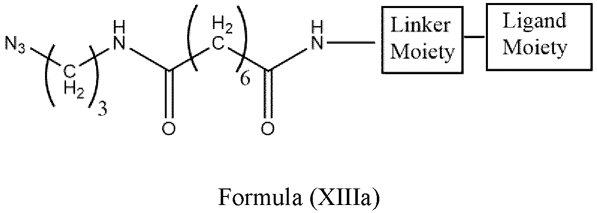
83. A compound of Formula (XIIIb): 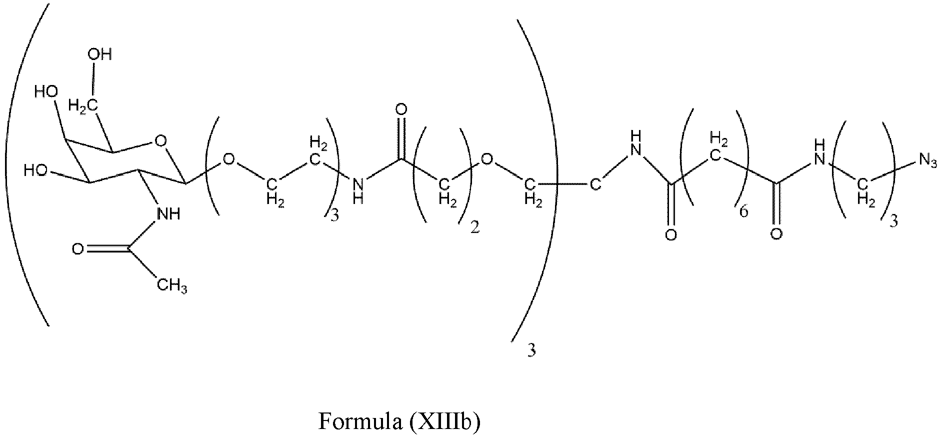
84. A compound of Formula (XIV): 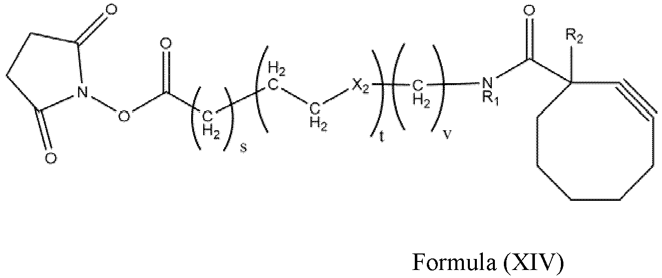 wherein:
   R₁ is selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₂ is selected from the group consisting of methylene, oxygen and sulfur;
   s, t, v are independently integers from 0 to 4, with the proviso that s, t and v cannot all be 0 at the same time.
85. A compound of Formula (XIVa): 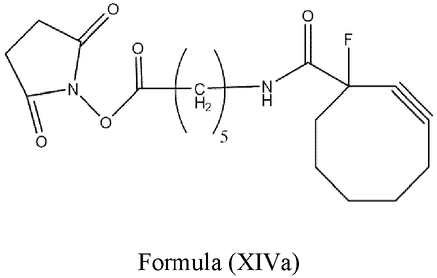
86. A compound of Formula (XIVb): 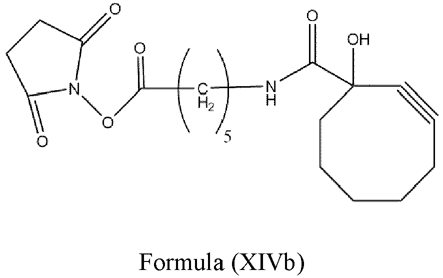
87. A compound of Formula (XV): 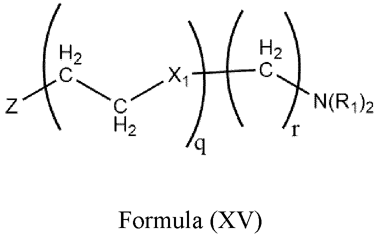 wherein:
   R₁ at each occurrence is independently selected from the group consisting of hydrogen, methyl and ethyl;
   X₁ is selected from the group consisting of methylene, oxygen and sulfur;
   q and r are independently integers from 0 to 4, with the proviso that q and r cannot both be 0 at the same time;
   Z is an oligonucleoside moiety.
88. A compound of Formula (XVa): 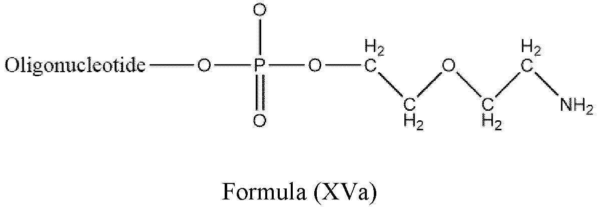
89. A compound of Formula (XVb): 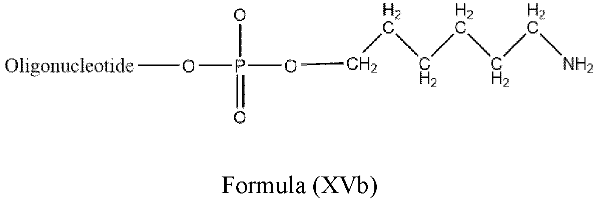
90. Use of a compound according to any of Sentences 76, 81 to 84, 87, for the preparation of a compound according to any of Sentences 1 to 29, 32 to 34, 37 to 56, 59 to 61, and 64 to 67, and / or a composition according to any of Sentences 30, 31, 35, 36, 57, 58, 62 and 63.
91. Use of a compound according to Sentence 85, for the preparation of a compound according to any of Sentences 1 to 29, 32 to 34, 37 to 56, 59 to 61, and 64 to 67, and / or a composition according to any of Sentences 30, 31, 35, 36, 57, 58, 62 and 63, wherein R₂ = F.
92. Use of a compound according to Sentence 86, for the preparation of a compound according to any of Sentences 1 to 29, 32 to 34, 37 to 56, 59 to 61, and 64 to 67, and / or a composition according to any of Sentences 30, 31, 35, 36, 57, 58, 62 and 63, wherein R₂ = OH.
93. Use of a compound according to Sentence 77, for the preparation of a compound according to any of Sentences 20, 25, 27, 29, 54, 56, and / or a composition according to any of Sentences 30, 31, 57, 58.
94. Use of a compound according to Sentence 78, for the preparation of a compound according to any of Sentences 20, 25, 28, 29, 55, 56, and / or a composition according to any of Sentences 30, 31, 57, 58.
95. Use of a compound according to Sentence 79, for the preparation of a compound according to any of Sentences 21, 26, 32, 34, 59, 61, and / or a composition according to any of Sentences 35, 36, 62, 63.
96. Use of a compound according to Sentence 80, for the preparation of a compound according to any of Sentences 21, 26, 33, 34, 60, 61, and / or a composition according to any of Sentences 35, 36, 62, 63.
97. Use of a compound according to Sentence 88, for the preparation of a compound according to any of Sentences 20, 25, 27 to 29, 54 to 56, and / or a composition according to any of Sentences 30, 31, 57, 58.
98. Use of a compound according to Sentence 89, for the preparation of a compound according to any of Sentences 21, 26, 32 to 34, 59 to 61, and / or a composition according to any of Sentences 35, 36, 62, 63.
99. A compound or composition obtained, or obtainable by a process according to any of Sentences 68 to 75.
100. A pharmaceutical composition comprising of a compound according to any of Sentences 1 to 29, 32 to 34, 37 to 56, 59 to 61, and 64 to 67, and / or a composition according to any of Sentences 30, 31, 35, 36, 57, 58, 62 and 63, together with a pharmaceutically acceptable carrier, diluent or excipient.
101. A compound according to any of Sentences 1 to 29, 32 to 34, 37 to 56, 59 to 61, and 64 to 67, and / or a composition according to any of Sentences 30, 31, 35, 36, 57, 58, 62 and 63, for use in therapy.

In another aspect the present invention may be applied in the compounds, processes, compositions or uses of the following Clauses numbered 1-56 wherein reference to any Formula in the Clauses refers only to those Formulas that are defined within Clause 1-56. These formulae are reproduced in Figure 6. Specifically, an oligonucleoside moiety as represented by Z in any of the following clauses can comprise a nucleic acid for inhibiting expression of ZPI as defined in any of the claims hereinafter.
1. A compound comprising the following structure: 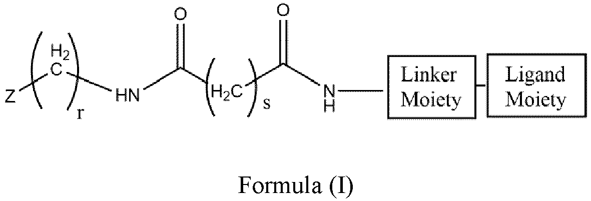 wherein:
   r and s are independently an integer selected from 1 to 16; and
   Z is an oligonucleoside moiety.
2. A compound according to Clause 1, wherein s is an integer selected from 4 to 12.
3. A compound according to Clause 2, wherein s is 6.
4. A compound according to any of Clauses 1 to 3, wherein r is an integer selected from 4 to 14.
5. A compound according to Clause 4, wherein r is 6.
6. A compound according to Clause 4, wherein r is 12.
7. A compound according to Clause 5, which is dependent on Clause 3.
8. A compound according to Clause 6, which is dependent on Clause 3.
9. A compound according to any of Clauses 1 to 8, wherein Z is: 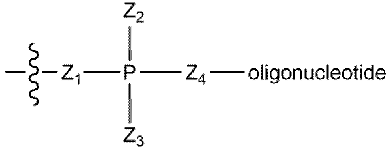 wherein:
   Z₁, Z₂, Z₃, Z₄ are independently at each occurrence oxygen or sulfur; and
   one the bonds between P and Z₂, and P and Z₃ is a single bond and the other bond is a double bond.
10. A compound according to any of Clauses 1 to 9, wherein said oligonucleoside is an RNA compound capable of modulating, preferably inhibiting, expression of a target gene.
11. A compound according to any of Clause 10, wherein said RNA compound comprises an RNA duplex comprising first and second strands, wherein the first strand is at least partially complementary to an RNA sequence of a target gene, and the second strand is at least partially complementary to said first strand, and wherein each of the first and second strands have 5' and 3' ends.
12. A compound according to Clause 11, preferably also dependent on Clauses 3 and 6, wherein the RNA compound is attached at the 5' end of its second strand to the adjacent phosphate.
13. A compound according to Clause 11, preferably also dependent on Clauses 3 and 5, wherein the RNA compound is attached at the 3' end of its second strand to the adjacent phosphate.
14. A compound of Formula (II), preferably dependent on Clause 12: 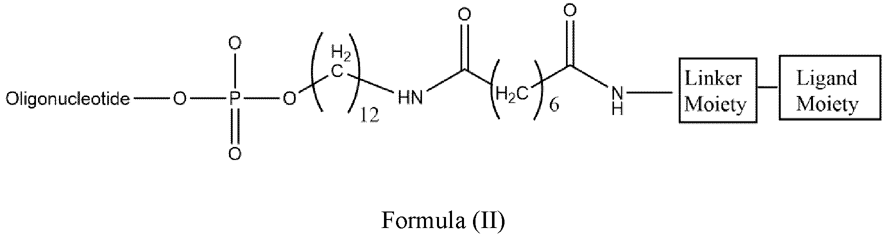
15. A compound of Formula (III), preferably dependent on Clause 13: 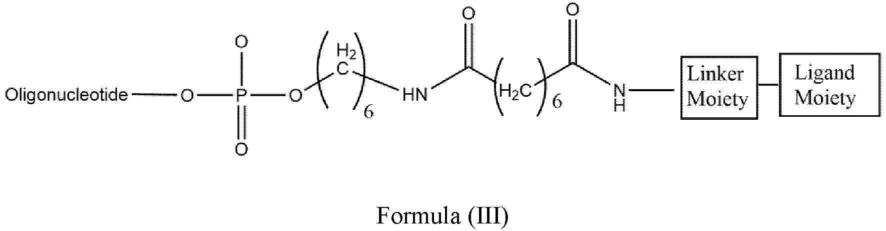
16. A compound as defined in any of Clauses 1 to 15, wherein the oligonucleoside comprises an RNA duplex which further comprises one or more riboses modified at the 2' position, preferably a plurality of riboses modified at the 2' position.
17. A compound according to Clause 16, wherein the modifications are chosen from 2'-O-methyl, 2'-deoxy-fluoro, and 2'-deoxy.
18. A compound according to any of Clauses 1 to 17, wherein the oligonucleoside further comprises one or more degradation protective moieties at one or more ends.
19. A compound according to Clause 18, wherein said one or more degradation protective moieties are not present at the end of the oligonucleoside strand that carries the linker / ligand moieties, and / or wherein said one or more degradation protective moieties is selected from phosphorothioate internucleoside linkages, phosphorodithioate internucleoside linkages and inverted abasic nucleosides, wherein said inverted abasic nucleosides are present at the distal end of the same strand to the end that carries the linker / ligand moieties.
20. A compound according to any of Clauses 1 to 19, wherein said ligand moiety as depicted in Formula (I) in Clause 1 comprises one or more ligands.
21. A compound according to Clause 20, wherein said ligand moiety as depicted in Formula (I) in Clause 1 comprises one or more carbohydrate ligands.
22. A compound according to Clause 21, wherein said one or more carbohydrates can be a monosaccharide, disaccharide, trisaccharide, tetrasaccharide, oligosaccharide or polysaccharide.
23. A compound according to Clause 22, wherein said one or more carbohydrates comprise one or more galactose moieties, one or more lactose moieties, one or more N-AcetylGalactosamine moieties, and / or one or more mannose moieties.
24. A compound according to Clause 23, wherein said one or more carbohydrates comprise one or more N-Acetyl-Galactosamine moieties.
25. A compound according to Clause 24, which comprises two or three N-AcetylGalactosamine moieties.
26. A compound according to any of the preceding Clauses, wherein said one or more ligands are attached in a linear configuration, or in a branched configuration.
27. A compound according to Clause 26, wherein said one or more ligands are attached as a biantennary or triantennary branched configuration.
28. A compound according to Clauses 20 to 27, wherein said moiety: 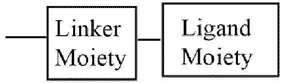 as depicted in Formula (I) in Clause 1 is any of Formulae (IV), (V) or (VI), preferably Formula (IV): 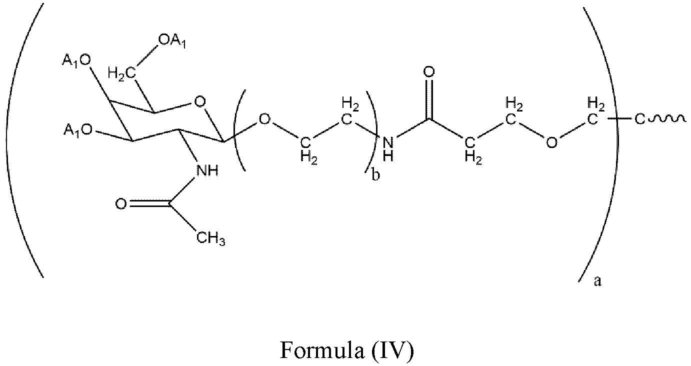 wherein:
   A_{I} is hydrogen, or a suitable hydroxy protecting group;
   a is an integer of 2 or 3; and
   b is an integer of 2 to 5; or 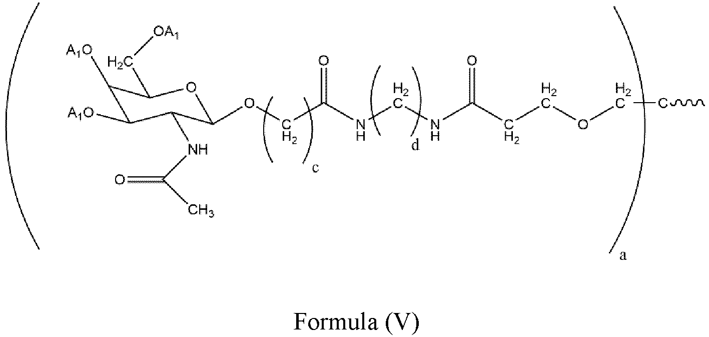 wherein:
      A_{I} is hydrogen, or a suitable hydroxy protecting group;
      a is an integer of 2 or 3; and
      c and d are independently integers of 1 to 6; or 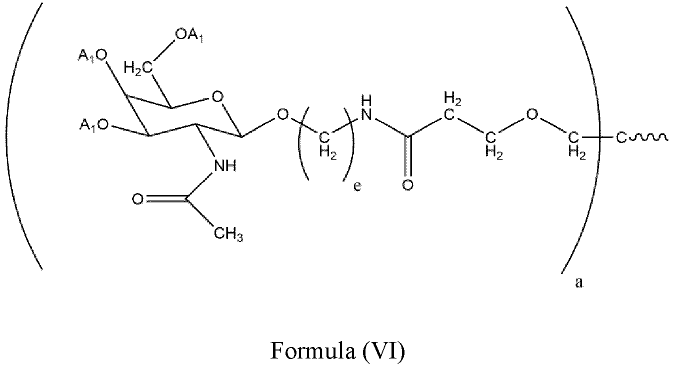 wherein:
         A_{I} is hydrogen, or a suitable hydroxy protecting group;
         a is an integer of 2 or 3; and
         e is an integer of 2 to 10.
29. A compound according to any of Clauses 1 to 28, wherein said moiety: 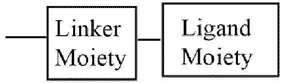 as depicted in Formula (I) in Clause 1 is Formula (VII): 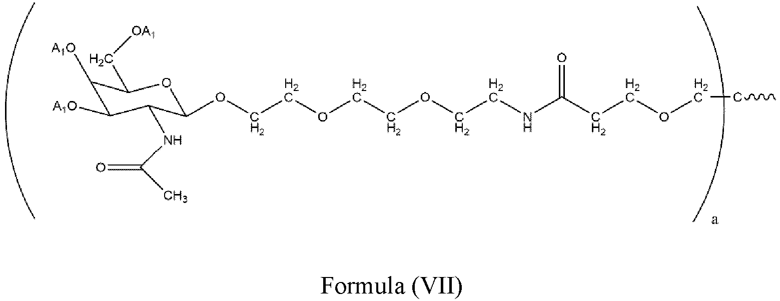 wherein:
   A_{I} is hydrogen;
   a is an integer of 2 or 3.
30. A compound according to Clause 28 or 29, wherein a = 2.
31. A compound according to Clause 28 or 29, wherein a = 3.
32. A compound according to Clause 28, wherein b = 3.
33. A compound of Formula (VIII): 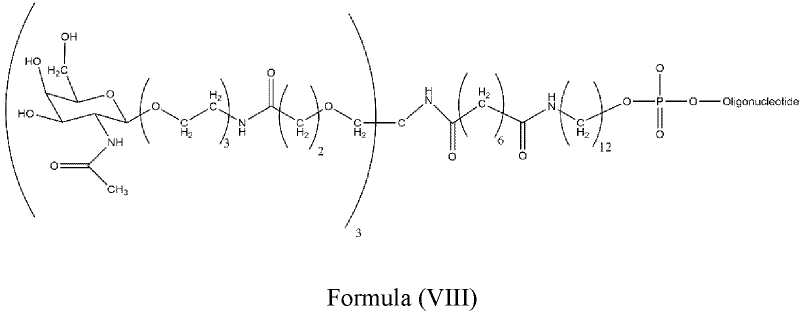
34. A compound of Formula (IX): 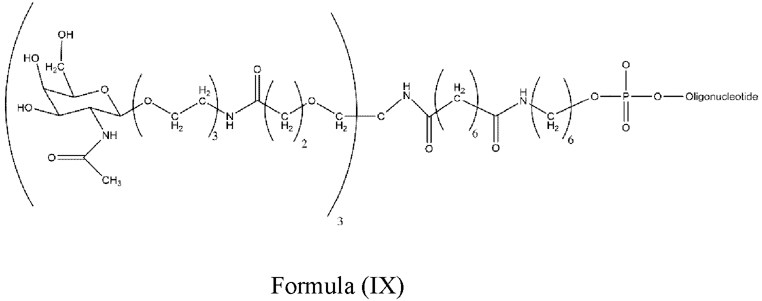
35. A compound according to Clause 33 or 34, wherein the oligonucleoside comprises an RNA duplex which further comprises one or more riboses modified at the 2' position, preferably a plurality of riboses modified at the 2' position.
36. A compound according to Clause 35, wherein the modifications are chosen from 2'-O-methyl, 2'-deoxy-fluoro, and 2'-deoxy.
37. A compound according to any of Clauses 33 to 36, wherein the oligonucleoside further comprises one or more degradation protective moieties at one or more ends.
38. A compound according to Clause 37, wherein said one or more degradation protective moieties are not present at the end of the oligonucleoside strand that carries the linker / ligand moieties, and / or wherein said one or more degradation protective moieties is selected from phosphorothioate internucleoside linkages, phosphorodithioate internucleoside linkages and inverted abasic nucleosides, wherein said inverted abasic nucleosides are present at the distal end of the same strand to the end that carries the linker / ligand moieties.
39. A compound according to Clause 33, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
40. A compound according to Clause 34, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
41. A process of preparing a compound according to any of Clauses 1 to 40, which comprises reacting compounds of Formulae (X) and (XI): 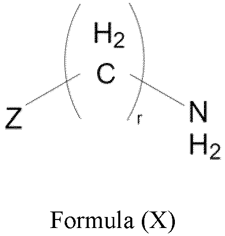 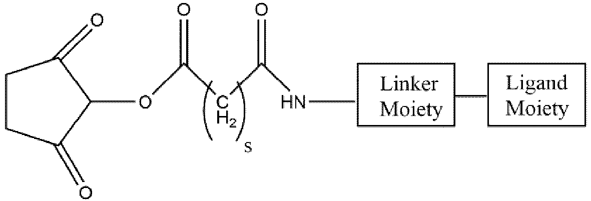 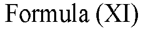 wherein:
   r and s are independently an integer selected from 1 to 16; and
   Z is an oligonucleoside moiety;
   and where appropriate carrying out deprotection of the ligand and / or annealing of a second strand for the oligonucleoside.
42. A process according to Clause 41, to prepare a compound according to any of Clauses 6, 8 to 14, 16 to 33, and 35 to 40, wherein: compound of Formula (X) is Formula (Xa): 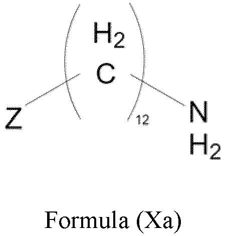 and compound of Formula (XI) is Formula (XIa): 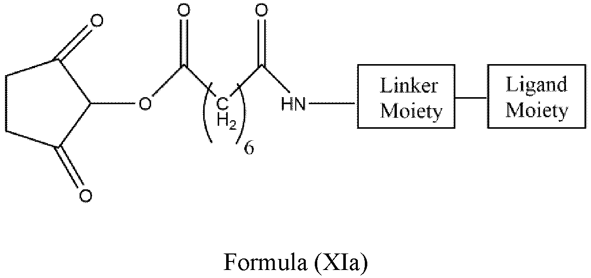 wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
43. A process according to Clause 41, to prepare a compound according to any of Clauses 5, 7, 9 to 13, 15 to 32, and 34 to 40, wherein:
   compound of Formula (X) is Formula (Xb): 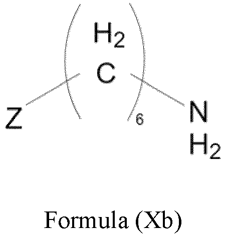 and compound of Formula (XI) is Formula (XIa): 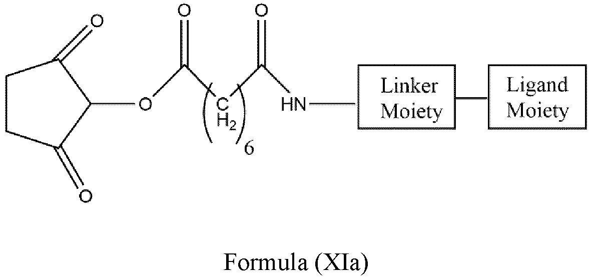 wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
44. A process according to Clauses 42 or 43, wherein:
   compound of Formula (XIa) is Formula (XIb): 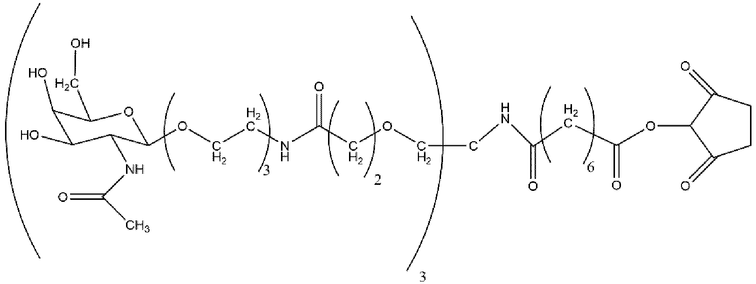 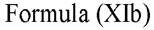
45. A compound of Formula (X): 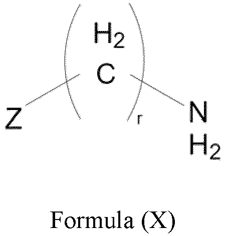 wherein:
   r is independently an integer selected from 1 to 16; and
   Z is an oligonucleoside moiety.
46. A compound of Formula (Xa): 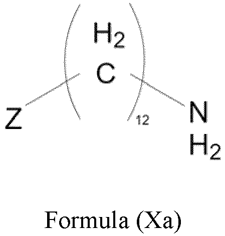
47. A compound of Formula (Xb): 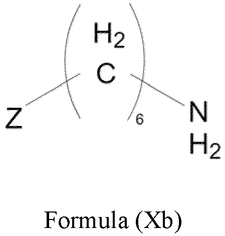
48. A compound of Formula (XI): 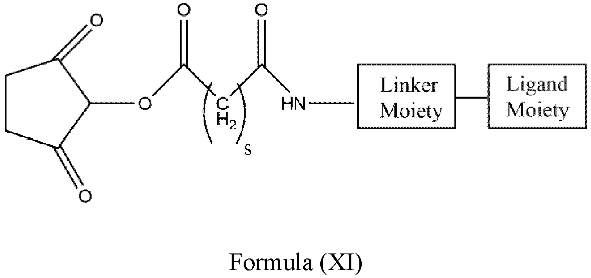 wherein:
   s is independently an integer selected from 1 to 16; and
   Z is an oligonucleoside moiety.
49. A compound of Formula (XIa): 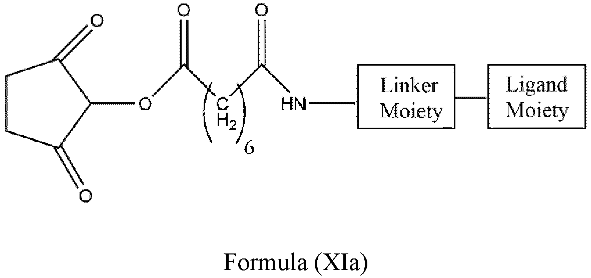
50. A compound of Formula (XIb): 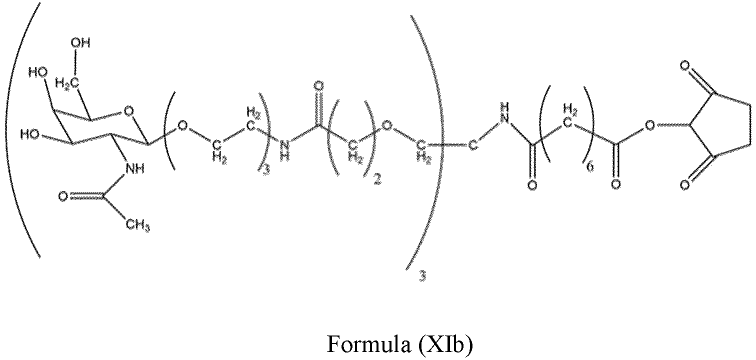
51. Use of a compound according to any of Clauses 45 and 48 to 50, for the preparation of a compound according to any of Clauses 1 to 40.
52. Use of a compound according to Clause 46, for the preparation of a compound according to any of Clauses 6, 8 to 14, 16 to 33, and 35 to 40.
53. Use of a compound according to Clause 47, for the preparation of a compound according to any of Clauses 5, 7, 9 to 13, 15 to 32, and 34 to 40.
54. A compound or composition obtained, or obtainable by a process according to any of Clauses 41 to 44.
55. A pharmaceutical composition comprising of a compound according to any of Clauses 1 to 40, together with a pharmaceutically acceptable carrier, diluent or excipient.
56. A compound according to any of Clauses 1 to 40, for use in therapy.

### EXAMPLES

The invention will be more fully understood by reference to the following examples. They should not, however, be construed as limiting the scope of the invention. It is understood that the examples and embodiments described herein are for illustrative purposes only and that various modifications or changes in light thereof will be suggested to persons skilled in the art and are to be included within the spirit and purview of this application and scope of the appended Clauses.

### Example 1: Synthesis of tether 1

### General Experimental conditions:

Thin layer chromatography (TLC) was performed on silica-coated aluminium plates with fluorescence indicator 254 nm from Macherey-Nagel. Compounds were visualized under UV light (254 nm), or after spraying with the 5% H₂SO₄ in methanol (MeOH) or ninhydrin reagent according to Stahl (from Sigma-Aldrich), followed by heating. Flash chromatography was performed with a Biotage Isolera One flash chromatography instrument equipped with a dual variable UV wavelength detector (200-400 nm) using Biotage Star Silica 10, 25, 50 or 100 g columns (Uppsala, Sweden).

All moisture-sensitive reactions were carried out under anhydrous conditions using dry glassware, anhydrous solvents, and argon atmosphere. All commercially available reagents were purchased from Sigma-Aldrich and solvents from Carl Roth GmbH + Co. KG. D-Galactosamine pentaacetate was purchased from AK scientific.

HPLC/ESI-MS was performed on a Dionex UltiMate 3000 RS UHPLC system and Thermo Scientific MSQ Plus Mass spectrometer using an Acquity UPLC Protein BEH C4 column from Waters (300Å, 1.7 µm, 2.1 × 100 mm) at 60 °C. The solvent system consisted of solvent A with H₂O containing 0.1% formic acid and solvent B with acetonitrile (ACN) containing 0.1% formic acid. A gradient from 5-100% of B over 15 min with a flow rate of 0.4 mL/min was employed. Detector and conditions: Corona ultra-charged aerosol detection (from esa). Nebulizer Temp.: 25 °C. N₂ pressure: 35.1 psi. Filter: Corona.

¹H and ¹³C NMR spectra were recorded at room temperature on a Varian spectrometer at 500 MHz (¹H NMR) and 125 MHz (¹³C NMR). Chemical shifts are given in ppm referenced to the solvent residual peak (CDCl₃ - ¹H NMR: δ at 7.26 ppm and ¹³C NMR δ at 77.2 ppm; DMSO-*d*₆ - 1H NMR: δ at 2.50 ppm and ¹³C NMR δ at 39.5 ppm). Coupling constants are given in Hertz. Signal splitting patterns are described as singlet (s), doublet (d), triplet (t) or multiplet (m).

### Synthesis route for the conjugate building block TriGalNAc _Tether1:

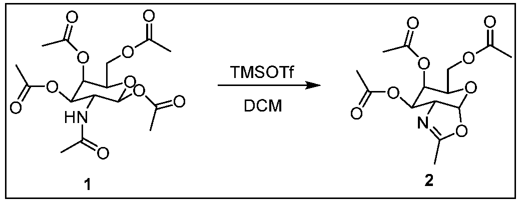

Preparation of compound 2: D-Galactosamine pentaacetate (3.00 g, 7.71 mmol, 1.0 eq.) was dissolved in anhydrous dichloromethane (DCM) (30 mL) under argon and trimethylsilyl trifluoromethanesulfonate (TMSOTf, 4.28 g, 19.27 mmol, 2.5 eq.) was added. The reaction was stirred at room temperature for 3 h. The reaction mixture was diluted with DCM (50 mL) and washed with cold saturated aq. NaHCO3 (100 mL) and water (100 mL). The organic layer was separated, dried over Na₂SO₄ and concentrated to afford the title compound as yellow oil, which was purified by flash chromatography (gradient elution: 0-10% MeOH in DCM in 10 CV). The product was obtained as colourless oil (2.5 g, 98%, rf= 0.45 (2% MeOH in DCM)).

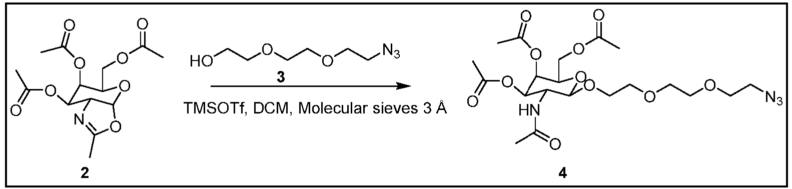

Preparation of compound **4:** Compound **2** (2.30 g, 6.98 mmol, 1.0 eq.) and azido-PEG3-OH (1.83 g, 10.5 mmol, 1.5 eq.) were dissolved in anhydrous DCM (40 mL) under argon and molecular sieves 3 Å (5 g) were added to the solution. The mixture was stirred at room temperature for 1 h. TMSOTf (0.77 g, 3.49 mmol, 0.5 eq.) was then added to the mixture and the reaction was stirred overnight. The molecular sieves were filtered, the filtrate was diluted with DCM (100 mL) and washed with cold saturated aq. NaHCO₃ (100 mL) and water (100 mL). The organic layer was separated, dried over Na₂SO₄ and the solvent was removed under reduced pressure. The crude material was purified by flash chromatography (gradient elution: 0-3% MeOH in DCM in 10 CV) to afford the title product as light yellow oil (3.10 g, 88%, rf = 0.25 (2% MeOH in DCM)). MS: calculated for C₂₀H₃₂N₄O₁₁, 504.21. Found 505.4. ¹H NMR (500 MHz, CDCl₃) δ 6.21-6.14 (m, 1H), 5.30 (dd, *J =* 3.4, 1.1 Hz, 1H), 5.04 (dd, *J =* 11.2, 3.4 Hz,1H), 4.76 (d, *J =* 8.6 Hz, 1H), 4.23-4.08 (m, 3H), 3.91-3.80 (m, 3H), 3.74-3.59 (m, 9H), 3.49-3.41 (m, 2H), 2.14 (s, 3H), 2.02 (s, 3H), 1.97 (d, *J =* 4.2 Hz, 6H). ¹³C NMR (125 MHz, CDCl₃) δ 170.6 (C), 170.5 (C), 170.4 (C), 170.3 (C), 102.1 (CH), 71.6 (CH), 70.8 (CH), 70.6 (CH), 70.5 (CH), 70.3 (CH₂), 69.7 (CH₂), 68.5 (CH₂), 66.6 (CH₂), 61.5 (CH₂), 23.1 (CH₃), 20.7 (3×CH₃).

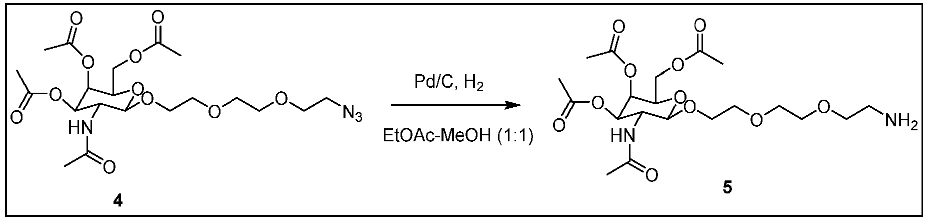

Preparation of compound 5: Compound 4 (1.00 g, 1.98 mmol, 1.0 eq.) was dissolved in a mixture of ethyl acetate (EtOAc) and MeOH (30 mL 1: 1 v/v) and Pd/C (100 mg) was added. The reaction mixture was degassed using vacuum/argon cycles (3x) and hydrogenated under balloon pressure overnight. The reaction mixture was filtered through celite and washed with EtOAc (30 mL). The solvent was removed under reduced pressure to afford the title compound as colourless oil (0.95 g, quantitative yield, rf = 0.25 (10% MeOH in DCM)). The compound was used without further purification. MS: calculated for C₂₀H₃₄N₂O₁₁, 478.2. Found 479.4.

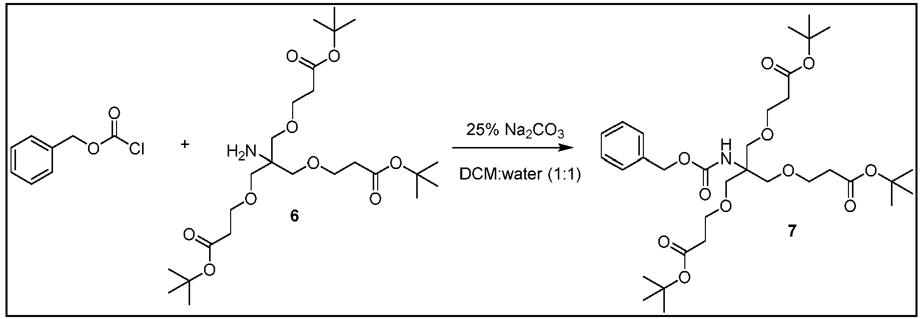

Preparation of compound 7: Tris{[2-(tert-butoxycarbonyl)ethoxy]methyl}-methylamine 6 (3.37 g, 6.67 mmol, 1.0 eq.) was dissolved in a mixture of DCM/water (40 mL 1:1 v/v) and Na₂CO₃ (0.18 g, 1.7 mmol, 0.25 eq.) was added while stirring vigorously. Benzyl chloroformate (2.94 mL, 20.7 mmol, 3.10 eq.) was added dropwise to the previous mixture and the reaction was stirred at room temperature for 24 h. The reaction mixture was diluted with CH₂Cl₂ (100 mL) and washed with water (100 mL). The organic layer was separated and dried over Na₂SO₄. The solvent was removed under reduced pressure and the resulting crude material was purified by flash chromatography (gradient elution: 0-10% EtOAc in cyclohexane in 12 CV) to afford the title compound as pale yellowish oil (3.9 g, 91%, rf = 0.56 (10% EtOAc in cyclohexane)). MS: calculated for C₃₃H₅₃NO₁₁, 639.3. Found 640.9. ¹H NMR (500 MHz, DMSO-*d*₆) δ 7.38-7.26 (m, 5H), 4.97 (s, 2H), 3.54 (t, 6H), 3.50 (s, 6H), 2.38 (t, 6H), 1.39 (s, 27H). ¹³C NMR (125 MHz, DMSO-*d*₆) δ 170.3 (3xC), 154.5 (C), 137.1 (C), 128.2 (2xCH), 127.7 (CH), 127.6 (2xCH), 79.7 (3xC), 68.4 (3×CH₂), 66.8 (3×CH₂), 64.9 (C), 58.7 (CH₂), 35.8 (3×CH₂), 27.7 (9×CH₃).

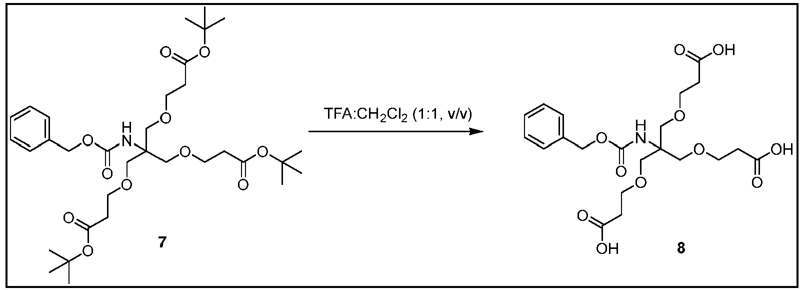

Preparation of compound **8:** Cbz-NH-tris-Boc-ester 7 (0.20 g, 0.39 mmol, 1.0 eq.) was dissolved in CH₂Cl₂ (1 mL) under argon, trifluoroacetic acid (TFA, 1 mL) was added and the reaction was stirred at room temperature for 1 h. The solvent was removed under reduced pressure, the residue was co-evaporated 3 times with toluene (5 mL) and dried under high vacuum to get the compound as its TFA salt (0.183 g, 98%). The compound was used without further purification. MS: calculated for C₂₁H₂₉NO₁₁, 471.6. Found 472.4.

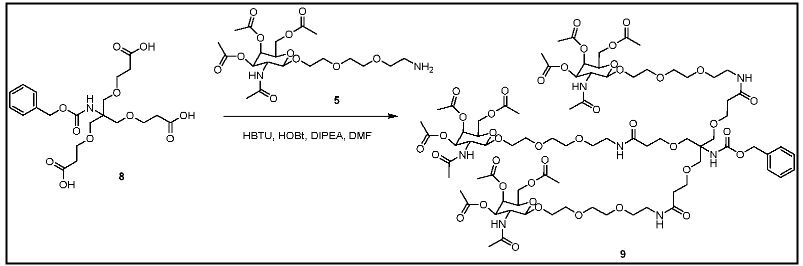

Preparation of compound **9:** CbzNH-tris-COOH **8** (0.72 g, 1.49 mmol, 1.0 eq.) and GalNAc-PEG3-NH₂ **5** (3.56 g, 7.44 mmol, 5.0 eq.) were dissolved in *N,N*-dimethylformamide (DMF) (25 mL). Then *N,N,N',N*'-tetramethyl-O-(1H-benzotriazol-1-yl)uronium hexafluorophosphate (HBTU) (2.78 g, 7.44 mmol, 5.0 eq.), 1-hydroxybenzotriazole hydrate (HOBt) (1.05 g, 7.44 mmol, 5.0 eq.) and *N,N*-diisopropylethylamine (DIPEA) (2.07 mL, 11.9 mmol, 8.0 eq.) were added to the solution and the reaction was stirred for 72 h. The solvent was removed under reduced pressure, the residue was dissolved in DCM (100 mL) and washed with saturated aq. NaHCO₃ (100 mL). The organic layer was dried over Na₂SO₄, the solvent evaporated and the crude material was purified by flash chromatography (gradient elution: 0-5% MeOH in DCM in 14 CV). The product was obtained as pale yellowish oil (1.2 g, 43%, rf = 0.20 (5% MeOH in DCM)). MS: calculated for C₈₁H₁₂₅N₇O₄₁, 1852.9. Found 1854.7. ¹H NMR (500 MHz, DMSO-*d*₆) δ 7.90-7.80 (m, 10H), 7.65-7.62 (m, 4H), 7.47-7.43 (m, 3H), 7.38-7.32 (m, 8H), 5.24-5.22 (m, 3H), 5.02-4.97 (m, 4H), 4.60-4.57 (m, 3 H), 4.07-3.90 (m 10H), 3.67-3.36 (m, 70H), 3.23-3.07 (m, 25H), 2.18 (s, 10H), 2.00 (s, 13H), 1.89 (s, 11H), 1.80-1.78 (m, 17H). ¹³C NMR (125 MHz, DMSO-*d*₆) δ 170.1 (C), 169.8 (C), 169.7 (C), 169.4 (C), 169.2 (C), 169.1 (C), 142.7 (C), 126.3 (CH), 123.9 (CH), 118.7 (CH), 109.7 (CH), 100.8 (CH), 70.5 (CH), 69.8 (CH), 69.6 (CH), 69.5 (CH), 69.3 (CH₂), 69.0 (CH₂), 68.2 (CH₂), 67.2 (CH₂), 66.7 (CH₂), 61.4 (CH₂), 22.6 (CH₂), 22.4 (3×CH₃), 20.7 (9×CH₃).

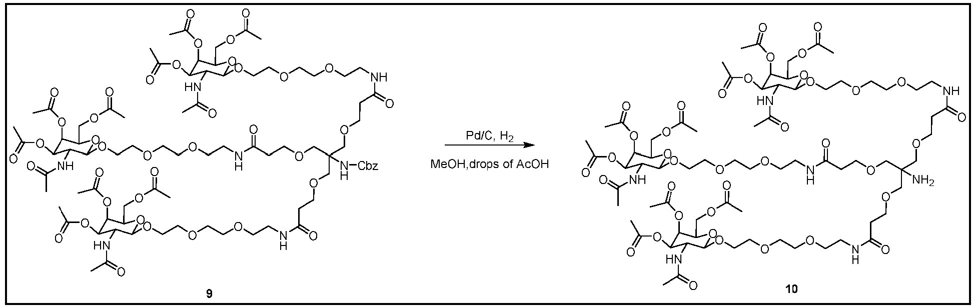

Preparation of compound **10:** Triantennary GalNAc compound **9** (0.27 g, 0.14 mmol, 1.0 eq.) was dissolved in MeOH (15 mL), 3 drops of acetic acid (AcOH) and Pd/C (30 mg) was added. The reaction mixture was degassed using vacuum/argon cycles (3x) and hydrogenated under balloon pressure overnight. The completion of the reaction was followed by mass spectrometry and the resulting mixture was filtered through a thin pad of celite. The solvent was evaporated and the residue obtained was dried under high vacuum and used for the next step without further purification. The product was obtained as pale yellowish oil (0.24 g, quantitative yield). MS: calculated for C₇₃H₁₁₉N₇O₃₉, 1718.8. Found 1719.3.

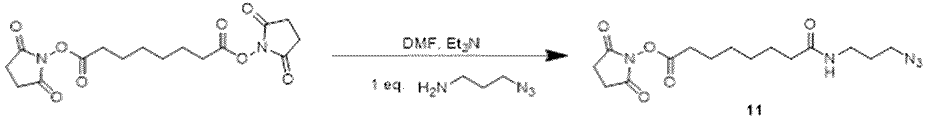

Preparation of compound **11:** Commercially available suberic acid bis(N-hydroxysuccinimide ester) (3.67 g, 9.9 mmol, 1.0 eq.) was dissolved in DMF (5 mL) and triethylamine (1.2 mL) was added. To this solution was added dropwise a solution of 3-azido-1-propylamine (1.0 g, 9.9 mmol, 1.0 eq.) in DMF (5 mL). The reaction was stirred at room temperature for 3 h. The reaction mixture was diluted with EtOAc (100 mL) and washed with water (50 mL). The organic layer was separated, dried over Na₂SO₄ and the solvent was removed under reduced pressure. The crude material was purified by flash chromatography (gradient elution: 0-5% MeOH in DCM in 16 CV). The product was obtained as white solid (1.54 g, 43%, rf = 0.71 (5% MeOH in DCM)). MS: calculated for C₁₅H₂₃N₅O₅, 353.4. Found 354.3.

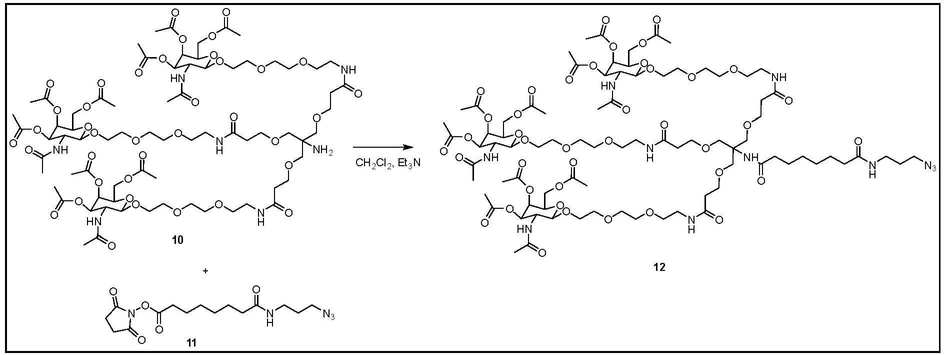

Preparation of TriGalNAc **(12):** Triantennary GalNAc compound **10** (0.35 g, 0.24 mmol, 1.0 eq.) and compound **11** (0.11 g, 0.31 mmol, 1.5 eq.) were dissolved in DCM (5 mL) under argon and triethylamine (0.1 mL, 0.61 mmol, 3.0 eq.) was added. The reaction was stirred at room temperature overnight. The solvent was removed under reduced pressure, the residue was dissolved in EtOAc (100 mL) and washed with water (100 mL). The organic layer was separated and dried over Na₂SO₄. The solvent was evaporated and the resulting crude material was purified by flash chromatography (elution gradient: 0-10% MeOH in DCM in 20 CV) to afford the title compound as white fluffy solid (0.27 g, 67%, rf = 0.5 (10% MeOH in DCM)). MS: calculated for C₈₄H₁₃₇N₁₁O₄₁, 1957.1. Found 1959.6.

### Conjugation of Tether 1 to a siRNA strand: Monofluoro cyclooctyne (MFCO) conjugation at 5'-or 3'-end

### 5'-end MFCO conjugation

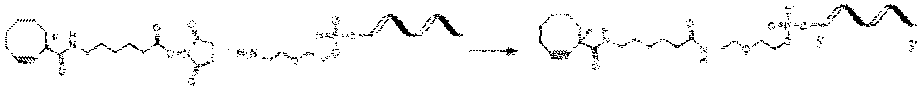

### 3'-end MFCO conjugation

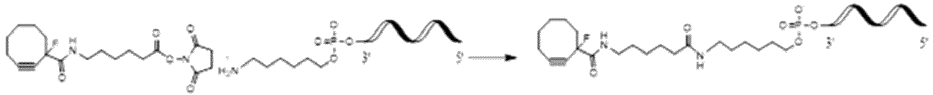

General conditions for MFCO conjugation: Amine-modified single strand was dissolved at 700 OD/mL in 50 mM carbonate/bicarbonate buffer pH 9.6/dimethyl sulfoxide (DMSO) 4:6 (v/v) and to this solution was added one molar equivalent of a 35 mM solution of MFCO-C6-NHS ester (Berry&Associates, Cat. # LK 4300) in DMF. The reaction was carried out at room temperature and after 1 h another molar equivalent of the MFCO solution was added. The reaction was allowed to proceed for an additional hour and was monitored by LC/MS. At least two molar equivalent excess of the MFCO NHS ester reagent relative to the amino modified oligonucleotide were needed to achieve quantitative consumption of the starting material. The reaction mixture was diluted 15-fold with water, filtered through a 1.2 µm filter from Sartorius and then purified by reserve phase (RP HPLC) on an Äkta Pure instrument (GE Healthcare).

Purification was performed using a XBridge C18 Prep 19 × 50 mm column from Waters. Buffer A was 100 mM TEAAc pH 7 and buffer B contained 95% acetonitrile in buffer A. A flow rate of 10 mL/min and a temperature of 60°C were employed. UV traces at 280 nm were recorded. A gradient of 0-100% B within 60 column volumes was employed.

Fractions containing full length conjugated oligonucleotide were pooled, precipitated in the freezer with 3 M NaOAc, pH 5.2 and 85% ethanol and the collected pellet was dissolved in water. Samples were desalted by size exclusion chromatography and concentrated using a speed-vac concentrator to yield the conjugated oligonucleotide in an isolated yield of 40-80%.

### 5'-GalNAc-T1 conjugates

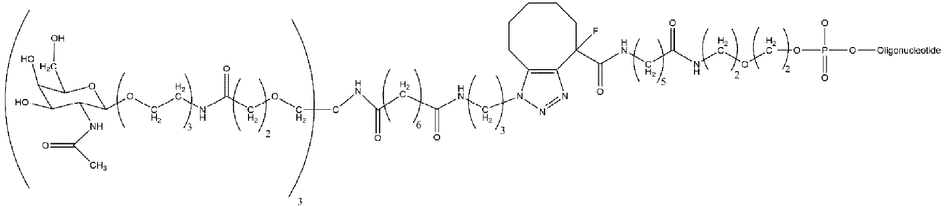

### 3'-GalNAc-T1 conjugates

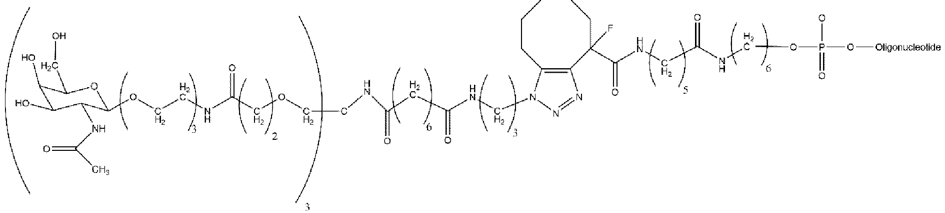

General procedure for TriGalNAc conjugation: MFCO-modified single strand was dissolved at 2000 OD/mL in water and to this solution was added one equivalent solution of compound 12 (10 mM) in DMF. The reaction was carried out at room temperature and after 3 h 0.7 molar equivalent of the compound 12 solution was added. The reaction was allowed to proceed overnight and completion was monitored by LCMS. The conjugate was diluted 15-fold in water, filtered through a 1.2 µm filter from Sartorius and then purified by RP HPLC on an Äkta Pure instrument (GE Healthcare).

RP HPLC purification was performed using a XBridge C18 Prep 19 × 50 mm column from Waters. Buffer A was 100 mM triethylammonium acetate pH 7 and buffer B contained 95% acetonitrile in buffer A. A flow rate of 10 mL/min and a temperature of 60°C were employed. UV traces at 280 nm were recorded. A gradient of 0-100% B within 60 column volumes was employed.

Fractions containing full-length conjugated oligonucleotide were pooled, precipitated in the freezer with 3 M NaOAc, pH 5.2 and 85% ethanol and the collected pellet was dissolved in water to give an oligonucleotide solution of about 1000 OD/mL. The O-acetates were removed by adding 20% aqueous ammonia. Quantitative removal of these protecting groups was verified by LC-MS.

The conjugates were desalted by size exclusion chromatography using Sephadex G25 Fine resin (GE Healthcare) on an Äkta Pure (GE Healthcare) instrument to yield the conjugated oligonucleotides in an isolated yield of 50-70%.

The following schemes further set out the routes of synthesis:

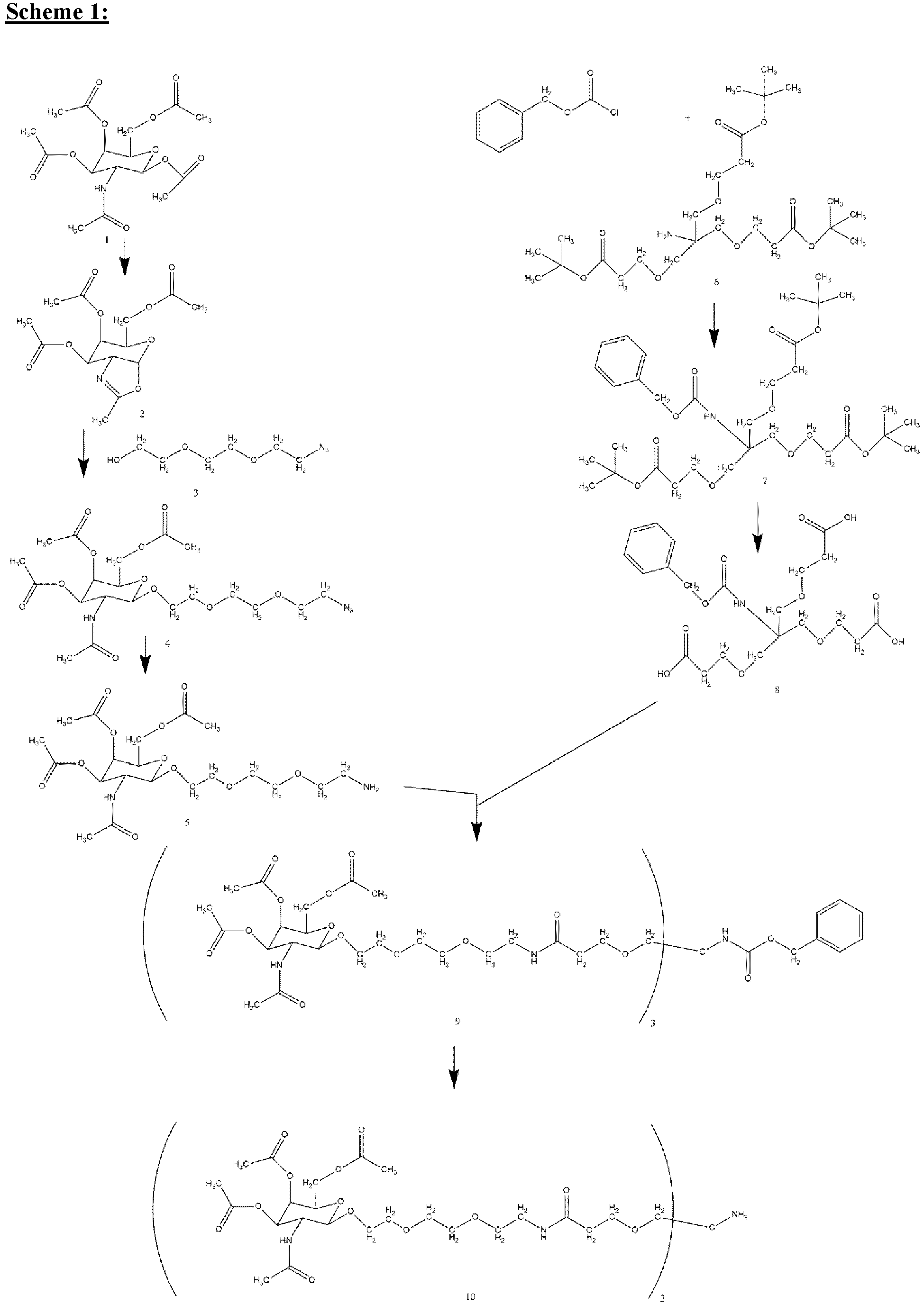

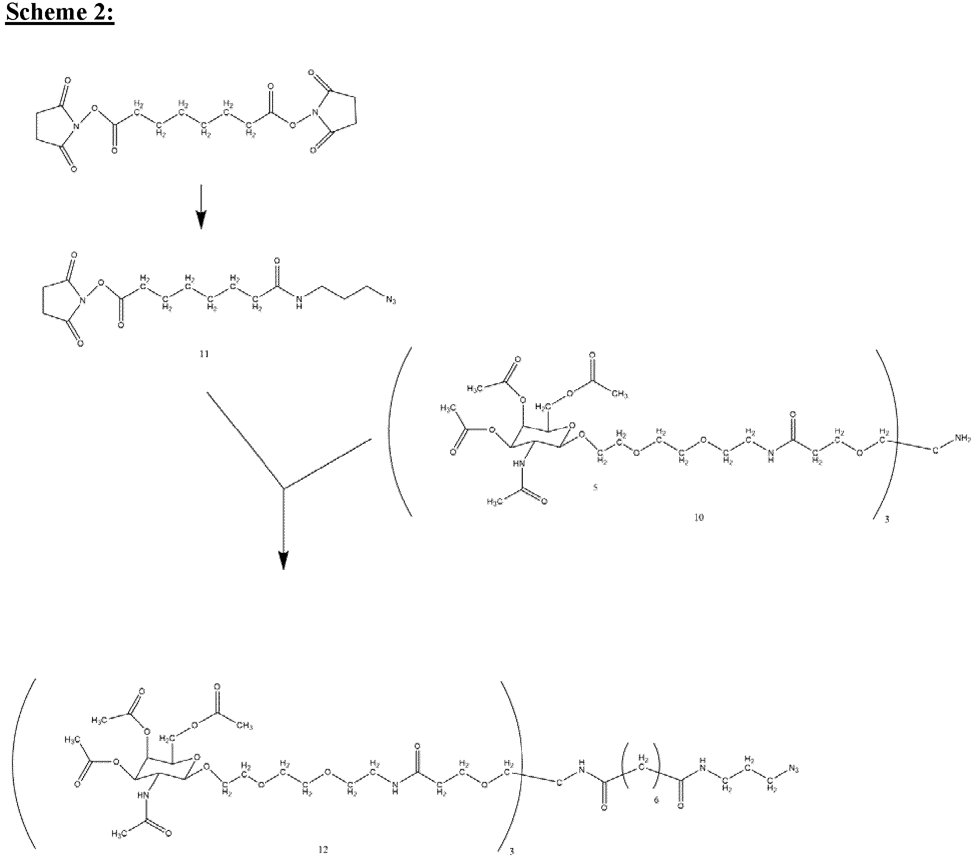

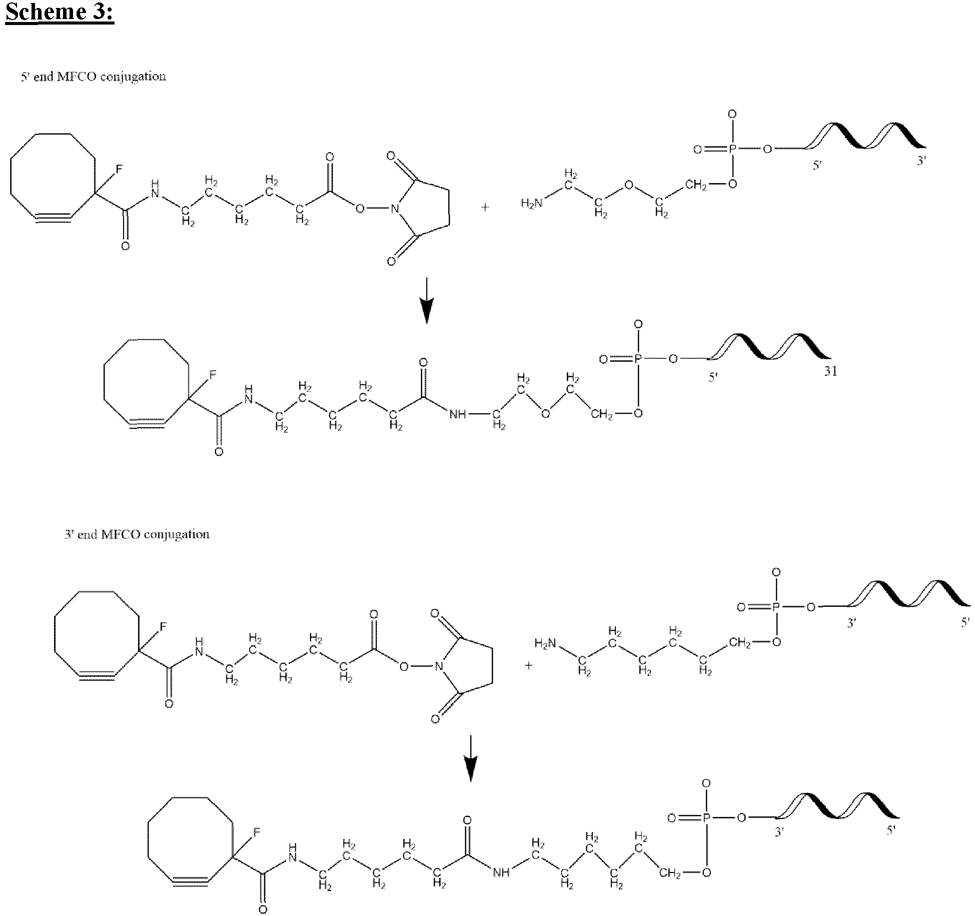

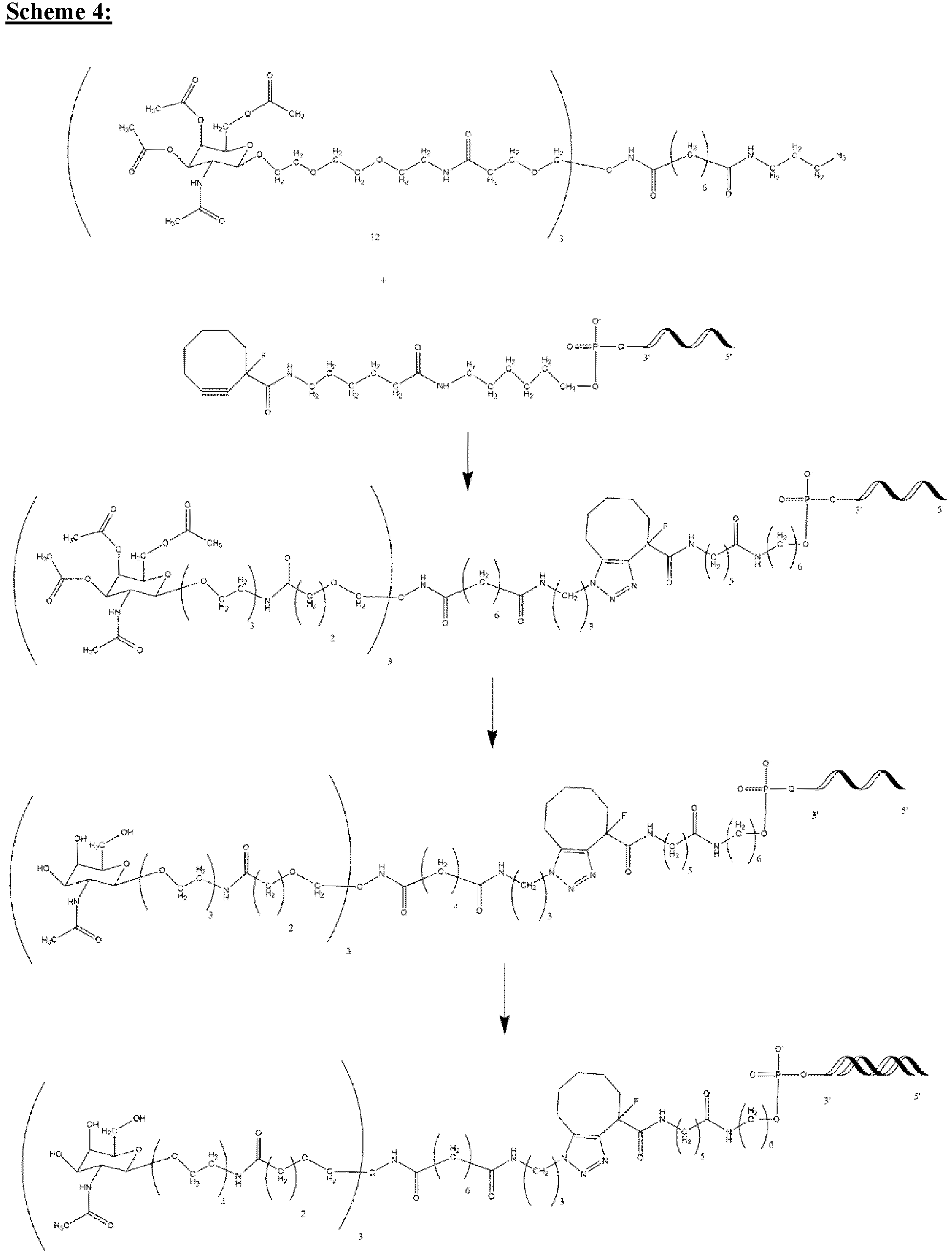

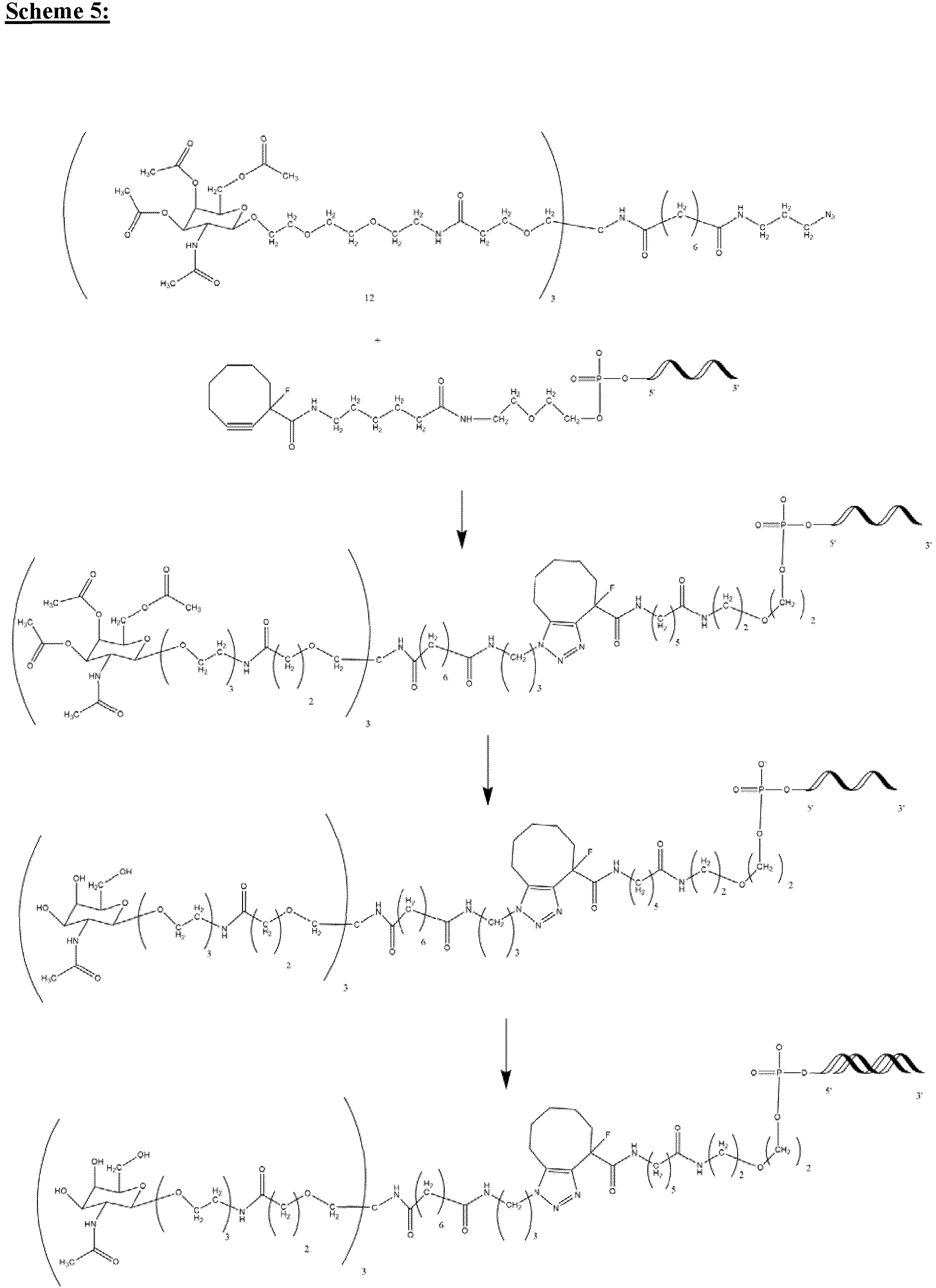

### Example 2: Duplex Annealing

To generate the desired siRNA duplex, the two complementary strands were annealed by combining equimolar aqueous solutions of both strands. The mixtures were placed into a water bath at 70°C for 5 minutes and subsequently allowed to cool to ambient temperature within 2 h. The duplexes were lyophilized for 2 days and stored at -20°C.

The duplexes were analyzed by analytical SEC HPLC on Superdex^{™} 75 Increase 5/150 GL column 5 × 153-158 mm (Cytiva) on a Dionex Ultimate 3000 (Thermo Fisher Scientific) HPLC system. Mobile phase consisted of 1x PBS containing 10% acetonitrile. An isocratic gradient was run in 10 min at a flow rate of 1.5 mL/min at room temperature. UV traces at 260 and 280 nm were recorded. Water (LC-MS grade) was purchased from Sigma-Aldrich and Phosphate-buffered saline (PBS; 10x, pH 7.4) was purchased from GIBCO (Thermo Fisher Scientific).

### Example 3: Synthesis of tether 2

### General Experimental conditions:

Thin layer chromatography (TLC) was performed on silica-coated aluminium plates with fluorescence indicator 254 nm from Macherey-Nagel. Compounds were visualized under UV light (254 nm), or after spraying with the 5% H₂SO₄ in methanol (MeOH) or ninhydrin reagent according to Stahl (from Sigma-Aldrich), followed by heating. Flash chromatography was performed with a Biotage Isolera One flash chromatography instrument equipped with a dual variable UV wavelength detector (200-400 nm) using Biotage Star Silica 10, 25, 50 or 100 g columns (Uppsala, Sweden).

All moisture-sensitive reactions were carried out under anhydrous conditions using dry glassware, anhydrous solvents, and argon atmosphere. All commercially available reagents were purchased from Sigma-Aldrich and solvents from Carl Roth GmbH + Co. KG. D-Galactosamine pentaacetate was purchased from AK scientific.

HPLC/ESI-MS was performed on a Dionex UltiMate 3000 RS UHPLC system and Thermo Scientific MSQ Plus Mass spectrometer using an Acquity UPLC Protein BEH C4 column from Waters (300Å, 1.7 µm, 2.1 × 100 mm) at 60 °C. The solvent system consisted of solvent A with H₂O containing 0.1% formic acid and solvent B with acetonitrile (ACN) containing 0.1% formic acid. A gradient from 5-100% of B over 15 min with a flow rate of 0.4 mL/min was employed. Detector and conditions: Corona ultra-charged aerosol detection (from esa). Nebulizer Temp.: 25 °C. N₂ pressure: 35.1 psi. Filter: Corona.

¹H and ¹³C NMR spectra were recorded at room temperature on a Varian spectrometer at 500 MHz (¹H NMR) and 125 MHz (¹³C NMR). Chemical shifts are given in ppm referenced to the solvent residual peak (CDCl₃ - ¹H NMR: δ at 7.26 ppm and ¹³C NMR δ at 77.2 ppm; DMSO-*d*₆ - ¹H NMR: δ at 2.50 ppm and ¹³C NMR δ at 39.5 ppm). Coupling constants are given in Hertz. Signal splitting patterns are described as singlet (s), doublet (d), triplet (t) or multiplet (m).

### Synthesis route for the conjugate building block TriGalNAc _Tether2:

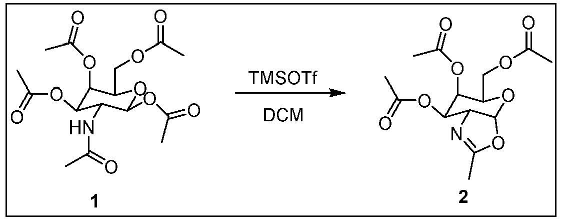

Preparation of compound 2: D-Galactosamine pentaacetate (3.00 g, 7.71 mmol, 1.0 eq.) was dissolved in anhydrous dichloromethane (DCM) (30 mL) under argon and trimethylsilyl trifluoromethanesulfonate (TMSOTf, 4.28 g, 19.27 mmol, 2.5 eq.) was added. The reaction was stirred at room temperature for 3 h. The reaction mixture was diluted with DCM (50 mL) and washed with cold saturated aq. NaHCO₃ (100 mL) and water (100 mL). The organic layer was separated, dried over Na₂SO₄, and concentrated to afford the title compound as yellow oil, which was purified by flash chromatography (gradient elution: 0-10% MeOH in DCM in 10 CV). The product was obtained as colourless oil (2.5 g, 98%, rf= 0.45 (2% MeOH in DCM)).

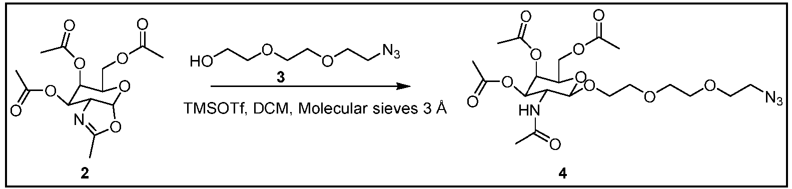

Preparation of compound **4:** Compound 2 (2.30 g, 6.98 mmol, 1.0 eq.) and azido-PEG3-OH (1.83 g, 10.5 mmol, 1.5 eq.) were dissolved in anhydrous DCM (40 mL) under argon and molecular sieves 3 Å (5 g) were added to the solution. The mixture was stirred at room temperature for 1 h. TMSOTf (0.77 g, 3.49 mmol, 0.5 eq.) was then added to the mixture and the reaction was stirred overnight. The molecular sieves were filtered, the filtrate was diluted with DCM (100 mL) and washed with cold saturated aq. NaHCO₃ (100 mL) and water (100 mL). The organic layer was separated, dried over Na₂SO₄ and the solvent was removed under reduced pressure. The crude material was purified by flash chromatography (gradient elution: 0-3% MeOH in DCM in 10 CV) to afford the title product as light-yellow oil (3.10 g, 88%, rf = 0.25 (2% MeOH in DCM)). MS: calculated for C₂₀H₃₂N₄O₁₁, 504.21. Found 505.4. ¹H NMR (500 MHz, CDCl₃) δ 6.21-6.14 (m, 1H), 5.30 (dd, *J* = 3.4, 1.1 Hz, 1H), 5.04 (dd, *J* = 11.2, 3.4 Hz,1H), 4.76 (d, *J =* 8.6 Hz, 1H), 4.23-4.08 (m, 3H), 3.91-3.80 (m, 3H), 3.74-3.59 (m, 9H), 3.49-3.41 (m, 2H), 2.14 (s, 3H), 2.02 (s, 3H), 1.97 (d, *J =* 4.2 Hz, 6H). ¹³C NMR (125 MHz, CDCl₃) δ 170.6 (C), 170.5 (C), 170.4 (C), 170.3 (C), 102.1 (CH), 71.6 (CH), 70.8 (CH), 70.6 (CH), 70.5 (CH), 70.3 (CH₂), 69.7 (CH₂), 68.5 (CH₂), 66.6 (CH₂), 61.5 (CH₂), 23.1 (CH₃), 20.7 (3×CH₃).

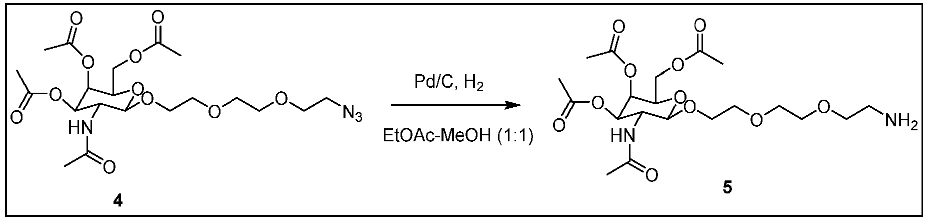

Preparation of compound **5:** Compound **4** (1.00 g, 1.98 mmol, 1.0 eq.) was dissolved in a mixture of ethyl acetate (EtOAc) and MeOH (30 mL 1: 1 v/v) and Pd/C (100 mg) was added. The reaction mixture was degassed using vacuum/argon cycles (3x) and hydrogenated under balloon pressure overnight. The reaction mixture was filtered through celite and washed with EtOAc (30 mL). The solvent was removed under reduced pressure to afford the title compound as colourless oil (0.95 g, quantitative yield, rf = 0.25 (10% MeOH in DCM)). The compound was used without further purification. MS: calculated for C₂₀H₃₄N₂O₁₁, 478.2. Found 479.4.

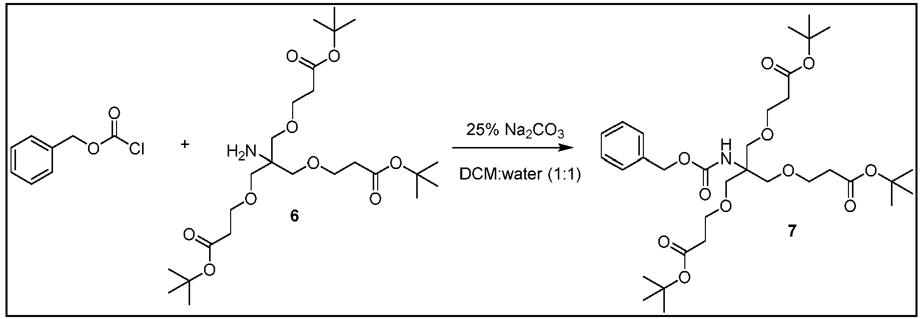

Preparation of compound 7: Tris{[2-(tert-butoxycarbonyl)ethoxy]methyl}-methylamine **6** (3.37 g, 6.67 mmol, 1.0 eq.) was dissolved in a mixture of DCM/water (40 mL 1:1 v/v) and Na₂CO₃ (0.18 g, 1.7 mmol, 0.25 eq.) was added while stirring vigorously. Benzyl chloroformate (2.94 mL, 20.7 mmol, 3.10 eq.) was added dropwise to the previous mixture and the reaction was stirred at room temperature for 24 h. The reaction mixture was diluted with CH₂Cl₂ (100 mL) and washed with water (100 mL). The organic layer was separated and dried over Na₂SO₄. The solvent was removed under reduced pressure and the resulting crude material was purified by flash chromatography (gradient elution: 0-10% EtOAc in cyclohexane in 12 CV) to afford the title compound as pale yellowish oil (3.9 g, 91%, rf = 0.56 (10% EtOAc in cyclohexane)). MS: calculated for C₃₃H₅₃NO₁₁, 639.3. Found 640.9. ¹H NMR (500 MHz, DMSO-*d*₆) δ 7.38-7.26 (m, 5H), 4.97 (s, 2H), 3.54 (t, 6H), 3.50 (s, 6H), 2.38 (t, 6H), 1.39 (s, 27H). ¹³C NMR (125 MHz, DMSO-*d*₆) δ 170.3 (3xC), 154.5 (C), 137.1 (C), 128.2 (2xCH), 127.7 (CH), 127.6 (2xCH), 79.7 (3xC), 68.4 (3×CH₂), 66.8 (3×CH₂), 64.9 (C), 58.7 (CH₂), 35.8 (3×CH₂), 27.7 (9×CH₃).

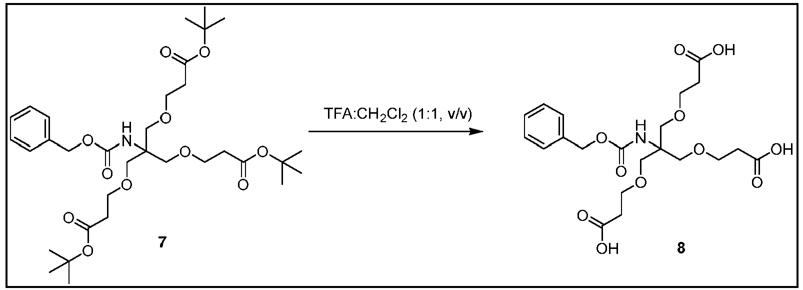

Preparation of compound **8:** Cbz-NH-tris-Boc-ester 7 (0.20 g, 0.39 mmol, 1.0 eq.) was dissolved in CH₂Cl₂ (1 mL) under argon, trifluoroacetic acid (TFA, 1 mL) was added and the reaction was stirred at room temperature for 1 h. The solvent was removed under reduced pressure, the residue was co-evaporated 3 times with toluene (5 mL) and dried under high vacuum to get the compound as its TFA salt (0.183 g, 98%). The compound was used without further purification. MS: calculated for C₂₁H₂₉NO₁₁, 471.6. Found 472.4.

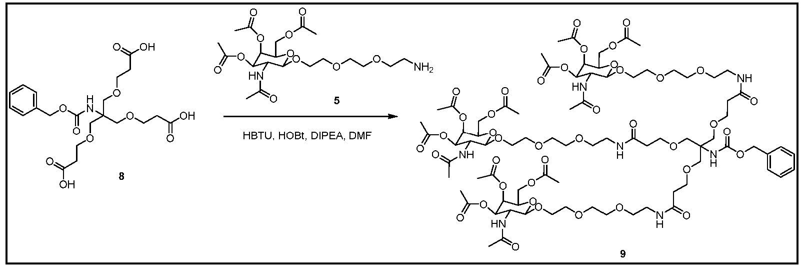

Preparation of compound **9:** CbzNH-tris-COOH **8** (0.72 g, 1.49 mmol, 1.0 eq.) and GalNAc-PEG3-NH₂ **5** (3.56 g, 7.44 mmol, 5.0 eq.) were dissolved in *N,N*-dimethylformamide (DMF) (25 mL). Then *N,N,N',N*'-tetramethyl-O-(1H-benzotriazol-1-yl)uronium hexafluorophosphate (HBTU) (2.78 g, 7.44 mmol, 5.0 eq.), 1-hydroxybenzotriazole hydrate (HOBt) (1.05 g, 7.44 mmol, 5.0 eq.) and *N,N-*diisopropylethylamine (DIPEA) (2.07 mL, 11.9 mmol, 8.0 eq.) were added to the solution and the reaction was stirred for 72 h. The solvent was removed under reduced pressure, the residue was dissolved in DCM (100 mL) and washed with saturated aq. NaHCO₃ (100 mL). The organic layer was dried over Na₂SO₄, the solvent evaporated and the crude material was purified by flash chromatography (gradient elution: 0-5% MeOH in DCM in 14 CV). The product was obtained as pale yellowish oil (1.2 g, 43%, rf = 0.20 (5% MeOH in DCM)). MS: calculated for C₈₁H₁₂₅N₇O₄₁, 1852.9. Found 1854.7. ¹H NMR (500 MHz, DMSO-*d*₆) δ 7.90-7.80 (m, 10H), 7.65-7.62 (m, 4H), 7.47-7.43 (m, 3H), 7.38-7.32 (m, 8H), 5.24-5.22 (m, 3H), 5.02-4.97 (m, 4H), 4.60-4.57 (m, 3 H), 4.07-3.90 (m 10H), 3.67-3.36 (m, 70H), 3.23-3.07 (m, 25H), 2.18 (s, 10H), 2.00 (s, 13H), 1.89 (s, 11H), 1.80-1.78 (m, 17H). ¹³C NMR (125 MHz, DMSO-*d*₆) δ 170.1 (C), 169.8 (C), 169.7 (C), 169.4 (C), 169.2 (C), 169.1 (C), 142.7 (C), 126.3 (CH), 123.9 (CH), 118.7 (CH), 109.7 (CH), 100.8 (CH), 70.5 (CH), 69.8 (CH), 69.6 (CH), 69.5 (CH), 69.3 (CH₂), 69.0 (CH₂), 68.2 (CH₂), 67.2 (CH₂), 66.7 (CH₂), 61.4 (CH₂), 22.6 (CH₂), 22.4 (3×CH₃), 20.7 (9×CH₃).

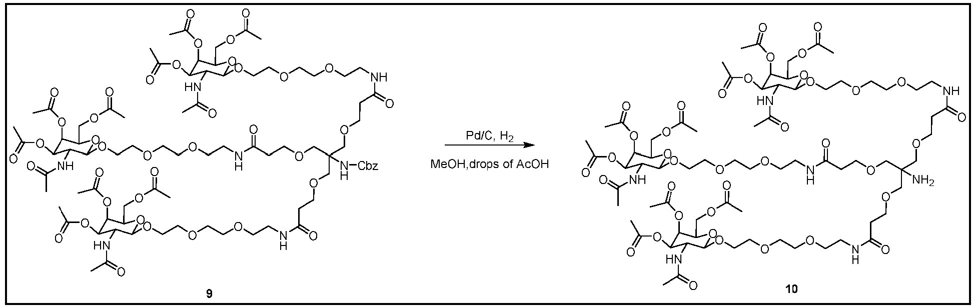

Preparation of compound **10:** Triantennary GalNAc compound **9** (0.27 g, 0.14 mmol, 1.0 eq.) was dissolved in MeOH (15 mL), 3 drops of acetic acid (AcOH) and Pd/C (30 mg) was added. The reaction mixture was degassed using vacuum/argon cycles (3x) and hydrogenated under balloon pressure overnight. The completion of the reaction was followed by mass spectrometry and the resulting mixture was filtered through a thin pad of celite. The solvent was evaporated, and the residue obtained was dried under high vacuum and used for the next step without further purification. The product was obtained as pale yellowish oil (0.24 g, quantitative yield). MS: calculated for C₇₃H₁₁₉N₇O₃₉, 1718.8. Found 1719.3.

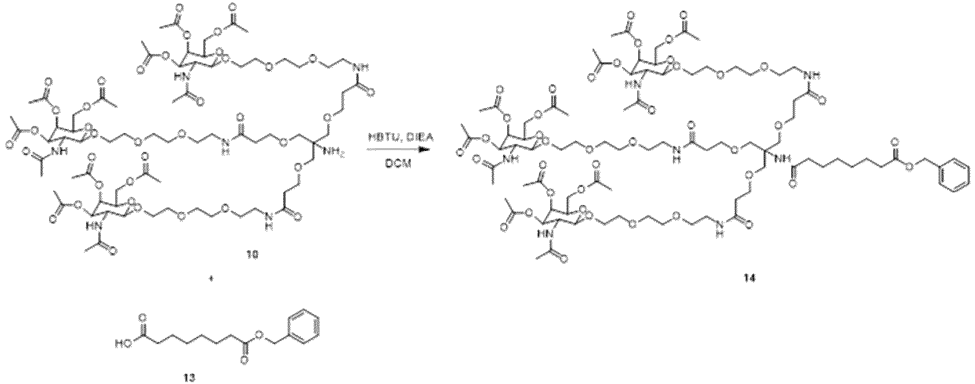

Preparation of compound 14: Triantennary GalNAc compound 10 (0.45 g, 0.26 mmol, 1.0 eq.), HBTU (0.19 g, 0.53 mmol, 2.0 eq.) and DIPEA (0.23 mL, 1.3 mmol, 5.0 eq.) were dissolved in DCM (10 mL) under argon. To this mixture, it was added dropwise a solution of compound 13 (0.14 g, 0.53 mmol, 2.0 eq.) in DCM (5 mL). The reaction was stirred at room temperature overnight. The solvent was removed, and the residue was dissolved in EtOAc (50 mL), washed with water (50 mL) and dried over Na₂SO₄. The solvent was evaporated, and the crude material was purified by flash chromatography (gradient elution: 0-5% MeOH in DCM in 20 CV). The product was obtained as white fluffy solid (0.25 g, 48%, rf = 0.4 (10% MeOH in DCM)). MS: calculated for C88H137N7O42, 1965.1. Found 1965.6.

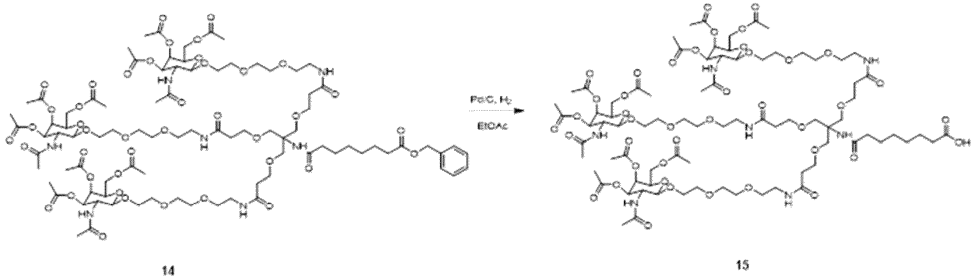

Preparation of TriGalNAc (15): Triantennary GalNAc compound 14 (0.31 g, 0.15 mmol, 1.0 eq.) was dissolved in EtOAc (15 mL) and Pd/C (40 mg) was added. The reaction mixture was degassed by using vacuum/argon cycles (3x) and hydrogenated under balloon pressure overnight. The completion of the reaction was monitored by mass spectrometry and the resulting mixture was filtered through a thin pad of celite. The solvent was removed under reduced pressure and the resulting residue was dried under high vacuum overnight. The residue was used for conjugations to oligonucleosides without further purification (0.28 g, quantitative yield). MS: calculated for C₈₁H₁₃₁N₇O₄₂, 1874.9. Found 1875.3.

### Conjugation of Tether 2 to a siRNA strand: TriGalNAc tether 2 (GalNAc-T2) conjugation at 5'-end or 3'-end

### 5'-GaINAc-T2 conjugates

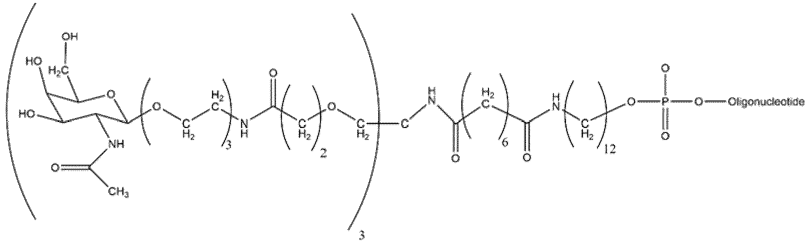

### 3'-GalNAc-T2 conjugates

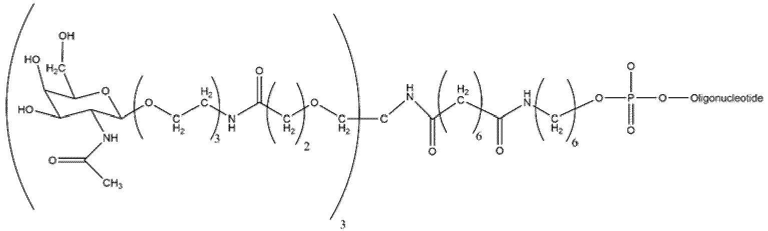

Preparation of TriGalNAc tether 2 NHS ester: To a solution of carboxylic acid tether 2 (compound 15, 227 mg, 121 µmol) in DMF (2.1 mL), N-hydroxysuccinimide (NHS) (15.3 mg, 133 µmol) and N,N'-diisopropylcarbodiimide (DIC) (19.7 µL, 127 µmol) were added. The solution was stirred at room temperature for 18 h and used without purification for the subsequent conjugation reactions.

General procedure for triGalNAc tether 2 conjugation: Amine-modified single strand was dissolved at 700 OD/mL in 50 mM carbonate/bicarbonate buffer pH 9.6/DMSO 4:6 (v/v) and to this solution was added one molar equivalent of Tether 2 NHS ester (57 mM) solution in DMF. The reaction was carried out at room temperature and after 1 h another molar equivalent of the NHS ester solution was added. The reaction was allowed to proceed for one more hour and reaction progress was monitored by LCMS. At least two molar equivalent excess of the NHS ester reagent relative to the amino modified oligonucleoside were needed to achieve quantitative consumption of the starting material. The reaction mixture was diluted 15-fold with water, filtered once through 1.2 µm filter from Sartorius and then purified by reserve phase (RP HPLC) on an Äkta Pure (GE Healthcare) instrument.

The purification was performed using a XBridge C18 Prep 19 x 50 mm column from Waters. Buffer A was 100 mM TEAA pH 7 and buffer B contained 95% acetonitrile in buffer A. A flow rate of 10 mL/min and a temperature of 60°C were employed. UV traces at 280 nm were recorded. A gradient of 0-100% B within 60 column volumes was employed.

Fractions containing full-length conjugated oligonucleosides were pooled together, precipitated in the freezer with 3 M NaOAc, pH 5.2 and 85% ethanol and then dissolved at 1000 OD/mL in water. The O-acetates were removed with 20% ammonium hydroxide in water until completion (monitored by LC-MS).

The conjugates were desalted by size exclusion chromatography using Sephadex G25 Fine resin (GE Healthcare) on an Äkta Pure (GE Healthcare) instrument to yield the conjugated oligonucleotides in an isolated yield of 60-80%.

The conjugates were characterized by HPLC-MS analysis with a 2.1 × 50 mm XBridge C18 column (Waters) on a Dionex Ultimate 3000 (Thermo Fisher Scientific) HPLC system equipped with a Compact ESI-Qq-TOF mass spectrometer (Bruker Daltonics). Buffer A was 16.3 mM triethylamine, 100 mM HFIP in 1% MeOH in H2O and buffer B contained 95% MeOH in buffer A. A flow rate of 250 µL/min and a temperature of 60°C were employed. UV traces at 260 and 280 nm were recorded. A gradient of 1-100% B within 31 min was employed.

The following schemes further set out the routes of synthesis:

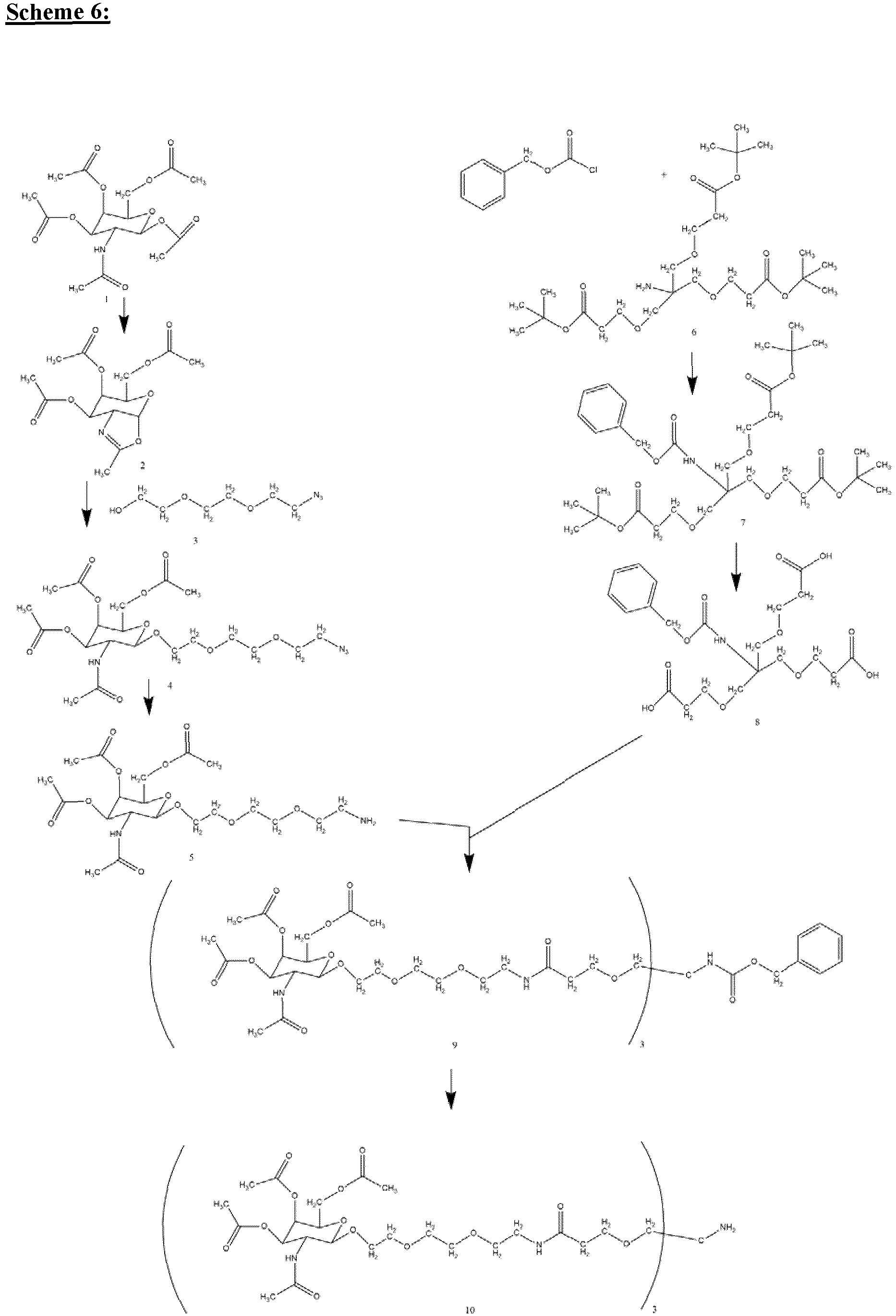

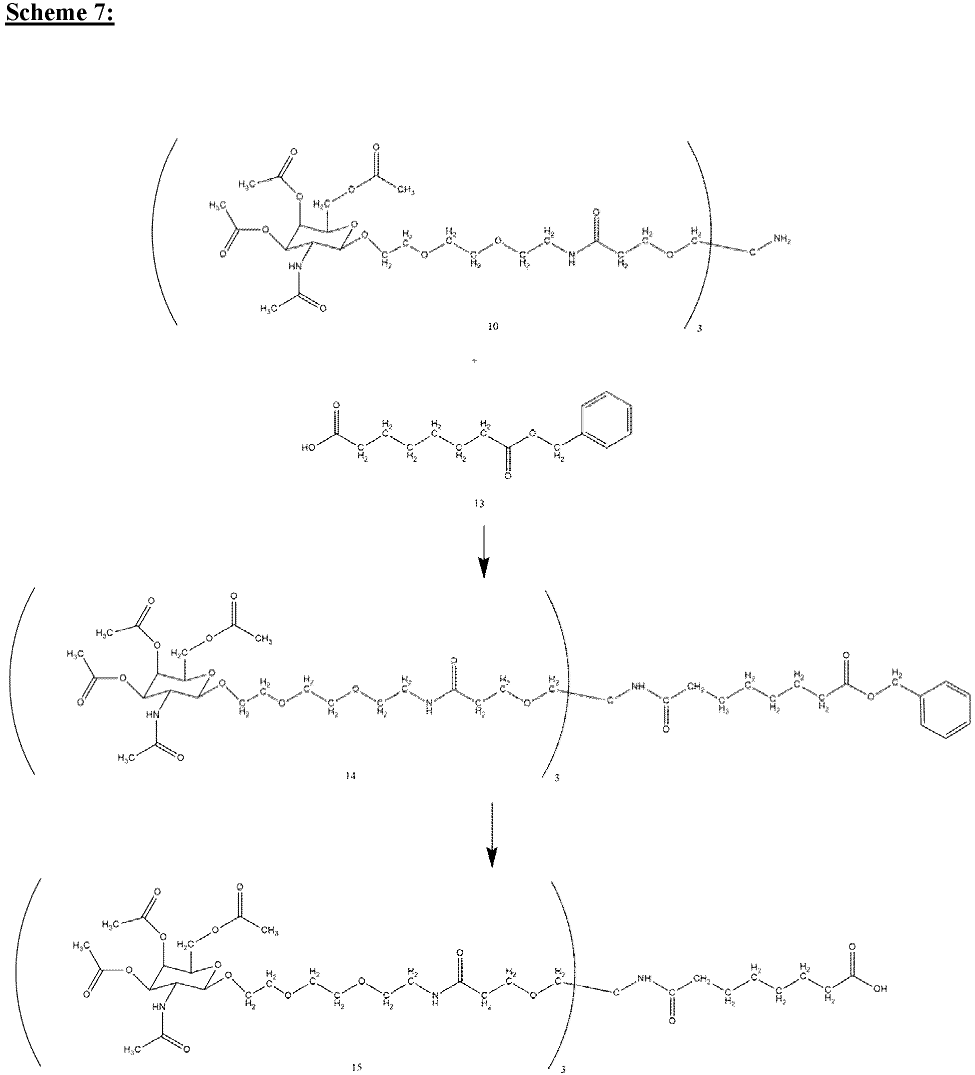

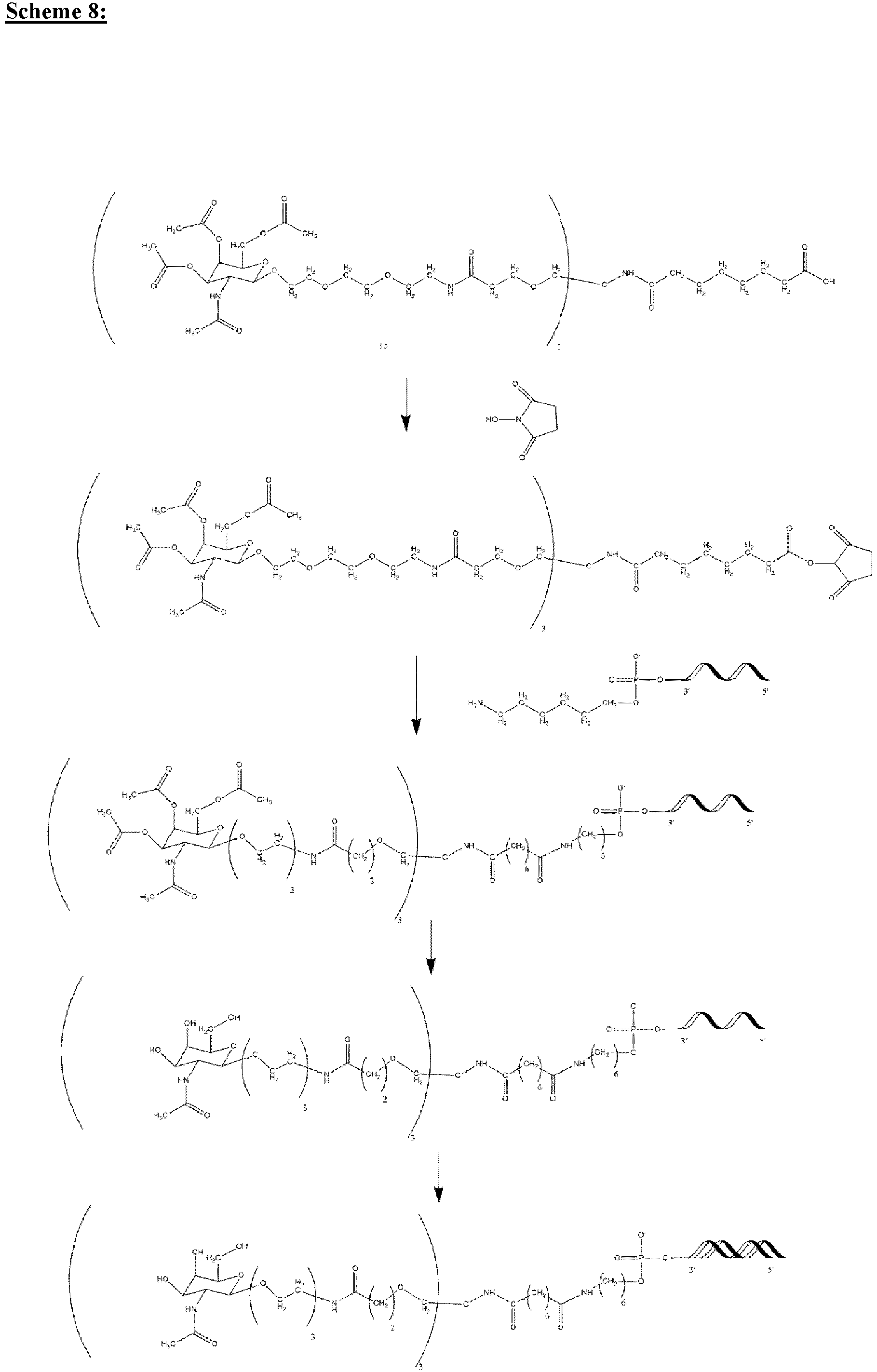

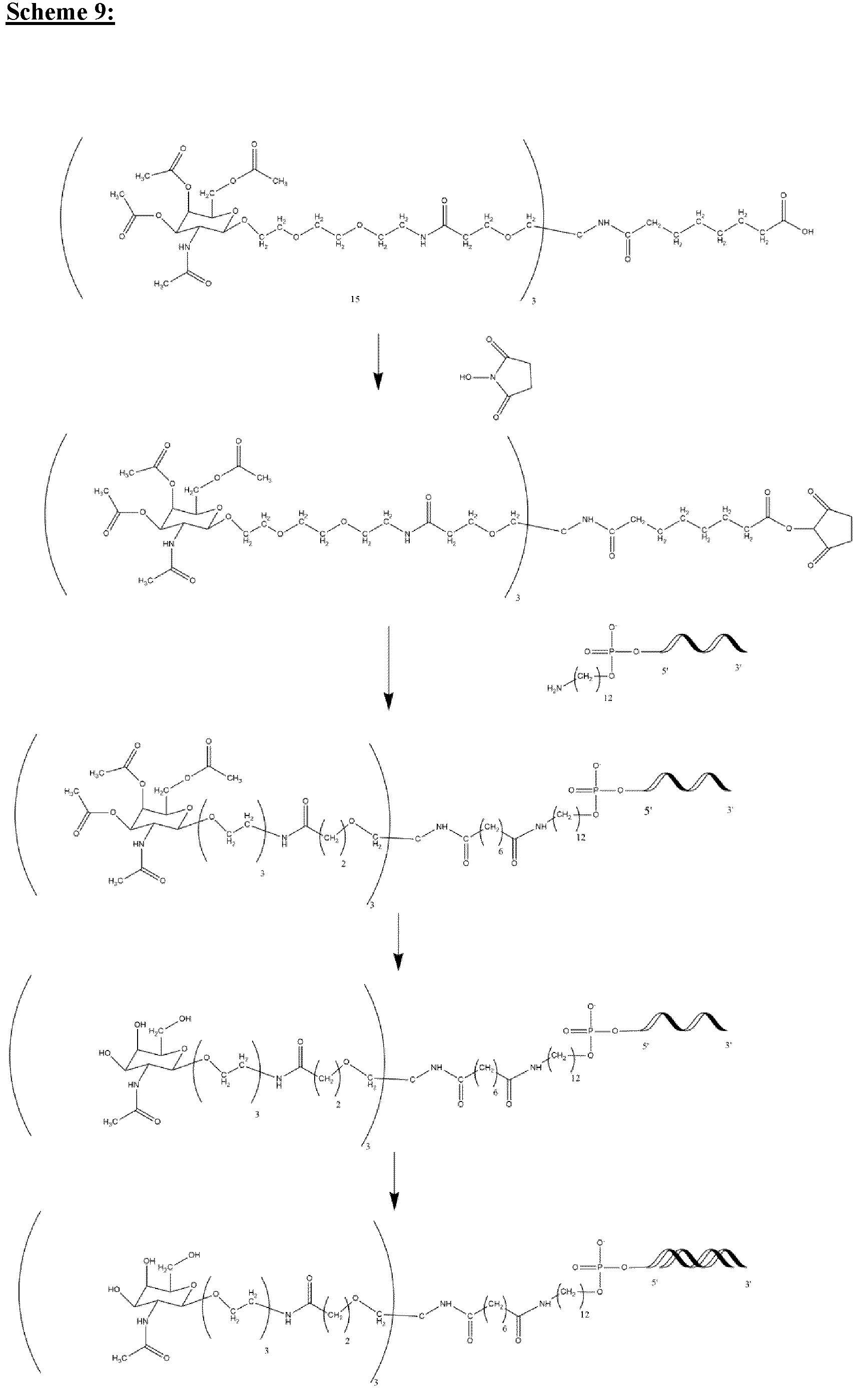

### Example 4: Duplex Annealing

To generate the desired siRNA duplex, the two complementary strands were annealed by combining equimolar aqueous solutions of both strands. The mixtures were placed into a water bath at 70°C for 5 minutes and subsequently allowed to cool to ambient temperature within 2 h. The duplexes were lyophilized for 2 days and stored at -20°C.

The duplexes were analyzed by analytical SEC HPLC on Superdex^{™} 75 Increase 5/150 GL column 5 x 153-158 mm (Cytiva) on a Dionex Ultimate 3000 (Thermo Fisher Scientific) HPLC system. Mobile phase consisted of 1x PBS containing 10% acetonitrile. An isocratic gradient was run in 10 min at a flow rate of 1.5 mL/min at room temperature. UV traces at 260 and 280 nm were recorded. Water (LC-MS grade) was purchased from Sigma-Aldrich and Phosphate-buffered saline (PBS; 10x, pH 7.4) was purchased from GIBCO (Thermo Fisher Scientific).

### Example 5: Alternative synthesis route for the conjugate building block TriGalNAc

### _Tether2:

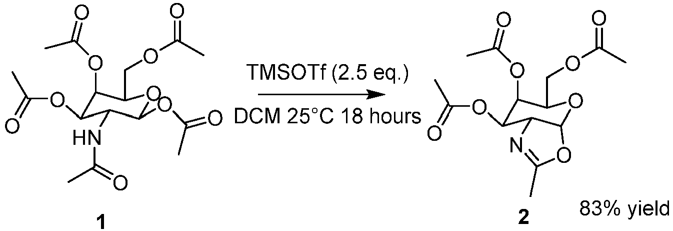

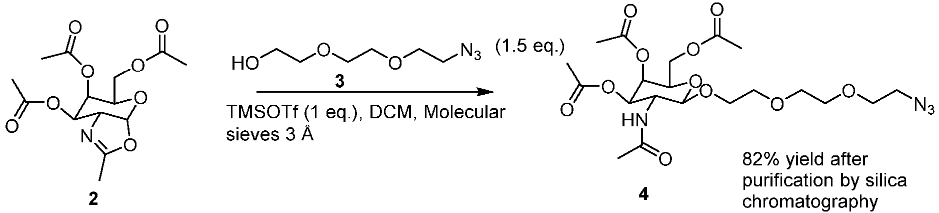

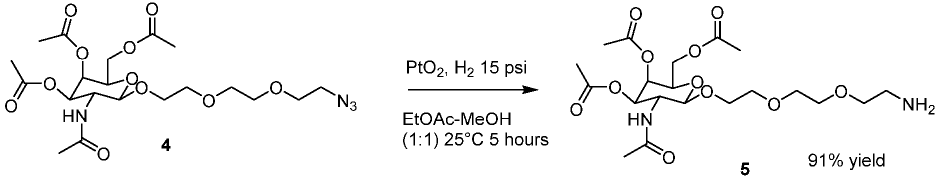

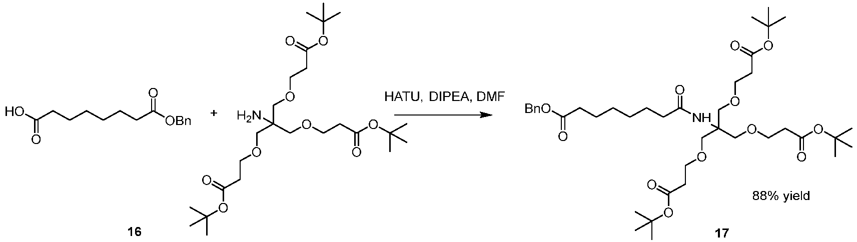

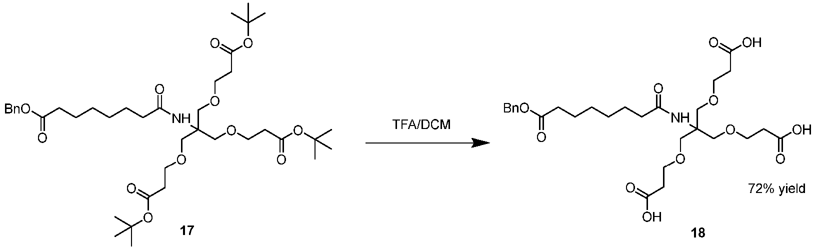

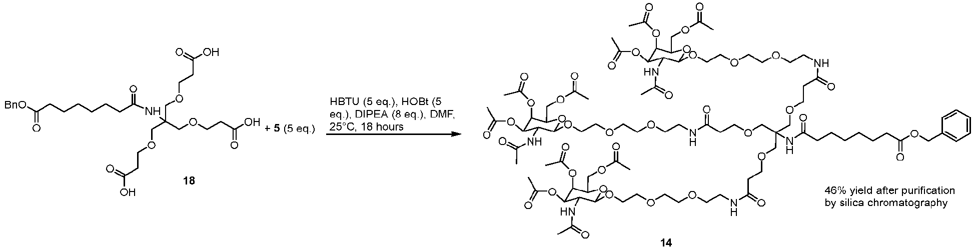

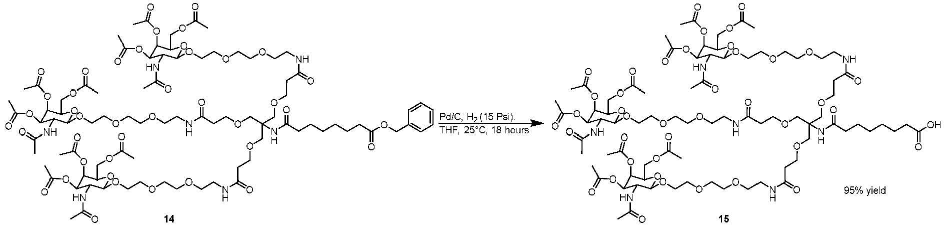

### Conjugation of Tether 2 to a siRNA strand: TriGalNAc tether 2 (GalNAc-T2) conjugation at 5'-end or 3'-end

### Conjugation conditions

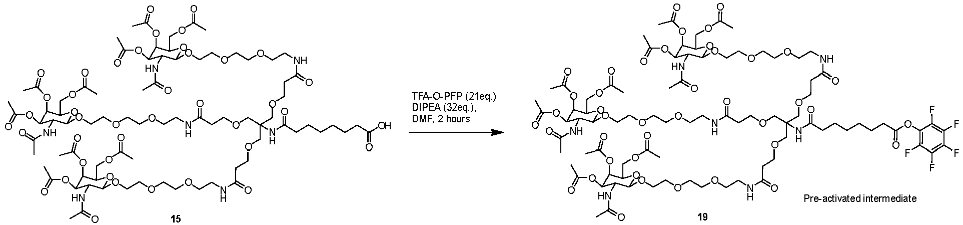

**Pre-activation:** To a solution of compound 15 (16 umol, 4 eq.) in DMF (160 µL) was added TFA-O-PFP (15 µl, 21 eq.) followed by DIPEA (23 µl, 32 eq.) at 25°C. The tube was shaken for 2 h at 25°C. The reaction was quenched with H₂O (10 µL).

**Coupling:** The resulting mixture was diluted with DMF (400 µl), followed by addition of oligo-amine solution (4.0 µmol in 10 x PBS, pH 7.4, 500 µL; final oligo concentration in organic and aqueous solution: 4 µmol/ml = 4 mM). The tube was shaken at 25°C for 16 h and the reaction was analysed by LCMS. The resulting mixture was treated with 28% NH₄OH (4.5 ml) and shaken for 2 h at 25°C. The mixture was analysed by LCMS, concentrated, and purified by IP-RP HPLC to produce the oligonucleotides conjugated to tether 2 GalNAc.

### 5'-GaINAc-T2 conjugates

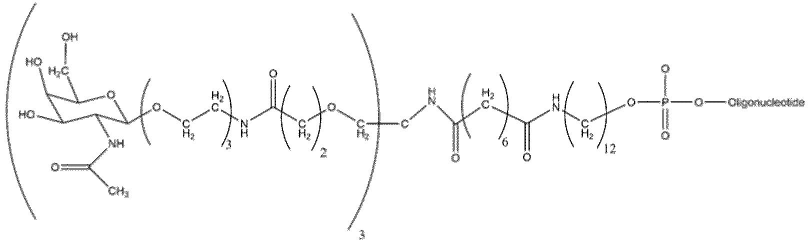

### 3'-GalNAc-T2 conjugates

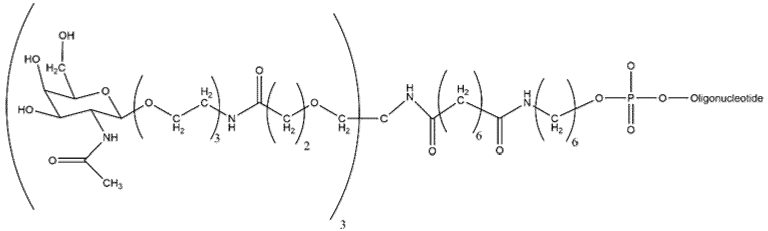

### Example 6: Solid phase synthesis method: scale ≤1µmol

Syntheses of siRNA sense and antisense strands were performed on a MerMade192X synthesiser with commercially available solid supports made of controlled pore glass with universal linker (Universal CPG, with a loading of 40 µmol/g; LGC Biosearch or Glen Research).

RNA phosphoramidites were purchased from ChemGenes or Hongene.

The 2'-O-Methyl phosphoramidites used were the following: 5'-(4,4'-dimethoxytrityl)-N-benzoyl-adenosine 2'-O-methyl-3'- [(2-cyanoethyl)-(N,N-diisopropyl)]-phosphoramidite, 5'-(4,4'-dimethoxytrityl)-N-acetyl-cytidine 2'-O-methyl-3'- [(2-cyanoethyl)-(N,N-diisopropyl)]-phosphoramidite, 5'-(4,4'-dimethoxytrityl)-N-isobutyryl-guanosine 2'-O-methyl-3'-[(2-cyanoethyl)-(N,N-diisopropyl)]-phosphoramidite, 5'-(4,4'-dimethoxytrityl)-uridine 2'-O-methyl-3'-[(2-cyanoethyl)- (N,N-diisopropyl)]-phosphoramidite.

The 2'-F phosphoramidites used were the following: 5'-dimethoxytrityl-N-benzoyl-deoxyadenosine 2'-fluoro-3'-[(2-cyanoethyl)-(N,N-diisopropyl)]-phosphoramidite, 5'-dimethoxytrityl-N-acetyl-deoxycytidine 2'-fluoro-3'-[(2-cyanoethyl)-(N,N-diisopropyl)]-phosphoramidite, 5'-dimethoxytrityl-N-isobutyryl-deoxyguanosine 2'-fluoro-3'- [(2-cyanoethyl)-(N,N-diisopropyl)]-phosphoramidite and 5'-dimethoxytrityl-deoxyuridine 2'-fluoro-3'-[(2-cyanoethyl)-(N,N-diisopropyl)]-phosphoramidite.

All phosphoramidites were dissolved in anhydrous acetonitrile (Honeywell Research Chemicals) at a concentration of 0.05M, except 2'-O-methyl-uridine phosphoramidite which was dissolved in DMF/MeCN (1:4, v/v). Iodine at 0.02M in acetonitrile/Pyridine/H2O (DNAchem) was used as oxidizing reagent. Thiolation for phosphorothioate linkages was performed with 0.2 M PADS (TCI) in acetonitrile/pyridine 1: 1 v/v. 5-Ethyl thiotetrazole (ETT), 0.25M mM in acetonitrile was used as activator solution.

Inverted abasic phosphoramidite, 3-O-Dimethoxytrityl-2-deoxyribose-5-[(2-cyanoethyl)-(N, N-diisopropyl)]-phosphoramidite were purchased from Chemgenes (ANP-1422) or Hongene (OP-040).

At each cycle, the DMT was removed by deblock solution, 3% TCA in DCM (DNAchem).

The coupling time was 180 seconds. The oxidizer contact time was set to 80 seconds and thiolation time was 2*100 seconds.

At the end of the synthesis, the oligonucleotides were cleaved from the solid support using a NH₄OH:EtOH solution 4:1 (v/v) for 20 hours at 45°C (TCI). The solid support was then filtered off, the filter was thoroughly washed with H₂O and the volume of the combined solution was reduced by evaporation under reduced pressure.

Oligonucleotide were treated to form the sodium salt by ultracentrifugation using Amicon Ultra-2 Centrifugal Filter Unit; PBS buffer (10x, Teknova, pH 7.4, Sterile) or by EtOH precipitation from 1M sodium acetate.

The single strands identity were assessed by MS ESI- and then, were annealed in water to form the final duplex siRNA and duplex purity were assessed by size exclusion chromatography.

### Example 7: Solid phase synthesis method: scale ≥5 µmol

Syntheses of siRNA sense and antisense strands were performed on a MerMade12 synthesiser with commercially available solid supports made of controlled pore glass with universal linker (Universal CPG, with a loading of 40 µmol/g; LGC Biosearch or Glen Research) at 5 µmol scale. Sense strand destined to 3' conjugation were sytnthesised at 12 µmol on 3'-PT-Amino-Modifier C6 CPG 500 Å solid support with a loading of 86 µmol/g (LGC).

RNA phosphoramidites were purchased from ChemGenes or Hongene.

The 2'-O-Methyl phosphoramidites used were the following: 5'-(4,4'-dimethoxytrityl)-N-benzoyl-adenosine 2'-O-methyl-3'- [(2-cyanoethyl)-(N,N-diisopropyl)]-phosphoramidite, 5'-(4,4'-dimethoxytrityl)-N-acetyl-cytidine 2'-O-methyl-3'- [(2-cyanoethyl)-(N,N-diisopropyl)]-phosphoramidite, 5'-(4,4'-dimethoxytrityl)-N-isobutyryl-guanosine 2'-O-methyl-3'-[(2-cyanoethyl)-(N,N-diisopropyl)]-phosphoramidite, 5'-(4,4'-dimethoxytrityl)-uridine 2'-O-methyl-3'-[(2-cyanoethyl)- (N,N-diisopropyl)]-phosphoramidite.

The 2'-F phosphoramidites used were the following: 5'-dimethoxytrityl-N-benzoyl-deoxyadenosine 2'-fluoro-3'-[(2-cyanoethyl)-(N,N-diisopropyl)]-phosphoramidite, 5'-dimethoxytrityl-N-acetyl-deoxycytidine 2'-fluoro-3'-[(2-cyanoethyl)-(N,N-diisopropyl)]-phosphoramidite, 5'-dimethoxytrityl-N-isobutyryl-deoxyguanosine 2'-fluoro-3'- [(2-cyanoethyl)-(N,N-diisopropyl)]-phosphoramidite and 5'-dimethoxytrityl-deoxyuridine 2'-fluoro-3'-[(2-cyanoethyl)-(N,N-diisopropyl)]-phosphoramidite.

Inverted abasic phosphoramidite, 3-O-Dimethoxytrityl-2-deoxyribose-5-[(2-cyanoethyl)-(N, N-diisopropyl)]-phosphoramidite were purchased from Chemgenes (ANP-1422) or Hongene (OP-040).

All phosphoramidites were dissolved in anhydrous acetonitrile (Honeywell Research Chemicals) at a concentration of 0.05M, except 2'-O-methyl-uridine phosphoramidite which was dissolved in DMF/MeCN (1:4, v/v). Iodine at 0.02M in acetonitrile/Pyridine/H₂O (DNAchem) was used as oxidizing reagent. Thiolation for phosphorothioate linkages was performed with 0.2 M PADS (TCI) in acetonitrile/pyridine 1:1 v/v. 5-Ethyl thiotetrazole (ETT), 0.25M mM in acetonitrile was used as activator solution.

At each cycle, the DMT was removed by deblock solution, 3% TCA in DCM (DNAchem).

For strands synthesised on universal CPG the coupling was performed with 8 eq. of amidite for 130 seconds. The oxidation time was 47 seconds, the thiolation time was 210 seconds.

For strands synthesised on 3'-PT-Amino-Modifier C6 CPG the coupling was performed with 8 eq. of amidite for 2*150 seconds. The oxidation time was 47 seconds, the thiolation time was 250 seconds

At the end of the synthesis, the oligonucleotides were cleaved from the solid support using a NH₄OH:EtOH solution 4:1 (v/v) for 20 hours at 45°C (TCI). The solid support was then filtered off, the filter was thoroughly washed with H₂O and the volume of the combined solution was reduced by evaporation under reduced pressure.

Oligonucleotide were treated to form the sodium salt by EtOH precipitation from 1M sodium acetate.

The single strand oligonucleotides were purified by IP-RP HPLC on Xbridge BEH C18 5 µm, 130 Å, 19x150 mm (Waters) column with an increasing gradient of B in A. Mobile phase A: 240 mM HFIP, 7 mM TEA and 5% methanol in water; mobile phase B: 240 mM HFIP, 7 mM TEA in methanol.

The single strands purity and identity were assessed by UPLC/MS ESI- on Xbridge BEH C18 2.5 µm, 3x50 mm (Waters) column with an increasing gradient of B in A. Mobile phase A: 100 mM HFIP, 5 mM TEA in water; mobile phase B: 20% mobile phase A: 80% Acetonitrile (v/v).

Sense strand were conjugated as per protocols provided in any of examples 1, 3 or 5.

Sense and Antisense strands were then annealed in water to form the final duplex siRNA and duplex purity were assessed by size exclusion chromatography.

### Example 8: Nucleic acid sequences

siRNA oligonucleosides according to the present invention target ZPI. The full DNA sequence of the ZPI target gene is as follows (SEQ ID NO:236):

AATGTGGGTTGGAGCCCCCATACAGAATCTCTATGGGGGCACTGCCTAGTGGAGCTGTGAGAAGACG
GCCACCGTCCTCCAGACCCCTGAATGGTAGATCCACCGACAGCTTGCGCCATTTATCCGGAAAAGCCA
CAGACACTCAACGCCAGCCCGTGAAAGCAGCCAGGAGGGAGGCTGTACCCTGCAAAGCCACAGGGGC
AGAGCTGCCCAAGACCAAGGGAAGCTACCTTTTGCATCAACGTGACCTGGACTCAAAGGAGATCATTT
TGGAGCTTTAAAATTTGACTGACCTGCTGGATTTCAGACTTGCATGGGCCCTGTAACCACTTCGTTTAG
GCCAATTTCTCCCATTTGGAACAGCCGTATTTACCCAATACCTGTAACCCCATTGTATCTAGGCAGTAA
CTAGCTTGCTTTTGATTTTACAGGCTCATAGGCAGAAGGGACTTGCCTTATCTCAGGTGAGACTTTGGA
TTGTGGACTTTTGGGTTAATGATGAAATGAGTTAAGACTTTGGGGGACTGTTGAGAAGGCATGATTGG
TTTTGAAATGTGAGGACATGAGATTTGGCAGGGCCAGAGGCGGAATGATATGGTTTGGCTCTGTATCC
CCACCCAAATCTCATCTTGAATTGTACTCCCATAATTCCCACATGTTGTGGGAAGGGACCCAGTGGGA
GATAATTTGAATCATGGGGGTGGTTCCGCCATACTGTTCTTGTGATAGTGAATAAGTCTCACAAGATCT
GATGCCTTTATTGGGGGTTTCTGCTTTTGCGTCTTCCTCATTTTCTCTTGCCGCCACCAGGTAAGCAGTG
CCTTTTGCCTCCCACCATGATTCTGAGGCCTCCCCAGCCACGTGGAGCTGTAAGTGCATTTAAACCTCT
TTCTCTTCCCAGTCTCGGGTATGTCTTTATCAGCGGCGTGAAAATGGACTAATACACTGTGGTTATGTA
TTATAGTCATATGATATTTTCATATTTTTGGAAGCTGGGTGAAGGGTAGATGTGGAGACCATGATTTTT
GCAAATTTTTTTAAGTTTAAAGTTATTTCTAAATTAGAAGTTTAAAAAGAAGAAATCACATAAGCCAT
AACACAATAGAAAGATGTCTTTAAAGTTCAAGGCAGGAGGGATGTCTGGAAATCAGCGAGAAATTTG
CACCTGTGTGTGCATGTGCATATGTGTGTGTGTATGTTGCAAGGACTTGGAAAGCCCTTTTTTTCCTAC
CTCTGTACTACTGTGGGGGGAGGCTAAACTTGACTTCTTCCCATCTTAGTTCTTTTTTGGGATAGACTC
CTGTAACAAAAGACAGACAAGAGAAAAATCAGCTTACAACATGGGCCATGCACTTCACACAGGAGAA
ACCTGCATGAAAAGTAACTCAAAATGGTGCCTTAGAACTCCACTTACCTTTAGTAAAGAGCAATAAAT
TAGCAGGAAAATCATGGATCGGGACAAGGGAAGTGGTTTTATGCTTCCAAGGGCAGGAAATCATGGA
AGGTAAATATATGGGAGGAAACTAAAGGAATAAGGCTTGTTTGCATATTCCTCTGATGCCATCTCTGG
GTTGATAAGAGTCTAGAGTCATTTCCAGTAAAGATGAATTTTTATCTGTCTTTAGGAAGAAAGGGGGA
AAGATAGAGAAAACTATTTCTCCATTTGCTGTTTCTTAATTACCTTCAGTTCAAAAATAATTTTTATATC
AGAAAGGCATATTTAGAGGTATGTTAGTTTATTTTCACACTGCTAATAAAGACATACCCAAGACTGGG
TAATTTATAAAGAAAAAGAGGTTTAATGGACTCACCGTTCCACATGGTTGGAGAGGCCTCACAATCAA
GGCAGGTCTTACATGGCAGCAGGCAAGAGGGAGAATGAGAGCCAAGCGAAAGGAATTTCCCCTTAAA
AATCCCCTTATAAAACCATCAGATCTCGTGAGACTTACTCACTACCACAAGAACAGTATGGGGGAAAC
CACCTCTATGATTCAATGATCTCCCACTGGGTACCCCCCAACAACACGTGGGAATTATGGGAGCTACA
ATTCAAGATAAGATTTGGGTGGGGACACAGACAGACCATATCAAGGGGTAACATAGTCTGGTTTCCTT
TACTACCCACCTACCCAAACACCCCCTTCATCTGATCCACACAAAGTAAACTCTTGCAGTTCTCTCACT
GTTTCCTGGAGTCTGCTTTTGGTCTCATAGGACTGCCCTAACGCTTGTTTTTCAGACGTTTAACCCTGTA
GGTCTCTGGACAAATTTGCTTTAGAAGCCCCTCGATGTCGCCCTGAAGAGTGGCTTTCAGAAGTTGTGC
CTCCTGCCTGAGGGGAGTTCCAGGAAGGGTTCTGCATCGCCTATGAGTTTATCTGGATCACCAGAGGC
CTTCCCGTCAGAGCTTTCCCAATCGTTTTTGGCCAAGGAGTGTGAGAAGCTAAAGTTCATAACAACTG

GAAGTCAGACAGCCTGGTCTATTCTGCTTTAACTCTAGCAGGAAAGGCCTTCATGGTGGGGCCTGAAT
ATCTTCCTTTATAAAATCAAAGCCTGGGGACAGGGTTACTTACTTCTGAGGTTCAATCTGGCTCTAAAA
TTATGCAACAAATGCCATTCCTTTAGCACTTCCTTCCTACCGGGCGAGATACTCAACTCCACAGGCACC
ACCTCAGTTCATCCTCTCAGAAGTCCTAACAGCTCAGCCTGGGGCACCCCATTTTACAGATTAGTAAAC
TGAGGCTAAGAGAGGTTAGGTAGCTTGTTCAGGGTCATGCTGCTGGTAAAAGAGCTCAGGCTACAGTG
CTATGCATTGAGTTTTCTCACTTTCCCATCTAACTGGAGGGCTAAAGGTCAAAGAGTGGGCAGCTCCCT
TGTTGGGAGCTGTACAGGAATAATGTCCTCCCTGAAGGAGGGGGACTTCTGAGCCACACCCTGGGGTC
CAGGGCTCACAGCCTTAGGAGCAAAATCGTCCACCCCCTTCCTGGTTCCTCGGTGCTGCAGAGATATT
CATAGGACAGAGTCTGAGTTCTGGCCACTTAACAGAGGAAGAAAGGCTGGCTCGGTGAGGTTAACTT
ACATCCCAGCAGCTAGGAACCGGGAGCAGAGGACCTCAGATTCACACCAGGGCAGGAGGCAATGGCC
TGGCTGAAGCCTTCACAATCTTCCCAATATACTCCGCTGCCTTCCTTTATAAGGATCCATTTCTGAAAC
CCTGTGCCCTGGCCAGGCACGGTGGCTCACACCTGTAATTCCAGTACTTTGGGAGGCCAAGGCAGGAG
GACCACGAGGTCAGGAGTTTGAGACCAGCCTGGCCAATATGGTGAAACCCCGTCTCTACTAAAAATAG
AAAAATTAGCGTGGTGGCAGGCGCCTGTAATCCCAGCTACTCGGGAGGCTGAGGCAGGAGAACTGTT
TGAACCTGGGAGGTGGAGGTCTCAGTGAGCTGAGACAGACAGTGCCTGGGTGACAGACAGAGACTCC
GTCTCAAAAAAAAAAAAAAAAAGAAAGAAACCCTGTGCCCTAAGACCTGCACACTCGCTGGCTCCGC
TCAGACATTTAGCAAAGCAGACACCTTCCCAGGCCTGGAGGAAACAGCCCCTGCTTTTTGGGAATCCA
CAAGCCCGCAGCTGCAGAGCTCGACCTGGATGGGCAGGCAAAGGCTGACTCCTGTGCGTGGTGTGAG
TCCAGCCTGGCCCCTCTACACCCTCACTTTCACCTCTTAAAGAACTGCCTATTAACAGAGCAGGTACTG
CCCAAAAGGAACACTCTGGAAACTTGTTGGGACACTTCTGCCTTTCACAAACGTTTGGGGGGAGTACT
ACTAGCATTTAAGGATTGAGGGTTAGCAATGCCAGACATACCAGAACACGCAGGGCAGTCTCCCATG
ATGAAGAGGCCGCCGGGTTCCCCAGGACTCACATGTCCACCTCAAGTTCACGTGGGATTATCTGAGCC
TAGACTGTCAGTCCTGGGGCTGCTTTATTTCATATAAAAATATAATATTTATCCAAGGTTTTACTACAC
ACTGCATTTTCTGTGAAGACAATGACCGTGTAAATCAGGGAAAGATCTATATTTTATTTTGTTTGAAAC
TTTACCAAGCATTATTTACCATTTCAAAAGCTCTATCCCTGGTAGTACCATTGGTTTTCTTGTTCACCGG
CCAGCAGTGAGCAGCACACAAGCGACCTCCCGTGGGCTCCACATTGGACAGCCTCACTGCACCTGCCC
AGGCCCTTAGGCCACAGCACTGCCATATTCAGGGACACATTATTCTCTTTTATTATGCCTCCATATTAT
CATTACAGCATTATCTTTTTTTTAATTTTGTGGGTAGATTATATTAGCTATACGTTTCACTTCAATGGTA
GTAGTAAGGGGCACATAACAAAATATTTACTTATATATATTAAAAAAGAGAGTCTGAGAAGTCTGAA
AAGTTTTGCCATAAACGGTCTCCACCAGCCTCAACTCTGAGTGCCCGAGGATTCAGTCTCAAGTCCAG
CAACATTGTGAAGCAGGAAATTTACCTTGAAAGGAGCTATGTACTCTAAGTAGTGATTTACCTGTCTG
CCTCCCCCACTGGATTGACCAGTTCCTTGGGGGCTGAGAGAACAGGTCCTGAACATTTCTGCTGTGCCC
CCCAACCCACATCCTCATAGTGTCCAGTACCAGGCTGGGGACTCAGGAAGCATCCATGGGATCCCCCA
GTGCCTTCTTTCTCGAGGTGTTCAGCACCTAGAACAGCTCAAGACAAATTCCCCACACCCCACCCAGA
CAGAGCTGAATCTTACTGGGGCGAAGCCTTGAGTTGCAAGGCAGAAGCTCTCGTGATGGGATTTGGGT
CATATTCCGGGTTATAGGAGGAGCTGGGGAGTATGGGAAGCCTCCCACTTGGTCTTTGGTTTTCCAGA
AACTCCACCATCACAAGCAGGATGTTAATCAGTAACCGTCCCACAGGGGATCATACTTTGGAATAGCA
AATATTTGCTGAAGGTTCTGGGCTGCAAAGCTGAAGCTTTGGTTTCTGCTCTAAATGAAGGACTTTTCC
AGGACCCAAGGCCACACACTGGTAAGAGGCAGTGGGTTACAGGAGACCTTCAATGAGTCTAATCAGG
GAGGGACCGGGAAGGATGGTATCATCCCTGGGCGGGCTCCAACGTGAGGGCTGTGTGGCTGAGCAGT
GCAAAGACCTCCATCCTACACTCCACAGGGACTGTACATACAGATTGGGAGCTGGAGTGGGGTAAGA
GGCGAATTATAGACACAAGGGGCTCCTCTGCAGGAAGGAGGCCAAGGGAAAGAGGCTTGAAAGGCTT
GATATTTCACCCACCACCACTCACTGCCGGAGTAAGCAGGTCTCCCCTTCCCAGGGCTGAGGGGAGGC
AGGGATGTGTGCTGTCCCAGGGCTGAGAAGTGGCAGGTGAGCTGGTGATTCCTTACTGCCCAGGTTCT
GTCTAGGAAGGTGCGTCCTCACCATGCTGGATGGTGTCCTAGTCCAGGAGCACCCCCTGAGCTCCTGG
CCTAGACTCCAAAGGGTTGGGTAGATGAGCAAAGACTTTACAAAGACCTTAGGCGATATATGTCCAGG
AGCACCCAGGAATTACTGGGCTACCACTGCAGACTGCAGGACAAGCTCCAAGAACAGGAAGGTAAGA
CTCAGCATTTGGAGGTGGTGACATCTAGTTGGCGTGCTGGGCTAATTTCCTGACCATTGTACAGGGAG
AAGTAACCTTGAATTCAGGAGTATTCTGTGTGGTCTTAATGTAGAAAGTAGCACTAAATGATGCCACG
TAATCGTTTTAGCTCAGGCTCCTCTAACAAAACACCACAGGCTGGGTGGCTCCAACAGCCATTGATTTT
TCACAGTTTTGGAGGCTGAAAGTCCGAGTCAGGGTGCCAGCGTGGCCGGATTCTGGTAGGGCTGTCTT
CTTGGCTTGCAGATGGCCACCTTCGCACCGTGTCCTCCCATGGAGAGGAGGTGCGGAGGGGGACTCTG
CTCTCTTCTTATGACAGCACTAGTGCTATCACAGGGGCCTTGCCCTCACGACCTCATCTAAACCTAATC
ACCTCCCAAGCGCCCCAACTCTATTGCCATCACAATGGTGGTTCGGGCTTCAACTTATTAATTCTCAGG
GGACACATTCAGTCCATAACAATAAAAGCGTGAAACTGGGCTGCGTTTACACTGAAAGAGCTATTTAC
CCAACGTTTACAATACTTGGGTGACCTGTTGAATGCAGGCTTGCCATTTAGAGTCAAAAAGAGCTTCC
TCAACAGTGTCCTTTGGGAAACACAGTGGAAGTATTTCACTGCTTCTACAGGGGAGAGGGTAGTGCCG
TTCAGACTGCAGAGTGAGGCCCTGAATTCCGGGGTGCCATTCAGCCCGAGCAAGGGGCAACATGCTG
GGCCCTGGCGCTGGAGGCGGTTTTGTCCCAGGCATAGATAAGGACTCAGCCCCTGCATCAGGAAGAG
GCCTGGCAGCACCGCCTGTCAATACATTTTGCCGCAGGTGACCTTGGTCAAGAATAAGGGTCTCTGCT
GATGGGAACTACTGTGAGGCCGGCAGCATCCACCCTGCGCTCACTGGGCTGGGTGGCCTACCCCACCC
AGACCCTCCCAGGGCAGTGGGCCCAGAGAGAGGATGAGGGAGGGCAGGTGTCCCAGGGGTTCTGCCC

AGCCAGCCTCTGGGATCAGGCCTGCAGTGTGGCTGAACACCAGAACTGAGTTTGGACACAGCCAGGT
GGCCCAGGCCAGTCCCAAGCCATGTATTTGGATGGAAAACATGGAAGTATTCAGGAGCCAGGCTCTGT
GTCCAAGGATGTGGAGGGAGCCTAAAAGGCGACAGAGAAGGGGACAGCTAACGGTGAAGAAGTGTA
GCTCCCACACTGCAGCCTAGGACAGTGAGAACCGGCATGCAGCCCAGGTGGCTGAGGGCTCTATGAA
GCCACAGTGGAGGGAGCCCAGAAGTGGGTTGTATGAATTGCGGGGCCTCCTGCTACCCGGGAGCTGC
AGCTATAGGAAGGAAGGAAGGAAGGAAGACCTCCAAGGAACTGTGTAGCAGAGGTGCAGTGCAAAG
AGAATTTTGATAAAAAATCCAGGAAAGCTCCAATACTTTCCCCCTTCCTTGCCTAACGGGCATGCAGG
CACTCCAATCCCCAGCCAAACAGGGCACTGGGCAAGGCCGGCCACCCATCTGGATGGGCAGCCTGAC
GACCAGATGGTCAGGGCAGTGAATGAAGCAGATCAAGGAAAGGTGTGTGAGGACCCCTGATTCCACC
TGCTTGGACCCCCACCTTCTGTGCTGCCTCCTGCTCCCAGAGTGGACTCTCTTGCCCTGGCCCTCAGGG
AGGAGACGGGATGAATGAAAACGGGGTCAGGACTGAGAGCTGCCTGCCGGCCTGGCAGGGAATGGG
AACTGGAGGAGGTTTTGCTCTGTGAAATAATGTCCCCTCTTTGGGTGAGCAAATGTCACCCACACTTGC
TCTAGGTCTCCCTGGGGCAGGGCTAACCTACTTGAGCCACAGGAAGGAGGCAGGGTCCCTGAAGAAG
CTTTTACTATCCACAAAGACATTTTAGGAGGCATTAAAACCATCTCTATCCTCTCCTCTCCACAGGAAG
TCTTGCAGCTGAAGGGAGGCACTCCTTGGCCTCCGCAGCCGATCACATGAAGGTGGTGCCAAGTCTCC
TGCTCTCCGTCCTCCTGGCACAGGTGTGGCTGGTACCCGGCTTGGCCCCCAGTCCTCAGTCGCCAGAGA
CCCCAGCCCCTCAGAACCAGACCAGCAGGGTAGTGCAGGCTCCCAAGGAGGAAGAGGAAGATGAGCA
GGAGGCCAGCGAGGAGAAGGCCAGTGAGGAAGAGAAAGCCTGGCTGATGGCCAGCAGGCAGCAGCT
TGCCAAGGAGACTTCAAACTTCGGATTCAGCCTGCTGCGAAAGATCTCCATGAGGCACGATGGCAACA
TGGTCTTCTCTCCATTTGGCATGTCCTTGGCCATGACAGGCTTGATGCTGGGGGCCACAGGGCCGACTG
AAACCCAGATCAAGAGAGGGCTCCACTTGCAGGCCCTGAAGCCCACCAAGCCCGGGCTCCTGCCTTCC
CTCTTTAAGGGACTCAGAGAGACCCTCTCCCGCAACCTGGAACTGGGCCTCACACAGGGGAGTTTTGC
CTTCATCCACAAGGATTTTGATGTCAAAGAGACTTTCTTCAATTTATCCAAGAGGTATTTTGATACAGA
GTGCGTGCCTATGAATTTTCGCAATGCCTCACAGGCCAAAAGGCTCATGAATCATTACATTAACAAAG
AGACTCGGGGGAAAATTCCCAAACTGTTTGATGAGATTAATCCTGAAACCAAATTAATTCTTGTGGAT
TACATCTTGTTCAAAGGTACTTTGATAATGTTCTGCTCTCCCAAGGCCACAGGGCCCTACGATTGTCTC
TCCCTTTCCTTTCGTTAGGCCAGCATATGATTAACGCTACGTGATTTTCTATGAATGTGTTTTCACGTTT
CAAAAACAGATTGATACACATATTGAACAGTGCCAGACGCTGTCATTTGAGGCCCTTCCCTGGTATCC
TATGTGCTTGTAGTCCTTATTATTTTCAGAGCACTCTACATAGCTCCCCTCTGACACTTAGAAGCATAG
GGTCTTTCCAAAAAACAGGGGGCTGGGGGATTATCTGGGGGATTTAGGATTGCATCATTGCTCCTTCA
TTTTTACTTTTTGACCAACTCTCTGCCCTTAGATTCCTATTATAGAAAATAGGGACACTCCACCTACTAC
AGTGTTAGAGGCTAAATGAGACAATGAATGTAAAGTGCCCAGATGGGCTTGGCACATAGCAGACACT
GAGTATCTATTGTTTACTTGTTCTTCCAAACTGCCAATCAGCAGGTAGAGCAGGAGTTGTCTCCTTTCT
AAAGATGAAACCAGCTCAGAGACGTTAGCTTGATCAAGGTCACACAGTAAGTGGCAGAGGCAAAACC
CAAACAAGGGCCTCCTGACCCCCTGATCCTAGGTTCTGTCCAGCCCTGCCTCCCTAATGGGGCACTGG
ACGTGGGTTGGATGCCACTTTCGCAGAGCTGGCACCAGACTTACAAAGCCCCGGCAGGGGAAGCCAC
TTTACAACCAGCCAGGCCACACCCCCAGGGCAGACGTTTATGTAGAGAGTAATGTACCTGCCTGCTAG
TAGCCTCTGCATTGTGGGGCCTTCTCTCAGAACCACACTAAACAGTGGGTGGGTGAGAAGTGTCACTC
CTGCCACCTTGGACTCTGCATGTGCTTGTGCCTGGTGTGAATGAGACAAAGTGGCAGTCAGAGGTGCC
AGGCAAAGGCTTTTCTCTAAGCTGGAGCCAACTATGAGGGAACGACTGTGAATTCCGTTCAGGTCCAG
GACAATGAGAGGAGCCAGGGATTGTTAGGAAACATTTCCCTGCTTTCGTGTGCGATTCCCAATAGGGC
CTGCGAGTGGAGCTGCATTTTGCTAGCTGGGCTAGAGGACGGGGAAAATTTTGGGGAAATTTATTTTG
CCTGCCTGAGCTGTGGAAAAGCCAACCCAATTAGGGAACGCCTTTCCTAGTTGGAACGAGAAGACGA
GAAGTGAGAGAAGTGAGATAGAAGGCTCCCTCTCTATTATTTGAGCAAGAACAATGCTTTTCAAAGAG
GGAATTTCTGCAATGAGTTCTTCTCTTACTTGTTCAGGGAAATGGTTGACCCCATTTGACCCTGTCTTC
ACCGAAGTCGACACTTTCCACCTGGACAAGTACAAGACCATTAAGGTGCCCATGATGTACGGTGCAGG
CAAGTTTGCCTCCACCTTTGACAAGAATTTTCGTTGTCATGTCCTCAAACTGCCCTACCAAGGAAATGC
CACCATGCTGGTGGTCCTCATGGAGAAAATGGGTGACCACCTCGCCCTTGAAGACTACCTGACCACAG
ACTTGGTGGAGACATGGCTCAGAAACATGAAAACCAGGTACAACTCTTGCCCACACCCTATACAAACT
CTACCTTTCTGTACTGGCAAACGCTCAGCACAATTTCATTGAATGCACCGTGATTTAATGTCTCCTCCA
GTGAGCTATAAGTTTCCTGAAGGCAGGGCAGCATTTGTCTTTTTTTCCACTCTATCCCCAGCATCTGTC
ACAGGGTGCCTGGCTGATTCATTCATTGAGTCCATCAGTATTTTACGTTCTGCGACTGTGATAAATATA
TGATGCCAGGGATCCATCAGCAAACAAAACAGGCAAAATTAGTCTGCCCTCATGCAGCTTACATTCTA
TTGAAGGAAGACAAAGAGTAAATTAAAAATAGGTAATAATGCAGGGAAGGGGACAAGAAGCATCAT
CAGGATGCAGATGGAGGTTAGACAAGGCCTCTCCAAGAAGGTAACAGTAAGCAAACATCTGAAGATG
AAGGATAAACCATGTGGATATATTCGGGGAGAGAAGTGTTATGTTACAGGCAGAAGTGTACAAGTTCT
GGGATGGGAGTGTACCTGGTGGGTTTGAAGAACATCAAGGAGACAAGTGTGGCTTCAGCAGTTGGAG
ATAAAATCAGAGAGGAAACAGGGGCCCAGTCCCCAGAAAAGACTTGGGCTTTCCTGAGAGAGGCAGG
AAGCCACTGGATGGTTCTGAGTAGAGGAGCAACCTGATTTTGACTTCTGTTTTTAAAGGATCACATAA
GCTCCTGTGTTGAGAAAAGACACTAGGGGGTAAGGATGGAAGCAAGGGAGAGTGGTTAGAAAGTTAC
TAGCAATCCAGGTAGAGATGCTGCTACCTGGACTGCGGTGGTGGTAGTGGAAGTGGTGAGAAGTGGC
TGGATTCTGGATCTATTAGGAAGTGCAGGATCTGCTAATCGATTGGATGTGGGTGAGAGAGGTGTCAA

AGGTGATCACAAAGTTTTTGGCCTTAGCAACTGGAAAGACGGATTTGCCATTTACTGAAAGGGGGAGG
AACAGGTCTGGGGTAAGTGCAGAAGTTCAGTCTTAAACACTTGGATCAGAAATATCTATTAGACATCC
AAGTTGAGATGTCAAGACGACAGGTGGATCTGGAGTCTAGGGTGAGGTCCAGGCCGGAGATATAAAT
TCGGTCATCAACACAGAACTAGAATCTAGACACATGACAGGGTTGGGGTCTGTAAATATAGAGGAGA
GGAAAAGAAAGCACAGAGTGGGCACTGAAATGTCTGCCCAATAAATTAATCCACCTATTGGAGTACA
AGGAAAATGGCTGCAATACGAATTCCATGGCTATGGCTTCTGAATCCTGTGACTCAGATTTTGGCAGA
CAAGTGCAGCTAAAGGTCCCCAGGGTTAGTTTTATCTTCATTATTCTTCTTTCATTTTTCTTCATATCTT
TAGCACCTAACAATGAACCCCAAACATCATAAGCCCTCAAGTAATGTTTGCTGAATGAATAACTTTTT
AAATTAATCTTCAAGACACGTCATGTCCTCAATTATTTTTAAATAAATAAAAAAATTTTATTTTGAGCC
ACAGAACTCATCTTTTCAAGCAACATATTTTCAAAGGAGGACTCCAGTATACAAAATAGATGGTATCA
GAGCTTCTCTGGCTAAAGACGGGTAGGGGTTGAAAGTTTTCTTTGCTCCCCTCCCCATCCATCCCCAGA
CTCCTCGGGTCTGCAGAATCCAGGAGCTGAAAACAGCCATCATCCAGGAGGCTGCAGGACTGCTGAA
AGCAGCTGTTAACTCAGGTTTTTTTTAAAATATAGGGAAATGAACACATAAGTACTTTGCTAAAGAAA
ACGTGAGTCACTGGCTGAGGAATAAAACTCATTCACTGAAGCTGAAGTACTATTTGATAAGCTAGAAA
TATTTTCCCTGAGTAGACCACTGTAAAAGAATGGCATGAACTACATAGTCAACTGAAAGACTCATTAA
TGGAAATAATCTTAAAGAACAAAAATTGTGACCTTTTTGGTGTCCACAGACTAGGGCTTTGTCTACATT
TCACCATCATCTGTTCTTGTACCACAGAAACATGGAAGTTTTCTTTCCGAAGTTCAAGCTAGATCAGAA
GTATGAGATGCATGAGCTGCTTAGGCAGATGGGAATCAGAAGAATCTTCTCACCCTTTGCTGACCTTA
GTGAACTCTCAGCTACTGGAAGAAATCTCCAAGTATCCAGGGTAAGTCAGGATCTTTCATCAGAGCCC
AACCTCAGCATGAAATGTCACCAAAACAAATGCTTTTACAAACCATTTAACTTTGATAAAATACCTAA
TTGTAGTGGAAAATTAGATTTAAGTCCCAAATACTTGAAATAGCACCCAGGTTGGATGTTTTAAGAAT
TTCAAGCAACTTCATTAAAATAACTTTTCAACTAATTTATTTTAAGCAGACCTCTCCCCCTCTGCTTAA
AGTGCTCAGGGAGAAATTTGACCCTGAAATAGAACTGGTTTACAGAGGCATCATCATTTATGTTGAAT
ACAACTTGAATAGTTCATGAAATTACACCACCTTTACAATGAAACAAACCCCTAGACATCATCTAGCC
CAACTTCTCCCTCCTTGTGGAAATCCCCTCCATAGCCCTACGAAATAGCCCTCCAACTTCTCTTCCTCTT
CATGCTTCCAGTGACATCAAACTCACCATTTCTTTGAAGAGCTGCCCAATCCACAAATAGCTAAAATT
GTTATATGTATATATATATATGTGTGTATATATATGTATATATGTATGTGTGTATAAATGTATATGTGTG
TATATGTGTGTGTGTATATATATATACACACACATATATATATATATGGAGAGAGACATACATATATAT
ATGGAGAGAGAGAGAGAGAGAGTCCTGTAACTTCTGATTCATACTTTTTGGTCCTAGTTCTATCTCTAA
AACTTCTAAGAACAAGTTTAGTCACCATCCACATAGAATCCCTTCAGTTACTCAGTGTTTCTCAGTGGA
AGGGTTCTTGGTTTTGAGGGGAACTGCTTGTTGTCCAGAGCAGTTGTGCATGTTGCAGGGAACTGGTT
AGCATTGCTGGCCCATGTTCACTAATGCCAGTAGGAAACTCCAGTCATCACTATAAAAATGCTCCCAC
ACATTTCCAAATGGCAGCTACATCTCTCTACATTCTTCCTTAGCTGTGTGGTTTAATATTTTCTTATACA
ATTGCAATTTTCAATTCCAAGAGAGACTAAAAATGGCATCCACTTAAGTAGGACACAGTAGGGTAACT
GTGGCCTGGAATCAGGTCTTACAACCTCAAGAGAGGTAAGACAATTAAATAAAACAATCCGTCAGAC
CAGCACCTGAAAGTGTTTCTGCTATGAACACATGAAAAACTGAAATGCGCTGCTGCTTTATGAAGGGT
CATCATGAAATTTAAACTGTAAATGATTAAATATTCTCCCTCTGTTTGCTCTGGGGAATTAATTTTCCTC
TAGGAAATCAGGGAATTTCCTGGAGTGAAAATCAGTGTAATTACATGTTATGTTTTCATTATCTCTTAT
AACACAGTAATTATATAGGTACATCACTCATATCACATCTTGTTTCTGTAAAAAAGGGCCTCCCAAAC
ATAGCAAGCAGCCACAGTATAGGCAGCCAGAATTCAGGAAGGCTCCAGGGACCCCTGGGCTTGGCCC
AGAAAAATGCCTCAGAGTAGTACCAGGTGCTGGGAAGCTGCTACAGAAGACTAGCCATTCCCTGCCTC
CACCTTGCCTGCCAAAAGGAAAGTCAGAGGACTCAAGGGATCCAGGGATCAAGGGATCCAGGCAGCT
TGAAAACCTTTTAGGAGCACCAGCTCAGCTCAAGAATTAGTAGCATAAATTACATGCTCAATAAAGAT
TTGATGCATGAGTGCATCCTGAGTCCATGCCCGGAATGTGTTTCACATATTCCACAATACTTCACATTG
GGTTCCTGAGGTCTCCTGGTATTGTTTAAGACTCCTGTGGCAGTCCCTGGTGCAACCCCAGACCACTCC
TCTTAACGTAGATGGGCCTGCTCCACTAAATCCCAGGAGCATGACCCCATGGGTAGGACCACTGTGAA
GAATTTCAAGGGGCTCATTTAATTCCTCCTTTGCACTGCCACACAAATGGTTTTTCACATTATTTCCTTT
TTCCAGGTTTTACAAAGAACAGTGATTGAAGTTGATGAAAGGGGCACTGAGGCAGTGGCAGGAATCTT
GTCAGAAATTACTGCTTATTCCATGCCTCCTGTCATCAAAGTGGACCGGCCATTTCATTTCATGATCTA
TGAAGAAACCTCTGGAATGCTTCTGTTTCTGGGCAGGGTGGTGAATCCGACTCTCCTATAATTCAGGA
CACGCATAAGCACTTCGTGCTGTAGTAGATGCTGAATCTGAGGTATCAAACACACACAGGATACCAGC
AATGGATGGCAGGGGAGAGTGTTCCTTTTGTTCTTAACTAGTTTAGGGTGTTCTCAAATAAATACAGTA
GTCCCCACTTATCTGAGGGGGATACATTCAAAGACCCCCAGCAGATGCCTGAAACGGTGGACAGTGCT
GAACCTTATATATATTTTTTCCTACACATACATACCTATGATAAAGTTTAATTTATAAATTAGGCACAG
TAAGAGATTAACAATAATAACAACATTAAGTAAAATGAGTTACTTGAATGCAAGCACTGCAATACCAT
AACAGTCAAACTGATTATAGAGAAGGCTACTAAGTGACTCATGGGCGAGGAGCATAGACAGTGTGGA
GACATTGGGCAAGGGGAGAATTCACATCCTGGGTGGGACAGAGCAGGACAATGCAAGATTCCATCCC
ACTACTCAGAATGGCATGCTGCTTAAGACTTTTAGATTGTTTATTTCTGGAATTTTTCATTTAATGTTTT
TGGACCATGGTTGACCATGGTTAACTGAGACTGCAGAAAGCAAAACCATGGATAAGGGAGGACTACT
ACAAAAGCATTAAATTGATACATATTTTTTAAGATGTTTGTGCAATCTGTCTGGTATTTTAAGCTTGTTT
CTAAGAACCTTAGTTACTTGGCTAAAGACTAGCTGGGTAGAATATCTTTTCTCTGTTGCTCACATATTT
TCATTTTTAAAAAGTTGCAGATGAGAACACTATGTCAAGATAAAGCCTTTGGGAGGAACACATGTAAA

CATTCTCCTTGAGTCATGTGCTTCTCTCTCTTTCCTTCTCTCTGGTGCAAAATAAGTGTTTTATTTTAATC
TATTACGGAGTCATTTCTTGCTGACTGACATCAGAAGAAAATAGCTCTAACCAGTCCTGATCACAGCA
TCTGCTTCCATGGTGCATCAAATCGCTTGGCAGAGGCATTGGCTGAATCACAGATCATCTAGTTCAATA
CCTTCATTTTACAAAGGAAAGAAAGAGGGACCCAGAAACAGGTCCATATTCTTACTTTCATGGGCCCT
AGGCACGTTTAACCTTGTAGACTCCTCCTTCCTTCATGAAGATATATATGTTCTATGGCTGCATTGGTA
GAAAGATGAATATATTCGTCTTTCAAAGTTGCATATCTAGCTTCAAAGTTATATGTCTAGCATATGGCA
ATAAGCAAAACACCTTCATGGGCCCTTACAGTACTGTCAGCCTTGGGCACTGTGTCTTCTGCATCTAGT
GGATAAGTCATACCTTATATACCAGTGGGAACAAAATACTTGTCCAAGGTCTTCCAGTGTGGCAATGG
CAGAGTCAGAAGCCTACCTTTCCTGAGTCTAGTCTCCAAGCCCTTTTTACTCTTCCTTCCATCTAAAAC
ATCTGATGGGGACCAGGTAAACAGCATGCACTACAGCTACCCATGGGGGTTAAACAGAATATAAGCA
TGAACTTTGTCCCAGGGTGAAAAGGAAAATCGTAAATATCCCTGATCTTCCTTAGGCAGTTATTTTCTG
TCACAGAAACAGAAAAGACTATATTCAGAGAATCCTGAATAGAGCTGATTTACAGTGTGAACTATGTT
AACTAAATGCCTAATTGGATTTCTGTCTGTCTGCTATCTAATGTTTAAAAAAACCTAAAATTCATTTAT
TGATTAGTTGTTTAATATAATTCAGAGTAATGTGAATAGGTAATAATATTAATATGCAGTCTAAATACT
GACTTTTCATCATTCCATAACCTGGACTGATGAAAAGTCAGTATTTAGACTGCATATTAATAAAATAA
AATTCATTCCTGTATTCATTCCAAGAGTACTAATTGACACTTATGAAGGGACAGGCAATTCTAGGCCCT
AGAGGGCCAAAGACAGAGGACTAACTCTATCTGACATTCTTAAGTCACCTTGTTTGTGTTCAATTAGTC
AGATTTGTTTGTGGAAAAATAGTAGAAAGAGGAATAAAGTAGCATCCAGTCCAATTTCCCACTTTTAA
GAGATGAAATCTGGAAAAATAAGTCTGTGAGAGCACAATACTCACTGAAATCAATATGGCCAAACCC
AGTAATAAAAAGGTACATTATTATTGAAGGATTCATATAGCATGCAGATAAAAAACTCCTGCCTTCTT
CCCACCACATACACTGCAAAGCAACAACAGCATAATAATTGTATTTAATATACTACTCTTTAAGGTAG
AAAATGGACCTATTCTATATTTTAAATATACTTTTTAATGTTCCCTCACATTTGCTTTAAGAAGTTCCTA
AGACACTCAGTTTCAGATTTCCCAAGTACACAGGCATGACAGAAAAACGCAGACCAATAAAAAATGT
AACTTACCTTACACAAATACATACACACAAATTCAGGGTTTCCAACCGAGCGGGGGAAATCTTAACAT
TGTAGAAGTCTTCACTATATATGTGTCGAGTTTTTGTTTTTGTTTTTGTTTTTGTTTTGAGACAGAGTCTT
GCTCTGTCACCCAGGCTGGAGTGCAGTGGTGCGATCTCAGCTCACTGCAACCTCCACCTCCCGGGTTC
GTGCCATTCTCCTGCCTCAGCCTCCCGAGTAGCTGGGACTACAGGCACCTGCCACCACGACCGGCTAA
TTTTTTGTATTTTAAGTAGAGATGGGGTTTCACTGTGTTAGTCAGGATGGTCTTGATCTCCTGACCTTGT
GATCTGCCCGCCTCGGCCTCCCAAAGTGCTGGGATTACAGGCATGAGCCACCACGCCCGGCCAAGTGT
CGAGTCTTAAAAATTGTTCCTACACAGACACACTCAACCACACGTTCTCACATATATATGCTGTAACA
ACTGAGAACAGGTTACTGACTTAATCTAATTCATTCTATCTTCATTGTAAAACTTCCACTCCAGCTGAA
GAGCCTGTTTCATTTCAATTCAAAGATTTCTCATATATCCACTAATTGTATGGCAAAACTGACTCATCT
CCAGACTAAGATATTCAAGCTCAGGAAGTCAAATAATAGAAATGATTTTTTAAATGTGTAAGAGGTTA
TAAAGAAAAACTTTATGTGCTCCTTATTTAACCTCTATTAAGTAAAATCCTTTATAGACCTATCTCCAT
TTCTGCAGTAAAAGTGAGCTCTACAGTTAGCTTGTAAGGCTAACTAGTGAAATTCCTGGACTTGTTCTT
AAAAATGCAAGTTTTAGTAATTAACAAAATGATGATGAAGATGTCCCTTTTCCCTACAACTACAGATG
GAGGGAGATTTTTCTTTGCCATACAACTAGCTTAAAGGATTAATTTGATAAGTTGTTAAACTGAGAACT
TTCACAAAAGTATCCATCTTGTTTTTGATATAAATGGAGATACATGTAGTTATTCATAACTGTCAGTAA
TTTGCTGTTTATCCTGTTTCTATATATCTGTCCTTGAGAGTATAATTTTAATAAATATTTCAAAGATTTT
AGGAAATGTCATGTTCTGTTAAAAAACTTCCAAAAGTAATTTTGATGAACAGTTTTGATAACTTAGTAC
TAACTAGGACTAAGACTGCAATTGACTGCTCTACATTCCTGAACTTTATAAGCAGTAGTTGTTTCTCTC
TGTCAAATCAGTGTCCCCTTTTCCCATTTGCATCATGGGAAAGTGAAACCTTATAATTCTGCTAAATTT
ATTATAACAAATACATTGAAATTCTCCATTTTATTAAATTAATAGAATGTTATGAATCAAAGCACCAA
AAAAACTGATGCAATTTTGATGTCTCGTTCTGTACCACATTCTCCAGATCTTAATATATTCAGTTCCAC
ATTATTGGTGCTAGTAGGAGACATAATGAAAACAGTTAAATGAAATCCACAGCGAGTATACTGATTAA
CCAGTACTGTCAAATTTCTCATACCTATTGAATTTTAACTACTGACAAAATGAGCAGTAACAATTCCAT
TTACCTGATTGTCCTTTGGCAAAGGATATTATTAAGAATCACTAAAAATAGCCATAAAGAAGCCATAT
GGAAGGAAGAAGGAAAACAAATGGCATGAAAAGGTCTCTCACTGAGTAACTATGCTCTTATAGTTGA
CGCTGGTATATTTCTTTTATTCACTACCTAAAAATGAACTATCTTACTCTTTAATTATAGAATAAAAAC
TGCAGGAAAGTATTTAAGACTTTTTTTCACAAACACAGGTATCTCATTAACCTATGTTTTATTTTGAGT
AAATTCATTATTCATTATTTCACATTATAAAAAGTAACCACACATACATATGCATTCACAAATTAGATC
ATCTTTATCATACATCAATATATTTTAAAAAACAAATATCTTCTAATATCAATATAGTTATATGCTGAT
TGCATTTTGAAATAGAGAAGCTGACAATAGCTTCACACGGTATATCTCAAGAACTGACAGTTTAAAAT
TAAGAACTGTATATATTCCACAGGCAAATTTTGATGGAAATATTAGCATTAGTACAAATAAATGCTGT
TGACATAGCTTAAGCATGATAGCTTGGAATAACAGCTGATTCAGACTAGATTCATCATTTTAAATAAA
GACAAGTACAATCTAAAATGTAAACAAAGTATTTATAAAATAAATTCTCTAGGAAATAAAGAAAATC
ATCAATCTATTATTTTTAAGGTATTTATAGCTCAAAGTTACCAGAAATCTTTGTGGAATTTTCACTGCC
AAATTTAAATTTGGGAATGTCCGGGTACAACATATTGTCACCACAATCCGGAGGGCCGCCAAAATCGC
AGACGGCTATTTGCATCCTTTCAGTGTGACTTTTCAAGTGGGCTTGGAGACTCATGAGAAAATGCAGT
ATCTTTCTCACCTTCCAAGTCCCCCTCCAAGTGCTTATCAAGCTAGGACAATTCAGCTGATGTAGACTT
TCATACGATTTTTAAATGCTAAAACTCTAGAACAATTAAATGGCTGGTTTCCTGCACAAATAAATGCA
GACTTGTCTCTTTTGCAGCAGTGGTTAAAGCACATTCCTAGAGATGTTTTTCATTACACTTCACTATAA

CATTGGAATTCCGTAACCACATTATTACTCAAGAAATATATATTATACCTCCTAGGGAATCTAATTTGA
AATATGAAAAGTTTAACATCAGCTGTCATTATGTCTCTCTTTCTGCTCATTAACAACAACAAAAAAAA
AAACCCAAAATTTAAAAACAAAGCCCCAGCCACTGCTTTAGCTTTTGTGTACCAATCACATTATCTCCT
GCTGCCTTTGTTTTGCCTCCTTCATCAAGCAGTTGATTTAAGGATTGGATTTTCTGGATTTTCTTTGGGA
AGAAAGAAATGAAGGAAGAGAGGGAGGGTGGGGAAGGAGGGAGTGAGAAAGGGAGAAAAAGAAAA
AAATATGAAAAATGTTATTCATATAATGTGTACAAAGTAAATTAAAAATATATAGATACTCTACTTTG
AATAATTCTAATATATGAGAAGT

Following Table 1 provides oligonucleoside mRNA target sequences of ZPI, together with the corresponding positions in transcript ENST00000261994.9. It is to be understood that SEQ ID NO: 1 to 47 refer to human (Homo sapiens) mRNA sequences.

**Table 1**

| SEQ ID NO | Oligonucleoside mRNA target sequence 5' → 3' | Starting position on ENST00000261994.9 |
|---|---|---|
| SEQ ID NO: 1 | ACCUCUGGAAUGCUUCUGUUUCU | 1412 |
| SEQ ID NO: 2 | AGUUCAAGCUAGAUCAGAAGUAU | 1146 |
| SEQ ID NO: 3 | UUGUGGAUUACAUCUUGUUCAAA | 825 |
| SEQ ID NO: 4 | ACCUUAGUGAACUCUCAGCUACU | 1227 |
| SEQ ID NO: 5 | CCUGAAACCAAAUUAAUUCUUGU | 806 |
| SEQ ID NO: 6 | UACUGGAAGAAAUCUCCAAGUAU | 1246 |
| SEQ ID NO: 7 | UUCCCAAACUGUUUGAUGAGAUU | 780 |
| SEQ ID NO: 8 | CUUGUGGAUUACAUCUUGUUCAA | 824 |
| SEQ ID NO: 9 | CAGGCAAGUUUGCCUCCACCUUU | 945 |
| SEQ ID NO: 10 | AAAACCAGAAACAUGGAAGUUUU | 1115 |
| SEQ ID NO: 11 | GGCUCAUGAAUCAUUACAUUAAC | 738 |
| SEQ ID NO: 12 | ACCAGAAACAUGGAAGUUUUCUU | 1118 |
| SEQ ID NO: 13 | UCUCCAAGUAUCCAGGGUUUUAC | 1258 |
| SEQ ID NO: 14 | AAACCAAAUUAAUUCUUGUGGAU | 810 |
| SEQ ID NO: 15 | GAAACCAAAUUAAUUCUUGUGGA | 809 |
| SEQ ID NO: 16 | AUCCUGAAACCAAAUUAAUUCUU | 804 |
| SEQ ID NO: 17 | UUCUUGUGGAUUACAUCUUGUUC | 822 |
| SEQ ID NO: 18 | UCUUGUGGAUUACAUCUUGUUCA | 823 |
| SEQ ID NO: 19 | AGAACAGUGAUUGAAGUUGAUGA | 1283 |
| SEQ ID NO: 20 | AUUAAUUCUUGUGGAUUACAUCU | 817 |
| SEQ ID NO: 21 | ACUCUCAGCUACUGGAAGAAAUC | 1237 |
| SEQ ID NO: 22 | ACCAAAUUAAUUCUUGUGGAUUA | 812 |
| SEQ ID NO: 23 | CCUCCACCUUUGACAAGAAUUUU | 957 |
| SEQ ID NO: 24 | AUCAUUACAUUAACAAAGAGACU | 747 |
| SEQ ID NO: 25 | AAUUCUUGUGGAUUACAUCUUGU | 820 |
| SEQ ID NO: 26 | GAACAGUGAUUGAAGUUGAUGAA | 1284 |
| SEQ ID NO: 27 | AUUCUUGUGGAUUACAUCUUGUU | 821 |
| SEQ ID NO: 28 | CCAGAAACAUGGAAGUUUUCUUU | 1119 |
| SEQ ID NO: 29 | UUAUCCAAGAGGUAUUUUGAUAC | 671 |
| SEQ ID NO: 30 | AUCUCCAAGUAUCCAGGGUUUUA | 1257 |
| SEQ ID NO: 31 | UCCUGAAACCAAAUUAAUUCUUG | 805 |
| SEQ ID NO: 32 | ACUGGAAGAAAUCUCCAAGUAUC | 1247 |
| SEQ ID NO: 33 | AACCAAAUUAAUUCUUGUGGAUU | 811 |
| SEQ ID NO: 34 | AAACCAGAAACAUGGAAGUUUUC | 1116 |
| SEQ ID NO: 35 | AAUUAAUUCUUGUGGAUUACAUC | 816 |
| SEQ ID NO: 36 | UCAUGAAUCAUUACAUUAACAAA | 741 |
| SEQ ID NO: 37 | UAGAUCAGAAGUAUGAGAUGCAU | 1155 |
| SEQ ID NO: 38 | CUGGAAGAAAUCUCCAAGUAUCC | 1248 |
| SEQ ID NO: 39 | UAUCCAAGAGGUAUUUUGAUACA | 672 |
| SEQ ID NO: 40 | CCAAAUUAAUUCUUGUGGAUUAC | 813 |
| SEQ ID NO: 41 | GAUCAGAAGUAUGAGAUGCAUGA | 1157 |
| SEQ ID NO: 42 | UGACCUUAGUGAACUCUCAGCUA | 1225 |
| SEQ ID NO: 43 | AAAUUAAUUCUUGUGGAUUACAU | 815 |
| SEQ ID NO: 44 | AAAUCUCCAAGUAUCCAGGGUUU | 1255 |
| SEQ ID NO: 45 | AACAGUGAUUGAAGUUGAUGAAA | 1285 |
| SEQ ID NO: 46 | GGAGACUUCAAACUUCGGAUUCA | 361 |
| SEQ ID NO: 47 | UUAAUUCUUGUGGAUUACAUCUU | 818 |

Table 2 provides the unmodified first (antisense) and corresponding unmodified second (sense) strand sequences for siRNA oligonucleosides according to the present invention, together with the corresponding positions in the overall gene sequence of SEQ ID NO:236 as follows.

**Table 2**

| SEQ ID NO (AS) | First (Antisense) Strand Base Sequence 5' → 3' (Shown as an Unmodified Nucleoside Sequence) | SEQ ID NO (SS) | Second (Sense) Strand Base Sequence 5' → 3' (Shown as an Unmodified Nucleoside Sequence) | Corresponding positions on ENST000002 61994.9 |
|---|---|---|---|---|
| SEQ ID NO: 48 | AGAAACAGAAGCAUUCCAG AGGU | SEQ ID NO: 95 | CUCUGGAAUGCUUCUGU UUCU | 1412-1435 |
| SEQ ID NO: 49 | AUACUUCUGAUCUAGCUUG AACU | SEQ ID NO: 96 | UUCAAGCUAGAUCAGAA GUAU | 1146-1169 |
| SEQ ID NO: 50 | UUUGAACAAGAUGUAAUCC ACAA | SEQ ID NO: 97 | GUGGAUUACAUCUUGUU CAAA | 825-848 |
| SEQ ID NO: 51 | AGUAGCUGAGAGUUCACUA AGGU | SEQ ID NO: 98 | CUUAGUGAACUCUCAGC UACU | 1227-1250 |
| SEQ ID NO: 52 | ACAAGAAUUAAUUUGGUUU CAGG | SEQ ID NO: 99 | UGAAACCAAAUUAAUUC UUGU | 806-829 |
| SEQ ID NO: 53 | AUACUUGGAGAUUUCUUCC AGUA | SEQ ID NO: 100 | CUGGAAGAAAUCUCCAA GUAU | 1246-1269 |
| SEQ ID NO: 54 | AAUCUCAUCAAACAGUUUG GGAA | SEQ ID NO: 101 | CCCAAACUGUUUGAUGA GAUU | 780-803 |
| SEQ ID NO: 55 | UUGAACAAGAUGUAAUCCA CAAG | SEQ ID NO: 102 | UGUGGAUUACAUCUUGU UCAA | 824-847 |
| SEQ ID NO: 56 | AAAGGUGGAGGCAAACUUG CCUG | SEQ ID NO: 103 | GGCAAGUUUGCCUCCAC CUUU | 945-968 |
| SEQ ID NO: 57 | AAAACUUCCAUGUUUCUGG UUUU | SEQ ID NO: 104 | AACCAGAAACAUGGAAG UUUU | 1115-1138 |
| SEQ ID NO: 58 | GUUAAUGUAAUGAUUCAUG AGCC | SEQ ID NO: 105 | CUCAUGAAUCAUUACAU UAAC | 738-761 |
| SEQ ID NO: 59 | AAGAAAACUUCCAUGUUUC UGGU | SEQ ID NO: 106 | CAGAAACAUGGAAGUUU UCUU | 1118-1141 |
| SEQ ID NO: 60 | GUAAAACCCUGGAUACUUG GAGA | SEQ ID NO: 107 | UCCAAGUAUCCAGGGUU UUAC | 1258-1281 |
| SEQ ID NO: 61 | AUCCACAAGAAUUAAUUUG GUUU | SEQ ID NO: 108 | ACCAAAUUAAUUCUUGU GGAU | 810-833 |
| SEQ ID NO: 62 | UCCACAAGAAUUAAUUUGG UUUC | SEQ ID NO: 109 | AACCAAAUUAAUUCUUG UGGA | 809-832 |
| SEQ ID NO: 63 | AAGAAUUAAUUUGGUUUCA GGAU | SEQ ID NO: 110 | CCUGAAACCAAAUUAAU UCUU | 804-827 |
| SEQ ID NO: 64 | GAACAAGAUGUAAUCCACA AGAA | SEQ ID NO: 111 | CUUGUGGAUUACAUCUU GUUC | 822-845 |
| SEQ ID NO: 65 | UGAACAAGAUGUAAUCCAC AAGA | SEQ ID NO: 112 | UUGUGGAUUACAUCUUG UUCA | 823-846 |
| SEQ ID NO: 66 | UCAUCAACUUCAAUCACUG UUCU | SEQ ID NO: 113 | AACAGUGAUUGAAGUUG AUGA | 1283-1306 |
| SEQ ID NO: 67 | AGAUGUAAUCCACAAGAAU UAAU | SEQ ID NO: 114 | UAAUUCUUGUGGAUUAC AUCU | 817-840 |
| SEQ ID NO: 68 | GAUUUCUUCCAGUAGCUGA GAGU | SEQ ID NO: 115 | UCUCAGCUACUGGAAGA AAUC | 1237-1260 |
| SEQ ID NO: 69 | UAAUCCACAAGAAUUAAUU UGGU | SEQ ID NO: 116 | CAAAUUAAUUCUUGUGG AUUA | 812-835 |
| SEQ ID NO: 70 | AAAAUUCUUGUCAAAGGUG GAGG | SEQ ID NO: 117 | UCCACCUUUGACAAGAA UUUU | 957-980 |
| SEQ ID NO: 71 | AGUCUCUUUGUUAAUGUAA UGAU | SEQ ID NO: 118 | CAUUACAUUAACAAAGA GACU | 747-770 |
| SEQ ID NO: 72 | ACAAGAUGUAAUCCACAAG AAUU | SEQ ID NO: 119 | UUCUUGUGGAUUACAUC UUGU | 820-843 |
| SEQ ID NO: 73 | UUCAUCAACUUCAAUCACU GUUC | SEQ ID NO: 120 | ACAGUGAUUGAAGUUGA UGAA | 1284-1307 |
| SEQ ID NO: 74 | AACAAGAUGUAAUCCACAA GAAU | SEQ ID NO: 121 | UCUUGUGGAUUACAUCU UGUU | 821-844 |
| SEQ ID NO: 75 | AAAGAAAACUUCCAUGUUU CUGG | SEQ ID NO: 122 | AGAAACAUGGAAGUUUU CUUU | 1119-1142 |
| SEQ ID NO: 76 | GUAUCAAAUACCUCUUGG AUAA | SEQ ID NO: 123 | AUCCAAGAGGUAUUUUG AUAC | 671-694 |
| SEQ ID NO: 77 | UAAAACCCUGGAUACUUGG AGAU | SEQ ID NO: 124 | CUCCAAGUAUCCAGGGU UUUA | 1257-1280 |
| SEQ ID NO: 78 | CAAGAAUUAAUUUGGUUUC AGGA | SEQ ID NO: 125 | CUGAAACCAAAUUAAUU CUUG | 805-828 |
| SEQ ID NO: 79 | GAUACUUGGAGAUUUCUUC CAGU | SEQ ID NO: 126 | UGGAAGAAAUCUCCAAG UAUC | 1247-1270 |
| SEQ ID NO: 80 | AAUCCACAAGAAUUAAUUU GGUU | SEQ ID NO: 127 | CCAAAUUAAUUCUUGUG GAUU | 811-834 |
| SEQ ID NO: 81 | GAAAACUUCCAUGUUUCUG GUUU | SEQ ID NO: 128 | ACCAGAAACAUGGAAGU UUUC | 1116-1139 |
| SEQ ID NO: 82 | GAUGUAAUCCACAAGAAUU AAUU | SEQ ID NO: 129 | UUAAUUCUUGUGGAUUA CAUC | 816-839 |
| SEQ ID NO: 83 | UUUGUUAAUGUAAUGAUUC AUGA | SEQ ID NO: 130 | AUGAAUCAUUACAUUAA CAAA | 741-764 |
| SEQ ID NO: 84 | AUGCAUCUCAUACUUCUGA UCUA | SEQ ID NO: 131 | GAUCAGAAGUAUGAGAU GCAU | 1155-1178 |
| SEQ ID NO: 85 | GGAUACUUGGAGAUUUCUU CCAG | SEQ ID NO: 132 | GGAAGAAAUCUCCAAGU AUCC | 1248-1271 |
| SEQ ID NO: 86 | UGUAUCAAAUACCUCUUG GAUA | SEQ ID NO: 133 | UCCAAGAGGUAUUUGA UACA | 672-695 |
| SEQ ID NO: 87 | GUAAUCCACAAGAAUUAAU UUGG | SEQ ID NO: 134 | AAAUUAAUUCUUGUGGA UUAC | 813-836 |
| SEQ ID NO: 88 | UCAUGCAUCUCAUACUUCU GAUC | SEQ ID NO: 135 | UCAGAAGUAUGAGAUGC AUGA | 1157-1180 |
| SEQ ID NO: 89 | UAGCUGAGAGUUCACUAAG GUCA | SEQ ID NO: 136 | ACCUUAGUGAACUCUCA GCUA | 1225-1248 |
| SEQ ID NO: 90 | AUGUAAUCCACAAGAAUUA AUUU | SEQ ID NO: 137 | AUUAAUUCUUGUGGAUU ACAU | 815-838 |
| SEQ ID NO: 91 | AAACCCUGGAUACUUGGAG AUUU | SEQ ID NO: 138 | AUCUCCAAGUAUCCAGG GUUU | 1255-1278 |
| SEQ ID NO: 92 | UUUCAUCAACUUCAAUCAC UGUU | SEQ ID NO: 139 | CAGUGAUUGAAGUUGAU GAAA | 1285-1308 |
| SEQ ID NO: 93 | UGAAUCCGAAGUUUGAAGU CUCC | SEQ ID NO: 140 | AGACUUCAAACUUCGGA UUCA | 361-384 |
| SEQ ID NO: 94 | AAGAUGUAAUCCACAAGAA UUAA | SEQ ID NO: 141 | AAUUCUUGUGGAUUACA UCUU | 818-841 |

Table 3 provides the modified first (antisense) sequences, together with the corresponding unmodified first (antisense) sequences for siRNA oligonucleosides according to the present invention as follows.

**Table 3**

| Antisense strand ID | Modified First (Antisense) Strand 5' → 3' | SEQ ID NO (AS - mod) | Underlying Base Sequence 5' → 3' (Shown as an Unmodified Nucleoside Sequence) | SEQ ID NO (AS - unmod) |
|---|---|---|---|---|
| ETX-S00008806 | AmsGfsAmAmAmCfAmGfAfAmGmCm AmUfUmCfCmAmGmAfGmsGmsUm | SEQ ID NO: 142 | AGAAACAGAAGCAU UCCAGAGGU | SEQ ID NO: 48 |
| ETX-S00008808 | AmsUfsAmCmUmUfCmUfGfAmUmCm UmAfGmCfUmUmGmAfAmsCmsUm | SEQ ID NO: 143 | AUACUUCUGAUCUA GCUUGAACU | SEQ ID NO: 49 |
| ETX-S00008810 | UmsUfsUmGmAmAfCmAfAfGmAmUm GmUfAmAfUmCmCmAfCmsAmsAm | SEQ ID NO: 144 | UUUGAACAAGAUGU AAUCCACAA | SEQ ID NO: 50 |
| ETX-S00008812 | AmsGfsUmAmGmCfUmGfAfGmAmGm UmUfCmAfCmUmAmAfGmsGmsUm | SEQ ID NO: 145 | AGUAGCUGAGAGUU CACUAAGGU | SEQ ID NO: 51 |
| ETX-S00008814 | AmsCfsAmAmGmAfAmUfUfAmAmUm UmUfGmGfUmUmUmCfAmsGmsGm | SEQ ID NO: 146 | ACAAGAAUUAAUUU GGUUUCAGG | SEQ ID NO: 52 |
| ETX-S00008816 | AmsUfsAmCmUmUfGmGfAfGmAmUm UmUfCmUfUmCmCmAfGmsUmsAm | SEQ ID NO: 147 | AUACUUGGAGAUUU CUUCCAGUA | SEQ ID NO: 53 |
| ETX-S00008818 | AmsAfsUmCmUmCfAmUfCfAmAmAm CmAfGmUfUmUmGmGfGmsAmsAm | SEQ ID NO: 148 | AAUCUCAUCAAACA GUUUGGGAA | SEQ ID NO: 54 |
| ETX-S00008820 | UmsUfsGmAmAmCfAmAfGfAmUmGm UmAfAmUfCmCmAmCfAmsAmsGm | SEQ ID NO: 149 | UUGAACAAGAUGUA AUCCACAAG | SEQ ID NO: 55 |
| ETX-S00008822 | AmsAfsAmGmGmUfGmGfAfGmGmCm AmAfAmCfUmUmGmCfCmsUmsGm | SEQ ID NO: 150 | AAAGGUGGAGGCAA ACUUGCCUG | SEQ ID NO: 56 |
| ETX-S00008824 | AmsAfsAmAmCmUfUmCfCfAmUmGm UmUfUmCfUmGmGmUfUmsUmsUm | SEQ ID NO: 151 | AAAACUUCCAUGUU UCUGGUUUU | SEQ ID NO: 57 |
| ETX-S00008826 | GmsUfsUmAmAmUfGmUfAfAmUmGm AmUfUmCfAmUmGmAfGmsCmsCm | SEQ ID NO: 152 | GUUAAUGUAAUGAU UCAUGAGCC | SEQ ID NO: 58 |
| ETX-S00008828 | AmsAfsGmAmAmAfAmCfUfUmCmCm AmUfGmUfUmUmCmUfGmsGmsUm | SEQ ID NO: 153 | AAGAAAACUUCCAU GUUUCUGGU | SEQ ID NO: 59 |
| ETX-S00008830 | GmsUfsAmAmAmAfCmCfCfUmGmGm AmUfAmCfUmUmGmGfAmsGmsAm | SEQ ID NO: 154 | GUAAAACCCUGGAU ACUUGGAGA | SEQ ID NO: 60 |
| ETX-S00008832 | AmsUfsCmCmAmCfAmAfGfAmAmUm UmAfAmUfUmUmGmGfUmsUmsUm | SEQ ID NO: 155 | AUCCACAAGAAUUA AUUUGGUUU | SEQ ID NO: 61 |
| ETX-S00008834 | UmsCfsCmAmCmAfAmGfAfAmUmUm AmAfUmUfUmGmGmUfUmsUmsCm | SEQ ID NO: 156 | UCCACAAGAAUUAA UUUGGUUUC | SEQ ID NO: 62 |
| ETX-S00008836 | AmsAfsGmAmAmUfUmAfAfUmUmUm GmGfUmUfUmCmAmGfGmsAmsUm | SEQ ID NO: 157 | AAGAAUUAAUUUGG UUUCAGGAU | SEQ ID NO: 63 |
| ETX-S00008838 | GmsAfsAmCmAmAfGmAfUfGmUmAm AmUfCmCfAmCmAmAfGmsAmsAm | SEQ ID NO: 158 | GAACAAGAUGUAAU CCACAAGAA | SEQ ID NO: 64 |
| ETX-S00008840 | UmsGfsAmAmCmAfAmGfAfUmGmUm AmAfUmCfCmAmCmAfAmsGmsAm | SEQ ID NO: 159 | UGAACAAGAUGUAA UCCACAAGA | SEQ ID NO: 65 |
| ETX-S00008842 | UmsCfsAmUmCmAfAmCfUfUmCmAm AmUfCmAfCmUmGmUfUmsCmsUm | SEQ ID NO: 160 | UCAUCAACUUCAAU CACUGUUCU | SEQ ID NO: 66 |
| ETX-S00008844 | AmsGfsAmUmGmUfAmAfUfCmCmAm CmAfAmGfAmAmUmUfAmsAmsUm | SEQ ID NO: 161 | AGAUGUAAUCCACA AGAAUUAAU | SEQ ID NO: 67 |
| ETX-S00008846 | GmsAfsUmUmUmCfUmUfCfCmAmGm UmAfGmCfUmGmAmGfAmsGmsUm | SEQ ID NO: 162 | GAUUUCUUCCAGUA GCUGAGAGU | SEQ ID NO: 68 |
| ETX-S00008848 | UmsAfsAmUmCmCfAmCfAfAmGmAm AmUfUmAfAmUmUmUfGmsGmsUm | SEQ ID NO: 163 | UAAUCCACAAGAAU UAAUUUGGU | SEQ ID NO: 69 |
| ETX-S00008850 | AmsAfsAmAmUmUfCmUfUfGmUmCm AmAfAmGfGmUmGmGfAmsGmsGm | SEQ ID NO: 164 | AAAAUUCUUGUCAA AGGUGGAGG | SEQ ID NO: 70 |
| ETX-S00008852 | AmsGfsUmCmUmCfUmUfUfGmUmUm AmAfUmGfUmAmAmUfGmsAmsUm | SEQ ID NO: 165 | AGUCUCUUUGUUAA UGUAAUGAU | SEQ ID NO: 71 |
| ETX-S00008854 | AmsCfsAmAmGmAfUmGfUfAmAmUm CmCfAmCfAmAmGmAfAmsUmsUm | SEQ ID NO: 166 | ACAAGAUGUAAUCC ACAAGAAUU | SEQ ID NO: 72 |
| ETX-S00008856 | UmsUfsCmAmUmCfAmAfCfUmUmCm AmAfUmCfAmCmUmGfUmsUmsCm | SEQ ID NO: 167 | UUCAUCAACUUCAA UCACUGUUC | SEQ ID NO: 73 |
| ETX-S00008858 | AmsAfsCmAmAmGfAmUfGfUmAmAm UmCfCmAfCmAmAmGfAmsAmsUm | SEQ ID NO: 168 | AACAAGAUGUAAUC CACAAGAAU | SEQ ID NO: 74 |
| ETX-S00008860 | AmsAfsAmGmAmAfAmAfCfUmUmCm CmAfUmGfUmUmUmCfUmsGmsGm | SEQ ID NO: 169 | AAAGAAAACUUCCA UGUUUCUGG | SEQ ID NO: 75 |
| ETX-S00008862 | GmsUfsAmUmCmAfAmAfAfUmAmCm CmUfCmUfUmGmGmAfUmsAmsAm | SEQ ID NO: 170 | GUAUCAAAAUACCU CUUGGAUAA | SEQ ID NO: 76 |
| ETX-S00008864 | UmsAfsAmAmAmCfCmCfUfGmGmAm UmAfCmUfUmGmGmAfGmsAmsUm | SEQ ID NO: 171 | UAAAACCCUGGAUA CUUGGAGAU | SEQ ID NO: 77 |
| ETX-S00008866 | CmsAfsAmGmAmAfUmUfAfAmUmUm UmGfGmUfUmUmCmAfGmsGmsAm | SEQ ID NO: 172 | CAAGAAUUAAUUUG GUUUCAGGA | SEQ ID NO: 78 |
| ETX-S00008868 | GmsAfsUmAmCmUfUmGfGfAmGmAm UmUfUmCfUmUmCmCfAmsGmsUm | SEQ ID NO: 173 | GAUACUUGGAGAUU UCUUCCAGU | SEQ ID NO: 79 |
| ETX-S00008870 | AmsAfsUmCmCmAfCmAfAfGmAmAm UmUfAmAfUmUmUmGfGmsUmsUm | SEQ ID NO: 174 | AAUCCACAAGAAUU AAUUUGGUU | SEQ ID NO: 80 |
| ETX-S00008872 | GmsAfsAmAmAmCfUmUfCfCmAmUm GmUfUmUfCmUmGmGfUmsUmsUm | SEQ ID NO: 175 | GAAAACUUCCAUGU UUCUGGUUU | SEQ ID NO: 81 |
| ETX-S00008874 | GmsAfsUmGmUmAfAmUfCfCmAmCm AmAfGmAfAmUmUmAfAmsUmsUm | SEQ ID NO: 176 | GAUGUAAUCCACAA GAAUUAAUU | SEQ ID NO: 82 |
| ETX-S00008876 | UmsUfsUmGmUmUfAmAfUfGmUmAm AmUfGmAfUmUmCmAfUmsGmsAm | SEQ ID NO: 177 | UUUGUUAAUGUAAU GAUUCAUGA | SEQ ID NO: 83 |
| ETX-S00008878 | AmsUfsGmCmAmUfCmUfCfAmUmAm CmUfUmCfUmGmAmUfCmsUmsAm | SEQ ID NO: 178 | AUGCAUCUCAUACU UCUGAUCUA | SEQ ID NO: 84 |
| ETX-S00008880 | GmsGfsAmUmAmCfUmUfGfGmAmGm AmUfUmUfCmUmUmCfCmsAmsGm | SEQ ID NO: 179 | GGAUACUUGGAGAU UUCUUCCAG | SEQ ID NO: 85 |
| ETX-S00008882 | UmsGfsUmAmUmCfAmAfAfAmUmAm CmCfUmCfUmUmGmGfAmsUmsAm | SEQ ID NO: 180 | UGUAUCAAAAUACC UCUUGGAUA | SEQ ID NO: 86 |
| ETX-S00008884 | GmsUfsAmAmUmCfCmAfCfAmAmGm AmAfUmUfAmAmUmUfUmsGmsGm | SEQ ID NO: 181 | GUAAUCCACAAGAA UUAAUUUGG | SEQ ID NO: 87 |
| ETX-S00008886 | UmsCfsAmUmGmCfAmUfCfUmCmAm UmAfCmUfUmCmUmGfAmsUmsCm | SEQ ID NO: 182 | UCAUGCAUCUCAUA CUUCUGAUC | SEQ ID NO: 88 |
| ETX-S00008888 | UmsAfsGmCmUmGfAmGfAfGmUmUm CmAfCmUfAmAmGmGfUmsCmsAm | SEQ ID NO: 183 | UAGCUGAGAGUUCA CUAAGGUCA | SEQ ID NO: 89 |
| ETX-S00008890 | AmsUfsGmUmAmAfUmCfCfAmCmAm AmGfAmAfUmUmAmAfUmsUmsUm | SEQ ID NO: 184 | AUGUAAUCCACAAG AAUUAAUUU | SEQ ID NO: 90 |
| ETX-S00008892 | AmsAfsAmCmCmCfUmGfGfAmUmAm CmUfUmGfGmAmGmAfUmsUmsUm | SEQ ID NO: 185 | AAACCCUGGAUACU UGGAGAUUU | SEQ ID NO: 91 |
| ETX-S00008894 | UmsUfsUmCmAmUfCmAfAfCmUmUm CmAfAmUfCmAmCmUfGmsUmsUm | SEQ ID NO: 186 | UUUCAUCAACUUCA AUCACUGUU | SEQ ID NO: 92 |
| ETX-S00008896 | UmsGfsAmAmUmCfCmGfAfAmGmUm UmUfGmAfAmGmUmCfUmsCmsCm | SEQ ID NO: 187 | UGAAUCCGAAGUUU GAAGUCUCC | SEQ ID NO: 93 |
| ETX-S00008898 | AmsAfsGmAmUmGfUmAfAfUmCmCm AmCfAmAfGmAmAmUfUmsAmsAm | SEQ ID NO: 188 | AAGAUGUAAUCCAC AAGAAUUAA | SEQ ID NO: 94 |

Table 4 provides the modified second (sense) sequences, together with the corresponding unmodified second (sense) sequences for siRNA oligonucleosides according to the present invention as follows.

**Table 4**

| Sense strand ID | Modified Second (Sense) Strand 5' → 3' | SEQ ID NO (SS - mod) | Underlying Base Sequence 5' → 3' (Shown as an Unmodified Nucleoside Sequence) | SEQ ID NO (SS - unmod) |
|---|---|---|---|---|
| ETX-S00008805 | iaiaCmsUmsCmUmGmGmAmAmUfGf CfUmUmCmUmGmUmUmUmCmUm | SEQ ID NO: 189 | CUCUGGAAUGCUUCUG UUUCU | SEQ ID NO: 95 |
| ETX-S00008807 | iaiaUmsUmsCmAmAmGmCmUmAfGf AfUmCmAmGmAmAmGmUmAmUm | SEQ ID NO: 190 | UUCAAGCUAGAUCAGA AGUAU | SEQ ID NO: 96 |
| ETX-S00008809 | iaiaGmsUmsGmGmAmUmUmAmCfAf UfCmUmUmGmUmUmCmAmAmAm | SEQ ID NO: 191 | GUGGAUUACAUCUUGU UCAAA | SEQ ID NO: 97 |
| ETX-S00008811 | iaiaCmsUmsUmAmGmUmGmAmAfCf UfCmUmCmAmGmCmUmAmCmUm | SEQ ID NO: 192 | CUUAGUGAACUCUCAG CUACU | SEQ ID NO: 98 |
| ETX-S00008813 | iaiaUmsGmsAmAmAmCmCmAmAfAf UfUmAmAmUmUmCmUmUmGmUm | SEQ ID NO: 193 | UGAAACCAAAUUAAUU CUUGU | SEQ ID NO: 99 |
| ETX-S00008815 | iaiaCmsUmsGmGmAmAmGmAmAfAf UfCmUmCmCmAmAmGmUmAmUm | SEQ ID NO: 194 | CUGGAAGAAAUCUCCA AGUAU | SEQ ID NO: 100 |
| ETX-S00008817 | iaiaCmsCmsCmAmAmAmCmUmGfUf UfUmGmAmUmGmAmGmAmUmUm | SEQ ID NO: 195 | CCCAAACUGUUUGAUG AGAUU | SEQ ID NO: 101 |
| ETX-S00008819 | iaiaUmsGmsUmGmGmAmUmUmAfCf AfUmCmUmUmGmUmUmCmAmAm | SEQ ID NO: 196 | UGUGGAUUACAUCUUG UUCAA | SEQ ID NO: 102 |
| ETX-S00008821 | iaiaGmsGmsCmAmAmGmUmUmUfGf CfCmUmCmCmAmCmCmUmUmUm | SEQ ID NO: 197 | GGCAAGUUUGCCUCCA CCUUU | SEQ ID NO: 103 |
| ETX-S00008823 | iaiaAmsAmsCmCmAmGmAmAmAfCf AfUmGmGmAmAmGmUmUmUmUm | SEQ ID NO: 198 | AACCAGAAACAUGGAA GUUUU | SEQ ID NO: 104 |
| ETX-S00008825 | iaiaCmsUmsCmAmUmGmAmAmUfCf AfUmUmAmCmAmUmUmAmAmCm | SEQ ID NO: 199 | CUCAUGAAUCAUUACA UUAAC | SEQ ID NO: 105 |
| ETX-S00008827 | iaiaCmsAmsGmAmAmAmCmAmUfGf GfAmAmGmUmUmUmUmCmUmUm | SEQ ID NO: 200 | CAGAAACAUGGAAGUU UUCUU | SEQ ID NO: 106 |
| ETX-S00008829 | iaiaUmsCmsCmAmAmGmUmAmUfCf CfAmGmGmGmUmUmUmUmAmCm | SEQ ID NO: 201 | UCCAAGUAUCCAGGGU UUUAC | SEQ ID NO: 107 |
| ETX-S00008831 | iaiaAmsCmsCmAmAmAmUmUmAfAf UfUmCmUmUmGmUmGmGmAmUm | SEQ ID NO: 202 | ACCAAAUUAAUUCUUG UGGAU | SEQ ID NO: 108 |
| ETX-S00008833 | iaiaAmsAmsCmCmAmAmAmUmUfAf AfUmUmCmUmUmGmUmGmGmAm | SEQ ID NO: 203 | AACCAAAUUAAUUCUU GUGGA | SEQ ID NO: 109 |
| ETX-S00008835 | iaiaCmsCmsUmGmAmAmAmCmCfAf AfAmUmUmAmAmUmUmCmUmUm | SEQ ID NO: 204 | CCUGAAACCAAAUUAA UUCUU | SEQ ID NO: 110 |
| ETX-S00008837 | iaiaCmsUmsUmGmUmGmGmAmUfUf AfCmAmUmCmUmGmUmUmCm | SEQ ID NO: 205 | CUUGUGGAUUACAUCU UGUUC | SEQ ID NO: 111 |
| ETX-S00008839 | iaiaUmsUmsGmUmGmGmAmUmUfAf CfAmUmCmUmGmUmUmCmAm | SEQ ID NO: 206 | UUGUGGAUUACAUCUU GUUCA | SEQ ID NO: 112 |
| ETX-S00008841 | iaiaAmsAmsCmAmGmUmGmAmUfUf GfAmAmGmUmUmGmAmUmGmAm | SEQ ID NO: 207 | AACAGUGAUUGAAGUU GAUGA | SEQ ID NO: 113 |
| ETX-S00008843 | iaiaUmsAmsAmUmUmCmUmUmGfUf GfGmAmUmUmAmCmAmUmCmUm | SEQ ID NO: 208 | UAAUUCUUGUGGAUUA CAUCU | SEQ ID NO: 114 |
| ETX-S00008845 | iaiaUmsCmsUmCmAmGmCmUmAfCf UfGmGmAmAmGmAmAmAmUmCm | SEQ ID NO: 209 | UCUCAGCUACUGGAAG AAAUC | SEQ ID NO: 115 |
| ETX-S00008847 | iaiaCmsAmsAmAmUmUmAmAmUfUf CfUmUmGmUmGmGmAmUmUmAm | SEQ ID NO: 210 | CAAAUUAAUUCUUGUG GAUUA | SEQ ID NO: 116 |
| ETX-S00008849 | iaiaUmsCmsCmAmCmCmUmUmUfGf AfCmAmAmGmAmAmUmUmUmUm | SEQ ID NO: 211 | UCCACCUUUGACAAGA AUUUU | SEQ ID NO: 117 |
| ETX-S00008851 | iaiaCmsAmsUmUmAmCmAmUmUfAf AfCmAmAmAmGmAmGmAmCmUm | SEQ ID NO: 212 | CAUUACAUUAACAAAG AGACU | SEQ ID NO: 118 |
| ETX-S00008853 | iaiaUmsUmsCmUmUmGmUmGmGfAf UfUmAmCmAmUmCmUmUmGmUm | SEQ ID NO: 213 | UUCUUGUGGAUUACAU CUUGU | SEQ ID NO: 119 |
| ETX-S00008855 | iaiaAmsCmsAmGmUmGmAmUmUfGf AfAmGmUmUmGmAmUmGmAmAm | SEQ ID NO: 214 | ACAGUGAUUGAAGUUG AUGAA | SEQ ID NO: 120 |
| ETX-S00008857 | iaiaUmsCmsUmUmGmUmGmGmAfUf UfAmCmAmUmCmUmUmGmUmUm | SEQ ID NO: 215 | UCUUGUGGAUUACAUC UUGUU | SEQ ID NO: 121 |
| ETX-S00008859 | iaiaAmsGmsAmAmAmCmAmUmGfGf AfAmGmUmUmUmUmCmUmUmUm | SEQ ID NO: 216 | AGAAACAUGGAAGUUU UCUUU | SEQ ID NO: 122 |
| ETX-S00008861 | iaiaAmsUmsCmCmAmAmGmAmGfGf UfAmUmUmUmUmGmAmUmAmCm | SEQ ID NO: 217 | AUCCAAGAGGUAUUUU GAUAC | SEQ ID NO: 123 |
| ETX-S00008863 | iaiaCmsUmsCmCmAmAmGmUmAfUf CfCmAmGmGmGmUmUmUmUmAm | SEQ ID NO: 218 | CUCCAAGUAUCCAGGG UUUUA | SEQ ID NO: 124 |
| ETX-S00008865 | iaiaCmsUmsGmAmAmAmCmCmAfAf AfUmUmAmAmUmUmCmUmUmGm | SEQ ID NO: 219 | CUGAAACCAAAUUAAU UCUUG | SEQ ID NO: 125 |
| ETX-S00008867 | iaiaUmsGmsGmAmAmGmAmAmAfUf CfUmCmCmAmAmGmUmAmUmCm | SEQ ID NO: 220 | UGGAAGAAAUCUCCAA GUAUC | SEQ ID NO: 126 |
| ETX-S00008869 | iaiaCmsCmsAmAmAmUmUmAmAfUf UfCmUmUmGmUmGmGmAmUmUm | SEQ ID NO: 221 | CCAAAUUAAUUCUUGU GGAUU | SEQ ID NO: 127 |
| ETX-S00008871 | iaiaAmsCmsCmAmGmAmAmAmCfAf UfGmGmAmAmGmUmUmUmUmCm | SEQ ID NO: 222 | ACCAGAAACAUGGAAG UUUUC | SEQ ID NO: 128 |
| ETX-S00008873 | iaiaUmsUmsAmAmUmUmCmUmUfGf UfGmGmAmUmUmAmCmAmUmCm | SEQ ID NO: 223 | UUAAUUCUUGUGGAUU ACAUC | SEQ ID NO: 129 |
| ETX-S00008875 | iaiaAmsUmsGmAmAmUmCmAmUfUf AfCmAmUmUmAmAmCmAmAmAm | SEQ ID NO: 224 | AUGAAUCAUUACAUUA ACAAA | SEQ ID NO: 130 |
| ETX-S00008877 | iaiaGmsAmsUmCmAmGmAmAmGfUf AfUmGmAmGmAmUmGmCmAmUm | SEQ ID NO: 225 | GAUCAGAAGUAUGAGA UGCAU | SEQ ID NO: 131 |
| ETX-S00008879 | iaiaGmsGmsAmAmGmAmAmAmUfCf UfCmCmAmAmGmUmAmUmCmCm | SEQ ID NO: 226 | GGAAGAAAUCUCCAAG UAUCC | SEQ ID NO: 132 |
| ETX-S00008881 | iaiaUmsCmsCmAmAmGmAmGmGfUf AfUmUmUmUmGmAmUmAmCmAm | SEQ ID NO: 227 | UCCAAGAGGUAUUUUG AUACA | SEQ ID NO: 133 |
| ETX-S00008883 | iaiaAmsAmsAmUmUmAmAmUmUfCf UfUmGmUmGmGmAmUmUmAmCm | SEQ ID NO: 228 | AAAUUAAUUCUUGUGG AUUAC | SEQ ID NO: 134 |
| ETX-S00008885 | iaiaUmsCmsAmGmAmAmGmUmAfUf GfAmGmAmUmGmCmAmUmGmAm | SEQ ID NO: 229 | UCAGAAGUAUGAGAUG CAUGA | SEQ ID NO: 135 |
| ETX-S00008887 | iaiaAmsCmsCmUmUmAmGmUmGfAf AfCmUmCmUmCmAmGmCmUmAm | SEQ ID NO: 230 | ACCUUAGUGAACUCUC AGCUA | SEQ ID NO: 136 |
| ETX-S00008889 | iaiaAmsUmsUmAmAmUmUmCmUfUf GfUmGmGmAmUmUmAmCmAmUm | SEQ ID NO: 231 | AUUAAUUCUUGUGGAU UACAU | SEQ ID NO: 137 |
| ETX-S00008891 | iaiaAmsUmsCmUmCmCmAmAmGfUf AfUmCmCmAmGmGmGmUmUmUm | SEQ ID NO: 232 | AUCUCCAAGUAUCCAG GGUUU | SEQ ID NO: 138 |
| ETX-S00008893 | iaiaCmsAmsGmUmGmAmUmUmGfAf AfGmUmUmGmAmUmGmAmAmAm | SEQ ID NO: 233 | CAGUGAUUGAAGUUGA UGAAA | SEQ ID NO: 139 |
| ETX-S00008895 | iaiaAmsGmsAmCmUmUmCmAmAfAf CfUmUmCmGmGmAmUmUmCmAm | SEQ ID NO: 234 | AGACUUCAAACUUCGG AUUCA | SEQ ID NO: 140 |
| ETX-S00008897 | iaiaAmsAmsUmUmCmUmUmGmUfGf GfAmUmUmAmCmAmUmCmUmUm | SEQ ID NO: 235 | AAUUCUUGUGGAUUAC AUCUU | SEQ ID NO: 141 |

Some of the modified second strand sequences as illustrated above in Table 4 include the preferred 5' iaia motif. However, it should also be understood that the scope of these modified second strand sequences additionally includes the Me / F modified second strand in the absence of the 5'iaia motif.

Table 5 identifies duplexes with Duplex IDs referencing the modified antisense and sense IDs from previous Tables 3 and 4.

**Table 5**

| Duplex ID | First (Antisense) strand ID | Second (Sense) strand ID |
|---|---|---|
| ETX-M00002599 | ETX-S00008806 | ETX-S00008805 |
| ETX-M00002600 | ETX-S00008808 | ETX-S00008807 |
| ETX-M00002601 | ETX-S00008810 | ETX-S00008809 |
| ETX-M00002602 | ETX-S00008812 | ETX-S00008811 |
| ETX-M00002603 | ETX-S00008814 | ETX-S00008813 |
| ETX-M00002604 | ETX-S00008816 | ETX-S00008815 |
| ETX-M00002605 | ETX-S00008818 | ETX-S00008817 |
| ETX-M00002606 | ETX-S00008820 | ETX-S00008819 |
| ETX-M00002607 | ETX-S00008822 | ETX-S00008821 |
| ETX-M00002608 | ETX-S00008824 | ETX-S00008823 |
| ETX-M00002609 | ETX-S00008826 | ETX-S00008825 |
| ETX-M00002610 | ETX-S00008828 | ETX-S00008827 |
| ETX-M00002611 | ETX-S00008830 | ETX-S00008829 |
| ETX-M00002612 | ETX-S00008832 | ETX-S00008831 |
| ETX-M00002613 | ETX-S00008834 | ETX-S00008833 |
| ETX-M00002614 | ETX-S00008836 | ETX-S00008835 |
| ETX-M00002615 | ETX-S00008838 | ETX-S00008837 |
| ETX-M00002616 | ETX-S00008840 | ETX-S00008839 |
| ETX-M00002617 | ETX-S00008842 | ETX-S00008841 |
| ETX-M00002618 | ETX-S00008844 | ETX-S00008843 |
| ETX-M00002619 | ETX-S00008846 | ETX-S00008845 |
| ETX-M00002620 | ETX-S00008848 | ETX-S00008847 |
| ETX-M00002621 | ETX-S00008850 | ETX-S00008849 |
| ETX-M00002622 | ETX-S00008852 | ETX-S00008851 |
| ETX-M00002623 | ETX-S00008854 | ETX-S00008853 |
| ETX-M00002624 | ETX-S00008856 | ETX-S00008855 |
| ETX-M00002625 | ETX-S00008858 | ETX-S00008857 |
| ETX-M00002626 | ETX-S00008860 | ETX-S00008859 |
| ETX-M00002627 | ETX-S00008862 | ETX-S00008861 |
| ETX-M00002628 | ETX-S00008864 | ETX-S00008863 |
| ETX-M00002629 | ETX-S00008866 | ETX-S00008865 |
| ETX-M00002630 | ETX-S00008868 | ETX-S00008867 |
| ETX-M00002631 | ETX-S00008870 | ETX-S00008869 |
| ETX-M00002632 | ETX-S00008872 | ETX-S00008871 |
| ETX-M00002633 | ETX-S00008874 | ETX-S00008873 |
| ETX-M00002634 | ETX-S00008876 | ETX-S00008875 |
| ETX-M00002635 | ETX-S00008878 | ETX-S00008877 |
| ETX-M00002636 | ETX-S00008880 | ETX-S00008879 |
| ETX-M00002637 | ETX-S00008882 | ETX-S00008881 |
| ETX-M00002638 | ETX-S00008884 | ETX-S00008883 |
| ETX-M00002639 | ETX-S00008886 | ETX-S00008885 |
| ETX-M00002640 | ETX-S00008888 | ETX-S00008887 |
| ETX-M00002641 | ETX-S00008890 | ETX-S00008889 |
| ETX-M00002642 | ETX-S00008892 | ETX-S00008891 |
| ETX-M00002643 | ETX-S00008894 | ETX-S00008893 |
| ETX-M00002644 | ETX-S00008896 | ETX-S00008895 |
| ETX-M00002645 | ETX-S00008898 | ETX-S00008897 |

For duplexes of Table 5:
ETX-M00002599 - ETX-M00002645 have a duplex structure according to Figure 7b.

Definitions as provided in the above Tables:
A - adenosine
C - cytidine
G - guanosine
T - thymidine
m - 2'-O-methyl
f - 2'fluro
s - phosphorothioate bond
ia - inverted abasic nucleoside

In a preferred embodiment, the invention relates to a nucleic acid comprising first and second strands that comprise, consist of, or consist essentially of a nucleotide sequence differing by 0 or 1 nucleotides from any one of the following first and second sequences:

| Duplex ID | Modified first strand | Modified second strand |
|---|---|---|
| ETX-M00002910 | ETX-S00008818 (SEQ ID NO: 148) | ETX-S00008817 (SEQ ID NO: 195) |
| ETX-M00002907 | ETX-S00008812 (SEQ ID NO: 145) | ETX-S00008811 (SEQ ID NO: 192) |

In a more preferred embodiment, the invention relates to a nucleic acid comprising first and second strands that comprise, consist of, or consist essentially of a nucleoside sequence differing by 0 or 1 nucleosides from any one of the following first and second sequences:

| Duplex ID | Modified first strand | Modified second strand |
|---|---|---|
| ETX-M00002910 | ETX-S00008818 (SEQ ID NO: 148) | ETX-S00008817 (SEQ ID NO: 195) |

### Example 9: Inhibition Screen for ZPI Expression in Human Huh7 Cells

Huh7 cells (human hepatocyte-derived cell line, obtained from JCRB Cell Bank) are maintained in Dulbecco's Modified Eagle Medium (DMEM) supplemented with 10% FBS at 37°C in at atmosphere of 5% CO2. Cells are transfected with siRNA duplexes targeting ZPI mRNA or a negative control siRNA (siRNA-control; sense strand 5'-UUCUCCGAACGUGUCACGUTT-3' (SEQ ID NO:297), antisense strand 5'-ACGUGACACGUUCGGAGAATT-3' (SEQ ID NO:298)) at a final duplex concentration of 3 nM. Transfection is carried out by adding 9.7 µL Opti-MEM (ThermoFisher) plus 0.3 µL Lipofectamine RNAiMAX (ThermoFisher) to 10 µL of each siRNA duplex. The mixture is incubated at room temperature for 15 minutes before being added to 100 µL of complete growth medium containing 20,000 Huh7 cells. Cells are incubated for 24 hours at 37°C/5% CO2 prior to total RNA purification using a RNeasy 96 Kit (Qiagen). Each duplex is tested by transfection in duplicate wells in two independent experiments.

cDNA synthesis is performed using FastQuant RT (with gDNase) Kit (Tiangen). Real-time quantitative PCR (qPCR) is performed on an ABI Prism 7900HT or ABI QuantStudio 7 with primers specific for human ZPI (Hs01547819_m1) and human GAPDH (Hs02786624_g1) using FastStart Universal Probe Master Kit (Roche).

qPCR is performed in duplicate on cDNA derived from each well and the mean Ct calculated. Relative ZPI expression is calculated from mean Ct values using the comparative Ct (ΔΔCt) method, normalised to GAPDH and relative to untreated cells. Sequences of RNAi molecules are depicted in Table 5.

### Example 10: Prediction of ZPI inhibition by nucleic acids of the invention

siRNAdesignR is a computational tool developed by the inventors for the engineering, selection and optimisation of features for the prediction of siRNA efficacy. In the first step of the process siRNAdesignR identified all possible siRNA target sequences in a gene (consensus regions found in all transcripts) and then filtered out sequences with undesirable characteristics (sequences that match any other human gene with 0 or 1 mismatches, sequences that are commonly mutated (in >1% of the population), sequences that encode immunostimulatory motifs or have degenerate bases and sequences in the 5' untranslated region).

For the remaining sequences, the inventors then derived over 400 features describing the nucleotide sequence of an siRNA target sequence and how these nucleotides interacted with each other and other nucleotides on the messenger RNA at a distance from the target sequence. These nucleotide interactions were predicted using a large language model trained on messenger RNA sequences. The most informative features were then selected with a regression analysis and used to train a machine learning model, which learnt how individual, and combinations of, features encode information on whether an siRNA is likely to be efficacious in vitro i.e. cause a significant reduction in mRNA levels.

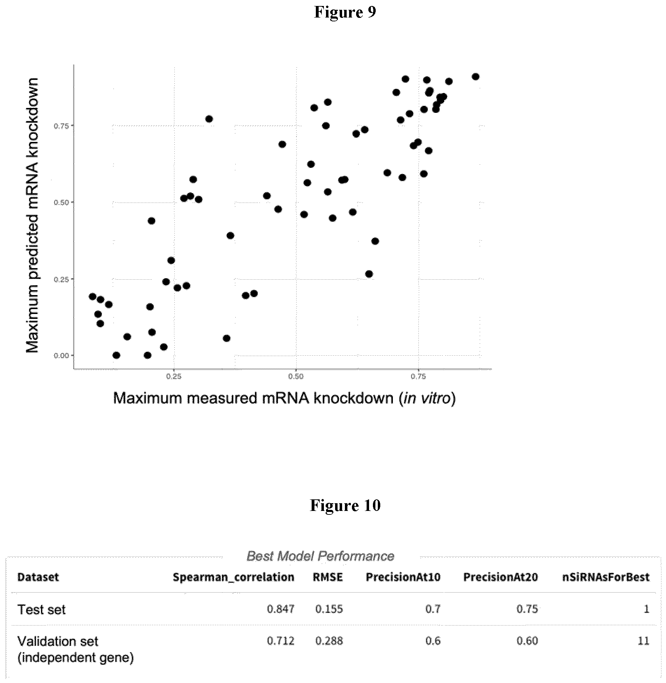

For the development of the machine learning model a database of siRNA sequences was built and their corresponding efficacy determined by screening hundreds of siRNAs in *in vitro* (cell line) models. This data was split into training (90% of the data) and test (10% of the data) sets. These datasets were used to train and test different machine learning models with optimised model hyperparameters. Over 10,000 models were evaluated with this method using an automated pipeline that tracked the hyperparameters and model performance. Performance was evaluated using five metrics; the Spearman correlation coefficient (Figure 9), the root mean squared error, precision@10, precision@20 and the rank of the best performing siRNA (determined experimentally).

The best performing model (ranked over all five metrics) was then validated using a previously unseen dataset (Figure 10). Models were considered accurate if precision@20 was greater than 0.5, and if the most efficacious siRNA (determined experimentally) was in the top 20 predictions.

As a final selection step, siRNAdesignR checked for cross species reactivity of the ranked siRNA sequences. This was not applied as a hard filter, but rather siRNA sequences would be preferred if they had cross species reactivity with Macaca fascicularis, Mus musculus and Rattus norvegicus but accepted if they had high efficacy predictions and Macaca fascicularis cross reactivity.

The outcome of the prediction is shown in Table 6.

**Table 6: siRNAdesignR predictions for efficacious antisense sequences of siRNAs targeting ZPI**

| **sequence** | **prediction** |
|---|---|
| AGAAACAGAAGCAUUCCAGAGGU (SEQ ID NO:48) | 0.87 |
| AUACUUCUGAUCUAGCUUGAACU (SEQ ID NO:49) | 0.86 |
| UUUGAACAAGAUGUAAUCCACAA (SEQ ID NO:50) | 0.86 |
| AGUAGCUGAGAGUUCACUAAGGU (SEQ ID NO:51) | 0.85 |
| ACAAGAAUUAAUUUGGUUUCAGG (SEQ ID NO:52) | 0.85 |
| AUACUUGGAGAUUUCUUCCAGUA (SEQ ID NO:53) | 0.84 |
| AAUCUCAUCAAACAGUUUGGGAA (SEQ ID NO:54) | 0.83 |
| UUGAACAAGAUGUAAUCCACAAG (SEQ ID NO:55) | 0.83 |
| AAAGGUGGAGGCAAACUUGCCUG (SEQ ID NO:56) | 0.82 |
| AAAACUUCCAUGUUUCUGGUUUU (SEQ ID NO:57) | 0.82 |
| GUUAAUGUAAUGAUUCAUGAGCC (SEQ ID NO:58) | 0.82 |
| AAGAAAACUUCCAUGUUUCUGGU (SEQ ID NO:59) | 0.81 |
| GUAAAACCCUGGAUACUUGGAGA (SEQ ID NO:60) | 0.80 |
| AUCCACAAGAAUUAAUUUGGUUU (SEQ ID NO:61) | 0.80 |
| UCCACAAGAAUUAAUUUGGUUUC (SEQ ID NO:62) | 0.80 |
| AAGAAUUAAUUUGGUUUCAGGAU (SEQ ID NO:63) | 0.79 |
| GAACAAGAUGUAAUCCACAAGAA (SEQ ID NO:64) | 0.79 |
| UGAACAAGAUGUAAUCCACAAGA (SEQ ID NO:65) | 0.79 |
| UCAUCAACUUCAAUCACUGUUCU (SEQ ID NO:66) | 0.78 |
| AGAUGUAAUCCACAAGAAUUAAU (SEQ ID NO:67) | 0.78 |
| GAUUUCUUCCAGUAGCUGAGAGU (SEQ ID NO:68) | 0.78 |
| UAAUCCACAAGAAUUAAUUUGGU (SEQ ID NO:69) | 0.77 |
| AAAAUUCUUGUCAAAGGUGGAGG (SEQ ID NO:70) | 0.77 |
| AGUCUCUUUGUUAAUGUAAUGAU (SEQ ID NO:71) | 0.77 |
| ACAAGAUGUAAUCCACAAGAAUU (SEQ ID NO:72) | 0.77 |
| UUCAUCAACUUCAAUCACUGUUC (SEQ ID NO:73) | 0.77 |
| AACAAGAUGUAAUCCACAAGAAU (SEQ ID NO:74) | 0.76 |
| AAAGAAAACUUCCAUGUUUCUGG (SEQ ID NO:75) | 0.76 |
| GUAUCAAAAUACCUCUUGGAUAA (SEQ ID NO:76) | 0.76 |
| UAAAACCCUGGAUACUUGGAGAU (SEQ ID NO:77) | 0.75 |
| CAAGAAUUAAUUUGGUUUCAGGA (SEQ ID NO:78) | 0.75 |
| GAUACUUGGAGAUUUCUUCCAGU (SEQ ID NO:79) | 0.75 |
| AAUCCACAAGAAUUAAUUUGGUU (SEQ ID NO:80) | 0.75 |
| GAAAACUUCCAUGUUUCUGGUUU (SEQ ID NO:81) | 0.75 |
| GAUGUAAUCCACAAGAAUUAAUU (SEQ ID NO:82) | 0.75 |
| UUUGUUAAUGUAAUGAUUCAUGA (SEQ ID NO:83) | 0.75 |
| AUGCAUCUCAUACUUCUGAUCUA (SEQ ID NO:84) | 0.74 |
| GGAUACUUGGAGAUUUCUUCCAG (SEQ ID NO:85) | 0.74 |
| UGUAUCAAAAUACCUCUUGGAUA (SEQ ID NO:86) | 0.74 |
| GUAAUCCACAAGAAUUAAUUUGG (SEQ ID NO:87) | 0.74 |
| UCAUGCAUCUCAUACUUCUGAUC (SEQ ID NO:88) | 0.74 |
| UAGCUGAGAGUUCACUAAGGUCA (SEQ ID NO:89) | 0.74 |
| AUGUAAUCCACAAGAAUUAAUUU (SEQ ID NO:90) | 0.73 |
| AAACCCUGGAUACUUGGAGAUUU (SEQ ID NO:91) | 0.73 |
| UUUCAUCAACUUCAAUCACUGUU (SEQ ID NO:92) | 0.73 |
| UGAAUCCGAAGUUUGAAGUCUCC (SEQ ID NO:93) | 0.73 |
| AAGAUGUAAUCCACAAGAAUUAA (SEQ ID NO:94) | 0.72 |

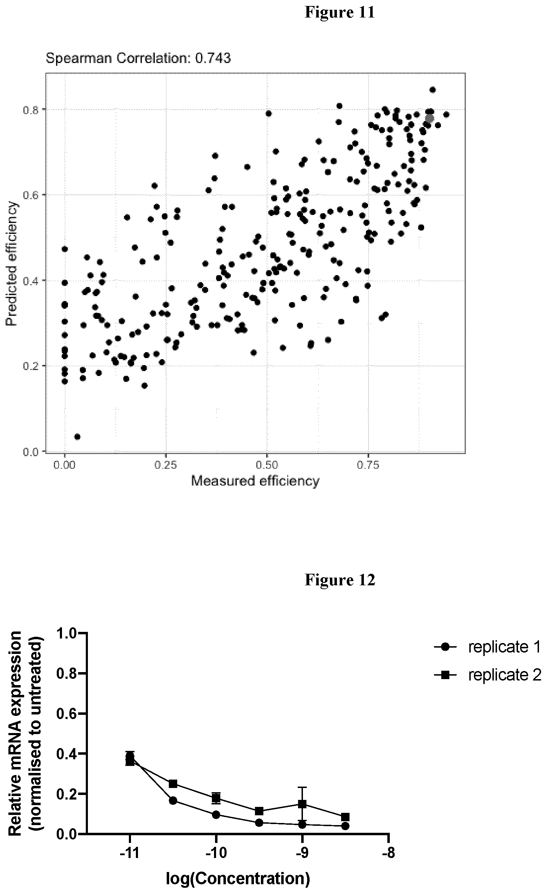

In order to demonstrate that siRNAdesignR could be used to find lead sequences for clinical projects, the inventors validated predictions for a different target, SLC25A5, in relevant preclinical models (Figures 11-14).

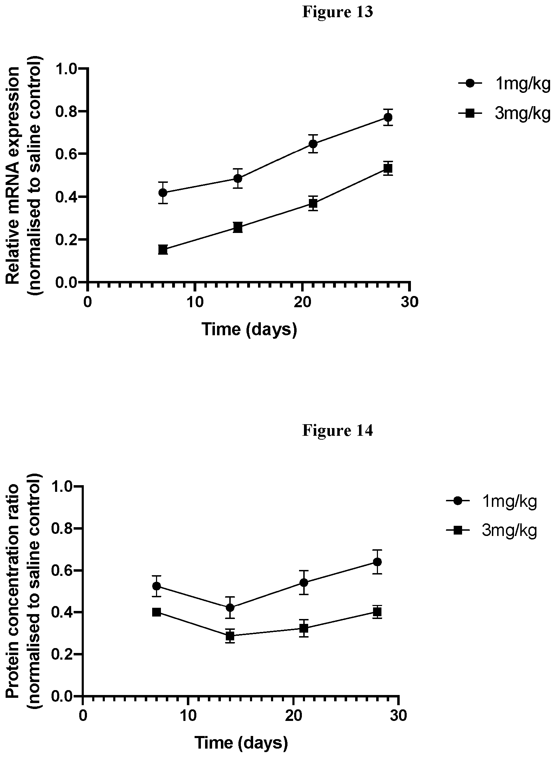

Highlighted in Figure 11 is an siRNA sequence that ranked within the top 20 predictions by siRNAdesignR for the target SLC25A5. This sequence was subsequently tested in dose response in vitro (in a Huh7 cell line), measuring the amount of mRNA knockdown (Figure 12), and in vivo (in C57BL/6 mice), measuring mRNA and protein knockdown (Figures 13 and 14).

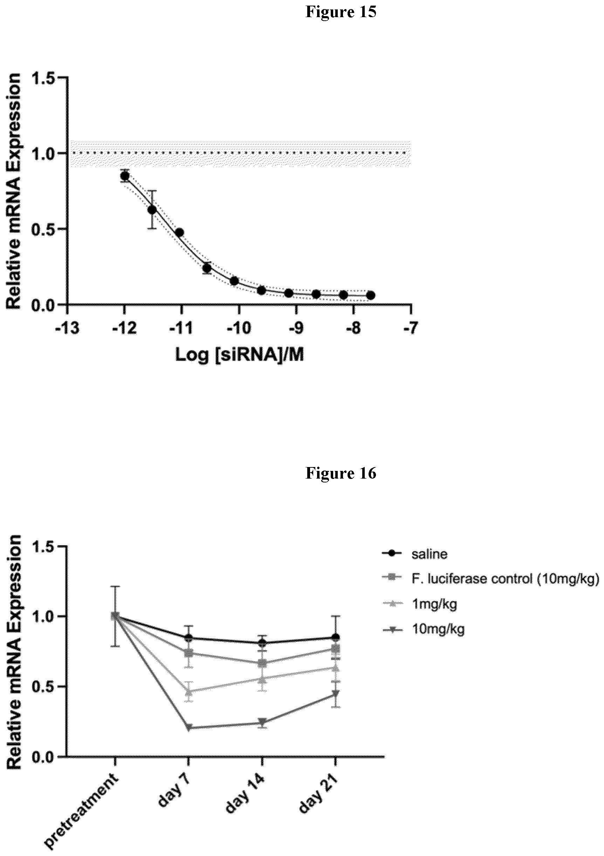

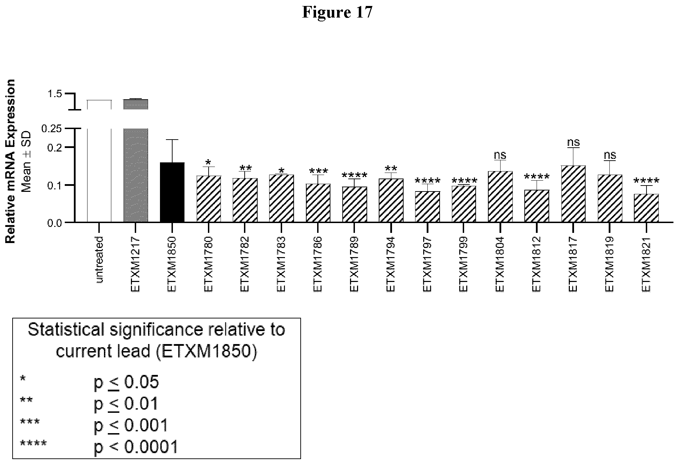

To further validate this approach, the inventors investigated whether siRNAdesignR predictions could identify more potent siRNA molecules than those identified by in vitro screening. 100 siRNAs were originally selected for in vitro screening based upon the cross reactivity between the Homo sapiens, Macaca fascicularis and Mus musculus B4GALT1 messenger RNA sequences. This screen identified siRNAs that were potent in vitro (Figure 15) and in vivo (Figure 16). Subsequent to performing this screen efficacy predictions were generated using siRNAdesignR as described above, this identified sequences targeting B4GALT1 that were predicted to be more efficacious than the lead sequence from the original screen shown in Figures 15 and 16. Screening of the predicted sequences in an in vitro model revealed 13 sequences demonstrating similar or greater mRNA knockdown than the original screening lead (ETXM-1850, see Figure 17). Accordingly, the inventors were able to validate that siRNAdesignR is capable to predict highly potent siRNA sequences.

### Example 11: Confirmation of Lead siRNA Activity in a Human hydrodynamic injection (HDI) Mouse Model

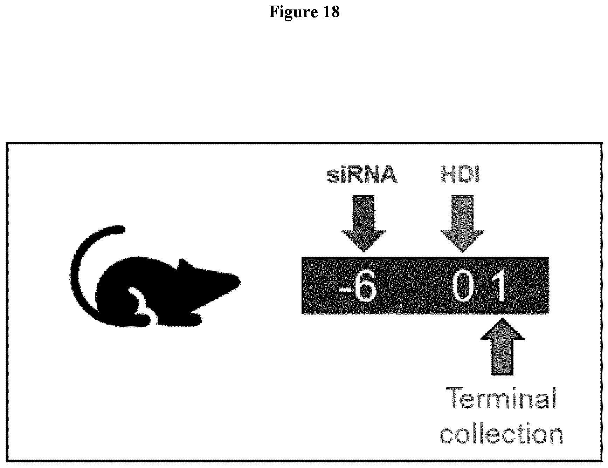

The transient expression of human mRNA in mice was evaluated to assess the activity of siRNAs against human mRNA sequences. A single subcutaneous dose of siRNAs (3 mg/kg) was administered on Day -6. Hydrodynamic injection (HDI) of a plasmid expressing human ZPI mRNA was performed on Day 0. The study was terminated, and mRNA expression was analysed on Day 1. As a negative control, saline was injected in place of an siRNA in the vehicle control group. An overview of the study is shown in Figure 18.

The siRNAs tested in this study are shown in Table 7 below.

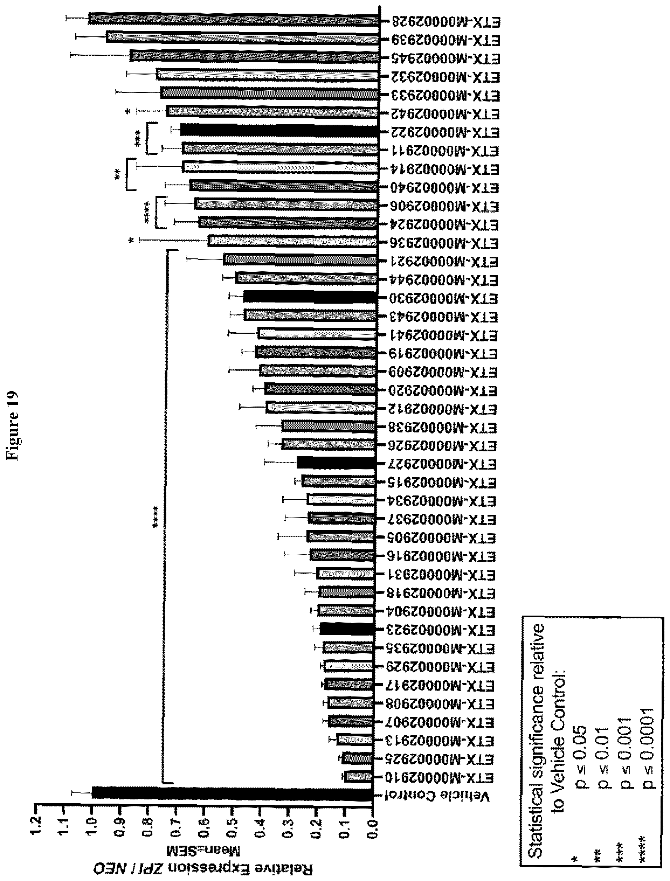

Activity of all tested siRNAs was confirmed in the human HDI mouse model (Figure 19).

qPCR was performed in duplicate on cDNA derived from each well and the mean Ct calculated. Relative ZPI expression was calculated from mean Ct values using the comparative Ct (ΔΔCt) method, normalised to NEO and relative to vehicle-injected controls. Sequences of RNAi molecules are depicted in the relevant Tables herein.

As can be seen from Figure 19, and Table 7 below, a particularly large effect is seen for a number of siRNAs. Preferred siRNAs for use in the invention are ETX-M00002910 and ETX-M00002907, and these are specifically contemplated throughout this disclosure, even when not singled out in a specific passage of this disclosure.

**Table 7**

| **ETX ID** | **Antisense SEQ ID NO** | **Sense SEQ ID NO** | **Mean Relative Expression** | **Standard Deviation** |
|---|---|---|---|---|
| ETX-M00002904 | SEQ ID NO 48 | SEQ ID NO 95 | 0.2 | 0.05 |
| ETX-M00002905 | SEQ ID NO 49 | SEQ ID NO 96 | 0.24 | 0.1 |
| ETX-M00002906 | SEQ ID NO 50 | SEQ ID NO 97 | 0.65 | 0.1 |
| ETX-M00002907 | SEQ ID NO 51 | SEQ ID NO 98 | 0.16 | 0.04 |
| ETX-M00002908 | SEQ ID NO 52 | SEQ ID NO 99 | 0.16 | 0.03 |
| ETX-M00002909 | SEQ ID NO 53 | SEQ ID NO 100 | 0.42 | 0.11 |
| ETX-M00002910 | SEQ ID NO 54 | SEQ ID NO 101 | 0.1 | 0.02 |
| ETX-M00002911 | SEQ ID NO 56 | SEQ ID NO 103 | 0.7 | 0.07 |
| ETX-M00002912 | SEQ ID NO 57 | SEQ ID NO 104 | 0.39 | 0.09 |
| ETX-M00002913 | SEQ ID NO 58 | SEQ ID NO 105 | 0.13 | 0.06 |
| ETX-M00002914 | SEQ ID NO 59 | SEQ ID NO 106 | 0.7 | 0.16 |
| ETX-M00002915 | SEQ ID NO 60 | SEQ ID NO 107 | 0.26 | 0.02 |
| ETX-M00002916 | SEQ ID NO 61 | SEQ ID NO 108 | 0.23 | 0.09 |
| ETX-M00002917 | SEQ ID NO 62 | SEQ ID NO 109 | 0.17 | 0.02 |
| ETX-M00002918 | SEQ ID NO 63 | SEQ ID NO 110 | 0.2 | 0.05 |
| ETX-M00002919 | SEQ ID NO 64 | SEQ ID NO 111 | 0.43 | 0.05 |
| ETX-M00002920 | SEQ ID NO 66 | SEQ ID NO 113 | 0.4 | 0.04 |
| ETX-M00002921 | SEQ ID NO 67 | SEQ ID NO 114 | 0.55 | 0.13 |
| ETX-M00002922 | SEQ ID NO 68 | SEQ ID NO 115 | 0.71 | 0.03 |
| ETX-M00002923 | SEQ ID NO 69 | SEQ ID NO 116 | 0.19 | 0.05 |
| ETX-M00002924 | SEQ ID NO 70 | SEQ ID NO 117 | 0.64 | 0.09 |
| ETX-M00002925 | SEQ ID NO 71 | SEQ ID NO 118 | 0.11 | 0.02 |
| ETX-M00002926 | SEQ ID NO 73 | SEQ ID NO 120 | 0.34 | 0.05 |
| ETX-M00002927 | SEQ ID NO 76 | SEQ ID NO 123 | 0.28 | 0.12 |
| ETX-M00002928 | SEQ ID NO 77 | SEQ ID NO 124 | 1.04 | 0.08 |
| ETX-M00002929 | SEQ ID NO 78 | SEQ ID NO 125 | 0.18 | 0.02 |
| ETX-M00002930 | SEQ ID NO 79 | SEQ ID NO 126 | 0.48 | 0.05 |
| ETX-M00002931 | SEQ ID NO 80 | SEQ ID NO 127 | 0.21 | 0.08 |
| ETX-M00002932 | SEQ ID NO 81 | SEQ ID NO 128 | 0.79 | 0.1 |
| ETX-M00002933 | SEQ ID NO 82 | SEQ ID NO 129 | 0.78 | 0.16 |
| ETX-M00002934 | SEQ ID NO 83 | SEQ ID NO 130 | 0.24 | 0.08 |
| ETX-M00002935 | SEQ ID NO 84 | SEQ ID NO 131 | 0.18 | 0.06 |
| ETX-M00002936 | SEQ ID NO 85 | SEQ ID NO 132 | 0.61 | 0.24 |
| ETX-M00002937 | SEQ ID NO 86 | SEQ ID NO 133 | 0.24 | 0.08 |
| ETX-M00002938 | SEQ ID NO 87 | SEQ ID NO 134 | 0.34 | 0.09 |
| ETX-M00002939 | SEQ ID NO 88 | SEQ ID NO 135 | 0.97 | 0.11 |
| ETX-M00002940 | SEQ ID NO 89 | SEQ ID NO 136 | 0.67 | 0.08 |
| ETX-M00002941 | SEQ ID NO 90 | SEQ ID NO 137 | 0.43 | 0.1 |
| ETX-M00002942 | SEQ ID NO 91 | SEQ ID NO 138 | 0.76 | 0.1 |
| ETX-M00002943 | SEQ ID NO 92 | SEQ ID NO 139 | 0.47 | 0.05 |
| ETX-M00002944 | SEQ ID NO 93 | SEQ ID NO 140 | 0.51 | 0.04 |
| ETX-M00002945 | SEQ ID NO 94 | SEQ ID NO 141 | 0.89 | 0.21 |

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure.

In case of ambiguity between the sequences in this specification and the sequences in the attached sequence listing, the sequences provided herein are considered to be the correct sequences.

The invention also relates to the following numbered clauses:
1. A nucleic acid for inhibiting expression of ZPI, comprising a duplex region that comprises a first strand and a second strand that is at least partially complementary to the first strand, wherein said first strand:
(i) is at least partially complementary to a portion of RNA transcribed from the ZPI gene, and
(ii) comprises at least 17 contiguous nucleosides differing by 0 or 1 nucleosides from any one of the first strand sequences as listed in Table 2.
2. A nucleic acid for inhibiting expression of ZPI, comprising a duplex region that comprises a first strand and a second strand that is at least partially complementary to the first strand, wherein said first strand:
(i) is at least partially complementary to a portion of RNA transcribed from the ZPI gene, and
(ii) comprises at least 17 contiguous nucleosides differing by 0 or 1 nucleosides from any one of the first strand modified sequences as listed in Table 3.
3. A nucleic acid according to clause 1 or 2, wherein the first strand comprises nucleosides 2-18 of any one of the sequences defined in clause 1 or 2, in particular wherein the first strand comprises nucleosides 2-18 of any one of the sequences defined in Tables 2 or 3.
4. A nucleic acid according to clause 1, wherein the second strand comprises a nucleoside sequence of at least 17 contiguous nucleosides differing by 0 or 1 nucleosides from any one of the second strand sequences as listed in Table 2, and wherein the second strand has a region of at least 85% complementarity over the 17 contiguous nucleosides to the first strand.
5. A nucleic acid according to clause 2, wherein the second strand comprises a nucleoside sequence of at least 17 contiguous nucleosides differing by 0 or 1 nucleosides from any one of the second strand modified sequences as listed in Table 4, and wherein the second strand has a region of at least 85% complementarity over the 17 contiguous nucleosides to the first strand.
6. A nucleic acid according to clause 1, wherein the first strand comprises any one of the first strand sequences as listed in Table 2.
7. A nucleic acid according to clause 2, wherein the first strand comprises any one of the first strand modified sequences as listed in Table 3.
8. A nucleic acid according to clause 4, wherein the second strand comprises any one of the second strand sequences as listed in Table 2.
9. A nucleic acid according to clause 5, wherein the second strand comprises any one of the second strand modified sequences as listed in Table 4.
10. A nucleic acid according to any preceding clause, wherein the first strand has a length in the range of 17 to 30 nucleosides, preferably 19 to 25 nucleosides, more preferably 19 or 23 nucleosides.
11. A nucleic acid according to any preceding clause, wherein the second strand has a length in the range of 17 to 30 nucleosides, preferably 19 to 25 nucleosides, more preferably 19 or 21 or 23 nucleosides.
12. A nucleic acid according to any preceding clause, wherein the duplex region of the nucleic acid is between 17 and 30 nucleosides in length, more preferably is 19 or 21 or 23 nucleosides in length.
13. A nucleic acid according to any preceding clause, wherein the region of complementarity between the first strand and the portion of RNA transcribed from the ZPI gene is between 17 and 30 nucleosides in length.
14. A nucleic acid according to any preceding clause, wherein the nucleic acid further comprises one or more single-stranded nucleoside overhangs, optionally wherein the overhang is present on the first or second strand, preferably at the 3' terminus of the first or second strand, and/or wherein the overhang comprises 1 to 4 nucleosides, more preferably 2 nucleosides.
15. A nucleic acid according to any preceding clause, wherein the nucleic acid is an siRNA oligonucleoside.
16. A nucleic acid according to any one of clauses 5 or 9, wherein the second strand comprises 2 consecutive abasic nucleosides in the 5' terminal region of the second strand, wherein one such abasic nucleoside is a terminal nucleoside at the 5' terminal region of the second strand and the other abasic nucleoside is a penultimate nucleoside at the 5' terminal region of the second strand, wherein:
(a) said penultimate abasic nucleoside is connected to an adjacent first basic nucleoside in an adjacent 5' near terminal region through a reversed internucleoside linkage; and
(b) the reversed linkage is a 5-5' reversed linkage; and
(c) the linkage between the terminal and penultimate abasic nucleosides is 3'5' when reading towards the terminus comprising the terminal and penultimate abasic nucleosides.
17. A nucleic acid according to clause 16, wherein
(i) the first strand and the second strand each has a length of 23 nucleosides;
(ii) two phosphorothioate internucleoside linkages are respectively between three consecutive positions in said 5' near terminal region of the second strand, wherein a first phosphorothioate internucleoside linkage is present between said adjacent first basic nucleoside of (a) and an adjacent second basic nucleoside in said 5' near terminal region of the second strand, and a second phosphorothioate internucleoside linkage is present between said adjacent second basic nucleoside and an adjacent third basic nucleoside in said 5' near terminal region of the second strand;
(iii) two phosphorothioate internucleoside linkages are respectively between three consecutive positions in both 5' and 3' terminal regions of the first strand, whereby a terminal nucleoside respectively at each of the 5' and 3' terminal regions of said first strand is each attached to a respective 5' and 3' adjacent penultimate nucleoside by a phosphorothioate internucleoside linkage, and each first 5' and 3' penultimate nucleoside is attached to a respective 5' and 3' adjacent antepenultimate nucleoside by a phosphorothioate internucleoside linkage; and
(iv) the second strand of the nucleic acid is conjugated directly or indirectly to one or more ligand moieties at the 3' terminal region of the second strand.
18. A nucleic acid according to clause 16 or 17, wherein the 2 consecutive inverted abasic nucleosides in the 5' terminal region of the second strand present as the following 5' terminal motif: 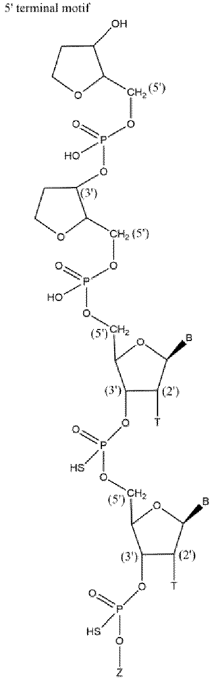 wherein:
T represents a 2'Me ribose modification,
B represents the nucleoside bases of the first two basic nucleosides in the 5' terminal region of the second strand, and
Z represents the remaining 19 contiguous basic nucleosides of said second strand.
19. A nucleic acid according to any preceding clause, wherein the nucleic acid is conjugated directly or indirectly to one or more ligand moieties, optionally wherein said ligand moiety is present at a terminal region of the second strand, preferably at the 3' terminal region thereof.
20. A nucleic acid according to clause 19, wherein the ligand moiety comprises:
(i) one or more N-acetyl galactosamine (GalNAc) ligands, and / or
(ii) one or more N-acetyl galactosamine (GalNAc) ligand derivatives.
21. A nucleic acid according to clause 20, wherein said one or more GalNAc ligands and / or GalNAc ligand derivatives are conjugated directly or indirectly to the 5' or 3' terminal region of the second strand of the nucleic acid, preferably at the 3' terminal region thereof.
22. A nucleic acid according to any one of clauses 19 to 21, comprising the structure: 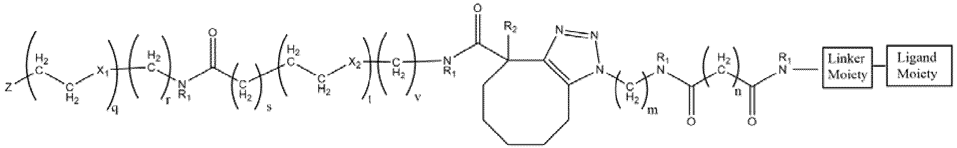 wherein:
R₁ at each occurrence is independently selected from the group consisting of hydrogen, methyl and ethyl;
R₂ is selected from the group consisting of hydrogen, hydroxy, -OC₁₋₃alkyl, -C(=O)OC₁₋₃alkyl, halo and nitro;
X₁ and X₂ at each occurrence are independently selected from the group consisting of methylene, oxygen and sulfur;
m is an integer of from 1 to 6;
n is an integer of from 1 to 10;
q, r, s, t, v are independently integers from 0 to 4, with the proviso that:
   (i) q and r cannot both be 0 at the same time; and
   (ii) s, t and v cannot all be 0 at the same time;
Z is an oligonucleoside moiety.
23. A nucleic acid according to clause 22, comprising the structure 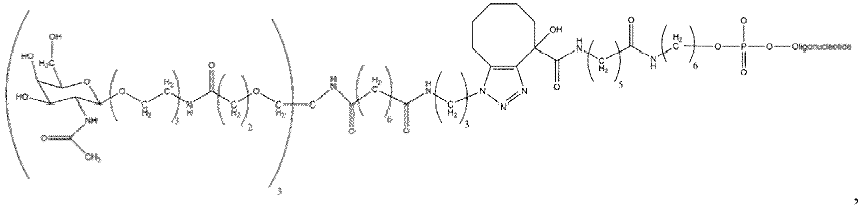 wherein oligonucleotide represents the contiguous nucleosides of the second strand.
24. A nucleic acid according to any one of clauses 19 to 21, comprising the structure: 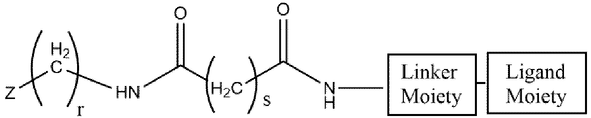 wherein:
r and s are independently an integer selected from 1 to 16; and
Z is an oligonucleoside moiety.
25. A nucleic acid according to clause 24, comprising the structure 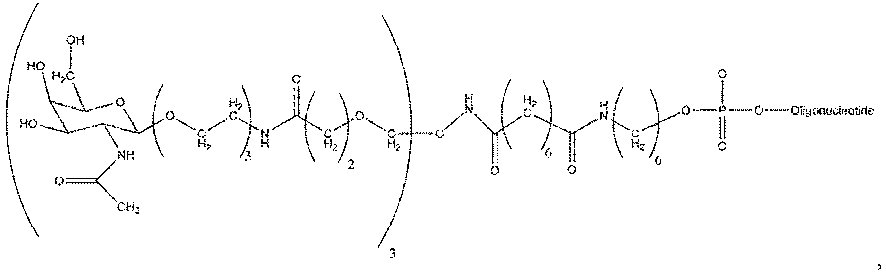 wherein oligonucleotide represents the contiguous nucleosides of the second strand.
26. A nucleic acid according to clause 23 or 25, wherein the structure is conjugated to the 3' terminal region of the second strand.
27. A nucleic acid as defined in clauses 18, 23 and 26.
28. A nucleic acid as defined in clauses 18, 25 and 26.
29. A nucleic acid according to any preceding clause, wherein the first strand comprises SEQ ID NO: 54 or SEQ ID NO: 51.
30. A nucleic acid according to any preceding clause, wherein the first strand comprises SEQ ID NO: 148 or SEQ ID NO: 145.
31. A nucleic acid according to any preceding clause, wherein the second strand comprises SEQ ID NO: 101 or SEQ ID NO: 98.
32. A nucleic acid according to any preceding clause, wherein the second strand comprises SEQ ID NO: 195 or SEQ ID NO: 192.
33. A nucleic acid according to any preceding clause, comprising first and second strands that comprise, consist of, or consist essentially of a nucleoside sequence differing by 0 or 1 nucleotides from any one of the following combinations of first and second sequences:

| Unmodified first strand | Unmodified second strand |
|---|---|
| SEQ ID NO: 54 | SEQ ID NO: 101 |
| SEQ ID NO: 51 | SEQ ID NO: 98 |

34. A nucleic acid according to any preceding clause, comprising first and second strands that comprise, consist of, or consist essentially of a nucleoside sequence differing by 0 or 1 nucleotides from any one of the following combinations of first and second sequences:

| Unmodified first strand | Unmodified second strand |
|---|---|
| SEQ ID NO: 54 | SEQ ID NO: 101 |

35. A nucleic acid according to any preceding clause, comprising first and second strands that comprise, consist of, or consist essentially of a nucleoside sequence differing by 0 or 1 nucleotides from any one of the following combinations of first and second sequences:

| Modified first strand | Modified second strand |
|---|---|
| SEQ ID NO: 148 | SEQ ID NO: 195 |
| SEQ ID NO: 145 | SEQ ID NO: 192 |

36. A nucleic acid according to any preceding clause, comprising first and second strands that comprise, consist of, or consist essentially of a nucleoside sequence differing by 0 or 1 nucleotides from any one of the following combinations of first and second sequences:

| Modified first strand | Modified second strand |
|---|---|
| SEQ ID NO: 148 | SEQ ID NO: 195 |

37. A pharmaceutical composition comprising a nucleic acid according to any preceding clause, in combination with a pharmaceutically acceptable excipient or carrier.
38. A nucleic acid or pharmaceutical composition according to any preceding clause, for use in therapy.
39. A nucleic acid or pharmaceutical composition according to any preceding clause, for use in prevention and/or treatment of a disease related to a disorder of haemostasis, such as haemophilia.
40. A nucleic acid or pharmaceutical composition according to any preceding clause, for use in prevention and/or treatment of Von Willebrand disease.
41. A nucleic acid or pharmaceutical composition according to any preceding clause, for use in prevention and/or treatment of Factor X Deficiency.

## Claims

1. A nucleic acid for inhibiting expression of ZPI, comprising a duplex region that comprises a first strand and a second strand that is at least partially complementary to the first strand, wherein said first strand:
(i) is at least partially complementary to a portion of RNA transcribed from the ZPI gene, and
(ii) comprises at least 17 contiguous nucleosides differing by 0 or 1 nucleosides from any one of the first strand sequences as listed in Table 2.

2. A nucleic acid for inhibiting expression of ZPI, comprising a duplex region that comprises a first strand and a second strand that is at least partially complementary to the first strand, wherein said first strand:
(i) is at least partially complementary to a portion of RNA transcribed from the ZPI gene, and
(ii) comprises at least 17 contiguous nucleosides differing by 0 or 1 nucleosides from any one of the first strand modified sequences as listed in Table 3.

3. A nucleic acid according to claim 1 or 2, wherein the first strand comprises nucleosides 2-18 of any one of the sequences defined in claim 1 or 2, in particular wherein the first strand comprises nucleosides 2-18 of any one of the sequences defined in Tables 2 or 3.

4. A nucleic acid according to claim 1, wherein the second strand comprises a nucleoside sequence of at least 17 contiguous nucleosides differing by 0 or 1 nucleosides from any one of the second strand sequences as listed in Table 2, and wherein the second strand has a region of at least 85% complementarity over the 17 contiguous nucleosides to the first strand.

5. A nucleic acid according to claim 2, wherein the second strand comprises a nucleoside sequence of at least 17 contiguous nucleosides differing by 0 or 1 nucleosides from any one of the second strand modified sequences as listed in Table 4, and wherein the second strand has a region of at least 85% complementarity over the 17 contiguous nucleosides to the first strand.

6. A nucleic acid according to claim 1, wherein the first strand comprises any one of the first strand sequences as listed in Table 2.

7. A nucleic acid according to claim 2, wherein the first strand comprises any one of the first strand modified sequences as listed in Table 3.

8. A nucleic acid according to claim 4, wherein the second strand comprises any one of the second strand sequences as listed in Table 2.

9. A nucleic acid according to claim 5, wherein the second strand comprises any one of the second strand modified sequences as listed in Table 4.

10. A nucleic acid according to any preceding claim, wherein the first strand comprises SEQ ID NO: 54 or SEQ ID NO: 51, or SEQ ID NO: 148 or SEQ ID NO: 145.

11. A nucleic acid according to any preceding claim, wherein the second strand comprises SEQ ID NO: 101 or SEQ ID NO: 98, or SEQ ID NO: 195 or SEQ ID NO: 192.

12. A nucleic acid according to any preceding claim, comprising first and second strands that comprise, consist of, or consist essentially of a nucleoside sequence differing by 0 or 1 nucleotides from any one of the following combinations of first and second sequences:
| Unmodified first strand | Unmodified second strand |
|---|---|
| SEQ ID NO: 54 | SEQ ID NO: 101 |
| SEQ ID NO: 51 | SEQ ID NO: 98 |
, preferably:
| Unmodified first strand | Unmodified second strand |
|---|---|
| SEQ ID NO: 54 | SEQ ID NO: 101 |

13. A nucleic acid according to any preceding claim, comprising first and second strands that comprise, consist of, or consist essentially of a nucleoside sequence differing by 0 or 1 nucleotides from any one of the following combinations of first and second sequences:
| Modified first strand | Modified second strand |
|---|---|
| SEQ ID NO: 148 | SEQ ID NO: 195 |
| SEQ ID NO: 145 | SEQ ID NO: 192 |
, preferably:
| Modified first strand | Modified second strand |
|---|---|
| SEQ ID NO: 148 | SEQ ID NO: 195 |

14. A pharmaceutical composition comprising a nucleic acid according to any preceding claim, in combination with a pharmaceutically acceptable excipient or carrier.

15. A nucleic acid or pharmaceutical composition according to any preceding claim, for use in therapy, for use in prevention and/or treatment of a disease related to a disorder of haemostasis, such as haemophilia, for use in prevention and/or treatment of Von Willebrand disease, or for use in prevention and/or treatment of Factor X Deficiency.
